# EUROPEAN PATENT SPECIFICATION

(11) **EP 1 660 186 B1**
(45) Date of publication and mention of the grant of the patent: **25.12.2013**
(21) Application number: 04816814.0
(22) Date of filing: 18.08.2004
(51) Int. Cl.: C07K 16/46, C07K 16/00, A61P 35/00

(54) **HUMANIZATION OF ANTIBODIES**
HUMANISIERUNG VON ANTIKÖRPERN
HUMANISATION D'ANTICORPS

(30) Priority: 18.08.2003 US 496367 P
(43) Date of publication of application: 31.05.2006
(60) Divisional application: 10185161.6
(73) Proprietor: MedImmune, LLC, Gaithersburg, MD 20878 (US)
(72) Inventor: DALL-ACQUA, William, Gaithersburg, MD 20878 (US); DAMSCHRODER, Melissa, Germantown, MD 20874 (US); WU, Herren, Boyds, MD 20841 (US)
(74) Representative: Bates, Rosica Florence
(86) International application number: PCT/US2004/026953
(87) International publication number: WO 2005/042743

(56) References cited:
- WJ HARRIS C. CUNNINGHAM: "Antibody therapeutics - Chapter 6 - Humanized antibodies" 1995, RG LANDES COMPANY , XP008056607 Paragraph page 96, "Germ line frameworks"; paragraph page 98, "Undesirable human framework regions"
- P. TAN ET AL.: ""Superhumanized" antibodies: reduction of immunogenic potential by complementarity-determining region grafting with human germline sequences: application to an anti-CD28" THE JOURNAL OF IMMUNOLOGY, vol. 169, 2002, pages 1119-1125, XP008056579
- JOHNSON SYD ET AL: "Development of a humanized monoclonal antibody (MEDI-493) with potent in vitro and in vivo activity against respiratory syncytial virus" JOURNAL OF INFECTIOUS DISEASES, CHICAGO, IL, US, vol. 176, no. 5, 1997, pages 1215-1224, XP002175659 ISSN: 0022-1899
- ROSOK M J ET AL: "A COMBINATORIAL LIBRARY STRATEGY FOR THE RAPID HUMANIZATION OF ANTICARCINOMA BR96 FAB" JOURNAL OF BIOLOGICAL CHEMISTRY, AMERICAN SOCIETY OF BIOLOCHEMICAL BIOLOGISTS, BIRMINGHAM,, US, vol. 271, no. 37, 13 September 1996 (1996-09-13), pages 22611-22618, XP002920742 ISSN: 0021-9258
- CALDAS C ET AL: "Design and synthesis of germline-based hemi-humanized single-chain Fv against the CD18 surface antigen" PROTEIN ENGINEERING, OXFORD UNIVERSITY PRESS, SURREY, GB, vol. 13, no. 5, May 2000 (2000-05), pages 353-360, XP002342730 ISSN: 0269-2139
- KANG ET AL: "Antibody redesign by chain shuffling from random combinatorial immunoglobulin libraries" PROCEEDINGS OF THE NATIONAL ACADEMY OF SCIENCES OF USA, NATIONAL ACADEMY OF SCIENCE, WASHINGTON, DC, US, vol. 88, December 1991 (1991-12), pages 11120-11123, XP002099780 ISSN: 0027-8424
- CLACKSON T ET AL: "MAKING ANTIBODY FRAGMENT USING PHAGE DISPLAY LIBRARIES" NATURE, NATURE PUBLISHING GROUP, LONDON, GB, vol. 352, 15 August 1991 (1991-08-15), pages 624-628, XP001019232 ISSN: 0028-0836
- GREG WINTER AND WILLIAM HARRIS: "Humanized antibodies" IMMUNOLOGY TODAY, vol. 14, no. 6, 1993, pages 243-246, XP002357357
- MATSUDA F ET AL: "THE COMPLETE NUCLEOTIDE SEQUENCE OF THE HUMAN IMMUNOGLOBULIN HEAVY CHAIN VARIABLE REGION LOCUS" JOURNAL OF EXPERIMENTAL MEDICINE, TOKYO, JP, vol. 188, no. 11, 7 December 1998 (1998-12-07), pages 2151-2162, XP001008659 ISSN: 0022-1007
- DALL'ACQUA W F ET AL: "Antibody humanization by framework shuffling" METHODS : A COMPANION TO METHODS IN ENZYMOLOGY, ACADEMIC PRESS INC., NEW YORK, NY, US, vol. 36, no. 1, May 2005 (2005-05), pages 43-60, XP004852552 ISSN: 1046-2023

## Description

### FIELD OF THE INVENTION

The present invention relates to methods of reengineering or reshaping antibodies to reduce the immunogenicity of the antibodies, while maintaining the immunospecificity of the antibodies for an antigen. In particular, the present invention provides methods of producing antibodies immunospecific for an antigen by synthesizing a combinatorial library comprising complementarity determining regions (CDRs) from a donor antibody fused in frame to framework regions from a sub-bank of framework regions.

### 1. BACKGROUND OF THE INVENTION

Antibodies play a vital role in our immune responses. They can inactivate viruses and bacterial toxins, and are essential in recruiting the complement system and various types of white blood cells to kill invading microorganisms and large parasites. Antibodies are synthesized exclusively by B lymphocytes, and are produced in millions of forms, each with a different amino acid sequence and a different binding site for an antigen. Antibodies, collectively called immunoglobulins (Ig), are among the most abundant protein components in the blood. Alberts et al., Molecular Biology of the Cell, 2nd ed., 1989, Garland Publishing, Inc.

A typical antibody is a Y-shaped molecule with two identical heavy (H) chains (each containing about 440 amino acids) and two identical light (L) chains (each containing about 220 amino acids). The four chains are held together by a combination of noncovalent and covalent (disulfide) bonds. The proteolytic enzymes, such as papain and pepsin, can split an antibody molecule into different characteristic fragments. Papain produces two separate and identical Fab fragments, each with one antigen-binding site, and one Fc fragment. Pepsin produces one F (ab')₂ fragment. Alberts et al., Molecular Biology of the Cell, 2nd ed., 1989, Garland Publishing, Inc.

Both L and H chains have a variable sequence at their amino-terminal ends but a constant sequence at their carboxyl-terminal ends. The L chains have a constant region about 110 amino acids long and a variable region of the same size. The H chains also have a variable region about 110 amino acids long, but the constant region of the H chains is about 330 or 440 amino acid long, depending on the class of the H chain. Alberts et al., Molecular Biology of the Cell, 2nd ed., 1989, Garland Publishing, Inc. at pp1019.

Only part of the variable region participates directly in the binding of antigen. Studies have shown that the variability in the variable regions of both L and H chains is for the most part restricted to three small hypervariable regions (also called complementarity-determining regions, or CDRs) in each chain. The remaining parts of the variable region, known as framework regions (FR), are relatively constant. Alberts et al., Molecular Biology of the Cell, 2nd ed., 1989, Garland Publishing, Inc. at pp 1019 - 1020.

Natural immunoglobulins have been used in assays, diagnosis and, to a more limited extent, therapy. However, such uses, especially in therapy, have been hindered by the polyclonal nature of natural immunoglobulins. The advent of monoclonal antibodies of defined specificity increased the opportunities for therapeutic use. However, most monoclonal antibodies are produced following immunization of a rodent host animal with the target protein, and subsequent fusion of a rodent spleen cell producing the antibody of interest with a rodent myeloma cell. They are, therefore, essentially rodent proteins and as such are naturally immunogenic in humans, frequently giving rise to an undesirable immune response termed the HAMA (Human Anti-Mouse Antibody) response.

Many groups have devised techniques to decrease the immunogenicity of therapeutic antibodies. Traditionally, a human template is selected by the degree of homology to the donor antibody, *i.e*., the most homologous human antibody to the non-human antibody in the variable region is used as the template for humanization. The rationale is that the framework sequences serve to hold the CDRs in their correct spatial orientation for interaction with an antigen, and that framework residues can sometimes even participate in antigen binding. Thus, if the selected human framework sequences are most similar to the sequences of the donor frameworks, it will maximize the likelihood that affinity will be retained in the humanized antibody. Winter (EP No. 0239400), for instance, proposed generating a humanized antibody by site-directed mutagenesis using long oligonucleotides in order to graft three complementarity determining regions (CDR1, CDR2 and CDR3) from each of the heavy and light chain variable regions. Although this approach has been shown to work, it limits the possibility of selecting the best human template supporting the donor CDRs.

Although a humanized antibody is less immunogenic than its natural or chimeric counterpart in a human, many groups find that a CDR grafted humanized antibody may demonstrate a significantly decreased binding affinity (*e.g.,* Riechmann et al., 1988, Nature 3 32:323-327). For instance, Reichmann and colleagues found that transfer of the CDR regions alone was not sufficient to provide satisfactory antigen binding activity in the CDR-grafted product, and that it was also necessary to convert a serine residue at position 27 of the human sequence to the corresponding rat phenylalanine residue. These results indicated that changes to residues of the human sequence outside the CDR regions may be necessary to obtain effective antigen binding activity. Even so, the binding affinity was still significantly less than that of the original monoclonal antibody.

For example, Queen et al (U.S. Patent No. 5,530,101) described the preparation of a humanized antibody that binds to the interleukin-2 receptor, by combining the CDRs of a murine monoclonal (anti-Tac MAb) with human immunoglobulin framework and constant regions. The human framework regions were chosen to maximize homology with the anti-Tac MAb sequence. In addition, computer modeling was used to identify framework amino acid residues which were likely to interact with the CDRs or antigen, and mouse amino acids were used at these positions in the humanized antibody. The humanized anti-Tac antibody obtained was reported to have an affinity for the interleukin-2 receptor (p55) of 3 X 10⁹ M⁻¹, which was still only about one-third of that of the murine MAb.

Other groups identified further positions within the framework of the variable regions (*i.e*., outside the CDRs and structural loops of the variable regions) at which the amino acid identities of the residues may contribute to obtaining CDR-grafted products with satisfactory binding affinity. See, *e.g.,* U.S. Patent Nos. 6,054,297 and 5,929,212. Still, it is impossible to know beforehand how effective a particular CDR grafting arrangement will be for any given antibody of interest.

Leung (U.S. Patent Application Publication No. US 2003/0040606) describes a framework patching approach, in which the variable region of the immunoglobulin is compartmentalized into FR1, CDR1, FR2, CDR2, FR3, CDR3 and FR4, and the individual FR sequence is selected by the best homology between the non-human antibody and the human antibody template. This approach, however, is labor intensive, and the optimal framework regions may not be easily identified.

As more therapeutic antibodies are being developed and are holding more promising results, it is important to be able to reduce or eliminate the body's immune response elicited by the administered antibody. Thus, new approaches allowing efficient and rapid engineering of antibodies to be human-like, and/or allowing a reduction in labor to humanize an antibody provide great benefits and medical value.

Citation or discussion of a reference herein shall not be construed as an admission that such is prior art to the present invention.

### 2. SUMMARY OF THE INVENTION

The present invention provides a method of generating a combinatorial library comprising a plurality of nucleotide sequences encoding humanized heavy chain variable regions, said nucleotide sequences encoding the humanized heavy chain variable regions each produced by fusing together a nucleic acid sequence encoding a human heavy chain framework region I, a nucleic acid sequence encoding a heavy chain complementarity determining region (CDR) 1, a nucleic acid sequence encoding a human heavy chain framework region 2, a nucleic acid sequence encoding a heavy chain CDR2, a nucleic acid sequence encoding a human heavy chain framework region 3, a nucleic acid sequence encoding a heavy chain CDR3, and a nucleic acid sequence encoding a human heavy chain framework region 4, wherein the CDRs are derived from a non-human donor antibody heavy chain variable region and each nucleic acid sequence encoding a heavy chain framework region is from a sub-bank comprising a plurality of nucleic acid sequences encoding said human heavy chain framework region from human germline framework sequences and/or framework regions from functional human antibody sequences. In accordance with this embodiment, the method may further comprise introducing the combinatorial library of nucleic acid sequences into a population of cells.

In another embodiment, the present invention provides a method of generating a combinatorial library comprising a plurality of nucleotide sequences encoding humanized light chain variable regions), said nucleotide sequences encoding the humanized light chain variable regions each produced by fusing together a nucleic acid sequence encoding a human light chain framework region 1, a nucleic acid sequence encoding a light chain CDR1, a nucleic acid sequence encoding a human light chain framework region 2, a nucleic acid sequence encoding a light chain CDR2, a nucleic acid sequence encoding a human light chain framework region 3, a nucleic acid sequence encoding a light chain CDR3, and a nucleic acid sequence encoding a human light chain framework region 4, wherein the CDRs are derived from a non-human donor antibody light chain variable region and each nucleic acid sequence encoding a light chain framework region is from a sub-bank comprising a plurality of nucleic acid sequences encoding said human light chain framework region from human germline framework sequences and/ or framework regions from functional human antibody sequences. In accordance with this embodiment, the method may further comprise introducing the combinatorial library of nucleic acid sequences into a population of cells. In another embodiment, the present invention provides a method of generating a combinatorial library comprising nucleic acid sequence comprising: (i) a first set of nucleotide sequences encoding humanized heavy chain variable regions, said first set of nucleotide sequences encoding humanized heavy chain variable regions each produced by fusing together a nucleic acid sequence encoding a human heavy chain framework region 1, a nucleic acid sequence encoding a heavy chain CDR1, a nucleic acid sequence encoding a human heavy chain framework region 2, a nucleic acid sequence encoding a heavy chain CDR2, a nucleic acid sequence encoding a human heavy chain framework region 3, a nucleic acid sequence encoding a heavy chain CDR3, and a nucleic acid sequence encoding a human heavy chain framework region 4, and, (ii) a second set of nucleotide sequences encoding humanized light chain variable regions, said second set of nucleotide sequences encoding humanized light chain variable regions each produced by fusing together a nucleic acid sequence encoding a human light chain framework region 1, a nucleic acid sequence encoding a light chain CDR1, a nucleic acid sequence encoding a human light chain framework region 2, a nucleic acid sequence encoding a light chain CDR2, a nucleic acid sequence encoding a human light chain framework region 3, a nucleic acid sequence encoding a light chain CDR3, and a nucleic acid sequence encoding a human light chain framework region 4, wherein the the heavy chain variable region CDRs are derived from a non-human donor antibody heavy chain variable region, the light chain variable region CDRs are derived from a non-human donor antibody light chain variable region, each nucleic acid sequence encoding a human heavy chain framework region is from a sub-bank comprising a plurality of nucleic acid sequences encoding said human heavy chain framework region from human germline framework sequences and/or framework regions from functional human antibody sequences and each nucleic acid sequence encoding a human light chain framework region is from a sub-bank comprising a plurality of nucleic acid sequences encoding said human light chain framework region from human germline framework sequences and/or framework regions from functional human antibody sequences. In accordance with this embodiment, the method may further comprise introducing the combinatorial library of nucleic acid sequences into a population of cells.

The methods described herein may provide cells comprising, containing or engineered to express the nucleic acid sequences described herein.

The present invention provides a method of producing a humanized antibody that immunospecifically binds to an antigen, said method comprising: (a) synthesizing a plurality of polynucleotide sequences each comprising a nucleotide sequence encoding a humanized heavy chain variable region, said nucleotide sequence produced by fusing together a nucleic acid sequence encoding a human heavy chain framework region 1, a nucleic acid sequence encoding a heavy chain CDR1, a nucleic acid sequence encoding a human heavy chain framework region 2, a nucleic acid sequence encoding heavy chain CDR2, a nucleic acid sequence encoding a human heavy chain framework region 3, a nucleic acid sequence encoding a heavy chain CDR3, and a nucleic acid sequence encoding a human heavy chain framework region 4, wherein the CDRs are derived from a non-human donor antibody heavy chain variable region that immunospecifically binds said antigen and each heavy chain framework region nucleic acid sequence is from a sub-bank comprising a plurality of nucleic acid sequences encoding said human heavy chain framework region from human germline framework sequences and/or framework regions from functional human antibody sequences; (b) introducing the polynucleotide sequences into a population of cells each containing a polynucleotide sequence encoding a light chain variable region; and (c) expressing the polynucleotide sequences encoding the heavy chain variable regionand the light chain variable region. Sub-banks of heavy chain framework region sequences may be generated prior to step (a) of the above-described method. The light chain variable region can be a humanized or human light chain variable region. In accordance with this embodiment, the method may further comprise a step (d) comprising screening for an antibody that immunospecifically binds to the antigen.

The present invention provides a method of producing a humanized antibody that immunospecifically binds to an antigen, said method comprising: (a) synthesizing a plurality of polynucleotide sequences each comprising a nucleotide sequence encoding a humanized light chain variable region, said nucleotide sequence produced by fusing together a nucleic acid sequence encoding a human light chain framework region 1, a nucleic acid sequence encoding a light chain CDR1, a nucleic acid sequence encoding a human light chain framework region 2, a nucleic acid sequence encoding a light chain CDR2, a nucleic acid sequence encoding a human light chain framework region 3, a nucleic acid sequence encoding a light chain CDR3, and a nucleic acid sequence encoding a human light chain framework region 4, wherein the CDRs are derived from a non-human donor antibody light chain variable region that immunospecifically binds said antigen and each light chain framework region nucleic acid sequence is from a sub-bank comprising a plurality of nucleic acid sequences encoding said human light chain framework region from human germline framework sequences and/or framework regions from functional human antibody sequences; (b) introducing the polynucleotide sequences into a population of cells each containing a polynucleotide encoding a heavy chain variable region; and (c) expressing the polynucleotide sequences encoding the heavy chain variable region and the light chain variable region. Sub-banks of light chain framework region sequences may be generated prior to step (a) of the above-described method. The heavy chain variable region can be a humanized or human variable heavy chain variable region. In accordance with this embodiment, the method may further comprise a step (d) comprising screening for an antibody that immunospecifically binds to the antigen.

In another embodiment, the present invention provides a method of producing a humanized antibody that immunospecifically binds to an antigen, said method comprising: (a) synthesizing a plurality of first polynucleotide sequences each comprising a nucleotide sequence encoding a humanized heavy chain variable region, said nucleotide sequence produced by fusing together a nucleic acid sequence encoding a human heavy chain framework region 1, a nucleic acid sequence encoding a heavy chain CDR1, a nucleic acid sequence encoding a human heavy chain framework region 2, a nucleic acid sequence encoding heavy chain CDR2, a nucleic acid sequence encoding a human heavy chain framework region 3, a nucleic acid sequence encoding a heavy chain CDR3, and a nucleic acid sequence encoding a human heavy chain framework region 4, wherein the CDRs are derived from a non-human donor antibody heavy chain variable region that immunospecifically binds said antigen and each heavy chain framework region nucleic acid sequence is from a sub-bank comprising a plurality of nucleic acid sequences encoding said human heavy chain framework region from human germline framework sequences and/or framework regions from functional human antibody sequences; (b) synthesizing a plurality of second polynucleotide sequences each comprising a nucleotide sequence encoding a humanized light chain variable region, said nucleotide sequence produced by fusing together a nucleic acid sequence encoding a human light chain framework region 1, a nucleic acid sequence encoding a light chain CDR1, a nucleic acid sequence encoding a human light chain framework region 2, a nucleic acid sequence encoding a light chain CDR2, a nucleic acid sequence encoding a human light chain framework region 3, a nucleic acid sequence encoding a light chain CDR3, and a nucleic acid sequence encoding a human light chain framework region 4, wherein the CDRs are derived from a non-human donor antibody light chain variable region that immunospecifically binds said antigen and each light chain framework region nucleic acid sequence is from a sub-bank comprising a plurality of nucleic acid sequences encoding said human light chain framework region from human germline framework sequences and/or framework regions from functional human antibody sequences; (c) introducing the nucleic acid sequences generated in steps (a) and (b) into a population of cells; and (d) expressing the nucleotide sequences encoding the heavy chain variable region and the light chain variable region. In accordance with this embodiment, the method may further comprise a step (e) comprising screening for an antibody that immunospecifically binds to the antigen. Sub-banks of light chain framework region sequences may be generated prior to step (a) of the above-described method.

In methods of the invention, the human framework region sub-bank(s) comprise framework regions from human germline framework sequences and/or framework regions from functional human antibody sequences into which one or more mutations have been introduced. The framework region sub-banks can be readily used to synthesize a combinatorial library of antibodies which can be screened for their immunospecificity for an antigen of interest, as well as their immunogencity in an organism of interest.

The invention is based, in part, on the synthesis of framework region sub-banks for the variable heavy chain framework regions and the variable light chain framework regions of antibodies and on the synthesis of combinatorial libraries of antibodies comprising a variable heavy chain region and/or a variable light chain region with the variable chain region(s) produced by fusing together in frame complementarity determining regions (CDRs) derived from a donor antibody and framework regions derived from framework region sub-banks. The synthesis of framework region sub-banks allows for the fast, less labor intensive production of combinatorial libraries of antibodies (with or without constant regions) which can be readily screened for their immunospecificity for an antigen of interest, as well as their immunogenicity in an organism of interest. The library approach described herein allows for efficient selection and identification of human acceptor frameworks. In addition to the synthesis of framework region sub-banks, sub-banks of CDRs can be generated and randomly fused in frame with framework regions from framework region sub-banks to produce combinatorial libraries of antibodies (with or without constant regions) that can be screened for their immunospecificity for an antigen of interest, as well as their immunogenicity in an organism of interest. The combinatorial library methodology of the invention is exemplified herein for the production of humanized antibodies for use in human beings. However, the combinatorial library methodology can readily be applied to the production of antibodies for use in any organism of interest.

In some embodiments, one or more of the CDRs derived from the donor antibody heavy and/or light chain variable region(s) contain(s) one or more mutations relative to the nucleic acid sequence encoding the corresponding CDR in the donor antibody.

Humanized antibodies can be produced by the methods described herein; such antibodies may be formulated into a composition comprising a humanized antibody produced by the methods described herein and a carrier, diluent or excipient, which may be a pharmaceutical composition in a form for its intended use.

The humanized antibodies generated as described herein comprise a light chain variable region and/or a heavy chain variable region. The antibodies generated as described herein may further comprise a constant region(s).

Humanized antibodies generated in accordance with the invention can be conjugated or fused to a moiety (e.g., a therapeutic agent or drug). Such antibodies may be formulated into compositions, preferably pharmaceutical compositions, comprising an antibody generated and/or identified in accordance with the present invention and a carrier, diluent or excipient. The compositions, preferably pharmaceutical compositions,can comprise a humanized antibody (or fragment thereof) conjugated or fused to a moiety (e.g., a therapeutic agent or drug), and a carrier, diluent or excepient. A humanized antibody generated and/or identified in accordance with the present invention may be used alone, or in combination with other therapies, to prevent, treat, manage or ameliorate a disorder or a symptom thereof.

The pharmaceutical compositions may be used for the prevention, management, treatment or amelioration of a disease or one or more symptoms thereof. Preferably, the pharmaceutical compositions are sterile and in suitable form for a particular method of administration to a subject with a disease.

Methods of detecting, diagnosing and/or monitoring the progression of a disorder may utilize one or more humanized antibodies generated and/or identified in accordance with the methods of the invention.

### 2.1 Terminology

As used herein, the terms "acceptor" and "acceptor antibody" refer to the antibody or nucleic acid sequence providing or encoding at least 80%, at least 85%, at least 90%, or at least 95% amino acid sequences of one or more of the framework regions. In some embodiments, the term "acceptor" refers to the antibody or nucleic acid sequence providing or encoding the constant region(s). In a specific embodiment, the term "acceptor" refers to a human antibody or nucleic acid sequence that provides or encodes at least 80%, preferably, at least 85%, at least 90%, or at least 95% amino acid sequences of one or more of the framework regions. An acceptor framework region and/or acceptor constant region(s) may be, *e.g.,* derived or obtained from a germline antibody gene, a mature antibody gene, a functional antibody (*e.g.,* antibodies well-known in the art, antibodies in development, or antibodies commercially available).

As used herein, the terms "antibody" and "antibodies" refer to monoclonal antibodies, multispecific antibodies, human antibodies, humanized antibodies, camelised antibodies, chimeric antibodies, single-chain Fvs (scFv), single chain antibodies, single domain antibodies, Fab fragments, F(ab) fragments, disulfide-linked Fvs (sdFv), anti-idiotypic (anti-Id) antibodies, and epitope-binding fragments of any of the above. In particular, antibodies include immunoglobulin molecules and immunologically active fragments of immunoglobulin molecules, *i.e*., molecules that contain an antigen binding site. Immunoglobulin molecules can be of any type (*e.g*., IgG, IgE, IgM, IgD, IgA and IgY), class (*e.g*., IgG₁, IgG₂, IgG₃, IgG₄, IgA₁ and IgA₂) or subclass.

A typical antibody contains two heavy chains paired with two light chains. A full-length heavy chain is about 50 kD in size (approximately 446 amino acids in length), and is encoded by a heavy chain variable region gene (about 116 amino acids) and a constant region gene. There are different constant region genes encoding heavy chain constant region of different isotypes such as alpha, gamma (IgG1, IgG2, IgG3, IgG4), delta, epsilon, and mu sequences. A full-length light chain is about 25 Kd in size (approximately 214 amino acids in length), and is encoded by a light chain variable region gene (about 110 amino acids) and a kappa or lambda constant region gene. The variable regions of the light and/or heavy chain are responsible for binding to an antigen, and the constant regions are responsible for the effector functions typical of an antibody.

As used herein, the term "CDR" refers to the complement determining region within antibody variable sequences. There are three CDRs in each of the variable regions of the heavy chain and the light chain, which are designated CDR1, CDR2 and CDR3, for each of the variable regions. The exact boundaries of these CDRs have been defined differently according to different systems. The system described by Kabat (Kabat et al., Sequences of Proteins of Immunological Interest (National Institutes of Health, Bethesda, MD (1987) and (1991)) not only provides an unambiguous residue numbering system applicable to any variable region of an antibody, but also provides precise residue boundaries defining the three CDRs. These CDRs may be referred to as Kabat CDRs. Chothia and coworkers (Chothia & Lesk, J. Mol. Biol. 196:901-917 (1987) and Chothia et al., Nature 342:877-883 (1989)) found that certain sub-portions within Kabat CDRs adopt nearly identical peptide backbone conformations, despite having great diversity at the level of amino acid sequence. These sub-portions were designated as L1, L2 and L3 or H1, H2 and H3 where the "L" and the "H" designates the light chain and the heavy chains regions, respectively. These regions may be referred to as Chothia CDRs, which have boundaries that overlap with Kabat CDRs. Other boundaries defining CDRs overlapping with the Kabat CDRs have been described by Padlan (FASEB J. 9:133-139 (1995)) and MacCallum (J Mol Biol 262(5):732-45 (1996)). Still other CDR boundary definitions may not strictly follow one of the above systems, but will nonetheless overlap with the Kabat CDRs, although they may be shortened or lengthened in light of prediction or experimental findings that particular residues or groups of residues or even entire CDRs do not significantly impact antigen binding. The methods used herein may utilize CDRs defined according to any of these systems, although preferred embodiments use Kabat or Chothia defined CDRs.

As used herein, the term "derivative" in the context of proteinaceous agent (*e.g.,* proteins, polypeptides, and peptides, such as antibodies) refers to a proteinaceous agent that comprises an amino acid sequence which has been altered by the introduction of amino acid residue substitutions, deletions, and/or additions. The term "derivative" as used herein also refers to a proteinaceous agent which has been modified, *i.e*., by the covalent attachment of any type of molecule to the proteinaceous agent. For example, but not by way of limitation, an antibody may be modified, *e.g*., by glycosylation, acetylation, pegylation, phosphorylation, amidation, derivatization by known protecting/blocking groups, proteolytic cleavage, linkage to a cellular ligand or other protein, etc. A derivative of a proteinaceous agent may be produced by chemical modifications using techniques known to those of skill in the art, including, but not limited to specific chemical cleavage, acetylation, formylation, metabolic synthesis of tunicamycin, etc. Further, a derivative of a proteinaceous agent may contain one or more non-classical amino acids. A derivative of a proteinaceous agent possesses a similar or identical function as the proteinaceous agent from which it was derived.

As used herein, the terms "disorder" and "disease" are used interchangeably for a condition in a subject.

As used herein, the term "donor antibody" refers to an antibody providing one or more CDRs. In a preferred embodiment, the donor antibody is an antibody from a species different from the antibody from which the framework regions are derived. In the context of a humanized antibody, the term "donor antibody" refers to a non-human antibody providing one or more CDRs.

As used herein, the term "effective amount" refers to the amount of a therapy which is sufficient to reduce or ameliorate the severity and/or duration of a disorder or one or more symptoms thereof, prevent the advancement of a disorder, cause regression of a disorder, prevent the recurrence, development, onset or progression of one or more symptoms associated with a disorder, detect a disorder, or enhance or improve the prophylactic or therapeutic effect(s) of another therapy (*e.g.,* prophylactic or therapeutic agent).

As used herein, the term "epitopes" refers to fragments of a polypeptide or protein having antigenic or immunogenic activity in an animal, preferably in a mammal, and most preferably in a human. An epitope having immunogenic activity is a fragment of a polypeptide or protein that elicits an antibody response in an animal. An epitope having antigenic activity is a fragment of a polypeptide or protein to which an antibody immunospecifically binds as determined by any method well-known to one of skill in the art, for example by immunoassays. Antigenic epitopes need not necessarily be immunogenic.

As used herein, the term "fusion protein" refers to a polypeptide or protein (including, but not limited to an antibody) that comprises an amino acid sequence of a first protein or polypeptide or functional fragment, analog or derivative thereof, and an amino acid sequence of a heterologous protein, polypeptide, or peptide (*i.e*., a second protein or polypeptide or fragment, analog or derivative thereof different than the first protein or fragment, analog or derivative thereof). In one embodiment, a fusion protein comprises a prophylactic or therapeutic agent fused to a heterologous protein, polypeptide or peptide. In accordance with this embodiment, the heterologous protein, polypeptide or peptide may or may not be a different type of prophylactic or therapeutic agent. For example, two different proteins, polypeptides or peptides with immunomodulatory activity may be fused together to form a fusion protein. In a preferred embodiment, fusion proteins retain or have improved activity relative to the activity of the original protein, polypeptide or peptide prior to being fused to a heterologous protein, polypeptide, or peptide.

As used herein, the term "fragment" refers to a peptide or polypeptide (including, but not limited to an antibody) comprising an amino acid sequence of at least 5 contiguous amino acid residues, at least 10 contiguous amino acid residues, at least 15 contiguous amino acid residues, at least 20 contiguous amino acid residues, at least 25 contiguous amino acid residues, at least 40 contiguous amino acid residues, at least 50 contiguous amino acid residues, at least 60 contiguous amino residues, at least 70 contiguous amino acid residues, at least contiguous 80 amino acid residues, at least contiguous 90 amino acid residues, at least contiguous 100 amino acid residues, at least contiguous 125 amino acid residues, at least 150 contiguous amino acid residues, at least contiguous 175 amino acid residues, at least contiguous 200 amino acid residues, or at least contiguous 250 amino acid residues of the amino acid sequence of another polypeptide or protein. In a specific embodiment, a fragment of a protein or polypeptide retains at least one function of the protein or polypeptide.

As used herein, the term "functional fragment" refers to a peptide or polypeptide (including, but not limited to an antibody) comprising an amino acid sequence of at least 5 contiguous amino acid residues, at least 10 contiguous amino acid residues, at least 15 contiguous amino acid residues, at least 20 contiguous amino acid residues, at least 25 contiguous amino acid residues, at least 40 contiguous amino acid residues, at least 50 contiguous amino acid residues, at least 60 contiguous amino residues, at least 70 contiguous amino acid residues, at least contiguous 80 amino acid residues, at least contiguous 90 amino acid residues, at least contiguous 100 amino acid residues, at least contiguous 125 amino acid residues, at least 150 contiguous amino acid residues, at least contiguous 175 amino acid residues, at least contiguous 200 amino acid residues, or at least contiguous 250 amino acid residues of the amino acid sequence of second, different polypeptide or protein, wherein said polypeptide or protein retains at least one function of the second, different polypeptide or protein. In a specific embodiment, a fragment of a polypeptide or protein retains at least two, three, four, or five functions of the protein or polypeptide. Preferably, a fragment of an antibody that immunospecifically binds to a particular antigen retains the ability to immunospecifically bind to the antigen.

As used herein, the term "framework" or "framework sequence" refers to the remaining sequences of a variable region minus the CDRs. Because the exact definition of a CDR sequence can be determined by different systems, the meaning of a framework sequence is subject to correspondingly different interpretations. The six CDRs (CDR1, 2, and 3 of light chain and CDR1, 2, and 3 of heavy chain) also divide the framework regions on the light chain and the heavy chain into four sub-regions (FR1, FR2, FR3 and FR4) on each chain, in which CDR1 is positioned between FR1 and FR2, CDR2 between FR2 and FR3, and CDR3 between FR3 and FR4. Without specifying the particular sub-regions as FR1, FR2, FR3 or FR4, a framework region, as referred by others, represents the combined FR's within the variable region of a single, naturally occurring immunoglobulin chain. As used herein, a FR represents one of the four sub-regions, and FRs represents two or more of the four sub-regions constituting a framework region. As an example, Table 1-4 list the germline sequences of FR1, 2, 3, and 4 of kappa light chain, respectively. Table 5-7 list the germline sequences of FR1, 2, and 3 of heavy chain according to the Kabat definition, respectively. Table 8-10 list the germline sequences of FR 1, 2 and 3 of heavy chain according to the Chothia definition, respectively. Table 11 lists the germline sequence of FR4 of the heavy chain.

### Tables 1-65

The SEQ ID Number for each sequence described in tables 1-65 is indicated in the first column of each table.

**Table 1. FR1 of Light Chains**

| | |
|---|---|
| 1 | GATGTTGTGATGACTCAGTCTCCACTCTCCCTGCCCGTCACCCTTGGACAGCCGGCCTCCATCTCCTGC |
| 2 | GATGTTGTGATGACTCAGTCTCCACTCTCCCTGCCCGTCACCCTTGGACAGCCGGCCTCCATCTCCTGC |
| 3 | GATATTGTGATGACCCAGACTCCACTCTCTCTGTCCGTCACCCCTGGACAGCCGGCCTCCATCTCCTGC |
| 4 | GATATTGTGATGACTCAGTCTCCACTCTCCCTGCCCGTCACCCCTGGAGAGCCGGCCTCCATCTCCTGC |
| 5 | GATATTGTGATGACCCAGACTCCACTCTCTCTGTCCGTCACCCCTCGACAGCCGGCCTCCATCTCCTGC |
| 6 | GATATTGTGATGACCCAGACTCCACTCTCCTCACCTGTCACCCTTGGACAGCCGGCCTCCATCTCCTGC |
| 7 | GATATTGTGATGACTCAGTCTCCACTCTCCCTGCCCGTCACCCCTGGAGAGCCGGCCTCCATCTCCTGC |
| 8 | GAGATTGTGATGACCCAGACTCCACTCTCCTTGTCTATCACCCCTGGAGAGCAGGCCTCCATCTCCTGC |
| 9 | GATATTGTGATGACCCAGACTCCACTCTCCTCGCCTGTCACCCTTGGACAGCCGGCCTCCATCTCCTTC |
| 10 | GAAATTGTGCTGACTCAGTCTCCAGACTTTCAGTCTGTGACTCCAAAGGAGAAAGTCACCATCACCTGC |
| 11 | GATGTTGTGATGACACAGTCTCCAGCTTTCCTCTCTGTGACTCCAGGGGAGAAAGTCACCATCACCTGC |
| 12 | GACATCCAGATGACCCAGTCTCCATCCTCCCTGTCTGCATCTGTAGGAGACAGAGTCACCATCACTTGC |
| 13 | GAAATTGTGCTGACTCAGTCTCCAGACTTTCAGTCTGTGACTCCAAAGGAGAAAGTCACCATCACCTGC |
| 14 | GACATCCAGATGACCCAGTCTCCATCCTCCCTGTCTGCATCTGTAGGAGACAGAGTCACCATCACTTGC |
| 15 | GAAACGACACTCACGCAGTCTCCAGCATTCATGTCAGCGACTCCAGGAGACAAAGTCAACATCTCCTGC |
| 16 | GACATCCAGATGACCCAGTCTCCATCCTCACTGTCTGCATCTGTAGGAGACAGAGTCACCATCACTTGT |
| 17 | GCCATCCAGATGACCCAGTCTCCATCCTCCCTGTCTGCATCTGTAGGAGACAGAGTCACCATCACTTGC |
| 18 | GACATCCAGATGACCCAGTCTCCTTCCACCCTGTCTGCATCTGTAGGAGACAGAGTCACCATCACTTGC |
| 19 | AACATCCAGATGACCCAGTCTCCATCTGCCATGTCTGCATCTGTAGGAGACAGAGTCACCATCACTTGT |
| 20 | GACATCCAGATGACCCAGTCTCCATCCTCACTGTCTGCATCTGTAGGAGACAGAGTCACCATCACTTGT |
| 21 | GAAATAGTGATGATGCAGTCTCCAGCCACCCTGTCTGTGTCTCCAGGGGAAAGAGCCACCCTCTCCTGC |
| 22 | GCCATCCAGTTGACCCAGTCTCCATCCTCCCTGTCTGCATCTGTAGGAGACAGAGTCACCATCACTTGC |
| 23 | GACATCCAGATGACCCAGTCTCCATCTTCTGTGTCTGCATCTGTAGGAGACAGAGTCACCATCACTTGT |
| 24 | GAAATAGTGATGACGCAGTCTCCAGCCACCCTGTCTGTGTCTCCAGGGGAAAGAGCCACCCTCTCCTGC |
| 25 | GAAATTGTGTTGACACAGTCTCCAGCCACCCTGTCTTTGTCTCCAGGGGAAAGAGCCACCCTCTCCTGC |
| 26 | GACATCCAGATGATCCAGTCTCCATCTTTCCTGTCTGCATCTGTAGGAGACAGAGTCAGTATCATTTGC |
| 27 | GCCATCCGGATGACCCAGTCTCCATTCTCCCTGTCTGCATCTGTAGGAGACAGAGTCACCATCACTTGC |
| 28 | GTCATCTGGATGACCCAGTCTCCATCCTTACTCTCTGCATCTACAGGAGACAGAGTCACCATCAGTTGT |
| 29 | GCCATCCAGTTGACCCAGTCTCCATCCTCCCTGTCTGCATCTGTAGGAGACAGAGTCACCATCACTTGC |
| 30 | GACATCCAGATGACCCAGTCTCCATCTTCCGTGTCTGCATCTGTAGGAGACAGAGTCACCATCACTTGT |
| 31 | GAAATTGTGTTGACACAGTCTCCAGCCACCCTGTCTTTGTCTCCAGGGGAAAGAGCCACCCTCTCCTGC |
| 32 | GACATCCAGTTGACCCAGTCTCCATCCTTCCTGTCTGCATCTGTAGGAGACAGAGTCACCATCACTTGC |
| 33 | GCCATCCGGATGACCCAGTCTCCATCCTCATTCTCTGCATCTACAGGAGACAGAGTCACCATCACTTGT |
| 34 | GACATCCAGATGACCCAGTCTCCATCCTCCCTGTCTGCATCTGTAGGAGACAGAGTCACCATCACTTGC |
| 35 | GACATCCAGTTGACCCAGTCTCCATCCTCCCTGTCTGCATCTGTAGGAGACAGAGTCACCATCACTTGC |
| 36 | GACATCCAGATGACCCAGTCTCCATCCTCCCTGTCTGCATCTGTAGGAGACAGAGTCACCATCACTTGC |
| 37 | GACATCCAGATGACCCAGTCTCCATCCTCCCTGTCTGCATCTGTAGGAGACAGAGTCACCATCACTTGC |
| 38 | GACATCCAGTTGACCCAGTCTCCATCCTCCCTGTCTGCATCTGTAGGAGACAGAGTCACCATCACTTGC |
| 39 | GACATCCAGATGACCCAGTCTCCATCCTCCCTGTCTGCATCTGTAGGAGACAGAGTCACCATCACTTGC |
| 40 | GAAATTGTAATGACACAGTCTCCACCCACCCTGTTTGTCTCCAGGGGAAAGAGTCACCCTCTCCTGC |
| 41 | GAAATTGTAATGACACAGTCTCCAGCCACCCTGTCTTTGTCTCCAGGGGAAAGAGCCACCCTCTCCTGC |
| 42 | GAAATTGTGTTGACGCAGTCTCCAGCCACCCTGTCTTTGTCTCCAGGGGAAAGAGCCACCCTCTCCTGC |
| 43 | GAAATTGGTTGACGCAGTCTCCAGGCACCCTGTCTTTGTCTCCAGGGGAAAGAGCCACCCTCTCCTGC |
| 44 | GACATCGTGATGACCCAGTCTCCAGACTCCCTGGCTGTGTCTCTGGGCGAGAGGGCCACCATCAACTGC |
| 45 | GATATTGTGATGACCCAGACTCCACTCTCCCTGCCCGTCACCCCTGGAGAGCCGGCCTCCATCTCCTGC |
| 46 | GATATTGTGATGACCCAGACTCCACTCTCCCTGCCCGTCACCCCTGGAGAGCCOOCCTCCATCTCCTGC |

**Table 2. FR2 of Light Chains**

| | |
|---|---|
| 47 | TGGTTTCAGCAGAGGCCAGGCCAATCTCCAAGGCGCCTAATTTAT |
| 48 | TGGTTTCAGCAGAGGCCAGGCCAATCTCCAAGGCGCCTAATTTAT |
| 49 | TGGTACCTGCAGAAGCCAGGCCAGTCTCCACAGCTCCTGATCTAT |
| 50 | TGGTACCTGCAGAAGCCAGGGCAGTCTCCACAGCTCCTGATCTAT |
| 51 | TGGTACCTGCAGAAGCCAGGCCAGCCTCCACAGCTCCTGATCTAT |
| 52 | TGGCTTCAGCAGAGGCCAGGCCAGCCTCCAAGACTCCTAATTTAT |
| 53 | TGGTACCTGCAGAAGCCAGGGCAGTCTCCACAGCTCCTGATCTAT |
| 54 | TGGTTTCTGCAGAAAGCCAGGCCAGTCTCCACACTCCTGATCTAT |
| 55 | TGGCTTCAGCAGAGGCCAGGCCAGCCTCCAAGACTCCTAATTTAT |
| 56 | TGGTACCAGCAGAAACCAGATCAGTCTCCAAAGCTCCTCATCAAG |
| 57 | TGGTACCAGCAGAAACCAGATCAAGCCCCAAAGCTCCTCATCAAG |
| 58 | TGGTATCAGCAGAAACCAGGGAAAGTTCCTAAGCTCCTGATCTAT |
| 59 | TGGTACCAGCAGAAACCAGATCAGTCTCCAAAGCTCCTCATCAAG |
| 60 | TGGTATCAGCAGAAACCAGGGAAAGCCCCTAAGCGCCTGATCTAT |
| 61 | TGGTACCAACAGAAACCAGGAGAAGCTGCTATTTTCATTATTCAA |
| 62 | TGGTTTCAGCAGAAACCAGGGAAAGCCCCTAAGTCCCTGATCTAT |
| 63 | TGGTATCAGCAGAAACCAGGGAAAGCCCCTAAGCTCCTGATCTAT |
| 64 | TGGTATCAGCAGAAACCAGGGAAAGCCCCTAAGCTCCTGATCTAT |
| 65 | TGGTTTCAGCAGAAACCAGGGAAAGTCCCTAAGCACCTGATCTAT |
| 66 | TGGTATCAGCAGAAACCAGAGAAAGCCCCTAAGTCCCTGATCTAT |
| 67 | TGGTACCAGCAGAAACCTGGCCAGGCTCCCAGGCTCCTCATCTAT |
| 68 | TGGTATCAGCAGAAACCAGGGAAAGCTCCTAAGCTCCTGATCTAT |
| 69 | TGGTATCAGCAGAAACCAGGGAAAGCCCCTAAGCTCCTGATCTAT |
| 70 | TGGTACCAGCAGAAACCTGGCCAGGCTCCCAGGCTCCTCATCTAT |
| 71 | TGGTACCAGCAGAAACCTGGCCAGGCTCCCAGGCTCCTCATCTAT |
| 72 | TGGTATCTGCAGAAACCAGGGAAATCCCCTAAGCTCTTCCTCTAT |
| 73 | TGGTATCAGCAAAAACCAGCAAAAGCCCCTAAGCTCTTCATCTAT |
| 74 | TGGTATCAGCAAAAACCAGGGAAAGCCCCTGAGCTCCTGATCTAT |
| 75 | TGGTATCAGCAGAAACCAGGGAAAGCTCCTAAGCTCCTGATCTAT |
| 76 | TGGTATCAGCAGAAACCAGGGAAAGCCCCTAAGCTCCTGATCTAT |
| 77 | TGGTACCAACAGAAACCTGGCCAGGCTCCCAGGCTCCTCATCTAT |
| 78 | TGGTATCAGCAAAAACCAGGGAAAGCCCCTAAGCTCCTGATCTAT |
| 79 | TGGTATCAGCAAAAACCAGGGAAAGCCCCTAAGCTCCTGATCTAT |
| 80 | TGGTATCAGCAGAAACCAGGGAAAGCCCCTAAGCTCCTGATCTAT |
| 81 | TGGTATCGGCAGAAACCAGGGAAAGTTCCTAAGCTCCTGATCTAT |
| 82 | TGGTATCAGCAGAAACCAGGGAAAGCCCCTAAGCTCCTGATCTAC |
| 83 | TGGTATCAGCAGAAACCAGGGAAAGCCCCTAAGCTCCTGATCTAT |
| 84 | TGGTATCGGCAGAAACCAGGGAAAGTTCCTAAGCTCCTGATCTAT |
| 85 | TGGTATCAGCAGAAACCAGGGAAAGCCCCTAAGCTCCTGATCTAC |
| 86 | TGGTATCAGCAGAAACCTGGCCAGGCGCCCAGGCTCCTCATCTAT |
| 87 | TGGTACCAGCAGAAACCTGGGCAGGCTCCCAGGCTCCTCATCTAT |
| 88 | TGGTACCAGCAGAAACCTGGCCTGGCGCCCAGGCTCCTCATCTAT |
| 89 | TGGTACCAGCAGAAACCTGGCCAGGCTCCCAGGCTCCTCATCTAT |
| 90 | TGGTACCAGCAGAAACCAGGACAGCCTCCTAAGCTGCTCATTTAC |
| 91 | TGGTACCTGCAGAAGCCAGGGCAGTCTCCACAGCTCCTGATCTAT |
| 92 | TGGTACCTGCAGAAGCCAGGGCAGTCTCCACAGCTCCTGATCTAT |

**Table 3. FR3 of Light Chains**

| |
|---|
| 93 |
| GGGGTCCCAGACAGATTCAGCGGCAGTGGGTCAGGCACTGATTTCACACTGAAAATCAGCAGGGTGGAGGC TGAGGATGTTGGGGTTTATTACTGC |
| 94 |
| GGGGTCCCAGACAGATTCAGCGGCAGTGGGTCAGGCACTGATTTCACACTGAAAATCAGCAGGGTGGAGGC TGAGGATGTTGGGGTTTATTACTGC |
| 95 |
| GGAGTGCCAGATAGGTTCAGTGGCAGCGGGTCAGGGACAGATTTCACACTGAAAATCAGCCGGGTGGAGGC TGAGGATGTTGGGGTTTATTACTGA |
| 96 |
| GGGGTCCCTGACAGGTTCAGTGGCAGTGGATCAGGCACAGATTTTACACTGAAAATCAGCAGAGTGGAGGC TGAGGATGTTGGGGTTTATTACTGC |
| 97 |
| GGAGTGCCAGATAGGTTCAGTGGCAGCGGGTCAGGGACAGATTTCACACTGAAAATCAGCCGGGTGGAGGC TGAGGATGTTGGGGTTTATTACTGC |
| 98 |
| GGGGTCCAGACAGATTCAGTGGCAGTGGGCAGGGACAGATTTCACACTGAAAATCAGCAGGGTGGAAGC TGAGGATGTCGGGGTTTATTACTGC |
| 99 |
| GGGGTCCCTGACAGGTTCAGTGGCAGTGGATCAGGCACAGATTTTACACTGAAAATCAGCAGAGTGGAGGC TGAGGATGTTGGGGTTTATTACTGC |
| 100 |
| GGAGTGCCAGATAGGTTCAGTGGCAGCGGGTCAGGGACAGATTTCACACTGAAAATCAGCCGGGTGGAGGC TGAGGATTTTGGAGTTTATTACTGC |
| 101 |
| GGGGTCCCAGACAGATTCAGTGGCAGTGGGGCAGGGACAGATTTCACACTGAAAATCAGCAGGGTGGAAGC TGAGGATGTCGGGGTTTATTACTGC |
| 102 |
| GGGGTCCCCTCGAGGTTCAGTGGCAGTGGATCTGGGACAGATTTCACCCTCACCATCAATAGCCTGGAAGCT GAAGATGCTGCAACGTATTACTGT |
| 103 |
| GGGGTCCCCTCGAGGTTCAGTGGCAGTGGATCTGGGACAGATTTCACCTTTACCATCAGTAGCCTGGAAGCT GAAGATGCTGCAACATATTACTGT |
| 104 |
| GGGGTCCCATCTCGGTTCAGTGGCAGTGGATCTGGGACAGATTTCACTCTCACCATCAGCAGCCTGCAGCCT GAAGATGTTGCAACTTATTACTGT |
| 105 |
| GGGGTCCCCTCGAGGTTCAGTGGCAGTGGATCTGGGACAGATTTCACCCTCACCATCAATAGCCTGGAAGCT GAAGATGCTGCAACGTATTACTGT |
| 106 |
| GGGGTCCCATCAAGGTTCAGCGGCAGTGGATCTGGGACAGAATTCACTCTCACAATCAGCAGCCTGCAGCCT GAAGATTTTGCAACTTATTACTGT |
| 107 |
| GGAATCCCACCTCGATTCAGTGGCAGCGGGTATGGAACAGATTTTACCCTCACAATTAATAACATAGAATCT GAGGATGCTGCATATTACTTCTGT |
| 108 |
| GGGGTCCCATCAAGGTTCAGCGGCAGTGGATCTGGGACAGATTTCACTCTCACCATCAGCAGCCTGCAGCCT GAAGATTTTGCAACTTATTACTGC |
| 109 |
| GGGGTCCCATCAAGGTTCAGCGGCAGTGGATCTGGCACAGATTTCACTCTCACCATCAGCAGCCTGCAGCCT GAAGATTTTGCAACTTATTACTGT |
| 110 |
| GGGGTCCCATCAAGGTTCAGCGGCAGTGGATCTGGGACAGAATTCACTCTCACCATCAGCAGCCTGCAGCCT GATGATTTTGCAACTTATTACTGC |
| 111 |
| GGGGTCCCATCAAGGTTCAGCGGCAGTGGATCTGGGACAGAATTCACTCTCACAATCAGCAGCCTGCAGCCT GAAGATTTTGCAACTTATTACTGT |
| 112 |
| GGGGTCCCATCAAGGTTCAGCGGCAGTGGATCTGGGACAGATTTCACTCTCACCATCAGCAGCCTGCAGCCT GAAGATTTTGCAACTTATTACTGC |
| 113 |
| GGCATCCCAGCCAGGTTCAGTGGCAGTGGGTCTGGGACAGAGTTCACTCTCACCATCAGCAGCCTGCAGTCT GAAGATTTTGCAGTTTATTACTGT |
| 114 |
| GGGGTCCCATCAAGGTTCAGCGGCAGTGGATCTGGGACAGATTTCACTCTCACCATCAGCAGCCTGCAGCCT GAAGATTTTGCAACTTATTACTGT |
| 115 |
| GGGGTCCCATCAAGGTTCAGCGGCAGTGGATCTGGGACAGATTTCACTCTCACTATCAGCAGCCTGCAGCCT GAAGATTTTGCAACTTACTATTGT |
| 116 |
| GGTATCCCAGCCAGGTTCAGTGGCAGTGGGTCTGGGACAGAGTTCACTCTCACCATCAGCAGCCTGCAGTCT GAAGATTTTGCAGTTTATTACTGT |
| 117 |
| GGCATCCCAGCCAGGTTCAGTGGCAGTGGGCCTGGGACAGACTTCACTCTCACCATCAGCAGCCTAGAGCCT GAAGATTTTGCAGTTTATTACTGT |
| 118 |
| GGGGTCTCATCGAGGTTCAGTGGCAGGGGATCTGGGACGGATTTCACTCTCACCATCATCAGCCTGAAGCCT GAAGATTTTGCAGCTTATTACTGT |
| 119 |
| GGGGTCCCATCAAGGTTCAGCGGCAGTGGATCTGGGACGGATTACACTCTCACCATCAGCAGCCTGCAGCCT GAAGATTTTGCAACTTATTACTGT |
| 120 |
| GGGGTCCCATCAAGGTTCAGTGGCAGTGGATCTGGGACAGATTTCACTCTCACCATCAGTTGCCTGCAGTCT GAAGATTTTGCAACTTATTACTGT |
| 121 |
| GGGGTCCCATCAAGGTTCAGCGGCAGTGGATCTGGGACAGATTTCACTCTCACCATCAGCAGCCTGCAGCCT GAAGATTTTGCAACTTATTACTGT |
| 122 |
| GGGGTCCCATCAAGGTTCAGCGGCAGTGGATCTGGGACAGATTTCACTCTCACCATCAGCAGCCTGCAGCCT GAAGATTTTGCAACTTACTATTGT |
| 123 |
| GGCATCCCAGCCAGGTTCAGTGGCAGTGGGTCTGGGACAGACTTCACTCTCACCATCAGCAGCCTAGAGCCT GAAGATTTTGCAGTTTATTACTGT |
| 124 |
| GGGGTCCCATCAAGGTTCAGCGGCAGTGGATCTGGGACAGAATTCACTCTCACAATCAGCAGCCTGCAGCCT GAAGATTTTGCAACTTATTACTGT |
| 125 |
| GGGGTCCCATCAAGGTTCAGCGGCAGTGGATCTGGGACAGATTTCACTCTCACCATCAGCTGCCTGCAGTCT GAAGATTTTGCAACTTATTACTGT |
| 126 |
| GGGGTCCCATCAAGGTTCAGTGGCAGTGGATCTGGGACAGATTTCACTCTCACCATCAGCAGTCTGCAACCT GAAGATTTTGCAACTTACTACTGT |
| 127 |
| GGAGTCCCATCTCGGTTCAGTGGCAGTGGATCTGGGACAGATTTCACTCTCACTATCAGCAGCCTGCAGCCT GAAGATGTTGCAACTTATTACGGT |
| 128 |
| GGGGTCCCATCAAGGTTCAGTGGAAGTGGATCTGGGACAGATTTTACTTTCACCATCAGCAGCCTGCAGCCT GAAGATATTGCAACATATTACTGT |
| 129 |
| GGGGTCCCATCAAGGTTCAGTGGCAGTGGATCTGGGACAGATTTCACTCTCACCATCAGCAGTCTGCAACCT GAAGATTTTGCAACTTACTACTGT |
| 130 |
| GGAGTCCCATCTCGGTTCAGTGGCAGTGGATCTGGGACAGATTTCACTCTCACTATCAGCAGCCTGCAGCCT GAAGATGTTGCAACTTATTACGGT |
| 131 |
| GGGGTCCCATCAAGGTTCAGTGGAAGTGGATCTGGGACAGATTTTACTTTCACCATCAGCAGCCTGCAGCCT GAAGATATTGCAACATATTACTGT |
| 132 |
| AGCATCCCAGCCAGGTTCAGTGGCAGTGGGTCTGGGACAGACTTCACTCTCACCATCAGCAGCCTGCAGCCT GAAGATTTTGCAGTTTATTACTGT |
| 133 |
| GGCATCCCAGCCAGGTTCAGTGGCAGTGGGTCTGGGACAGACTTCACTCTCACCATCAGCAGCCTGCAGCCT GAAGATTTTGCAGTTTATTACTGT |
| 134 |
| GGCATCCCAGACAGGTTCAGTGGCAGTGGGTCTGGGACAGACTTCACTCTCACCATCAGCAGACTGGAGCCT GAAGATTTTGCAGTGTATTACTGT |
| 135 |
| GGCATCCCAGACAGGTTCAGTGGCAGTGGGTCTGGGACAGACTTCACTCTCACCATCAGCAGACTGGAGCCT GAAGATTTTGCAGTGTATTACTGT |
| 136 |
| GGGGTCCCTGACCGATTCAGTGGCAGCGGGTCTGGGACAGATTTCACTCTCACCATCAGCAGCCTGCAGGCT GAAGATGTGGCAGTTTATTACTGT |
| 137 |
| GGAGTCCCAGACAGGTTCAGTGGCAGTGGGTCAGGCACTGATTTCACACTGAAAATCAGCAGGGTGGAGGC TGAGGATGTTGGAGTTTATTACTGC |
| 138 |
| GGAGTCCCAGACAGGTTCAGTGGCAGTGGGTCAGGCACTGATTTCACACTGAAAATCAGCAGGGTGGAGGC TGAGGATGTTGGAGTTTATTACTGC |

**Table 4. FR4 of Light Chains**

| | |
|---|---|
| 139 | TTCGGCCAAGGGACCAAGGTGGAAATCAAA |
| 140 | TTTGGCCAGGGGACCAAGCTGGAGATCAAA |
| 141 | TTCGGCCCTGGGACCAAAGTGGATATCAAA |
| 142 | TTCGGCGGAGGGACCAAGGTGGAGATCAAA |
| 143 | TTCGGCCAAGGGACACGACTGGAGATTAAA |

**Table 5. FR1 of Heavy Chains (Kabat definition)**

| |
|---|
| 144 |
| CAGGTTCAGCTGGTGCAGTCTGGAGCTGAGGTGAAGAAGCCTGGGGCCTCAGTGAAGGTCTCCTGCAAGGCT TCTGGTTACACCTTTACC |
| 145 |
| CAGGTGCAGCTGGTGCAGTCTGGGGCTGAGGTGAAGAAGCCTGGGGCCTCAGTGAAGGTCTCCTGCAAGGC TTCTGGATACACCTTCACC |
| 146 |
| CAGGTCCAGCTGGTACAGTCTGGGGCTGAGGTGAAGAAGCCTGGGGCCTCAGTGAAGGTCTCCTGCAAGGTT TCCGGATACACCCTCACT |
| 147 |
| CAGGTTCAGCTGGTGCAGTCTGGGGCTGAGGTGAAGAAGCCTGGGGCCTCAGTGAAGGTTTCCTGCAAGGCT TCTGGATACACCTTCACT |
| 148 |
| CAGATGCAGCTGGTGCAGTCTGGGGCTGAGGTGAAGAAGACTGGGTCCTCAGTGAAGGTTTCCTGCAAGGCT TCCGGATACACCTTCACC |
| 149 |
| CAGGTGCAGCTGGTGCAGTCTGGGGCTGAGGTGAAGAAGCCTGGGGCCTCAGTGAAGGTTTCCTGCAAGGC ATCTGGATACACCTTCACC |
| 150 |
| CAAATGCAGCTGGTGCAGTCTGGGCCTGAGGTGAAGAAGCCTGGGACCTCAGTGAAGGTCTCCTGCAAGGCT TCTGGATTCACCTTTACT |
| 151 |
| CAGGTGCAGCTGGTGCAGTCTGGGGCTGAGGTGAAGAAGCCTGGGTCCTCGGTGAAGGTCTCCTGCAAGGCT TCTGGAGGCACCTTCAGC |
| 152 |
| CAGGTGCAGCTGGTGCAGTCTGGGGCTGAGGTGAAGAAGCCTGGGGCCTCAGTGAAGGTCTCCTGCAAGGC TTCTGGATACACCTTCACC |
| 153 |
| CAGGTCACCTTGAAGGAGTCTGGTCCTGTGCTGGTGAAACCCACAGAGACCCTCACGCTGACCTGCACCGTC TCTGGGTTCTCACTCAGC |
| 154 |
| CAGATCACCTTGAAGGAGTCTGGTCCTACGCTGGTGAAACCCACACAGACCCTCACGCTGACCTGCACCTTC TCTGGGTTCTCACTCAGC |
| 155 |
| CAGGTCACCTTGAGGGAGTCTGGTCCTGCGCTGGTGAAACCCACACAGACCCTCACACTGACCTGCACCTTC TCTGGGTTCTCACTCAGC |
| 156 |
| CAGGTGCAGCTGGTGGAGTCTGGGGGAGGCTTGGTCAAGCCTGGAGGGTCCCTGAGACTCTCCTGTGCAGCC TCTGGATTCACCTTCAGT |
| 157 |
| GAGGTGCAGCTGGTGGAGTCTGGGGGAGGCTTGGTACAGCCTGGGGGGTCCCTGAGACTCTCCTGTGCAGCC TCTGGATTCACCTTCAGT |
| 158 |
| GAGGTGCAGCTGGTGGAGTCTGGGGGAGGCTTGGTAAAGCCTGGGGGGTCCCTTAGACTCTCCTGTGCAGCC TCTGGATTCACTTTCAGT |
| 159 |
| GAGGTGCAGCTGGTGGAGTCTGGGGGAGGCTTGGTACAGCCTGGGGGGTCCCTGAGACTCTCCTGTGCAGCC TCTGGATTCACCTTCAGT |
| 160 |
| GAGGTGCAGCTGGTGGAGTCTGGGGGAGGTGTGGTACGGCCTGGGGGGTCCCTGAGACTCTCCTGTGCAGCC TCTGGATTCACCTTTGAT |
| 161 |
| GAGGTGCAGCTGGTGGAGTCTGGGGGAGGCCTGGTCAAGCCTGGGGGGTCCCTGAGACTCTCCTGTGCAGCC TCTGGATTCACCTTCAGT |
| 162 |
| GAGGTGCAGCTGTTGGAGTCTGGGGGAGGCTTGGTACAGCCTGGGGGGTCCCTGAGACTCTCCTGTGCAGCC TCTGGATTCACCTTTAGC |
| 163 |
| CAGGTGCAGCTGGTGGAGTCTGGGGGAGGCGTGGTCCAGCCTGGGAGGTCCCTGAGACTCTCCTGTGCAGCC TCTGGATTCACCTTCAGT |
| 164 |
| CAGGTGCAGCTGGTGGAGTCTGGGGGAGGCGTGGTCCAGCCTGGGAGGTCCCTGAGACTCTCCTGTGCAGCG TCTGGATTCACCTTCAGT |
| 165 |
| GAGGTGCAGCTGGTGGAGTCTGGGGGAGGCTTGGTACAGCCTGGGGGATCCCTGAGACTCTCCTGTGCAGCC TCTGGATTCACCTTCAGT |
| 166 |
| GAGGTGCAGCTGGTGGAGTCTGGGGGAGGCTTGGTACAGCCTAGGGGGTCCCTGAGACTCTCCTGTGCAGCC TCTGGATTCACCGTCAGT |
| 167 |
| GAAGTGCAGCTGGTGGAGTCTGGGGGAGTCGTGGTACAGCCTGGGGGGTCCCTGAGACTCTCCTGTGCAGCC TCTGGATTCACCTTTGAT |
| 168 |
| GAGGTGCAGCTGGTGGAGTCTGGGGGAGGCTTGGTACAGCCTGGGGGGTCCCTGAGACTCTCCTGTGCAGCC TCTGGATTCACCTTCAGT |
| 169 |
| GAGGTGCAGCTGGTGGAGTCTGGGGGAGGCTTGGTACAGCCAGGGCGGTCCCTGAGACTCTCCTGTACAGCT TCTGGATTCACCTTTGGT |
| 170 |
| GAGGTGCAGCTGGTGGAGTCTGGAGGAGGCTTGATCCAGCCTGGGGGGTCCCTGAGACTCTCCTGTGCAGCC TCTGGGTTCACCGTCAGT |
| 171 |
| GAGGTGCAGCTGGTGGAGTCTGGGGAAGGCTTGGTCCAGCCTGGGGGGTCCCTGAGACTCTCCTGTGCAGCC TCTGGATTCACCTTCAGT |
| 172 |
| GAGGTGCAGCTGGTGGAGTCTGGAGGAGGCTTGATCCAGCCTGGGGGGTCCCTGAGACTCTCCTGTGCAGCC TCTGGGTTCACCGTCAGT |
| 173 |
| GAGGTGCAGCTGGTGGAGTCTGGGGGAGGCTTGGTCCAGCCTGGGGGGTCCCTGAGACTCTCCTGTGCAGCC TCTGGATTCACCTTTAGT |
| 174 |
| GAGGTGCAGCTGGTGGAGTCTGGGGGAGGCTTGGTCCAGCCTGGAGGGTCCCTGAGACTCTCCTGTGCAGCC TCTGGATTCACCTTCAGT |
| 175 |
| GAGGTGCAGCTGGTGGAGTCCGGGGGAGGCTTGGTCCAGCCTGGGGGGTCCCTGAAACTCTCCTGTGCAGCC TCTGGGTTCACCTTCAGT |
| 176 |
| GAGGTGCAGCTGGTGGAGTCCGGGGGAGGCTTAGTTCAGCCTGGGGGGTCCCTGAGACTCTCCTGTGCAGCC TCTGGATTCACCTTCAGT |
| 177 |
| GAAGTGCAGCTGGTGGAGTCTGGGGGAGGCTTGGTACAGCCTGGCAGGTCCCTGAGACTCTCCTGTGCAGCC TCTGGATTCACCTTTGAT |
| 178 |
| CAGGTGCAGCTGCAGGAGTCGGGCCCAGGACTGGTGAAGCCTTCGGACACCCTGTCCCTCACCTGCGCTGTC TCTGGTTACTCCATCAGC |
| 179 |
| CAGGTGCAGCTGCAGGAGTCGGGCCCAGGACTGGTGAAGCCTTCACAGACCCTGTCCCTCACCTGTACTGTC TCTGGTGGCTCCATCAGC |
| 180 |
| CAGGTGCAGCTACAGCAGTGGGGCGCAGGACTGTTGAAGCCTTCGGAGACCCTGTCCCTCACCTGCGCTGTC TATGGTGGGTCCTTCAGT |
| 181 |
| CAGCTGCAGCTGCAGGAGTCGGGCCCAGGACTGGTGAAGCCTTCGGAGACCCTGTCCCTCACCTGCACTGTC TCTGGTGGCTCCATCAGC |
| 182 |
| CAGGTGCAGCTGCAGGAGTCGGGCCCAGGACTGGTGAAGCCTTCGGAGACCCTGTCCCTCACCTGCACTGTC TCTGGTGGCTCCATCAGT |
| 183 |
| CAGGTGCAGCTGCAGGAGTCGGGCCCAGGACTGGTGAAGCCTTCGGAGACCCTGTCCCTCACCTGCACTGTC TCTGGTGGCTCCATCAGT |
| 184 |
| CAGGTGCAGCTGCAGGAGTCGGGCCCAGGACTGGTGAAGCCTTCGGAGACCCTGTCCCTCACCTGCACTGTC TCTGGTGGCTCCGTCAGC |
| 185 |
| GAGGTGCAGCTGGTGCAGTCTGGAGCAGAGGTGAAAAAGCCCGGGGAGTCTCTGAAGATCTCCTGTAAGGG TTCTGGATACAGCTTTACC |
| 186 |
| CAGGTACAGCTGCAGCAGTCAGGTCCAGGACTGGTGAAGCCCTCGCAGACCCTCTCACTCACCTGTGCCATC TCCGGGGACAGTGTCTCT |
| 187 |
| CAGGTGCAGCTGGTGCAGTCTGGCCATGAGGTGAAGCAGCCTGGGGCCTCAGTGAAGGTCTCCTGCAAGGCT TCTGGTTACAGTTTCACC |

**Table 6. FR2 of Heavy Chains (Kabat definition)**

| | |
|---|---|
| 188 | TGGGTGCGACAGGCCCCTGGACAAGGGCTTGAGTGGATGGGA |
| 189 | TGGGTGCGACAGGCCCCTGGACAAGGGCTTGAGTGGATGGGA |
| 190 | TGGGTGCGACAGGCTCCTGGAAAAGGGCTTGAGTGGATGGGA |
| 191 | TGGGTGCGCCAGGCCCCCGGACAAAGGCTTGAGTGGATGGGA |
| 192 | TGGGTGCGACAGGCCCCCGGACAAGCGCTTGAGTGGATGGGA |
| 193 | TGGGTGCGACAGGCCCCTGGACAAGGGCTTGAGTGGATGGGA |
| 194 | TGGGTGCGACAGGCTCGTGGACAACGCCTTGAGTGGATAGGA |
| 195 | TGGGTGCGACAGGCCCCTGGACAAGGGCTTGAGTGGATGGGA |
| 196 | TGGGTGCGACAGGCCACTGGACAAGGGCTTGAGTGGATGGGA |
| 197 | TGGATCCGTCAGCCCCCAGGGAAGGCCCTGGAGTGGCTTGCA |
| 198 | TGGATCCGTCAGCCCCCAGGAAAGGCCCTGGAGTGGCTTGCA |
| 199 | TGGATCCGTCAGCCCCCAGGGAAGGCCCTGGAGTGGCTTGCA |
| 200 | TGGATCCGCCAGGCTCCAGGGAAGGGGCTGGAGTGGGTTTCA |
| 201 | TGGGTCCGCCAAGCTACAGGAAAAGGTCTGGAGTGGGTCTCA |
| 202 | TGGGTCCGCCAGGCTCCAGGGAAGGGGCTGGAGTGGGTTGGC |
| 203 | TGGGCCCGCAAGGCTCCAGGAAAGGGGCTGGAGTGGGTATCG |
| 204 | TGGGTCCGCCAAGCTCCAGGGAAGGGGCTGGAGTGGGTCTCT |
| 205 | TGGGTCCGCCAGGCTCCAGGGAAGGGGCTGGAGTGGGTCTCA |
| 206 | TGGGTCCGCCAGGCTCCAGGGAAGGGGCTGGAGTGGGTCTCA |
| 207 | TGGGTCCGCCAGGCTCCAGGCAAGGGGCTGGAGTGGGTGGCA |
| 208 | TGGGTCCGCCAGGCTCCAGGCAAGGGGCTGGAGTGGGTGGCA |
| 209 | TGGGTCCATCAGGCTCCAGGAAAGGGGCTGGAGTGGGTATCG |
| 210 | TGGATCCGCCAGGCTCCAGGGAAGGGGCTGGAGTGGGTCTCA |
| 211 | TGGGTCCGTCAAGCTCCGGGGAAGGGTCTGGAGTGGGTCTCT |
| 212 | TGGGTCCGCCAGGCTCCAGGGAAGGGGCTGGAGTGGGTTTCA |
| 213 | TGGTTCCGCCAGGCTCCAGGGAAGGGGCTGGAGTGGGTAGGT |
| 214 | TGGGTCCGCCAGGCTCCAGGGAAGGGGCTGGAGTGGGTCTCA |
| 215 | TGGGTCCGCCAGGCTCCAGGGAAGGGACTGGAATATGTTTCA |
| 216 | TGGGTCCGCCAGGCTCCAGGGAAGGGGCTGGAGTGGGTCTCA |
| 217 | TGGGTCCGCCAGGCTCCAGGGAAGGGGCTGGAGTGGGTGGCC |
| 218 | TGGGTCCGCCAGGCTCCAGGGAAGGGGCTGGAGTGGGTTGGC |
| 219 | TGGGTCCGCCAGGCTTCCGGGAAAGGGCTGGAGTGGGTTGGC |
| 220 | TGGGTCCGCCAAGCTCCAGGGAAGGGGCTGGTGTGGGTCTCA |
| 221 | TGGGTCCGGCAAGCTCCAGGGAAGGGCCTGGAGTGGGTCTCA |
| 222 | TGGATCCGGCAGCCCCCAGGGAAGGGACTGGAGTGGATTGGG |
| 223 | TGGATCCGCCAGCACCCAGGGAAGGGCCTGGAGTGGATTGGG |
| 224 | TGGATCCGCCAGCCCCCAGGGAAGGGGCTGGAGTGGATTGGG |
| 225 | TGGATCCGCCAGCCCCCAGGGAAGGGGCTGGAGTGGATTGGG |
| 226 | TGGATCCGGCAGCCCGCCGGGAAGGGACTGGAGTGGATTGGG |
| 227 | TGGATCCGGCAGCCCCCAGGGAAGGGACTGGAGTGGATTGGG |
| 228 | TGGATCCGGCAGCCCCCAGGGAAGGGACTGGAGTGGATTGGG |
| 229 | TGGGTGCGCCAGATGCCCGGGAAAGGCCTGGAGTGGATGGGG |
| 230 | TGGATCAGGCAGTCCCCATCGAGAGGCCTTGAGTGGCTGGGA |
| 231 | TGGGTGCCACAGGCCCCTGGACAAGGGCTTGAGTGGATGGGA |

**Table 7. FR3 of Heavy Chains (Kabat definition)**

| |
|---|
| 232 |
| AGAGTCACCATGACCACAGACACATCCACGAGCACAGCCTACATGGAGCTGAGGAGCCTGAGATCTGACGA CACGGCCGTGTATTACTGTGCGAGA |
| 233 |
| AGGGTCACCATGACCAGGGACACGTCCATCAGCACAGCCTACATGGAGCTGAGCAGGCTGAGATCTGACGA CACGGCCGTGTATTACTGTGCGAGA |
| 234 |
| AGAGTCACCATGACCGAGGACACATCTACAGACACAGCCTACATGGAGCTGAGCAGCCTGAGATCTGAGGA CACGGCCGTGTATTACTGTGCAACA |
| 235 |
| AGAGTCACCATTACCAGGGACACATCCGCGAGCACAGCCTACATGGAGCTGAGCAGCCTGAGATCTGAGGA CATGGCTGTGTATTACTGTGCGAGA |
| 236 |
| AGAGTCACCATTACCAGGGACAGGTCTATGAGCACAGCCTACATGGAGCTGAGCAGCCTGAGATCTGAGGA CACAGCCATGTATTACTGTGCAAGA |
| 237 |
| AGAGTCACCATGACCAGGGACACGTCCACGAGCACAGTCTACATGGAGCTGAGCAGCCTGAGATCTGAGGA CACGGCCGTGTATTACTGTGCGAGA |
| 238 |
| AGAGTCACCATTACCAGGACATGTCCACAAGCACAGCCTACATGGAGCTGAGCAGCCTGAGATCCGAGGA CACGGCCGTGTATTACTGTGCGGCA |
| 239 |
| AGAGTCACGATTACCGCGGACAAATCCACGAGCACAGCCTACATGGAGCTGAGCAGCCTGAGATCTGAGGA CACGGCCGTGTATTACTGTGCGAGA |
| 240 |
| AGAGTCACCATGACCAGGAACACCTCCATAAGCACAGCCTACATGGAGCTGAGCAGCCTGAGATCTGAGGA CACGGCCGTGTATTACTGTGCGAGA |
| 241 |
| AGGCTCACCATCTCCAAGGACACCTCCAAAAGCCAGGTGGTCCTTACCATGACCAACATGGACCCTGTGGAC ACAGCCACATATTACTGTGCACGG |
| 242 |
| AGGCTCACCATCACCAAGGACACCTCCAAAAACCAGGTGGTCCTTACAATGACCAACATGGACCCTGTGGAC ACAGCCACATATTACTGTGCACAC |
| 243 |
| AGGCTCACCATCTCCAAGGACACCTCCAAAAACCAGGTGGTCCTTACAATGACCAACATGGACCCTGTGGAC ACAGCCACGTATTATTGTGCACGG |
| 244 |
| CGATTCACCATCTCCAGGGACAACGCCAAGAACTCACTGTATCTGCAAATGAACAGCCTGAGAGCCGAGGA CACGGCCGTGTATTACTGTGCGAGA |
| 245 |
| CGATTCACCATCTCCAGAGAAAATGCCAAGAACTCCTTGTATCTTCAAATGAACAGCCTGAGAGCCGGGGAC ACGGCTGTGTATTACTGTGCAAGA |
| 246 |
| AGATTCACCATCTCAAGAGATGATTCAAAAAACACGCTGTATCTGCAAATGAACAGCCTGAAAACCGAGGA CACAGCCGTGTATTACTGTACCACA |
| 247 |
| CGATTCATCATCTCCAGAGACAATTCCAGGAACTCCCTGTATCTGCAAAAGAACAGACGGAGAGCCGAGGA CATGGCTGTGTATTACTGTGTGAGA |
| 248 |
| CGATTCACCATCTCCAGAGACAACGCCAAGAACTCCCTGTATCTGCAAATGAACAGTCTGAGAGCCGAGGAC ACGGCCTTGTATCACTGTGCGAGA |
| 249 |
| CGATTCACCATCTCCAGAGACAACGCCAAGAACTCACTGTATCTGCAAATGAACAGCCTGAGAGCCGAGGA CACGGCTGTGTATTACTGTGCGAGA |
| 250 |
| CGGTTCACCATCTCCAGAGACAATTCCAAGAACACGCTGTATCTGCAAATGAACAGCCTGAGAGCCGAGGA CACGGCCGTATATTACTGTGCGAAA |
| 251 |
| CGATTCACCATCTCCAGAGACAATTCCAAGAACACGCTGTATCTGCAAATGAACAGCCTGAGAGCTGAGGAC ACGGCTGTGTATTACTGTGCGAGA |
| 252 |
| CGATTCACCATCTCCAGAGACAATTCCAAGAACACGCTGTATCTGCAAATGAACAGCCTGAGAGCCGAGGA CACGGCTGTGTATTACTGTGCGAGA |
| 253 |
| CGATTCATCATCTCCAGAGACAATTCCAGGAACACCCTGTATCTGCAAACGAATAGCCTGAGGGCCGAGGAC ACGGCTGTGTATTACTGTGTGAGA |
| 254 |
| AGATTCACCATCTCCAGAGACAATTCCAAGAACACGCTGTATCTTCAAATGAACAACCTGAGAGCTGAGGGC ACGGCCGTGTATTACTGTGCCAGA |
| 255 |
| CGATTCACCATCTCCAGAGACAACAGCAAAAACTCCCTGTATCTGCAAATGAACAGTCTGAGAACTGAGGAC ACCGCCTTGTATTACTGTGCAAAA |
| 256 |
| CGATTCACCATCTCCAGAGACAATGCCAAGAACTCACTGTATCTGCAAATGAACAGCCTGAGAGACGAGGA CACGGCTGTGTATTACTGTGCGAGA |
| 257 |
| AGATTCACCATCTCAAGAGATGATTCCAAAAGCATCGCCTATCTGCAAATGAACAGCCTGAAAACCGAGGA CACAGCCGTGTATTACTGTACTAGA |
| 258 |
| CGATTCACCATCTCCAGAGACAATTCCAAGAACACGCTGTATCTTCAAATGAACAGCCTGAGAGCCGAGGAC ACGGCCGTGTATTACTGTGCGAGA |
| 259 |
| AGATTCACCATCTCCAGAGACAATTCCAAGAACACGCTGTATCTTCAAATGGGCAGCCTGAGAGCTGAGGAC ATGGCTGTGTATTACTGTGCGAGA |
| 260 |
| CGATTCACCATCTCCAGAGACAATTCCAAGAACACGCTGTATCTTCAAATGAACAGCCTGAGAGCTGAGGAC ACGGCTGTGTATTACTGTGCGAGA |
| 261 |
| CGATTCACCATCTCCAGAGACAACGCCAAGAACTCACTGTATCTGCAAATGAACAGCCTGAGAGCCGAGGA CACGGCTGTGTATTACTGTGCGAGA |
| 262 |
| AGATTCACCATCTCAAGAGATGATTCAAAGAACTCACTGTATCTGCAAATGAACAGCCTGAAAACCGAGGAC ACGGCCGTGTATTACTGTGCTAGA |
| 263 |
| AGGTTCACCATCTCCAGAGATGATTCAAAGAACACGGCGTATCTGCAAATGAACAGCCTGAAAACCGAGGA CACGGCCGTGTATTACTGTACTAGA |
| 264 |
| CGATTCACCATCTCCAGAGACAACGCCAAGAACACGCTGTATCTGCAAATGAACAGTCTGAGAGCCGAGGA CACGGCTGTGTATTACTGTGCAAGA |
| 265 |
| CGATTCACCATCTCCAGAGACAACGCCAAGAACTCCCTGTATCTGCAAATGAACAGTCTGAGAGCTGAGGAC ACGGCCTTGTATTACTGTGCAAAA |
| 266 |
| CGAGTCACCATGTCAGTAGACACGTCCAAGAACCAGTTCTCCCTGAAGCTGAGCTCTGTGACCGCCGTGGAC ACGGCCGTGTATTACTGTGCGAGA |
| 267 |
| CGAGTTACCATATCAGTAGACACGTCTAAGAACCAGTTCTCCCTGAAGCTGAGCTCTGTGACTGCCGCGGAC ACGGCCGTGTATTACTGTGCGAGA |
| 268 |
| CGAGTCACCATATCAGTAGACACGTCCAAGAACCAGTTCTCCCTGAAGCTGAGCTCTGTGACCGCCGCGGAC ACGGCTGTGTATTACTGTGCGAGA |
| 269 |
| CGAGTCACCATATCCGTAGACACGTCCAAGAACCAGTTCTCCCTGAAGCTGAGCTCTGTGACCGCCGCAGAC ACGGCTGTGTATTACTGTGCGAGA |
| 270 |
| CGAGTCACCATGTCAGTAGACACGTCCAAGAACCAGTTCTCCCTGAAGCTGAGCTCTGTGACCGCCGCGGAC ACGGCCGTGTATTACTGTGCGAGA |
| 271 |
| CGAGTCACCATATCAGTAGACACGTCCAAGAACCAGTTCTCCCTGAAGCTGAGCTCTGTGACCGCTGCGGAC ACGGCCGTGTATTACTGTGCGAGA |
| 272 |
| CGAGTCACCATATCAGTAGACACGTCCAAGAACCAGTTCTCCCTGAAGCTGAGCTCTGTGACCGCTGCGGAC ACGGCCGTGTATTACTGTGCGAGA |
| 273 |
| CAGGTCACCATCTCAGCCGACAAGTCCATCAGCACCGCCTACCTGCAGTGGAGCAGCCTGAAGGCCTCGGAC ACCGCCATGTATTACTGTGCGAGA |
| 274 |
| CGAATAACCATCAACCCAGACACATCCAAGAACCAGTTCTCCCTGCAGCTGAACTCTGTGACTCCCGAGGAC ACGGCTGTGTATTACTGTGCAAGA |
| 275 |
| CGGTTTGTCTTCTCCATGGACACCTCTGCCAGCACAGCATACCTGCAGATCAGCAGCCTAAAGGCTGAGGAC ATGGCCATGTATTACTGTGCGAGA |

**Table 8. FR1 of Heavy Chains (Chothia definition)**

| |
|---|
| 276 |
| CAGGTTCAGCTGGTGCAGTCTGGAGCTGAGGTGAAGAAGCCTGGGGCCTCAGTGAAGGTCTCCTGCAAGGCT TCT |
| 277 |
| CAGGTGCAGCTGGTGCAGTCTGGGGCTGAGGTGAAGAAGCCTGGGGCCTCAGTGAAGGTCTCCTGCAAGGC TTCT |
| 278 |
| CAGGTCCAGCTGGTACAGTCTGGGGCTGAGGTGAAGAAGCCTGGGGCCTCAGTGAAGGTCTCCTGCAAGGTT TCC |
| 279 |
| CAGGTTCAGCTGGTGCAGTCTGGGGCTGAGGTGAAGAAGCCTGGGGCCTCAGTGAAGGTTTCCTGCAAGGCT TCT |
| 280 |
| CAGATGCAGCTGGTGCAGTCTGGGGCTGAGGTGAAGAAGACTGGGTCCTCAGTGAAGGTTTCCTGCAAGGCT TCC |
| 281 |
| CAGGTGCAGCTGGTGCAGTCTGGGGCTGAGGTGAAGAAGCCTGGGGCCTCAGTGAAGGTTTCCTGCAAGGC ATCT |
| 282 |
| CAAATGCAGCTGGTGCAGTCTGGGCCTGAGGTGAAGAAGCCTGGGACCTCAGTGAAGGTCTCCTGCAAGGCT TCT |
| 283 |
| CAGGTGCAGCTGGTGCAGTCTGGGGCTGAGGTGAAGAAGCCTGGGTCCTCGGTGAAGGTCTCCTGCAAGGCT TCT |
| 284 |
| CAGGTGCAGCTGGTGCAGTCTGGGGCTGAGGTGAAGAAGCCTGGGGCCTCAGTGAAGGTCTCCTGCAAGGC TTCT |
| 285 |
| CAGGTCACCTTGAAGGAGTCTGGTCCTGTGCTGGTGAAACCCACAGAGACCCTCACGCTGACCTGCACCGTC TCT |
| 286 |
| CAGATCACCTTGAAGGAGTCTGGTCCTACGCTGGTGAAACCCACACAGACCCTCACGCTGACCTGCACCTTC TCT |
| 287 |
| CAGGTCACCTTGAGGGAGTCTGGTCCTGCGCTGGTGAAACCCACACAGACCCTCACACTGACCTGCACCTTC TCT |
| 288 |
| CAGGTGCAGCTGGTGGAGTCTGGGGGAGGCTTGGTCAAGCCTGGAGGGTCCCTGAGACTCTCCTGTGCAGCC TCT |
| 289 |
| GAGGTGCAGCTGGTGGAGTCTGGGGGAGGCTTGGTACAGCCTGGGGGGTCCCTGAGACTCTCCTGTGCAGCC TCT |
| 290 |
| GAGGTGCAGCTGGTGGAGTCTGGGGGAGGCTTGGTAAAGCCTGGGGGGTCCCTTAGACTCTCCTGTGCAGCC TCT |
| 291 |
| GAGGTGCAGCTGGTGGAGTCTGGGGGAGGCTTGGTACAGCCTGGGGGGTCCCTGAGACTCTCCTGTGCAGCC TCT |
| 292 |
| GAGGTGCAGCTGGTGGAGTCTGGGGGAGGTGTGGTACGGCCTGGGGGGTCCCTGAGACTCTCCTGTGCAGCC TCT |
| 293 |
| GAGGTGCAGCTGGTGGAGTCTGGGGGAGGCCTGGTCAAGCCTGGGGGGTCCCTGAGACTCTCCTGTGCAGCC TCT |
| 294 |
| GAGGTGCAGCTGTTGGAGTCTGGGGGAGGCTTGGTACAGCCTGGGGGGTCCCTGAGACTCTCCTGTGCAGCC TCT |
| 295 |
| CAGGTGCAGCTGGTGGAGTCTGGGGGAGGCGTGGTCCAGCCTGGGAGGTCCCTGAGACTCTCCTGTGCAGCC TCT |
| 296 |
| CAGGTGCAGCTGGTGGAGTCTGGGGGAGGCGTGGTCCAGCCTGGGAGGTCCCTGAGACTCTCCTGTGCAGCG TCT |
| 297 |
| GAGGTGCAGCTGGTGGAGTCTGGGGGAGGCTTGGTACAGCCTGGGGGATCCCTGAGACTCTCCTGTGCAGCC TCT |
| 298 |
| GAGGTGCAGCTGGTGGAGTCTGGGGGAGGCTTGGTACAGCCTAGGGGGTCCCTGAGACTCTCCTGTGCAGCC TCT |
| 299 |
| GAAGTGCAGCTGGTGGAGTCTGGGGGAGTCGTGGTACAGCCTGGGGGGTCCCTGAGACTCTCCTGTGCAGCC TCT |
| 300 |
| GAGGTGCAGCTGGTGGAGTCTGGGGGAGGCTTGGTACAGCCTGGGGGGTCCCTGAGACTCTCCTGTGCAGCC TCT |
| 301 |
| GAGGTGCAGCTGGTGGAGTCTGGGGGAGGCTTGGTACAGCCAGGGCGGTCCCTGAGACTCTCCTGTACAGCT TCT |
| 302 |
| GAGGTGCAGCTGGTGGAGTCTGGAGGAGGCTTGATCCAGCCTGGGGGGTCCCTGAGACTCTCCTGTGCAGCC TCT |
| 303 |
| GAGGTGCAGCTGGTGGAGTCTGGGGAAGGCTTGGTCCAGCCTGGGGGGTCCCTGAGACTCTCCTGTGCAGCC TCT |
| 304 |
| GAGGTGCAGCTGGTGGAGTCTGGAGGAGGCTTGATCCAGCCTGGGGGGTCCCTGAGACTCTCCTGTGCAGCC TCT |
| 305 |
| GAGGTGCAGCTGGTGGAGTCTGGGGGAGGCTTGGTCCAGCCTGGGGGGTCCCTGAGACTCTCCTGTGCAGCC TCT |
| 306 |
| GAGGTGCAGCTGGTGGAGTCTGGGGGAGGCTTGGTCCAGCCTGGAGGGTCCCTGAGACTCTCCTGTGCAGCC TCT |
| 307 |
| GAGGTGCAGCTGGTGGAGTCCGGGGGAGGCTTGGTCCAGCCTGGGGGGTCCCTGAAACTCTCCTGTGCAGCC TCT |
| 308 |
| GAGGTGCAGCTGGTGGAGTCCGGGGGAGGCTTAGTTCAGCCTGGGGGGTCCCTGAGACTCTCCTGTGCAGCC TCT |
| 309 |
| GAAGTGCAGCTGGTGGAGTCTGGGGGAGGCTTGGTACAGCCTGGCAGGTCCCTGAGACTCTCCTGTGCAGCC TCT |
| 310 |
| CAGGTGCAGCTGCAGGAGTCGGGCCCAGGACTGGTGAAGCCTTCGGACACCCTGTCCCTCACCTGCGCTGTC TCT |
| 311 |
| CAGGTGCAGCTGCAGGAGTCGGGCCCAGGACTGGTGAAGCCTTCACAGACCCTGTCCCTCACCTGTACTGTC TCT |
| 312 |
| CAGGTGCAGCTACAGCAGTGGGGCGCAGGACTGTTGAAGCCTTCGGAGACCCTGTCCCTCACCTGCGCTGTC TAT |
| 313 |
| CAGCTGCAGCTGCAGGAGTCGGGCCCAGGACTGGTGAAGCCTTCGGAGACCCTGTCCCTCACCTGCACTGTC TCT |
| 314 |
| CAGGTGCAGCTGCAGGAGTCGGGCCCAGGACTGGTGAAGCCTTCGGAGACCCTGTCCCTCACCTGCACTGTC TCT |
| 315 |
| CAGGTGCAGCTGCAGGAGTCGGGCCCAGGACTGGTGAAGCCTTCGGAGACCCTGTCCCTCACCTGCACTGTC TCT |
| 316 |
| CAGGTGCAGCTGCAGGAGTCGGGCCCAGGACTGGTGAAGCCTTCGGAGACCCTGTCCCTCACCTGCACTGTC TCT |
| 317 |
| GAGGTGCAGCTGGTGCAGTCTGGAGCAGAGGTGAAAAAGCCCGGGGAGTCTCTGAAGATCTCCTGTAAGGG TTCT |
| 318 |
| CAGGTACAGCTGCAGCAGTCAGGTCCAGGACTGGTGAAGCCCTCGCAGACCCTCTCACTCACCTGTGCCATC TCC |
| 319 |
| CAGGTGCAGCTGGTGCAGTCTGGCCATGAGGTGAAGCAGCCTGGGGCCTCAGTGAAGGTCTCCTGCAAGGCT TCT |

**Table 9. FR2 of Heavy Chains (Chothia definition)**

| | |
|---|---|
| 320 | TATGGTATCAGCTGGGTGCGACAGGCCCCTGGACAAGGGCTTGAGTGGATGGGATGGATC |
| 321 | TACTATATGCACTGGGTGCGACAGGCCCCTGGACAAGGGCTTGAGTGGATGGGATGGATC |
| 322 | TTATCCATGCACTGGGTGCGACAGGCTCCTGGAAAAGGGCTTGAGTGGATGGGAGGTTTT |
| 333 | TATGCTATGCATTGGGTGCGCCAGGCCCCCGGACAAAGGCTTGAGTGGATGGGATGGAGC |
| 324 | CGCTACCTGCACTGGGTGCGACAGGCCCCCGGACAAGCGCTTGAGTGGATGGGATGGATC |
| 325 | TACTATATGCACTGGGTGCGACAGGCCCCTGGACAAGGGCTTGAGTGGATGGGAATAATC |
| 326 | TCTGCTATGCAGTGGGTGCGACAGGCTCGTGGACAACGCCTTGAGTGGATAGGATGGATC |
| 327 | TATGCTATCAGCTGGGTGCGACAGGCCCCTGGACAAGGGCTTGAGTGGATGGGAGGGATC |
| 328 | TATGATATCAACTGGGTGCGACAGGCCACTGGACAAGGGCTTGAGTGGATGGGATGGATG |
| 329 | ATGGGTGTGAGCTGGATCCGTCAGCCCCCAGGGAAGGCCCTGGAGTGGCTTGCACACATT |
| 330 | GTGGGTGTGGGCTGGATCCGTCAGCCCCCAGGAAAGGCCCTGGAGTGGCTTGCACTCATT |
| 331 | ATGTGTGTGAGCTGGATCCGTCAGCCCCCAGGGAAGGCCCTGGAGTGGCTTGCACTCATT |
| 332 | TACTACATGAGCTGGATCCGCCAGGCTCCAGGGAAGGGGCTGGAGTGGGTTTCATACATT |
| 333 | TACGACATGCACTGGGTCCGCCAAGCTACAGGAAAAGGTCTGGAGTGGGTCTCAGCTATT |
| 334 | GCCTGGATGAGCTGGGTCCGCCAGGCTCCAGGGAAGGGGCTGGAGTGGGTTGGCCGTATT |
| 335 | AGTGACATGAACTGGGCCCGCAAGGCTCCAGGAAAGGGGCTGGAGTGGGTATCGGGTGTT |
| 336 | TATGGCATGAGCTGGGTCCGCCAAGCTCCAGGGAAGGGGCTGGAGTGGGTCTCTGGTATT |
| 337 | TATAGCATGAACTGGGTCCGCCAGGCTCCAGGGAAGGGGCTGGAGTGGGTCTCATCCATT |
| 338 | TATGCCATGAGCTGGGTCCGCCAGGCTCCAGGGAAGGGGCTGGAGTGGGTCTCAGCTATT |
| 339 | TATGGCATGCACTGGGTCCGCCAGGGTCCAGGCAAGGGGCTGGAGTGGGTGGCAGTTATA |
| 340 | TATGGCATGCACTGGGTCCGCCAGGCTCCAGGCAAGGGGCTGGAGTGGGTGGCAGTTATA |
| 341 | AGTGACATGAACTGGGTCCATCAGGCTCCAGGAAAGGGGCTGGAGTGGGTATCGGGTGTT |
| 342 | AATGAGATGAGCTGGATCCGCCAGGCTCCAGGGAAGGGGCTGGAGTGGGTCTCATCCATT |
| 343 | TATACCATGCACTGGGTCCGTCAAGCTCCGGGGAAGGGTCTGGAGTGGGTCTCTCTTATT |
| 344 | TATAGCATGAACTGGGTCCGCCAGGCTCCAGGGAAGGGGCTGGAGTGGGTTTCATACATT |
| 345 | TATGCTATGAGCTGGTTCCGCCAGGCTCCAGGGAAGGGGCTGGAGTGGGTAGGTTTCATT |
| 346 | AACTACATGAGCTGGGTCCGCCAGGCTCCAGGGAAGGGGCTGGAGTGGGTCTCAGTTATT |
| 347 | TATGCTATGCACTGGGTCCGCCAGGCTCCAGGGAAGGGACTGGAATATGTTTCAGCTATT |
| 348 | AACTACATGAGCTGGGTCCGCCAGGCTCCAGGGAAGGGGCTGGAGTGGGTCTCAGTTATT |
| 349 | TATTGGATGAGCTGGGTCCGCCAGGCTCCAGGGAAGGGGCTGGAGTGGGTGGCCAACATA |
| 350 | CACTACATGGACTGGGTCCGCCAGGCTCCAGGGAAGGGGCTGGAGTGGGTTGGCCGTACT |
| 351 | TCTGCTATGCACTGGGTCCGCCAGGCCCGGGAAAGGGCTGGAGTGGGTTGGCCGTATT |
| 352 | TACTGGATGCACTGGGTCCGCCAAGCTCCAGGGAAGGGGCTGGTGTGGGTCTCACGTATT |
| 353 | TATGCCATGCACTGGGTCCGGCAAGCTCCAGGGAAGGGCCTGGAGTGGGTCTCAGGTATT |
| 354 | AACTGGTGGGGCTGGATCCGGCAGCCCCCAGGGAAGGGACTGGAGTGGATTGGGTACATC |
| 355 | TACTACTGGAGCTGGATCCGCCAGCACCCAGGGAAGGGCCTGGAGTGGATTGGGTACATC |
| 356 | TACTACTGGAGCTGGATCCGCCAGCCCCCAGGGAAGGGGCTGGAGTGGATTGGGGAAATC |
| 357 | TACTACTGGGGCTGGATCCGCCAGCCCCCAGGGAAGGGGCTGGAGTGGATTGGGAGTATC |
| 358 | TACTACTGGAGCTGGATCCGGCAGCCCGCCGGGAAGGGACTGGAGTGGATTGGGCGTATC |
| 359 | TACTACTGGAGCTGGATCCGGCAGCCCCCAGGGAAGGGACTGGAGTGGATTGGGTATATC |
| 360 | TACTACTGGAGCTGGATCCGGCAGCCCCCAGGGAAGGGACTGGAGTGGATTGGGTATATC |
| 361 | TACTGGATCGGCTGGGTGCGCCAGATGCCCGGGAAAGGCCTGGAGTGGATGGGGATCATC |
| 362 | GCTGCTTGGAACTGGATCAGGCAGTCCCCATCGAGAGGCCTTGAGTGGCTGGGAAGGACA |
| 363 | TATGGTATGAATTGGGTGCCACAGGCCCCTGGACAAGGGCTTGAGTGGATGGGATGGTTC |

**Table 10. FR3 of Heavy Chains (Chothia definition)**

| |
|---|
| 364 |
| ACAAACTATGCACAGAAGCTCCAGGGCAGAGTCACCATGACCACAGACACATCCACGAGCACAGCCTACAT GGAGCTGAGGAGCCTGAGATCTGACGACACGGCCGTGTATTACTGTGCGAGA |
| 365 |
| ACAAACTATGCACAGAAGTTTCAGGGCAGGGTCACCATGACCAGGGACACGTCCATCAGCACAGCCTACAT GGAGCTGAGCAGGCTGAGATCTGACGACACGGCCGTGTATTACTGTGCGAGA |
| 366 |
| ACAATCTACGCACAGAAGTTCCAGGGCAGAGTCACCATGACCGAGGACACATCTACAGACACAGCCTACAT GGAGCTGAGCAGCCTGAGATCTGAGGACACGGCCGTGTATTACTGTGCAACA |
| 367 |
| ACAAAATATTCACAGGAGTTCCAGGGCAGAGTCACCATTACCAGGGACACATCCGCGAGCACAGCCTACAT GGAGCTGAGCAGCCTGAGATCTGAGGACATGGCTGTGTATTACTGTGCGAGA |
| 368 |
| ACCAACTACGCACAGAAATTCCAGGACAGAGTCACCATTACCAGGGACAGGTCTATGAGCACAGCCTACAT GGAGCTGAGCAGCCTGAGATCTGAGGACACAGCCATGTATTACTGTGCAAGA |
| 369 |
| ACAAGCTACGCACAGAAGTTCCAGGGCAGAGTCACCATGACCAGGGACACGTCCACGAGCACAGTCTACAT GGAGCTGAGCAGCCTGAGATCTGAGGACACGGCCGTGTATTACTGTGCGAGA |
| 370 |
| ACAAACTACGCACAGAAGTTCCAGGAAAGAGTCACCATTACCAGGGACATGTCCACAAGCACAGCCTACAT GGAGCTGAGCAGCCTGAGATCCGAGGACACGGCCGTGTATTACTGTGCGGCA |
| 371 |
| GCAAACTACGCACAGAAGTTCCAGGGCAGAGTCACGATTACCGCGGACAAATCCACGAGCACAGCCTACAT GGAGCTGAGCAGCCTGAGATCTGAGGACACGGCCGTGTATTACTGTGCGAGA |
| 372 |
| ACAGGCTATGCACAGAAGTTCCAGGGCAGAGTCACCATGACCAGGAACACCTCCATAAGCACAGCCTACAT GGAGCTGAGCAGCCTGAGATCTGAGGACACGGCCGTGTATTACTGTGCGAGA |
| 373 |
| AAATCCTACAGCACATCTCTGAAGAGCAGGCTCACCATCTCCAAGGACACCTCCAAAAGCCAGGTGGTCCTT ACCATGACCAACATGGACCCTGTGGACACAGCCACATATTACTGTGCACGG |
| 374 |
| AAGCGCTACAGCCCATCTCTGAAGAGCAGGCTCACCATCACCAAGGACACCTCCAAAAACCAGGTGGTCCTT ACAATGACCAACATGGACCCTGTGGACACAGCCACATATTACTGTGCACAC |
| 375 |
| AAATACTACAGCACATCTCTGAAGACCAGGCTCACCATCTCCAAGGACACCTCCAAAAACCAGGTGGTCCTT ACAATGACCAACATGGACCCTGTGGACACAGCCACGTATTATTGTGCACGG |
| 376 |
| ATATACTACGCAGACTCTGTGAAGGGCCGATTCACCATCTCCAGGGACAACGCCAAGAACTCACTGTATCTG CAAATGAACAGCCTGAGAGCCGAGGACACGGCCGTGTATTACTGTGCGAGA |
| 377 |
| ACATACTATCCAGGCTCCGTGAAGGGCCGATTCACCATCTCCAGAGAAAATGCCAAGAACTCCTTGTATCTT CAAATGAACAGCCTGAGAGCCGGGGACACGGCTGTGTATTACTGTGCAAGA |
| 378 |
| ACAGACTACGCTGCACCCGTGAAAGGCAGATTCACCATCTCAAGAGATGATTCAAAAAACACGCTGTATCTG CAAATGAACAGCCTGAAAACCGAGGACACAGCCGTGTATTACTGTACCACA |
| 379 |
| ACGCACTATGTGGACTCCGTGAAGCGCCGATTCATCATCTCCAGAGACAATTCCAGGAACTCCCTGTATCTG CAAAAGAACAGACGGAGAGCCGAGGACATGGCTGTGTATTACTGTGTGAGA |
| 380 |
| ACAGGTTATGCAGACTCTGTGAAGGGCCGATTCACCATCTCCAGAGACAACGCCAAGAACTCCCTGTATCTG CAAATGAACAGTCTGAGAGCCGAGGACACGGCCTTGTATCACTGTGCGAGA |
| 381 |
| ATATACTACGCAGACTCAGTGAAGGGCCGATTCACCATCTCCAGAGACAACGCCAAGAACTCACTGTATCTG CAAATGAACAGCCTGAGAGCCGAGGACACGGCTGTGTATTACTGTGCGAGA |
| 382 |
| ACATACTACGCAGACTCCGTGAAGGGCCGGTTCACCATCTCCAGAGACAATTCCAAGAACACGCTGTATCTG CAAATGAACAGCCTGAGAGCCGAGGACACGGCCGTATATTACTGTGCGAAA |
| 383 |
| AAATACTATGCAGACTCCGTGAAGGGCCGATTCACCATCTCCAGAGACAATTCCAAGAACACGCTGTATCTG CAAATGAACAGCCTGAGAGCTGAGGACACGGCTGTGTATTACTGTGCGAGA |
| 384 |
| AAATACTATGCAGACTCCGTGAAGGGCCGATTCACCATCTCCAGAGACAATTCCAAGAACACGCTGTATCTG CAAATGAACAGCCTGAGAGCCGAGGACACGGCTGTGTATTACTGTGCGAGA |
| 385 |
| ACGCACTATGCAGACTCTGTGAAGGGCCGATTCATCATCTCCAGAGACAATTCCAGGAACACCCTGTATCTG CAAACGAATAGCCTGAGGGCCGAGGACACGGCTGTGTATTACTGTGTGAGA |
| 386 |
| ACATACTACGCAGACTCCAGGAAGGGCAGATTCACCATCTCCAGAGACAATTCCAAGAACACGCTGTATCTT CAAATGAACAACCTGAGAGCTGAGGGCACGGCCGTGTATTACTGTGCCAGA |
| 387 |
| ACATACTATGCAGACTCTGTGAAGGGCCGATTCACCATCTCCAGAGACAACAGCAAAAACTCCCTGTATCTG CAAATGAACAGTCTGAGAACTGAGGACACCGCCTTGTATTACTGTGCAAAA |
| 388 |
| ATATACTACGCAGACTCTGTGAAGGGCCGATTCACCATCTCCAGAGACAATGCCAAGAACTCACTGTATCTG CAAATGAACAGCCTGAGAGACGAGGACACGGCTGTGTATTACTGTGCGAGA |
| 389 |
| ACAGAATACGCCGCGTCTGTGAAAGGCAGATTCACCATCTCAAGAGATGATTCCAAAAGCATCGCCTATCTG CAAATGAACAGCCTGAAAACCGAGGACACAGCCGTGTATTACTGTACTAGA |
| 390 |
| ACATACTACGCAGACTCCGTGAAGGGCCGATTCACCATCTCCAGAGACAATTCCAAGAACACGCTGTATCTT CAAATGAACAGCCTGAGAGCCGAGGACACGGCCGTGTATTACTGTGCGAGA |
| 391 |
| ACATATTATGCAGACTCTGTGAAGGGCAGATTCACCATCTCCAGAGACAATTCCAAGAACACGCTGTATCTT CAAATGGGCAGCCTGAGAGCTGAGGACATGGCTGTGTATTACTGTGCGAGA |
| 392 |
| ACATACTACGCAGACTCCGTGAAGGGCCGATTCACCATCTCCAGAGACAATTCCAAGAACACGCTGTATCTT CAAATGAACAGCCTGAGAGCTGAGGACACGGCTGTGTATTACTGTGCGAGA |
| 393 |
| AAATACTATGTGGACTCTGTGAAGGGCCGATTCACCATCTCCAGAGACAACGCCAAGAACTCACTGTATCTG CAAATGAACAGCCTGAGAGCCGAGGACACGGCTGTGTATTACTGTGCGAGA |
| 394 |
| ACAGAATACGCCGCGTCTGTGAAAGGCAGATTCACCATCTCAAGAGATGATTCAAAGAACTCACTGTATCTG CAAATGAACAGCCTGAAAACCGAGGACACGGCCGTGTATTACTGTGCTAGA |
| 395 |
| ACAGCATATGCTGCGTCGGTGAAAGGCAGGTTCACCATCTCCAGAGATGATTCAAAGAACACGGCGTATCTG CAAATGAACAGCCTGAAAACCGAGGACACGGCCGTGTATTACTGTACTAGA |
| 396 |
| ACAAGCTACGCGGACTCCGTGAAGGGCCGATTCACCATCTCCAGAGACAACGCCAAGAACACGCTGTATCT GCAAATGAACAGTCTGAGAGCCGAGGACACGGCTGTGTATTACTGTGCAAGA |
| 397 |
| ATAGGCTATGCGGACTCTGTGAAGGGCCGATTCACCATCTCCAGAGACAACGCCAAGAACTCCCTGTATCTG CAAATGAACAGTCTGAGAGCTGAGGACACGGCCTTGTATTACTGTGCAAAA |
| 398 |
| ACCTACTACAACCCGTCCCTCAAGAGTCGAGTCACCATGTCAGTAGACACGTCCAAGAACCAGTTCTCCCTG AAGCTGAGCTCTGTGACCGCCGTGGACACGGCCGTGTATTACTGTGCGAGA |
| 399 |
| ACCTACTACAACCCGTCCCTCAAGAGTCGAGTTACCATATCAGTAGACACGTCTAAGAACCAGTTCTCCCTG AAGCTGAGCTCTGTGACTGCCGCGGACACGGCCGTGTATTACTGTGCGAGA |
| 400 |
| ACCAACTACAACCCGTCCCTCAAGAGTCGAGTCACCATATCAGTAGACACGTCCAAGAACCAGTTCTCCCTG AAGCTGAGCTCTGTGACCGCCGCGGACACGGCTGTGTATTACTGTGCGAGA |
| 401 |
| ACCTACTACAACCCGTCCCTCAAGAGTCGAGTCACCATATCCGTAGACACGTCCAAGAACCAGTTCTCCCTG AAGCTGAGCTCTGTGACCGCCGCAGACACGGCTGTGTATTACTGTGCGAGA |
| 402 |
| ACCAACTACAACCCCTCCCTCAAGAGTCGAGTCACCATGTCAGTAGACACGTCCAAGAACCAGTTCTCCCTG AAGCTGAGCTCTGTGACCGCCGCGGACACGGCCGTGTATTACTGTGCGAGA |
| 403 |
| ACCAACTACAACCCCTCCCTCAAGAGTCGAGTCACCATATCAGTAGACACGTCCAAGAACCAGTTCTCCCTG AAGCTGAGCTCTGTGACCGCTGCGGACACGGCCGTGTATTACTGTGCGAGA |
| 404 |
| ACCAACTACAACCCCTCCCTCAAGAGTCGAGTCACCATATCAGTAGACACGTCCAAGAACCAGTTCTCCCTG AAGCTGAGCTCTGTGACCGCTGCGGACACGGCCGTGTATTACTGTGCGAGA |
| 405 |
| ACCAGATACAGCCCGTCCTTCCAAGGCCAGGTCACCATCTCAGCCGACAAGTCCATCAGCACCGCCTACCTG CAGTGGAGCAGCCTGAAGGCCTCGGACACCGCCATGTATTACTGTGCGAGA |
| 406 |
| AATGATTATGCAGTATCTGTGAAAAGTCGAATAACCATCAACCCAGACACATCCAAGAACCAGTTCTCCCTG CAGCTGAACTCTGTGACTCCCGAGGACACGGCTGTGTATTACTGTGCAAGA |
| 407 |
| CCAACATATGCCCAGGGCTTCACAGGACGGTTTGTCTTCTCCATGGACACCTCTGCCAGCACAGCATACCTG CAGATCAGCAGCCTAAAGGCTGAGGACATGGCCATGTATTACTGTGCGAGA |

**Table 11. FR4 of Heavy Chain**

| | |
|---|---|
| 408 | TGGGGCCAGGGCACCCTGGTCACCGTCTCCTCA |
| 409 | TGGGGCCGTGGCACCCTGGTCACTGTCTCCTCA |
| 410 | TGGGGCCAAGGGACAATGGTCACCGTCTCTTCA |
| 411 | TGGGGCCAAGGAACCCTGGTCACCGTCTCCTCA |
| 412 | TGGGGCCAAGGAACCCTGGTCACCGTCTCCTCA |
| 413 | TGGGGGCAAGGGACCACGGTCACCGTCTCCTCA |

As used herein, the term "germline antibody gene" or "gene fragment" refers to an immunoglobulin sequence encoded by non-lymphoid cells that have not undergone the maturation process that leads to genetic rearrangement and mutation for expression of a particular immunoglobulin. (*See, e.g.,* Shapiro et al., Crit. Rev. Immunol. 22(3):183-200 (2002); Marchalonis et al., Adv Exp Med Biol. 484:13-30 (2001)). One of the advantages provided by various embodiments of the present invention stems from the recognition that germline antibody genes are more likely than mature antibody genes to conserve essential amino acid sequence structures characteristic of individuals in the species, hence less likely to be recognized as from a foreign source when used therapeutically in that species.

As used herein, the term "humanized antibody" is an antibody or a variant, derivative, analog or fragment thereof which immunospecifically binds to an antigen of interest and which comprises a framework (FR) region having substantially the amino acid sequence of a human antibody and a complementarity determining region (CDR) having substantially the amino acid sequence of a non-human antibody. As used herein, the term "substantially" in the context of a CDR refers to a CDR having an amino acid sequence at least 80%, preferably at least 85%, at least 90%, at least 95%, at least 98% or at least 99% identical to the amino acid sequence of a non-human antibody CDR. A humanized antibody comprises substantially all of at least one, and typically two, variable domains (Fab, Fab', F(ab')₂, FabC, Fv) in which all or substantially all of the CDR regions correspond to those of a non-human immunoglobulin (*i.e*., donor antibody) and all or substantially all of the framework regions are those of a human immunoglobulin sequence. Preferably, a humanized antibody also comprises at least a portion of an immunoglobulin constant region (Fc), typically that of a human immunoglobulin. In some embodiments, a humanized antibody contains both the light chain as well as at least the variable domain of a heavy chain. The antibody also may include the CH1, hinge, CH2, CH3, and CH4 regions of the heavy chain. In some embodiments, a humanized antibody only contains a humanized light chain. In some embodiments, a humanized antibody only contains a humanized heavy chain. In specific embodiments, a humanized antibody only contains a humanized variable domain of a light chain and/or humanized heavy chain.

The humanized antibody can be selected from any class of immunoglobulins, including IgM, IgG, IgD, IgA and IgE, and any isotype, including without limitation IgG₁, IgG₂, IgG₃ and IgG₄. The humanized antibody may comprise sequences from more than one class or isotype, and particular constant domains may be selected to optimize desired effector functions using techniques well-known in the art.

The framework and CDR regions of a humanized antibody need not correspond precisely to the parental sequences, *e.g*., the donor antibody CDR or the acceptor framework may be mutagenized by substitution, insertion and/or deletion of at least one amino acid residue so that the CDR or framework residue at that site does not correspond to either the donor antibody or the acceptor framework. Such mutations, however, will not be extensive. Usually, at least 80%, preferably at least 85%, more preferably at least 90%, and most preferably at least 95% of the humanized antibody residues will correspond to those of the parental FR and CDR sequences.

As used herein, the term "host cell" includes a to the particular subject cell transfected or transformed with a nucleic acid molecule and the progeny or potential progeny of such a cell. Progeny of such a cell may not be identical to the parent cell transfected with the nucleic acid molecule due to mutations or environmental influences that may occur in succeeding generations or integration of the nucleic acid molecule into the host cell genome.

As used herein, the term "immunospecifically binds to an antigen" and analogous terms refer to peptides, polypeptides, proteins (including, but not limited to fusion proteins and antibodies) or fragments thereof that specifically bind to an antigen or a fragment and do not specifically bind to other antigens. A peptide, polypeptide, or protein that immunospecifically binds to an antigen may bind to other antigens with lower affinity as determined by, *e.g.,* immunoassays, BIAcore, or other assays known in the art. Antibodies or fragments that immunospecifically bind to an antigen may be cross-reactive with related antigens. Preferably, antibodies or fragments that immunospecifically bind to an antigen do not cross-react with other antigens.

As used herein, the term "isolated" in the context of a proteinaceous agent (*e.g.,* a peptide, polypeptide or protein (such as fusion protein or antibody)) refers to a proteinaceous agent which is substantially free of cellular material or contaminating proteins, polypeptides, peptides and antibodies from the cell or tissue source from which it is derived, or substantially free of chemical precursors or other chemicals when chemically synthesized. The language "substantially free of cellular material" includes preparations of a proteinaceous agent in which the proteinaceous agent is separated from cellular components of the cells from which it is isolated or recombinantly produced. Thus, a proteinaceous agent that is substantially free of cellular material includes preparations of a proteinaceous agent having less than about 30%, 20%, 10%, or 5% (by dry weight) of heterologous protein, polypeptide or peptide (also referred to as a "contaminating protein"). When the proteinaceous agent is recombinantly produced, it is also preferably substantially free of culture medium, *i.e*., culture medium represents less than about 20%, 10%, or 5% of the volume of the proteinaceous agent preparation. When the proteinaceous agent is produced by chemical synthesis, it is preferably substantially free of chemical precursors or other chemicals, *i.e*., it is separated from chemical precursors or other chemicals which are involved in the synthesis of the proteinaceous agent. Accordingly, such preparations of a proteinaceous agent have less than about 30%, 20%, 10%, 5% (by dry weight) of chemical precursors or compounds other than the proteinaceous agent of interest. In a specific embodiment, proteinaceous agents disclosed herein are isolated. In a preferred embodiment, an antibody of the invention is isolated.

As used herein, the term "isolated" in the context of nucleic acid molecules refers to a nucleic acid molecule which is separated from other nucleic acid molecules which are present in the natural source of the nucleic acid molecule. Moreover, an "isolated" nucleic acid molecule, such as a cDNA molecule, is preferably substantially free of other cellular material, or culture medium when produced by recombinant techniques, or substantially free of chemical precursors or other chemicals when chemically synthesized. In a specific embodiment, nucleic acid molecules are isolated. In a preferred embodiment, a nucleic acid molecule encoding an antibody of the invention is isolated. As used herein, the term "substantially free" refers to the preparation of the "isolated" nucleic acid having less than about 30%, 20%, 10%, or 5% (by dry weight) of heterologous nucleic acids, and preferably other cellular material, culture medium, chemical precursors, or other chemicals.

As used herein, the term "in combination" refers to the use of more than one therapies (*e.g.,* more than one prophylactic agent and/or therapeutic agent). The use of the term "in combination" does not restrict the order in which therapies (*e.g.,* prophylactic and/or therapeutic agents) are administered to a subject. A first therapy (*e.g.,* a first prophylactic or therapeutic agent) can be administered prior to (*e.g.,* 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks before), concomitantly with, or subsequent to (*e.g.,* 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks after) the administration of a second therapy (*e.g.,* a second prophylactic or therapeutic agent) to a subject.

As used herein, the terms "manage," "managing," and "management" refer to the beneficial effects that a subject derives from a therapy (*e.g.,* a prophylactic or therapeutic agent), which does not result in a cure of the disease. In certain embodiments, a subject is administered one or more therapies (*e.g.,* one or more prophylactic or therapeutic agents) to "manage" a disease so as to prevent the progression or worsening of the disease.

As used herein, the term "mature antibody gene" refers to a genetic sequence encoding an immunoglobulin that is expressed, for example, in a lymphocyte such as a B cell, in a hybridoma or in any antibody producing cell that has undergone a maturation process so that the particular immunoglobulin is expressed. The term includes mature genomic DNA, cDNA and other nucleic acid sequences that encode such mature genes, which have been isolated and/or recombinantly engineered for expression in other cell types. Mature antibody genes have undergone various mutations and rearrangements that structurally distinguish them from antibody genes encoded in all cells other than lymphocytes. Mature antibody genes in humans, rodents, and many other mammals are formed by fusion of V and J gene segments in the case of antibody light chains and fusion of V, D, and J gene segments in the case of antibody heavy chains. Many mature antibody genes acquire point mutations subsequent to fusion, some of which increase the affinity of the antibody protein for a specific antigen.

As used herein, the term "pharmaceutically acceptable" refers approved by a regulatory agency of the federal or a state government, or listed in the U.S. Pharmacopeia, European Pharmacopeia, or other generally recognized pharmacopeia for use in animals, and more particularly, in humans.

As used herein, the terms "prevent," "preventing," and "prevention" refer to the inhibition of the development or onset of a disorder or the prevention of the recurrence, onset, or development of one or more symptoms of a disorder in a subject resulting from the administration of a therapy (*e.g.,* a prophylactic or therapeutic agent), or the administration of a combination of therapies (*e.g.,* a combination of prophylactic or therapeutic agents).

As used herein, the terms "prophylactic agent" and "prophylactic agents" refer to any agent(s) which can be used in the prevention of a disorder or one or more of the symptoms thereof. In certain embodiments, the term "prophylactic agent" refers to an antibody of the invention. In certain other embodiments, the term "prophylactic agent" refers to an agent other than an antibody of the invention. Preferably, a prophylactic agent is an agent which is known to be useful to or has been or is currently being used to prevent or impede the onset, development, progression and/or severity of a disorder or one or more symptoms thereof.

As used herein, the term "prophylactically effective amount" refers to the amount of a therapy (*e.g.,* prophylactic agent) which is sufficient to result in the prevention of the development, recurrence, or onset of a disorder or one or more symptoms thereof, or to enhance or improve the prophylactic effect(s) of another therapy (*e.g.,* a prophylactic agent).

As used herein, the phrase "protocol" refers to a regimen for dosing and timing the administration of one or more therapies (*e.g.,* therapeutic agents) that has a therapeutic effective.

As used herein, the phrase "side effects" encompasses unwanted and adverse effects of a prophylactic or therapeutic agent. Side effects are always unwanted, but unwanted effects are not necessarily adverse. An adverse effect from a therapy (*e.g.,* a prophylactic or therapeutic agent) might be harmful, uncomfortable, or risky.

As used herein, the term "small molecules" and analogous terms include, but are not limited to, peptides, peptidomimetics, amino acids, amino acid analogs, polynucleotides, polynucleotide analogs, nucleotides, nucleotide analogs, organic or inorganic compounds (*i.e*., including heteroorganic and organometallic compounds) having a molecular weight less than about 10,000 grams per mole, organic or inorganic compounds having a molecular weight less than about 5,000 grams per mole, organic or inorganic compounds having a molecular weight less than about 1,000 grams per mole, organic or inorganic compounds having a molecular weight less than about 500 grams per mole, and salts, esters, and other pharmaceutically acceptable forms of such agents.

As used herein, the terms "subject" and "patient" are used interchangeably. As used herein, the terms "subject" and "subjects" refer to an animal, preferably a mammal including a non-primate (*e.g.,* a cow, pig, horse, cat, dog, rat, and mouse) and a primate *(e.g.,* a monkey, such as a cynomolgous monkey, a chimpanzee, and a human), and most preferably a human. In one embodiment, the subject is a non-human animal such as a bird *(e.g.,* a quail, chicken, or turkey), a farm animal (*e.g*., a cow, horse, pig, or sheep), a pet *(e.g.,* a cat, dog, or guinea pig), or laboratory animal (*e.g.,* an animal model for a disorder). In a preferred embodiment, the subject is a human (*e.g.,* an infant, child, adult, or senior citizen).

As used herein, the term "synergistic" refers to a combination of therapies (*e.g.,* prophylactic or therapeutic agents) which is more effective than the additive effects of any two or more single therapies (*e.g.,* one or more prophylactic or therapeutic agents). A synergistic effect of a combination of therapies (*e.g.,* a combination of prophylactic or therapeutic agents) permits the use of lower dosages of one or more of therapies (*e.g*., one or more prophylactic or therapeutic agents) and/or less frequent administration of said therapies to a subject with a disorder. The ability to utilize lower dosages of therapies (*e.g.,* prophylactic or therapeutic agents) and/or to administer said therapies less frequently reduces the toxicity associated with the administration of said therapies to a subject without reducing the efficacy of said therapies in the prevention or treatment of a disorder. In addition, a synergistic effect can result in improved efficacy of therapies (*e.g.,* prophylactic or therapeutic agents) in the prevention or treatment of a disorder. Finally, synergistic effect of a combination of therapies (*e.g.,* prophylactic or therapeutic agents) may avoid or reduce adverse or unwanted side effects associated with the use of any single therapy.

As used herein, the terms "therapeutic agent" and "therapeutic agents" refer to any agent(s) which can be used in the prevention, treatment, management, or amelioration of a disorder or one or more symptoms thereof. In certain embodiments, the term "therapeutic agent" refers to an antibody of the invention. In certain other embodiments, the term "therapeutic agent" refers an agent other than an antibody of the invention. Preferably, a therapeutic agent is an agent which is known to be useful for, or has been or is currently being used for the prevention, treatment, management, or amelioration of a disorder or one or more symptoms thereof.

As used herein, the term "therapeutically effective amount" refers to the amount of a therapy (*e.g.,* an antibody of the invention), which is sufficient to reduce the severity of a disorder, reduce the duration of a disorder, ameliorate one or more symptoms of a disorder, prevent the advancement of a disorder, cause regression of a disorder, or enhance or improve the therapeutic effect(s) of another therapy.

As used herein, the terms "therapies" and "therapy" can refer to any protocol(s), method(s), and/or agent(s) that can be used in the prevention, treatment, management, and/or amelioration of a disorder or one or more symptoms thereof. In certain embodiments, the terms "therapy" and "therapy" refer to anti-viral therapy, anti-bacterial therapy, anti-fungal therapy, anti-cancer agent, biological therapy, supportive therapy, and/or other therapies useful in treatment, management, prevention, or amelioration of a disorder or one or more symptoms thereof known to one skilled in the art, for example, a medical professional such as a physician.

As used herein, the terms "treat," "treatment," and "treating" refer to the reduction or amelioration of the progression, severity, and/or duration of a disorder or amelioration of one or more symptoms thereof resulting from the administration of one or more therapies (including, but not limited to, the administration of one or more prophylactic or therapeutic agents).

### 3.2 BRIEF DESCRIPTION OF THE FIGURES

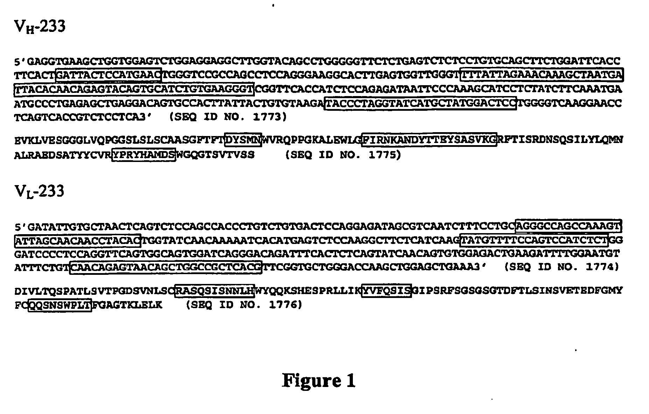

Figure 1. Nucleic acid and protein sequences of the heavy and light chains of the mouse anti-human EphA2 monoclonal antibody B233. CDR1, 2 and 3 regions as defined by Kabat are boxed. The full amino acid sequences of the variable heavy (V_{H}) and light (V_{L}) chains are given using the standard one letter code.

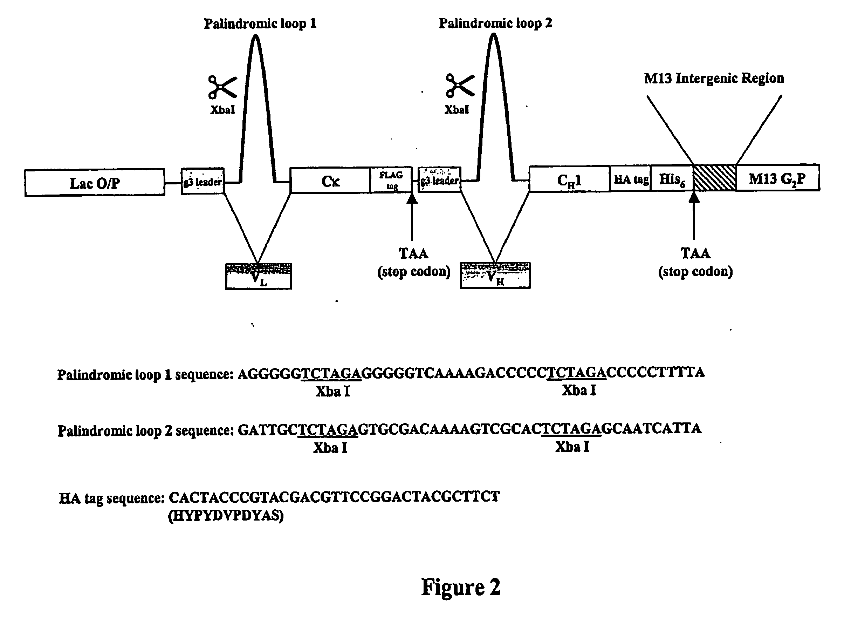

Figure 2. Phage vector used for screening of the framework shuffling libraries and expression of the corresponding Fab fragments. Streptavidin purified, single-stranded DNA of each of the V_{L} and V_{H} genes is annealed to the vector by hybridization mutagenesis using homology in the gene 3 leader/Cκ and gene 3 leader/Cκ1 regions, respectively. The unique Xba1 site in the palindromic loops allows elimination of the parental vector. V_{H} and V_{L} genes are then expressed in frame with the first constant domain of the human κ1 heavy chain and the constant domain of the human kappa (κ) light chain, respectively.

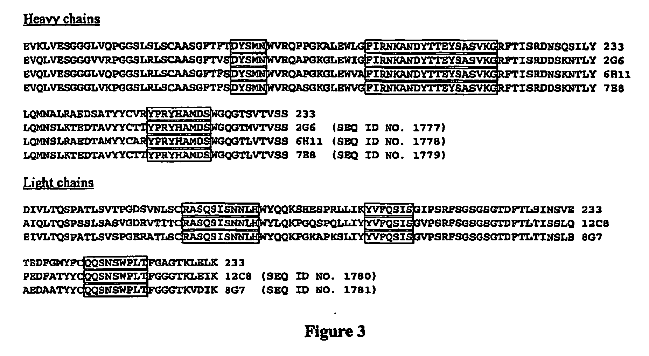

Figure 3. Protein sequences of framework-shuffled, humanized clones of the anti-human EphA2 monoclonal antibody B233 isolated after screening of libraries A and B. CDR1, 2 and 3 regions as defined by Kabat are boxed. The full amino acid sequences of the variable heavy (V_{H}) and light (V_{L}) chains are given using the standard one letter code.

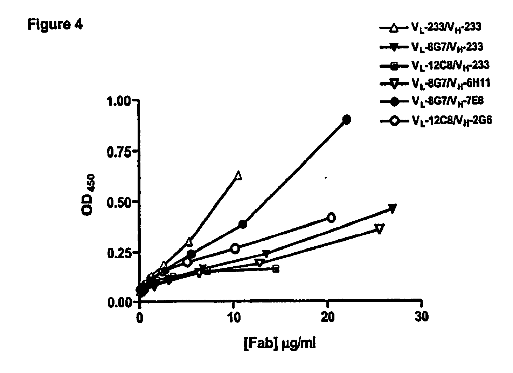

Figure 4. ELISA titration using Fab extracts on immobilized human EphA2-Fc.

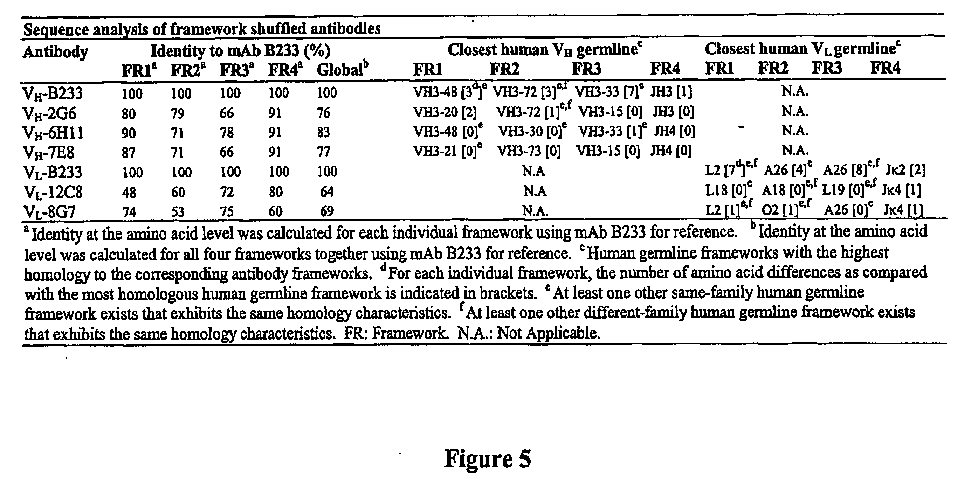

Figure 5. Sequence analysis of framework shuffled antibodies. ^{a}Percent identity at the amino acid level was calculated for each individual antibody framework using mAb B233 for reference

### DETAILED DESCRIPTION OF THE INVENTION

The present invention relates to methods of re-engineering or re-shaping an antibody from a first species, wherein the re-engineered or re-shaped antibody does not elicit undesired immune response in a second species, and the re-engineered or re-shaped antibody retains substantially the same antigen binding-ability of the antibody from the first species. A combinatorial library comprising the CDRs of the antibody from the first species fused in frame with framework regions from a bank of framework regions derived from a second species can be constructed and screened for the desired modified antibody.

Cells may comprise, contain or be engineered to express the nucleic acid sequences described herein. A method of producing a humanized heavy chain variable region as described herein may comprise expressing the nucleotide sequence encoding a humanized heavy chain variable region in a cell described herein. A method of producing a humanized light chain variable region may comprise expressing the nucleotide sequence encoding a humanized light chain variable region in a cell described herein. A method of producing a humanized antibody that immunospecifically binds to an antigen may comprise expressing the nucleic acid sequence(s) encoding the humanized antibody contained in the cell described herein.

Humanized antibodies can be produced by the methods described herein. A composition may comprise a humanized antibody produced by the methods described herein and a carrier, diluent or excipient, such compositions can be pharmaceutical compositions in a form suitable for their intended use.

For clarity of disclosure, and not by way of limitation, the detailed description of the invention section is divided into the following sub-sections:
(i) construction of a global bank of acceptor framework regions
(ii) selection of CDRs
(iii) construction of combinatorial sub-libraries
(iv) construction of combinatorial libraries
(v) expression of the combinatorial libraries
(vi) selection of humanized antibodies
(vii) production and characterization of humanized antibodies
(viii) antibody conjugates
(ix) uses of the compositions
(x) administration and formulations
(xi) dosage and frequency of administration
(xii) biological assays
(xiii) kits
(xiv) article of manufacture

### 2.2 Construction of a Global Bank of Acceptor Framework Regions

According to the present invention, a variable light chain region and/or variable heavy chain region of a donor non-human antibody can be humanized) by fusing together nucleic acid sequences encoding framework regions (FR1, FR2, FR3, FR4 of the light chain, and FR1, FR2, FR3, FR4 of the heavy chain) of an acceptor human antibody and nucleic acid sequences encoding complementarity-determining regions (CDR1, CDR2, CDR3 of the light chain, and CDR1, CDR2, CDR3 of the heavy chain) of the donor antibody. Preferably, the humanized antibody light chain comprises FR1, CDR1, FR2, CDR2, FR3, CDR3, and FR4. A humanized antibody heavy chain comprises FR1, CDR1, FR2, CDR2, FR3, CDR3, and FR4. Each acceptor human framework region (FR1, 2, 3, 4 of light chain, and FR1, 2, 3, 4 of heavy chain) can be generated from FR sub-banks for the light chain and FR sub-banks for the heavy chain, respectively. A global bank of acceptor human framework regions comprises two or more FR sub-banks.

### 2.2.1 Generation of Sub-banks for the Light Chain Frameworks

Light chain sub-banks 1, 2, 3 and 4 are constructed, wherein sub-bank 1 comprises plurality of nucleic acid sequences comprising nucleotide sequences, each nucleotide sequence encoding a light chain FR1; sub-bank 2 comprises a plurality of nucleic acid sequences comprising nucleotide sequences, each nucleotide sequence encoding a light chain FR2; sub-bank 3 comprises a plurality of nucleic acid sequences comprising nucleotide sequences, each nucleotide sequence encoding a light chain FR3; and sub-bank 4 comprises a plurality of nucleic acid sequences comprising nucleotide sequences, each nucleotide sequence encoding a light chain FR4. In some embodiments, the FR sequences are obtained or derived from functional human antibody sequences (*e.g.,* an antibody known in the art and/or commercially available). In some embodiments, the FR sequences are derived from human germline light chain sequences. In one embodiment, the sub-bank FR sequences are derived from a human germline kappa chain sequences. In another embodiment, the sub-bank FR sequences are derived from a human germline lambda chain sequences.

By way of example but not limitation, the following describes a method of generating 4 light chain FR sub-banks using Polymerase Chain Reaction (PCR), wherein human germline kappa chain sequences are used as templates. Light chain FR sub-banks 1, 2 and 3 (encoding FR1, 2, 3 respectively) encompass 46 human germline kappa chain sequences (A1, A10, A11, A14, A 17, A18, A19, A2, A20, A23, A26, A27, A3, A30, A5, A7, B2, B3, L1, L10, L11, L12, L14, L15, L16, L18, L 19, L2, L20, L22, L23, L24, L25, L4/18a, L5, L6, L8, L9, O1, O11, 012, 014, 018, 02, 04 and 08). See Kawasaki et al., 2001, Eur. J. Immunol., 31:1017-1028; Schable and Zachau, 1993, Biol. Chem. Hoppe Seyler 374:1001-1022; and Brensing-Kuppers et al., 1997, Gene 191:173-181. The sequences are summarized at the NCBI website: www.ncbi.nlm.nih.gov/igblast/showGermline.cgi?organism=human&chainType=VK&seqT ype=nucleotide. Light chain FR sub-bank 4 (encoding FR4) encompasses 5 human germline kappa chain sequences (Jκ1, Jκ2, Jκ3, Jκ4 and Jκ5). See Hieter et al., 1982, J. Biol. Chem. 257:1516-1522. The sequences are summarized at the NCBI website: www.ncbi.nlm.nih.gov/igblast/showGermline.cgi?organism=human&chainType=JK&seqT ype=nucleotide.

By way of example but not limitation, the construction of light chain FR1 sub-bank is carried out using the Polymerase Chain Reaction by overlap extension using the oligonucleotides listed in Table 12 and Table 13 (all shown in the 5' to 3' orientation, name followed by sequence):

**Table 12. Light Chain FR1 Forward Primers (for Sub-Bank 1)**

| | | |
|---|---|---|
| 414 | FR1L1 | GATGTTGTGATGACTCAGTCTCCACTCTCCCTGCCCGTCACCC |
| 415 | FR1L2 | GATGTTGTGATGACTCAGTCTCCACTCTCCCTGCCCGTCACCC |
| 416 | FR1L3 | GATATTGTGATGACCCAGACTCCACTCTCTCTGTCCGTCACCC |
| 417 | FR1L4 | GATATTGTGATGACTCAGTCTCCACTCTCCCTGCCCGTCACCC |
| 418 | FR1L5 | GATATTGTGATGACCCAGACTCCACTCTCTCTGTCCGTCACCC |
| 419 | FR1L6 | GATATTGTGATGACCCAGACTCCACTCTCCTCACCTGTCACCC |
| 420 | FR1L7 | GATATTGTGATGACTCAGTCTCCACTCTCCCTGCCCGTCACCC |
| 421 | FR1L8 | GAGATTGTGATGACCCAGACTCCACTCTCCTTGTCTATCACCC |
| 422 | FR1L9 | GATATTGTGATGACCCAGACTCCACTCTCCTCGCCTGTCACCC |
| 423 | FR1L10 | GAAATTGTGCTGACTCAGTCTCCAGACTTTCAGTCTGTGACTC |
| 424 | FR1L11 | GATGTTGTGATGACACAGTCTCCAGCTTTCCTCTCTGTGACTC |
| 425 | FR1L12 | GACATCCAGATGACCCAGTCTCCATCCTCCCTGTCTGCATCTG |
| 426 | FR1L13 | GAAATTGTGCTGACTCAGTCTCCAGACTTTCAGTCTGTGACTC |
| 427 | FR1L14 | GACATCCAGATGACCCAGTCTCCATCCTCCCTGTCTGCATCTG |
| 428 | FR1L15 | GAAACGACACTCACGCAGTCTCCAGCATTCATGTCAGCGACTC |
| 429 | FR1L16 | GACATCCAGATGACCCAGTCTCCATCCTCACTGTCTGCATCTG |
| 430 | FR1L17 | GCCATCCAGATGACCCAGTCTCCATCCTCCCTGTCTGCATCTG |
| 431 | FR1L18 | GACATCCAGATGACCCAGTCTCCTTCCACCCTGTCTGCATCTG |
| 432 | FR1L19 | AACATCCAGATGACCCAGTCTCCATCTGCCATGTCTGCATCTG |
| 433 | FR1L20 | GACATCCAGATGACCCAGTCTCCATCCTCACTGTCTGCATCTG |
| 434 | FR1L21 | GAAATAGTGATGATGCAGTCTCCAGCCACCCTGTCTGTGTCTC |
| 435 | FR1L22 | GCCATCCAGTTGACCCAGTCTCCATCCTCCCTGTCTGCATCTG |
| 436 | FR1L23 | GACATCCAGATGACCCAGTCTCCATCTTCTGTGTCTGCATCTG |
| 437 | FR1L24 | GAAATAGTGATGACGCAGTCTCCAGCCACCCTGTCTGTGTCTC |
| 438 | FR1L25 | GAAATTGTGTTGACACAGTCTCCAGCCACCCTGTCTTTGTCTC |
| 439 | FR1L36 | GACATCCAGATGATCCAGTCTCCATCTTTCCTGTCTGCATCTG |
| 440 | FR1L27 | GCCATCCGGATGACCCAGTCTCCATTCTCCCTGTCTGCATCTG |
| 441 | FR1L28 | GTCATCTGGATGACCCAGTCTCCATCCTTACTCTCTGCATCTA |
| 442 | FR1L29 | GCCATCCAGTTGACCCAGTCTCCATCCTCCCTGTCTGCATCTG |
| 443 | FR1L30 | GACATCCAGATGACCCAGTCTCCATCTTCCGTGTCTGCATCTG |
| 444 | FR1L31 | GAAATTGTGTTGACACAGTCTCCAGCCACCCTGTCTTTGTCTC |
| 445 | FR1L32 | GACATCCAGTTGACCCAGTCTCCATCCTTCCTGTCTGCATCTG |
| 446 | FR1L33 | GCCATCCGGATGACCCAGTCTCCATCCTCATTCTCTGCATCTA |
| 447 | FR1L34 | GACATCCAGATGACCCAGTCTCCATCCTCCCTGTCTGCATCTG |
| 448 | FR1L35 | GACATCCAGTTGACCCAGTCTCCATCCTCCCTGTCTGCATCTG |
| 449 | FR1L36 | GACATCCAGATGACCCAGTCTCCATCCTCCCTGTCTGCATCTG |
| 450 | FR1L37 | GACATCCAGATGACCCAGTCTCCATCCTCCCTGTCTGCATCTG |
| 451 | FR1L38 | GACATCCAGTTGACCCAGTCTCCATCCTCCCTGTCTGCATCTG |
| 452 | FR1L39 | GACATCCAGATGACCCAGTCTCCATCCTCCCTGTCTGCATCTG |
| 453 | FR1L40 | GAAATTGTAATGACACAGTCTCCACCCACCCTGTCTTTGTCTC |
| 454 | FR1L41 | GAAATTGTAATGACACAGTGTCCAGCCACCCTGTCTTTGTCTC |
| 455 | FR1L42 | GAAATTGTGTTGACGCAGTCTCCAGCCACCCTGTCTTTGTCTC |
| 456 | FR1L43 | GAAATTGTGTTGACGCAGTCTCCAGGCACCCTGTCTTTGTCTC |
| 457 | FR1L44 | GACATCGTGATGACCCAGTCTCCAGACTCCCTGGCTGTGTCTC |
| 458 | FR1L45 | GATATTGTGATGACCCAGACTCCACTCTCCCTGCCCGTCACCC |
| 459 | 4FR1L46 | GATATTGTGATGACCCAGACTCCACTCTCCCTGCCCGTCACCC |

**Table 13. Light Chain FR1 Reverse Primers (for Sub-Bank 1)**

| | | |
|---|---|---|
| 460 | FR1L1' | GCAGGAGATGGAGGCCGGCTGTCCAAGGGTGACGGGCAGGGAGAGTG |
| 461 | FR1L2' | GCAGGAGATGGAGGCCGGCTGTCCAAGGGTGACGGGCAGGGAGAGTG |
| 462 | FR1L3' | GCAGGAGATGGAGGCCGGCTGTCCAGGGGTGACGGACAGAGAGAGTG |
| 463 | FR1L4' | GCAGGAGATGGAGGCCGGCTCTCCAGGGGTGACGGGCAGGGAGAGTG |
| 464 | FR1L5' | GCAGGAGATGGAGGCCGGCTGTCCAGGGGTGACGGACAGAGAGAGTG |
| 465 | FR1L6' | GCAGGAGATGGAGGCCGGCTGTCCAAGGGTGACAGGTGAGGAGAGTG |
| 466 | FR1L7' | GCAGGAGATGGAGGCCGGCTCTCCAGGGGTGACGGGCAGGGAGAGTG |
| 467 | FR1L8' | GCAGGAGATGGAGGCCTGCTCTCCAGGGGTGATAGACAAGGAGAGTG |
| 468 | FR1L9' | GAAGGAGATGGAGGCCGGCTGTCCAAGGGTGACAGGCGAGGAGAGTG |
| 469 | FR1L10' | GCAGGTGATGGTGACTTTCTCCTTTGGAGTCACAGACTGAAAGTCTG |
| 470 | FR1L11' | GCAGGTGATGGTGACTTTCTCCCCTGGAGTCACAGAGAGGAAAGCTG |
| 471 | FR1L12' | GCAAGTGATGGTGACTCTGTCTCCTACAGATGCAGACAGGGAGGATG |
| 472 | FR1L13' | GCAGGTGATGGTGACTTTCTCCTTTGGAGTCACAGACTGAAAGTCTG |
| 473 | FR1L14' | GCAAGTGATGGTGACTCTGTCTCCTACAGATGCAGACAGGGAGGATG |
| 474 | FR1L15' | GCAGGAGATGTTGACTTTGTCTCCTGGAGTCGCTGACATGAATGCTG |
| 475 | FR1L16' | ACAAGTGATGGTGACTCTGTCTCCTACAGATGCAGACAGTGAGGATG |
| 476 | FR1L17' | GCAAGTGATGGTGACTCTGTCTCCTACAGATGCAGACAGGGAGGATG |
| 477 | FR1L18' | GCAAGTGATGGTGACTCTGTCTCCTACAGATGCAGACAGGGTGGAAG |
| 478 | FR1L19' | ACAAGTGATGGTGACTCTGTGTCCTACAGATGCAGACATGGCAGATG |
| 479 | FR1L20' | ACAAGTGATGGTGACTCTGTCTCCTACAGATGCAGACAGTGAGGATG |
| 480 | FR1L21' | GCAGGAGAGGGTGGCTCTTTCCCCTGGAGACACAGACAGGGTGGCTG |
| 481 | FR1L22' | GCAAGTGATGGTGACTCTGTCTCCTACAGATGCAGACAGGGAGGATG |
| 482 | FR1L23' | ACAAGTGATGGTGACTCTGTCTCCTACAGATGCAGACACAGAAGATG |
| 483 | FR1L24' | GCAGGAGAGGGTGGCTCTTTCCCCTGGAGACACAGACAGGGTGGCTG |
| 484 | FR1L25' | GCAGGAGAGGGTGGCTCTTTCCCCTGGAGACAAAGACAGGGTGGCTG |
| 485 | FR1L36' | GCAAATGATACTGACTCTGTCTCCTACAGATGCAGACAGGAAAGATG |
| 486 | FR1L27' | GCAAGTGATGGTGACTCTGTCTCCTACAGATGCAGACAGGGAGAATG |
| 487 | FR1L28' | ACAACTGATGGTGACTCTGTCTCCTGTAGATGCAGAGAGTAAGGATG |
| 488 | FR1L29' | GCAAGTGATGGTGACTCTGTCTCCTACAGATGCAGACAGGGAGGATG |
| 489 | FR1L30' | ACAAGTGATGGTGACTCTGTCTCCTACAGATGCAGACACGGAAGATG |
| 490 | FR1L31' | GCAGGAGAGGGTGGCTCTTTCCCCTGGAGACAAAGACAGGGTGGCTG |
| 491 | FR1L32' | GCAAGTGATGGTGACTCTGTCTCCTACAGATGCAGACAGGAAGGATG |
| 492 | FR1L33' | ACAAGTGATGGTGACTCTGTCTCCTGTAGATGCAGAGAATGAGGATG |
| 493 | FR1L34' | GCAAGTGATGGTGACTCTGTCTCCTACAGATGCAGACAGGGAGGATG |
| 494 | FR1L35' | GCAAGTGATGGTGACTCTGTCTCCTACAGATGCAGACAGGGAGGATG |
| 495 | FR1L36' | GCAAGTGATGGTGACTCTGTCTCCTACAGATGCAGACAGGGAGGATG |
| 496 | FR1L37' | GCAAGTGATGGTGACTCTGTCTCCTACAGATGCAGACAGGGAGGATG |
| 497 | FR1L38' | GCAAGTGATGGTGACTCTGTCTCCTACAGATGCAGACAGGGAGGATG |
| 498 | FR1L39' | GCAAGTGATGGTGACTCTGTCTCCTACAGATGCAGACAGGGAGGATG |
| 499 | FR1L40' | GCAGGAGAGGGTGACTCTTTCCCCTGGAGACAAAGACAGGGTGGGTG |
| 500 | FR1L41' | GCAGGAGAGGGTGGCTCTTTCCCCTGGAGACAAAGACAGGGTGGCTG |
| 501 | FR1L42' | GCAGGAGAGGGTGGCTCTTTCCCCTGGAGACAAAGACAGGGTGGCTG |
| 502 | FR1L43' | GCAGGAGAGGGTGGCTCTTTCCCCTGGAGACAAAGACAGGGTGCCTG |
| 503 | FR1L44' | GCAGTTGATGGTGGCCCTCTCGCCCAGAGACACAGCCAGGGAGTCTG |
| 504 | FR1L45' | GCAGGAGATGGAGGCCGGCTCTCCAGGGGTGACGGGCAGGGAGAGTG |
| 505 | FR1L46' | GCAGGAGATGGAGGCCGGCTCTCCAGGGGTGACGGGCAGGGAGAGTG |

PCR is carried out using the following oligonucleotide combinations (46 in total): FR1L1/FR1L1', FR1L2/FR1L2', FR1L3/FR1L3', FR1L4/FR1L4', FR1L5/FR1L5', FR1L6/FR1L6', FR1L7/FR1L7', FR1L8/FR1L8', FR1L9/FR1L9', FR1L10/FR1L10', FR1L11/FR1L11', FR1L12/FR1L12', FR1L13/FR1L13', FR1L14/FR1L14', FR1L15/FR1L15', FR1L16/FR1L16', FR1L17/FR1L17', FR1L18/FR1L18', FR1L19/FR1L19', FR1L20/FR1L20', FR1L21/FR1L21', FR1L22/FR1L22', FR1L23/FR1L23', FR1L24/FR1L24', FR1L25/FR1L25', FR1L26/FR1L26', FR1L27/FR1L27', FR1L28/FR1L28', FR1L29/FR1L29', FR1L30/FR1L30', FR1L31/FR1L31', FR1L32/FR1L32', FR1L33/FR1L33', FR1L34/FR1L34', FR1L35/FR1L35', FR1L36/FR1L36', FR1L37/FR1L37', FR1L38/FR1L38', FR1L39/FR1L39', FR1L40/FR1L40', FR1L41/FR1L41', FR1L42/FR1L42', FR1L43/FR1L43', FR1L44/FR1L44', FR1L45/FR1L45', and FR1L46/FR1L46'. The pooling of the PCR products generates sub-bank 1.

By way of example but not limitation, the construction of light chain FR2 sub-bank is carried out using the Polymerase Chain Reaction by overlap extension using the oligonucleotides listed in Table 14 and Table 15 (all shown in the 5' to 3' orientation, name followed by sequence):

**Table 14. Light Chain FR2 Forward Primers (for Sub-Bank 2)**

| | | |
|---|---|---|
| 506 | FR2L1 | TGGTTTCAGCAGAGGCCAGGCCAATCTCCAA |
| 507 | FR2L2 | TGGTTTCAGCAGAGGCCAGGCCAATCTCCAA |
| 508 | FR2L3 | TGGTACCTGCAGAAGCCAGGCCAGTCTCCAC |
| 509 | FR2L4 | TGGTACCTGCAGAAGCCAGGGCAGTCTCCAC |
| 510 | FR2L5 | TGGTACCTGCAGAAGCCAGGCCAGCCTCCAC |
| 511 | FR2L6 | TGGCTTCAGCAGAGGCCAGGCCAGCCTCCAA |
| 512 | FR2L7 | TGGTACCTGCAGAAGCCAGGGCAGTCTCCAC |
| 513 | FR2L8 | TGGTTTCTGCAGAAAGCCAGGCCAGTCTCCA |
| 514 | FR2L9 | TGGCTTCAGCAGAGGCCAGGCCAGCCTCCAA |
| 515 | FR2L10 | TGGTACCAGCAGAAACCAGATCAGTCTCCAA |
| 516 | FR2L11 | TGGTACCAGCAGAAACCAGATCAAGCCCCAA |
| 517 | FR2L12 | TGGTATCAGCAGAAACCAGGGAAAGTTCCTA |
| 518 | FR2L13 | TGGTACCAGCAGAAACCAGATCAGTCTCCAA |
| 519 | FR2L14 | TGGTATCAGCAGAAACCAGGGAAAGCCCCTA |
| 520 | FR2L15 | TGGTACCAACAGAAACCAGGAGAAGCTGCTA |
| 521 | FR2L16 | TGGTTTCAGCAGAAACCAGGGAAAGCCCCTA |
| 522 | FR2L17 | TGGTATCAGCAGAAACCAGGGAAAGCCCCTA |
| 523 | FR2L18 | TGGTATCAGCAGAAACCAGGGAAAGCCCCTA |
| 524 | FR2L19 | TGGTTTCAGCAGAAACCAGGGAAAGTCCCTA |
| 525 | FR2L20 | TGGTATCAGCAGAAACCAGAGAAAGCCCCTA |
| 526 | FR2L21 | TGGTACCAGCAGAAACCTGGCCAGGCTCCCA |
| 527 | FR2L22 | TGGTATCAGCAGAAACCAGGGAAAGCTCCTA |
| 528 | FR2L23 | TGGTATCAGCAGAAACCAGGGAAAGCCCCTA |
| 529 | FR2L24 | TGGTACCAGCAGAAACCTGGCCAGGCTCCCA |
| 530 | FR2L25 | TGGTACCAGCAGAAACCTGGCCAGGCTCCCA |
| 531 | FR2L26 | TGGTATCTGCAGAAACCAGGGAAATCCCCTA |
| 532 | FR2L27 | TGGTATCAGCAAAAACCAGCAAAAGCCCCTA |
| 533 | FR2L28 | TGGTATCAGCAAAAACCAGGGAAAGCCCCTG |
| 534 | FR2L29 | TGGTATCAGCAGAAACCAGGGAAAGCTCCTA |
| 535 | FR2L30 | TGGTATCAGCAGAAACCAGGGAAAGCCCCTA |
| 536 | FR2L31 | TGGTACCAACAGAAACCTGGCCAGGCTCCCA |
| 537 | FR2L32 | TGGTATCAGCAAAAACCAGGGAAAGCCCCTA |
| 538 | FR2L33 | TGGTATCAGCAAAAACCAGGGAAAGCCCCTA |
| 539 | FR2L34 | TGGTATCAGCAGAAACCAGGGAAAGCCCCTA |
| 540 | FR2L35 | TGGTATCGGCAGAAACCAGGGAAAGTTCCTA |
| 541 | FR2L36 | TGGTATCAGCAGAAACCAGGGAAAGCCCCTA |
| 542 | FR2L37 | TGGTATCAGCAGAAACCAGGGAAAGCCCCTA |
| 543 | FR2L38 | TGGTATCGGCAGAAACCAGGGAAAGTTCCTA |
| 544 | FR2L39 | TGGTATCAGCAGAAACCAGGGAAAGCCCCTA |
| 545 | FR2L40 | TGGTATCAGCAGAAACCTGGCCAGGCGCCCA |
| 546 | FR2L41 | TGGTACCAGCAGAAACCTGGGCAGGCTCCCA |
| 547 | FR2L42 | TGGTACCAGCAGAAACCTGGCCTGGCGCCCA |
| 548 | FR2L43 | TGGTACCAGCAGAAACCTGGCCAGGCTCCCA |
| 549 | FR2L44 | TGGTACCAGCAGAAACCAGGACAGCCTCCTA |
| 550 | FR2L45 | TGGTACCTGCAGAAGCCAGGGCAGTCTCCAC |
| 551 | FR2L46 | TGGTACCTGCAGAAGCCAGGGCAGTCTCCAC |

**Table 15. Light Chain FR2 Reverse Primers (for Sub-Bank 2)**

| | | |
|---|---|---|
| 552 | FR2L1' | ATAAATTAGGCGCCTTGGAGATTGGCCTGGCCTCT |
| 553 | FR2L2' | ATAAATTAGGCGCCTTGGAGATTGGCCTGGCCTCT |
| 554 | FR2L3' | ATAGATCAGGAGCTGTGGAGACTGGCCTGGCTTCT |
| 555 | FR2L4' | ATAGATCAGGAGCTGTGGAGACTGCCCTGGCTTCT |
| 556 | FR2L5' | ATAGATCAGGAGCTGTGGAGGCTGGCCTGGCTTCT |
| 557 | FR2L6' | ATAAATrAGGAGTCTTGGAGGCTGGCCTGGCCTCT |
| 558 | FR2L7' | ATAGATCAGGAGCTGTGGAGACTGCCCTGGCTTCT |
| 559 | FR2L8' | ATAGATCAGGAGTGTGGAGACTGGCCTGGCTTTCT |
| 560 | FR2L9' | ATAAATTAGGAGTCTTGGAGGCTGGCCTGGCCTCT |
| 561 | FR2L10' | CTTGATGAGGAGCTTTGGAGACTGATCTGGTTTCT |
| 562 | FR2L11' | CTTGATGAGGAGCTTTGGGGCTTGATCTGGTTTCT |
| 563 | FR2L12' | ATAGATCAGGAGCTTAGGAACTTTCCCTGGTTTCT |
| 564 | FR2L13' | CTTGATGAGGAGCTTTGGAGACTGATCTGGTTTCT |
| 565 | FR2L14' | ATAGATCAGGCGCTTAGGGGCTTTCCCTGGTTTCT |
| 566 | FR2L15' | TTGAATAATGAAAATAGCAGCTTCTCCTGGTTTCT |
| 567 | FR2L16' | ATAGATCAGGGACTTAGGGGCTTTCCCTGGTTTCT |
| 568 | FR2L17' | ATAGATCAGGAGCTTAGGGGCTTTCCCTGGTTTCT |
| 569 | FR2L18' | ATAGATCAGGAGCTTAGGGGCTTTCCCTGGTTTCT |
| 570 | FR2L19' | ATAGATCAGGTGCTTAGGGACTTTCCCTGGTTTCT |
| 571 | FR2L20' | ATAGATCAGGGACTTAGGGGCTTTCTCTGGTTTCT |
| 572 | FR2L21' | ATAGATGAGGAGCCTGGGAGCCTGGCCAGGTTTCT |
| 573 | FR2L22' | ATAGATCAGGAGCTTAGGAGCTCCCTGGTTTCT |
| 574 | FR2L23' | ATAGATCAGGAGCTTAGGGGCTTTCCCTGGTTTCT |
| 575 | FR2L24' | ATAGATGAGGAGCCTGGGAGCCTGGCCAGGTTTCT |
| 576 | FR2L25' | ATAGATGAGGAGCCTGGGAGCCTGGCCAGGTTTCT |
| 577 | FR2L26' | ATAGAGGAAGAGCTTAGGGGATTTCCCTGGTTTCT |
| 578 | FR2L27' | ATAGATGAAGAGCTTAGGGGCTTTTGCTGGTTTTT |
| 579 | FR2L28' | ATAGATCAGGAGCTCACGGGCTTTCCCTGGTTTTT |
| 580 | FR2L29' | ATAGATCAGGAGCTTAGGAGCTTTCCCTGGTTTCT |
| 581 | FR2L30' | ATAGATCAGGAGCTTAGGGGCTTTCCCTGGTTTCT |
| 582 | FR2L31' | ATAGATGAGGAGCCTGGGAGCCTGGCCAGGTTTCT |
| 583 | FR2L32' | ATAGATCAGGAGCTTAGGGGCTTTCCCTGGTTTTT |
| 584 | FR2L33' | ATAGATCAGGAGCTTAGGGGCTTTCCCTGGTTTTT |
| 585 | FR2L34' | ATAGATCAGGAGCTTAGGGGCTTTCCCTGGTTTCT |
| 586 | FR2L35' | ATAGATCAGGAGCTTAGGAACTTTCCCTGGTTTCT |
| 587 | FR2L36' | GTAGATCAGGAGCTTAGGGGCT7TCCCTGGTTTCT |
| 588 | FR2L37' | ATAGATCAGGAGCTTAGGGGCTTTCCCTGGTTTCT |
| 589 | FR2L38' | ATAGATCAGGAGCTTAGGAACTTTCCCTGGTTTCT |
| 590 | FR2L39' | GTAGATCAGGAGCTTAGGGGCTTTCCCTGGTTTCT |
| 591 | FR2L40' | ATAGATGAGGAGCCTGGGCGCCTGGCCAGGTTTCT |
| 592 | FR2L41' | ATAGATGAGGAGCCTGGGAGCCTGCCCAGGTTTCT |
| 593 | FR2L42' | ATAGATGAGGAGCCTGGGCGCCAGGCCAGGTTTCT |
| 594 | FR2L43' | ATAGATGAGGAGCCTGGGAGCCTGGCCAGGTTTCT |
| 595 | FR2L44' | GTAAATGAGCAGCTTAGGAGGCTGTCCTGGTTTCT |
| 596 | FR2L45' | ATAGATCAGGAGCTGTGGAGACTGCCCTGGCTTCT |
| 597 | FR2L46' | ATAGATCAGGAGCTGTGGAGACTGCCCTGGCTTCT |

PCR is carried out using the following oligonucleotide combinations (46 in total): FR2L1/FR2L1', FR2L2/FR2L2', FR2L3/FR2L3', FR2L4/FR2L4', FR2L5/FR2L5', FR2L6/FR2L6', FR2L7/FR2L7', FR2L8/FR2L8', FR2L9/FR2L9', FR2L10/FR2L10', FR2L11/FR2L11', FR2L12/FR2L12', FR2L13/FR2L13", FR2L14/FR2L14', FR2L15/FR2L15', FR2L16/FR2L16', FR2L17/FR2L17', FR2L18/FR2L18', FR2L 19/FR2L 19', FR2L20/FR2L20', FR2L21/FR2L21', FR2L22/FR2L22', FR2L23/FR2L23', FR2L24/FR2L24', FR2L25/FR2L25', FR2L26/FR2L26', FR2L27/FR2L27', FR2L28/FR2L28', FR2L29/FR2L29', FR2L30/FR2L30', FR2L31/FR2L31', FR2L32/FR2L32', FR2L33/FR2L33', FR2L34/FR2L34', FR2L35/FR2L35', FR2L36/FR2L36', FR2L37/FR2L37', FR2L38/FR2L38', FR2L39/FR2L39', FR2L40/FR2L40', FR2L41/FR2L41', FR2L42/FR2L42', FR2L43/FR2L43', FR2L44/FR2L44', FR2L45/FR2L45', and FR2L46/FR2L46'. The pooling of the PCR products generates sub-bank 2.

By way of example but not limitation, the construction of light chain FR3 sub-bank is carried out using the Polymerase Chain Reaction by overlap extension using the oligonucleotides listed in Table 16 and Table 17 (all shown in the 5' to 3' orientation, name followed by sequence):

**Table 16. Light Chain FR3 Forward Primers (for Sub-Bank 3)**

| | |
|---|---|
| 598 | FR3L1 |
| | GGGGTCCCAGACAGATTCAGCGGCAGTGGGTCAGGCACTGATTTCACACTGAAAATCAG |
| 599 | FR3L2 |
| | GGGGTCCCAGACAGATTCAGCGGCAGTGGGTCAGGCACTGATTTCACACTGAAAATCAG |
| 600 | FR3L3 |
| | GGAGTGCCAGATAGGTTCAGTGGCAGCGGGTCAGGGACAGATTTCACACTGAAAATCAG |
| 601 | FR3L4 |
| | GGGGTCCCTGACAGGTTCAGTGGCAGTGGATCAGGCACAGATTTTACACTGAAAATCAG |
| 602 | FR3L5 |
| | GGAGTGCCAGATAGGTTCAGTGGCAGCGGGTCAGGGACAGATTTCACACTGAAAATCAG |
| 603 | FR3L6 |
| | GGGGTCCCAGACAGATTCAGTGGCAGTGGGGCAGGGACAGATTTCACACTGAAAATCAG |
| 604 | FR3L7 |
| | GGGGTCCCTGACAGGTTCAGTGGCAGTGGATCAGGCACAGATTTTACACTGAAAATCAG |
| 605 | FR3L8 |
| | GGAGTGCCAGATAGGTTCAGTGGCAGCGGGTCAGGGACAGATTTCACACTGAAAATCAG |
| 606 | FR3L9 |
| | GGGGTCCCAGACAGATTCAGTGGCAGTGGGGCAGGGACAGATTTCACACTGAAAATCAG |
| 607 | FR3L10 |
| | GGGGTCCCCTCGAGGTTCAGTGGCAGTGGATCTGGGACAGATTTCACCCTCACCATCAA |
| 608 | FR3L11 |
| | GGGGTCCCCTCGAGGTTCAGTGGCAGTGGATCTGGGACAGATTTCACCTTTACCATCAG |
| 609 | FR3L12 |
| | GGGGTCCCATCTCGGTTCAGTGGCAGTGGATCTGGGACAGATTTCACTCTCACCATCAG |
| 610 | FR3L13 |
| | GGGGTCCCCTCGAGGTTCAGTGGCAGTGGATCTGGGACAGATTTCACCCTCACCATCAA |
| 611 | FR3L14 |
| | GGGGTCCCATCAAGGTTCAGCGGCAGTGGATCTGGGACAGAATTCACTCTCACAATCAG |
| 612 | FR3L15 |
| | GGAATCCCACCTCGATTCAGTGGCAGCGGGTATGGAACAGATTTTACCCTCACAATTAA |
| 613 | FR3L16 |
| | GGGGTCCCATCAAGGTTCAGCGGCAGTGGATCTGGGACAGATTTCACTCTCACCATCAG |
| 614 | FR3L17 |
| | GGGGTCCCATCAAGGTTCAGCGGCAGTGGATCTGGCACAGATTTCACTCTCACCATCAG |
| 615 | FR3L18 |
| | GGGGTCCCATCAAGGTTCAGCGGCAGTGGATCTGGGACAGAATTCACTCTCACCATCAG |
| 616 | FR3L19 |
| | GGGGTCCCATCAAGGTTCAGCGGCAGTGGATCTGGGACAGAATTCACACTCACAATCAG |
| 617 | FR3L20 |
| | GGGGTCCCATCAAGGTTCAGCGGCAGTGGATCTGGGACAGATTTCACTCTCACCATCAG |
| 618 | FR3L21 |
| | GGCATCCCAGCCAGGTTCAGTGGCAGTGGGTCTGGGACAGAGTTCACTCTCACCATCAG |
| 619 | FR3L22 |
| | GGGGTCCCATCAAGGTTCAGCGGCAGTGGATCTGGGACAGATTTCACTCTCACCATCAG |
| 620 | FR3L23 |
| | GGGGTCCCATCAAGGTTCAGCGGCAGTGGATCTGGGACAGATTTCACTCTCACTATCAG |
| 621 | FR3L24 |
| | GGTATCCCAGCCAGGTTCAGTGGCAGTGGGTCTGGGACAGAGTTCACTCTCACCATCAG |
| 622 | FR3L25 |
| | GGCATCCCAGCCAGGTTCAGTGGCAGTGGGCCTGGGACAGACTTCACTCTCACCATCAG |
| 623 | FR3L26 |
| | GGGGTCTCATCGAGGTTCAGTGGCAGGGGATCTGGGACGGATTTCACTCTCACCATCAT |
| 624 | FR3L27 |
| | GGGGTCCCATCAAGGTTCAGCGGCAGTGGATCTGGGACGGATTACACTCTCACCATCAG |
| 625 | FR3L28 |
| | GGGGTCCCATCAAGGTTCAGTGGCAGTGGATCTGGGACAGATTTCACTCTCACCATCAG |
| 626 | FR3L29 |
| | GGGGTCCCATCAAGGTTCAGCGGCAGTGGATCTGGGACAGATTTCACTCTCACCATCAG |
| 627 | FR3L30 |
| | GGGGTCCCATCAAGGTTCAGCGGCAGTGGATCTGGGACAGATTTCACTCTCACCATCAG |
| 628 | FR3L31 |
| | GGCATCCCAGCCAGGTTCAGTGGCAGTGGGTCTGGGACAGACTTCACTCTCACCATCAG |
| 629 | FR3L32 |
| | GGGGTCCCATCAAGGTTCAGCGGCAGTGGATCTGGGACAGAATTCACTCTCACAATCAG |
| 630 | FR3L33 |
| | GGGGTCCCATCAAGGTTCAGCGGCAGTGGATCTGGGACAGATTTCACTCTCACCATCAG |
| 631 | FR3L34 |
| | GGGGTCCCATCAAGGTTCAGTGGCAGTGGATCTGGGACAGATTTCACTCTCACCATCAG |
| 632 | FR3L35 |
| | GGAGTCCCATCTCGGTTCAGTGGCAGTGGATCTGGGACAGATTTCACTCTCACTATCAG |
| 633 | FR3L36 |
| | GGGGTCCCATCAAGGTTCAGTGGAAGTGGATCTGGGACAGATTTTACTTTCACCATCAG |
| 634 | FR3L37 |
| | GGGGTCCCATCAAGGTTCAGTGGCAGTGGATCTGGGACAGATTTCACTCTCACCATCAG |
| 635 | FR3L38 |
| | GGAGTCCCATCTCGGTTCAGTGGCAGTGGATCTGGGACAGATTTCACTCTCACTATCAG |
| 636 | FR3L39 |
| | GGGGTCCCATCAAGGTTCAGTGGAAGTGGATCTGGGACAGATTTTACTTTCACCATCAG |
| 637 | FR3L40 |
| | AGCATCCCAGCCAGGTTCAGTGGCAGTGGGTCTGGGACAGACTTCACTCTCACCATCAG |
| 638 | FR3L41 |
| | GGCATCCCAGCCAGGTTCAGTGGCAGTGGGTCTGGGACAGACTTCACTCTCACCATCAG |
| 639 | FR3L42 |
| | GGCATCCCAGACAGGTTCAGTGGCAGTGGGTCTGGGACAGACTTCACTCTCACCATCAG |
| 640 | FR3L43 |
| | GGCATCCCAGACAGGTTCAGTGGCAGTGGGTCTGGGACAGACTTCACTCTCACCATCAG |
| 641 | FR3L44 |
| | GGGGTCCCTGACCGATTCAGTGGCAGCGGGTCTGGGACAGATTTCACTCTCACCATCAG |
| 642 | FR3L45 |
| | GGAGTCCCAGACAGGTTCAGTGGCAGTGGGTCAGGCACTGATTTCACACTGAAAATCAG |
| 643 | FR3L46 |
| | GGAGTCCCAGACAGGTTCAGTGGCAGTGGGTCAGGCACTGATTTCACACTGAAAATCAG |

**Table 17. Light Chain FR3 Reverse Primers (for Sub-Bank 3)**

| | | |
|---|---|---|
| 644 | FR3L1' | GCAGTAATAAACCCCAACATCCTCAGCCTCCACCCTGCTGATTTTCAGTGTGAAA |
| 645 | FR3L2' | GCAGTAATAAACCCCAACATCCTCAGCCTCCACCCTGCTGATTTTCAGTGTGAAA |
| 646 | FR3L3 | TCAGTAATAAACCCCAACATCCTCAGCCTCCACCCGGCTGATTTTCAGTGTGAAA |
| 647 | FR3L4' | GCAGTAATAAACCCCAACATCGTCAGCCTCCACTCTGCTGATTTTCAGTGTAAAA |
| 648 | FR3L5' | GCAGTAATAAACCCCAACATCCTCAGCCTCCACCCGGCTGATTTTCAGTGTGAAA |
| 649 | FR3L6' | GCAGTAATAAACCCCGACATCCTCAGCTTCCACCCTGCTGATTTTCAGTGTGAAA |
| 650 | FR3L7' | GCAGTAATAAACCCCAACATCCTCAGCCTCCACTCTGCTGATTTTCAGTGTAAAA |
| 651 | FR3L8' | GCAGTAATAAACTCCAAAATCCTCAGCCTCCACCCGGCTGATTTTCAGTGTGAAA |
| 652 | FR3L9' | GCAGTAATAAACCCCGACATCCTCAGCTTCCACCCTGCTGATTTTCAGTGTGAAA |
| 653 | FR3L10' | ACAGTAATACGTTGCAGCATCTTCAGCTTCCAGGCTATTGATGGTGAGGGTGAAA |
| 654 | FR3L11' | ACAGTAATATGTTGCAGCATCTTCAGCTTCCAGGCTACTGATGGTAAAGGTGAAA |
| 655 | FR3L12' | ACAGTAATAAGTTGCAACATCTTCAGGCTGCAGGCTGCTGATGGTGAGAGTGAAA |
| 656 | FR3L13' | ACAGTAATACGTTGCAGCATCTTCAGCTTCCAGGGTATTGATGGTGAGGGTGAAA |
| 657 | FR3L14' | ACAGTAATAAGTTGCAAAATCTTCAGGCTGCAGGCTGCTGATTGTGAGAGTGAAT |
| 658 | FR3L15' | ACAGAAGTAATATGCAGCATCCTCAGATTCTATGTTATTAATTGTGAGGGTAAAA |
| 659 | FR3L16' | GCAGTAATAAGTTGCAAAATCTTCAGGCTGCAGGCTGCTGATGGTGAGAGTGAAA |
| 660 | FR3L17' | ACAGTAATAAGTTGCAAAATCTTCAGGCTGCAGGCTGCTGATGGTGAGAGTGAAA |
| 661 | FR3L18' | GCAGTAATAAGTTGCAAAATCATCAGGCTGCAGGCTGCTGATGGTGAGAGTGAAT |
| 662 | FR3L19' | ACAGTAATAAGTTGCAAAATCTTCAGGCTGCAGGCTGCTGATTGTGAGAGTGAAT |
| 663 | FR3L20' | GCAGTAATAAGTTGCAAAATCTTCAGGCTGCAGGCTGCTGATGGTGAGAGTGAAA |
| 664 | FR3L21' | ACAGTAATAAACTGCAAAATCTTCAGACTGCAGGCTGCTGATGGTGAGAGTGAAC |
| 665 | FR3L22' | ACAGTAATAAGTTGCAAAATCTTCAGGCTGCAGGCTGCTGATGGTGAGAGTGAAA |
| 666 | FR3L23' | ACAATAGTAAGTTGCAAAATCTTCAGGCTGCAGGCTGCTGATAGTGAGAGTGAAA |
| 667 | FR3L24' | ACAGTAATAAACTGCAAAATCTTCAGACTGCAGGCTGCTGATGGTGAGAGTGAAC |
| 668 | FR3L25' | ACAGTAATAAACTGCAAAATCTTCAGGCTCTAGGCTGCTGATGGTGAGAGTGAAG |
| 669 | FR3L26' | ACAGTAATAAGCTGCAAAATCTTCAGGCTTCAGGCTGATGATGGTGAGAGTGAAA |
| 670 | FR3L27' | ACAGTAATAAGTTGCAAAATCTTCAGGCTGCAGGCTGCTGATGGTGAGAGTGTAA |
| 671 | FR3L28' | ACAGTAATAAGTTGCAAAATCTTCAGACTGCAGGCAACTGATGGTGAGAGTGAAA |
| 672 | FR3L29' | ACAGTAATAAGTTGCAAAATCTTCAGGCTGCAGGCTGCTGATGGTGAGAGTGAAA |
| 673 | FR3L30' | ACAATAGTAAGTTGCAAAATCTTCAGGCTGCAGGCTGCTGATGGTGAGAGTGAAA |
| 674 | FR3L31' | ACAGTAATAAACTGCAAAATCTTCAGGCTCTAGGCTGCTGATGGTGAGAGTGAAG |
| 675 | FR3L32' | ACAGTAATAAGTTGCAAAATCTTCAGGCTGCAGGCTGCTGATTGTGAGAGTGAAT |
| 676 | FR3L33' | ACAGTAATAAGTTGCAAAATCTTCAGACTGCAGGCAGCTGATGGTGAGAGTGAAA |
| 677 | FR3L34' | ACAGTAGTAAGTTGCAAAATCTTCAGGTTGCAGACTGCTGATGGTGAGAGTGAAA |
| 678 | FR3L35' | ACCGTAATAAGTTGCAACATCTTCAGGCTGCAGGCTGCTGATAGTGAGAGTGAAA |
| 679 | FR3L36' | ACAGTAATATGTTGCAATATCITCAGGCTGCAGGCTGCTGATGGTGAAAGTAAAA |
| 680 | FR3L37' | ACAGTAGTAAGTTGCAAAATCTTCAGGTTGCAGACTGCTGATGGTGAGAGTGAAA |
| 681 | FR3L38' | ACCGTAATAAGTTGCAACATCTTCAGGCTGCAGGCTGCTGATAGTGAGAGTGAAA |
| 682 | FR3L39' | ACAGTAATATGTTGCAATATCTTCAGGCTGCAGGCTGCTGATGGTGAAAGTAAAA |
| 683 | FR3L40' | ACAGTAATAAACTGCAAAATCTTCAGGCTGCAGGCTGCTGATGGTGAGAGTGAAG |
| 684 | FR3L41' | ACAGTAATAAACTGCAAAATCTTCAGGCTGCAGGCTGCTGATGGTGAGAGTGAAG |
| 685 | FR3L42' | ACAGTAATACACTGCAAAATCTTCAGGCTCCAGTCTGCTGATGGTGAGAGTGAAG |
| 686 | FR3L43' | ACAGTAATACACTGCAAAATCTTCAGGCTCCAGTCTGCTGATGGTGAGAGTGAAG |
| 687 | FR3L44' | ACAGTAATAAACTGCCACATCTTCAGCCTGCAGGCTGCTGATGGTGAGAGTGAAA |
| 688 | FR3L45' | GCAGTAATAAACTCCAACATCCTCAGCCTCCACCCTGCTGATTTTCAGTGTGAAA |
| 689 | FR3L46' | GCAGTAATAAACTCCAACATCCTCAGCCTCCACCCTGCTGATTTTCAGTGTGAAA |

PCR is carried out using the following oligonucleotide combinations (46 in total): FR3L1/FR3L1', FR3L2/FR3L2', FR3L3/FR3L3', FR3L4/F'R3L4', FR3L5/FR3L5', FR3L6/FR3L6', FR3L7/FR3L7', FR3L8/FR3L8', FR3L9/FR3L9', FR3L10/FR3L10', FR3L11/FR3L11', FR3L12/FR3L12', FR3L13/FR3L13', FR3L14/FR3L14', FR3L15/FR3L15', FR3L16/FR3L16', FR3L17/FR3L17', FR3L18/FR3L18', FR3L19/FR3L19', FR3L20/FR3L20', FR3L21/FR3L21', FR3L22/FR3L22', FR3L23/FR3L23', FR3L24/FR3L24', FR3L25/FR3L25', FR3L26/FR3L26', FR3L27/FR3L27', FR3L28/FR3L28', FR3L29/FR3L29', FR3L30/FR3L30', FR3L31/FR3L31', FR3L32/FR3L32', FR3L33/FR3L33', FR3L34/FR3L34', FR3L35/FR3L35', FR3L36/FR3L36', FR3L37/FR3L37', FR3L38/FR3L38', FR3L39/FR3L39', FR3L40/FR3L40', FR3L41/FR3L41', FR3L42/FR3L42', FR3L43/FR3L43', FR3L44/FR3L44', FR3L45/FR3L45', and FR3L46/FR3L46'. The pooling of the PCR products generates sub-bank 3.

By way of example but not limitation, the construction of light chain FR4 sub-bank is carried out using the Polymerase Chain Reaction by overlap extension using the oligonucleotides listed in Table 18 and Table 19 (all shown in the 5' to 3' orientation, name followed by sequence):

**Table 18. Light Chain FR4 Forward Primers (for Sub-Bank 4)**

| | | |
|---|---|---|
| 690 | FR4L1 | TTCGGCCAAGGGACCAAGGTGGAAATCAAA |
| 691 | FR4L2 | TTTGGCCAGGGGACCAAGCTGGAGATCAAA |
| 692 | FR4L3 | TTCGGCCCTGGGACCAAAGTGGATATCAAA |
| 693 | FR4L4 | TTCGGCGGAGGGACCAAGGTGGAGATCAAA |
| 694 | FR4L5 | TTCGGCCAAGGGACACGACTGGAGATTAAA |

**Table 19. Light Chain FR4 Reverse Primers (for Sub-Bank 4)**

| | | |
|---|---|---|
| 695 | FR4L1' | TTTGATTTCCACCTTGGTCCCTTGGCCGAA |
| 696 | FR4L2' | TTTGATCTCCAGCTTGGTCCCCTGGCCAAA |
| 697 | FR4L3' | TTTGATATCCACTTTGGTCCCAGGGCCGAA |
| 698 | FR4L4' | TTTGATCTCCACCTTGGTCCCTCCGCCGAA |
| 699 | FR4L5' | TTTAATCTCCAGTCGTGTCCCTTGGCCGAA |

PCR is carried out using the following oligonucleotide combinations (5 in total): FR4L1/FR4L1', FR4L2/FR4L2', FR4L3/FR4L3', FR4L4/FR4L4', or FR4L5/FR4L5'. The pooling of the PCR products generates sub-bank 4.

### 2.2.2 Generation of Sub-banks for the Heavy Chain Frameworks

In some embodiments, heavy chain FR sub-banks 5, 6, 7 and 11 are constructed wherein sub-bank 5 comprises a plurality of nucleic acid sequences comprising nucleotide sequences, each nucleotide sequence encoding a heavy chain FR1; sub-bank 6 comprises a plurality of nucleic acid sequences comprising nucleotide sequences, each nucleotide sequence encoding a heavy chain FR2; sub-bank 7 comprises a plurality of nucleic acid sequences comprising nucleotide sequences, each nucleotide sequence encoding a heavy chain FR3; and sub-bank 11 comprises a plurality of nucleic acid sequences comprising nucleotide sequences, each nucleotide sequence encoding a heavy chain FR4, respectively; wherein the heavy chain FR1, FR2, and FR3 are defined according to Kabat definition for CDR H 1 and H2. In some embodiments, the FR sequences are derived from functional human antibody sequences. In other embodiments, the FR sequences are derived from human germline heavy chain sequences.

By way of example but not limitation, the following describes a method of generating 4 heavy chain FR sub-banks using Polymerase Chain Reaction (PCR), wherein human germline heavy chain sequences are used as templates. Heavy chain FR sub-banks 5, 6 and 7 (encoding FR1, 2, 3 respectively) encompass 44 human germline heavy chain sequences (VH1-18, VH1-2, VH1-24, VH1-3, VH1-45, VH1-46, VH1-58, VH1-69, VH1-8, VH2-26, VH2-5, VH2-70, VH3-11, VH3-13, VH3-15, VH3-16, VH3-20, VH3-21, VH3-23, VH3-30, VH3-33, VH3-35, VH3-38, VH3-43, VH3-48, VH3-49, VH3-53, VH3-64, VH3-66, VH3-7, VH3-72, VH3-73, VH3-74, VH3-9, VH4-28, VH4-31, VH4-34, VH4-39, VH4-4, VH4-59, VH4-61, VH5-51, VH6-1 and VH7-81). See Matsuda et al., 1998, J. Exp. Med., 188:1973-1975. The sequences are summarized at the NCBI website: www.ncbi.nlm.nih.gov/igblast/showGermline.cgi?organisrn=human&chainType=VH&seqT ype=nucleotide. Heavy chain FR sub-bank 11 (encoding FR4) encompasses 6 human germline heavy chain sequences (JH1, JH2, JH3, JH4, JH5 and JH6). See Ravetch et al., 1981, Cell 27(3 Pt 2):583-591. The sequences are summarized at the NCBI website: www.ncbi.nlm.nih.gov/igblast/showGermline.cgi?organism=human&chainType=JH&seqT ype=nucleotide.

By way of example but not limitation, the construction of heavy chain FR1 sub-bank (according to Kabat definition) is carried out using the Polymerase Chain Reaction by overlap extension using the oligonucleotides listed in Table 20 and Table 21 (all shown in the 5' to 3' orientation, name followed by sequence):

**Table 20. Heavy Chain FR1 (Kabat Definition) Forward Primers (for Sub-Bank 5):**

| | |
|---|---|
| 700 | FR1HK1 |
| | CAGGTTCAGCTGGTGCAGTCTGGAGCTGAGGTGAAGAAGCCTGGGGCCTCAGTGAAGGT |
| 701 | FR1HK2 |
| | CAGGTGCAGCTGGTGCAGTCTGGGGCTGAGGTGAAGAAGCCTGGGGCCTCAGTGAAGGT |
| 702 | FR1HK3 |
| | CAGGTCCAGCTGGTACAGTCTGGGGCTGAGGTGAAGAAGCCTGGGGCCTCAGTGAAGGT |
| 703 | FR1HK4 |
| | CAGGTTCAGCTGGTGCAGTCTGGGGCTGAGGTGAAGAAGCCTGGGGCCTCAGTGAAGGT |
| 704 | FR1HK5 |
| | CAGATGCAGCTGGTGCAGTCTGGGGCTGAGGTGAAGAAGACTGGGTCCTCAGTGAAGGT |
| 705 | FR1HK6 |
| | CAGGTGCAGCTGGTGCAGTCTGGGGCTGAGGTGAAGAAGCCTGGGGCCTCAGTGAAGGT |
| 706 | FR1HK7 |
| | CAAATGCAGCTGGTGCAGTCTGGGCCTGAGGTGAAGAAGCCTGGGACCTCAGTGAAGGT |
| 707 | FR1HK8 |
| | CAGGTGCAGCTGGTGCAGTCTGGGGCTGAGGTGAAGAAGCCTGGGTCCTCGGTGAAGGT |
| 708 | FR1HK9 |
| | CAGGTGCAGCTGGTGCAGTCTGGGGCTGAGGTGAAGAAGCCTGGGGCCTCAGTGAAGGT |
| 709 | FR1HK10 |
| | CAGGTCACCTTGAAGGAGTCTGGTCCTGTGCTGGTGAAACCCACAGAGACCCTCACGCT |
| 710 | FR1HK11 |
| | CAGATCACCTTGAAGGAGTCTGGTCCTACGCTGGTGAAACCCACACAGACCCTCACGCT |
| 711 | FR1HK12 |
| | CAGGTCACCTTGAGGGAGTCTGGTCCTGCGCTGGTGAAACCCACACAGACCCTCACACT |
| 712 | FR1KK13 |
| | CAGGTGCAGCTGGTGGAGTCTGGGGGAGGCTTGGTCAAGCCTGGAGGGTCCCTGAGACT |
| 713 | FR1HK14 |
| | GAGGTGCAGCTGGTGGAGTCTGGGGGAGGCTTGGTACAGCCTGGGGGGTCCCTGAGACT |
| 714 | FR1HK15 |
| | GAGGTGCAGCTGGTGGAGTCTGGGGGAGGCTTGGTAAAGCCTGGGGGGTCCCTTAGAGT |
| 715 | FR1HK16 |
| | GAGGTGCAGCTGGTGGAGTCTGGGGGAGGCTTGGTACAGCCTGGGGGGTCCCTGAGACT |
| 716 | FR1HK17 |
| | GAGGTGCAGCTGGTGGAGTCTGGGGGAGGTGTGGTACGGCCTGGGGGGTCCCTGAGACT |
| 717 | FR1HK18 |
| | GAGGTGCAGCTGGTGGAGTCTGGGGGAGGCCTGGTCAAGCCTGGGGGGTCCCTGAGACT |
| 718 | FR1HK19 |
| | GAGGTGCAGCTGTTGGAGTCTGGGGGAGGCTTGGTACAGCCTGGGGGGTCCCTGAGACT |
| 719 | FR1HK20 |
| | CAGGTGCAGCTGGTGGAGTCTGGGGGAGGCGTGGTCCAGCCTGGGAGGTCCCTGAGACT |
| 720 | FR1HK21 |
| | CAGGTGCAGCTGGTGGAGTCTGGGGGAGGCGTGGTCCAGCCTGGGAGGTCCCTGAGACT |
| 721 | FR1HK22 |
| | GAGGTGCAGCTGGTGGAGTCTGGGGGAGGCTTGGTACAGCCTGGGGGATCCCTGAGACT |
| 722 | FR1HK23 |
| | GAGGTGCAGCTGGTGGAGTCTGGGGGAGGCTTGGTACAGCCTAGGGGGTCCCTGAGACT |
| 723 | FR1HK24 |
| | GAAGTGCAGCTGGTGGAGTCTGGGGGAGTCGTGGTACAGCCTGGGGGGTCCCTGAGACT |
| 724 | FR1HK25 |
| | GAGGTGCAGCTGGTGGAGTCTGGGGGAGGCTTGGTACAGCCTGGGGGGTCCCTGAGACT |
| 725 | FR1HK26 |
| | GAGGTGCAGCTGGTGGAGTCTGGGGGAGGCTTGGTACAGCCAGGGCGGTCCCTGAGACT |
| 726 | FR1HK27 |
| | GAGGTGCAGCTGGTGGAGTCTGGAGGAGGCTTGATCCAGCCTGGGGGGTCCCTGAGACT |
| 727 | FR1HK28 |
| | GAGGTGCAGCTGGTGGAGTCTGGGGAAGGCTTGGTCCAGCCTGGGGGGTCCCTGAGACT |
| 728 | FR1HK29 |
| | GAGGTGCAGCTGGTGGAGTCTGGAGGAGGCTTGATCCAGCCTGGGGGGTCCCTGAGACT |
| 729 | FR1HK30 |
| | GAGGTGCAGCTGGTGGAGTCTGGGGGAGGCTTGGTCCAGCCTGGGGGGTCCCTGAGACT |
| 730 | FR1HK31 |
| | GAGGTGCAGCTGGTGGAGTCTGGGGGAGGCTTGGTCCAGCCTGGAGGGTCCCTGAGACT |
| 731 | FR1HK32 |
| | GAGGTGCAGCTGGTGGAGTCCGGGGGAGGCTTGGTCCAGCCTGGGGGGTCCCTGAAACT |
| 732 | FR1HK33 |
| | GAGGTGCAGCTGGTGGAGTCCGGGCGAGGCTTAGTTCAGCCTGGGGGGTCCCTGAGACT |
| 733 | FR1HK34 |
| | GAAGTGCAGCTGGTGGAGTCTGGGGGAGGCTTGGTACAGCCTGGCAGGTCCCTGAGACT |
| 734 | FR1HK35 |
| | CAGGTGCAGCTGCAGGAGTCGGGCCCAGGACTGGTGAAGCCTTCGGACACCCTGTCCCT |
| 735 | FR1HK36 |
| | CAGGTGCAGCTGCAGGAGTCGGGCCCAGGACTGGTGAAGCCTTCACAGACCCTGTCCCT |
| 736 | FR1HK37 |
| | CAGGTGCAGCTACAGCAGTGGGGCGCAGGACTGTTGAAGCCTTCGGAGACCCTGTCCCT |
| 737 | FR1HK38 |
| | CAGCTGCAGCTGCAGGAGTCGGGCCCAGGACTGGTGAAGCCTTCGGAGACCCTGTCCCT |
| 738 | FR1HK39 |
| | CAGGTGCAGCTGCAGGAGTCGGGCCCAGGACTGGTGAAGCCTTCGGAGACCCTGTCCCT |
| 739 | FR1HK40 |
| | CAGGTGCAGCTGCAGGAGTCGGGCCCAGGACTGGTGAAGCCTTCGGAGACCCTGTCCCT |
| 740 | FR1HK41 |
| | CAGGTGCAGCTGCAGGAGTCGGGCCCAGGACTGGTGAAGCCTTCGGAGACCCTGTCCCT |
| 741 | FR1HK42 |
| | GAGGTGCAGCTGGTGCAGTCTGGAGCAGAGGTGAAAAAGCCCGGGGAGTCTCTGAAGAT |
| 742 | FR1HK43 |
| | CAGGTACAGCTGCAGCAGTCAGGTCCAGGACTGGTGAAGCCCTCGCAGACCCTCTCACT |
| 743 | FR1HK44 |
| | CAGGTGCAGCTGGTGCAGTCTGGCCATGAGGTGAAGCAGCCTGGGGCCTCAGTGAAGGT |

**Table 21. Heavy Chain FR1 (Kabat Definition) Reverse Primers (for Sub-Bank 5):**

| | | |
|---|---|---|
| 744 | FR1HK1' | GGTAAAGGTGTAACCAGAAGCCTTGCAGGAGACCTTCACTGAGGCCCCAGGC |
| 745 | FR1HK2' | GGTGAAGGTGTATCCAGAAGCCTTGCAGGAGACCTTCACTGAGGCCCCAGGC |
| 746 | FR1HK3' | AGTGAGGGTGTATCCGGAAACCTTGCAGGAGACCTTCACTGAGGCCCCAGGC |
| 747 | FR1HK4' | AGTGAAGGTGTATCCAGAAGCCTTGCAGGAAACCTTCACTGAGGCCCCAGGC |
| 748 | FR1HK5' | GGTGAAGGTGTATCCGGAAGCCTTGCAGGAAACCTTCACTGAGGACCCAGTC |
| 749 | FR1HK6' | GGTGAAGGTGTATCCAGATGCCTTGCAGGAAACCTTCACTGAGGCCCCAGGC |
| 750 | FR1HK7' | AGTAAAGGTGAATCCAGAAGCCTTGCAGGAGACCTTCACTGAGGTCCCAGGC |
| 751 | FR1HK8' | GCTGAAGGTGCCTCCAGAAGCCTTGCAGGAGACCTTCACCGAGGACCCAGGC |
| 752 | FR1HK9' | GGTGAAGGTGTATCCAGAAGCCTTGCAGGAGACCTTCACTGAGGCCCCAGGC |
| 753 | FR1HK10' | GCTGAGTGAGAACCCAGAGACGGTGCAGGTCAGCGTGAGGGTCTCTGTGGGT |
| 754 | FR1HK11' | GCTGAGTGAGAACCCAGAGAAGGTGCAGGTCAGCGTGAGGGTCTGTGTGGGT |
| 755 | FR1HK12' | GCTGAGTGAGAACCCAGAGAAGGTGCAGGTCAGTGTGAGGGTCTGTGTGGGT |
| 756 | FR1HK13' | ACTGAAGGTGAATCCAGAGGCTGCACAGGAGAGTCTCAGGGACCCTCCAGGC |
| 757 | FR1HK14' | ACTGAAGGTGAATCCAGAGGCTGCACAGGAGAGTCTCAGGGACCCCCCAGGC |
| 758 | FR1HK15' | ACTGAAAGTGAATCCAGAGGCTGCACAGGAGAGTCTAAGGGACCCCCCAGGC |
| 759 | FR1HK16' | ACTGAAGGTGAATCCAGAGGCTGCACAGGAGAGTCTCAGGGACCCCCCAGGC |
| 760 | FR1HK17' | ATCAAAGGTGAATCCAGAGGCTGCACAGGAGAGTCTCAGGGACCCCCCAGGC |
| 761 | FR1HK18' | ACTGAAGGTGAATCCAGAGGCTGCACAGGAGAGTCTCAGGGACCCCCCAGGC |
| 762 | FR1HK19' | GCTAAAGGTGAATCCAGAGGCTGCACAGGAGAGTCTCAGGGACCCCCCAGGC |
| 763 | FR1HK20' | ACTGAAGGTGAATCCAGAGGCTGCACAGGAGAGTCTCAGGGACCTCCCAGGC |
| 764 | FR1HK21' | ACTGAAGGTGAATCCAGACGCTGCACAGGAGAGTCTCAGGGACCTCCCAGGC |
| 765 | FR1HK22' | ACTGAAGGTGAATCCAGAGGCTGCACAGGAGAGTCTCAGGGATCCCCCAGGC |
| 766 | FR1HK23' | ACTGACGGTGAATCCAGAGGCTGCACAGGAGAGTCTCAGGGACCCCCTAGGC |
| 767 | FR1HK24' | ATCAAAGGTGAATCCAGAGGCTGCACAGGAGAGTCTCAGGGACCCCCCAGGC |
| 768 | FR1HK25' | ACTGAAGGTGAATCCAGAGGCTGCACAGGAGAGTCTCAGGGACCCCCCAGGC |
| 769 | FR1HK26' | ACCAAAGGTGAATCCAGAAGCTGTACAGGAGAGTCTCAGGGACCGCCCTGGC |
| 770 | FR1HK27' | ACTGACGGTGAACCCAGAGGCTGCACAGGAGAGTCTCAGGGACCCCCCAGGC |
| 771 | FR1HK28' | ACTGAAGGTGAATCCAGAGGCTGCACAGGAGAGTCTCAGGGACCCCCCAGGC |
| 772 | FR1HK29' | ACTGACGGTGAACCCAGAGGCTGCACAGGAGAGTCTCAGGGACCCCCCAGGC |
| 773 | FR1HK30' | ACTAAAGGTGAATCCAGAGGCTGCACAGGAGAGTCTCAGGGACCCCCCAGGC |
| 774 | FR1HK31' | ACTGAAGGTGAATCCAGAGGCTGCACAGGAGAGTCTCAGGGACCCTCCAGGC |
| 775 | FR1HK32' | ACTGAAGGTGAACCCAGAGGCTGCACAGGAGAGTTTCAGGGACCCCCCAGGC |
| 776 | FR1HK33' | ACTGAAGGTGAATCCAGAGGCTGCACAGGAGAGTCTCAGGGACCCCCCAGGC |
| 777 | FR1HK34' | ATCAAAGGTGAATCCAGAGGCTGCACAGGAGAGTCTCAGGGACCTGCCAGGC |
| 778 | FR1HK35' | GCTGATGGAGTAACCAGAGACAGCGCAGGTGAGGGACAGGGTGTCCGAAGGC |
| 779 | FR1HK36' | GCTGATGGAGCCACCAGAGACAGTACAGGTGAGGGACAGGGTCTGTGAAGGC |
| 780 | FR1HK37' | ACTGAAGGACCCACCATAGACAGCGCAGGTGAGGGACAGGGTCTCCGAAGGC |
| 781 | FR1HK38' | GCTGATGGAGCCACCAGAGACAGTGCAGGTGAGGGACAGGGTCTCCGAAGGC |
| 782 | FR1HK39' | ACTGATGGAGCCACCAGAGACAGTGCAGGTGAGGGACAGGGTCTCCGAAGGC |
| 783 | FR1HK40' | ACTGATGGAGCCACCAGAGACAGTGCAGGTGAGGGACAGGGTCTCCGAAGGC |
| 784 | FR1HK41' | GCTGACGGAGCCACCAGAGACAGTGCAGGTGAGGGACAGGGTCTCCGAAGGC |
| 785 | FR1HK42' | GGTAAAGCTGTATCCAGAACCCTTACAGGAGATCTTCAGAGACTCCCCGGGC |
| 786 | FR1HK43' | AGAGACACTGTCCCCGGAGATGGCACAGGTGAGTGAGAGGGTCTGCGAGGGC |
| 787 | FR1HK44' | GGTGAAACTGTAACCAGAAGCCTTGCAGGAGACCTTCACTGAGGCCCCAGGC |

PCR is carried out using the following oligonucleotide combinations (44 in total): FR1HK1/FR1HK1', FR1HK2/FR1HK2', FR1HK3/FR1HK3', FR1HK4/FR1HK4', FR1HK5/FR1HK5', FR1HK6/FR1HK6', FR1HK7/FR1HK7', FR1HK8/FR1HK8', FR1HK9/FR1HK9', FR1HK10/FR1HK10', FR1HK11/FR1HK11', FR1HK12/FR1HK12', FR1HK13/FR1HK13', FR1HK14/FR1HK14', FR1HK15/FR1HK15', FR1HK16/FR1HK16', FR1HK17/FR1HK17', FR1HK18/FR1HK18', FR1HK19/FR1HK19', FR1HK20/FR1HK20', FR1HK21/FR1HK21', FR1HK22/FR1HK22', FR1HK23/FR1HK23', FR1HK24/FR1HK24', FR1HK25/FR1HK25', FR1HK26/FR1HK26', FR1HK27/FR1HK27', FR1HK28/FR1HK28', FR1HK29/FR1HK29', FR1HK30/FR1HK31', FR1HK32/FR1HK32', FR1HK33/FR1HK33', FR1HK34/FR1HK34', FR1HK35/FR1HK35', FR1HK36/FR1HK36', FR1HK37/FR1HK37', FR1HK38/FR1HK38', FR1HK39/FR1HK39', FR1HK40/FR1HK40', FR1HK41/FR1HK41', FR1HK42/FR1HK42', FR1HK43/FR1HK43', or FR1HK44/FR1HK44'. The pooling of the PCR products generates sub-bank 5.

By way of example but not limitation, the construction of heavy chain FR2 sub-bank (according to Kabat definition) is carried out using the Polymerase Chain Reaction by overlap extension using the oligonucleotides listed in Table 22 and Table 23 (all shown in the 5' to 3' orientation, name followed by sequence):

**Table 22. Heavy Chain FR2 (Kabat Definition) Forward Primers (for Sub-Bank 6):**

| | | |
|---|---|---|
| 788 | FR2HK1 | TGGGTGCGACAGGCCCCTGGACAAGGGCTTG |
| 789 | FR2HK2 | TGGGTGCGACAGGCCCCTGGACAAGGGCTTG |
| 790 | FR2HK3 | TGGGTGCGACAGGCTCCTGGAAAAGGGCTTG |
| 791 | FR2HK4 | TGGGTGCGCCAGGCCCCCGGACAAAGGCTTG |
| 792 | FR2HK5 | TGGGTGCGACAGGCCCCCGGACAAGCGCTTG |
| 793 | FR2HK6 | TGGGTGCGACAGGCCCCTGGACAAGGGCTTG |
| 794 | FR2HK7 | TGGGTGCGACAGGCTCGTGGACAACGCCTTG |
| 795 | FR2HK8 | TGGGTGCGACAGGCCCCTGGACAAGGGCTTG |
| 796 | FR2HK9 | TGGGTGCGACAGGCCACTGGACAAGGGCTTG |
| 797 | FR2HK10 | TGGATCCGTCAGCCCCCAGGGAAGGCCCTGG |
| 798 | FR2HK11 | TGGATCCGTCAGCCCCCAGGAAAGGCCCTGG |
| 799 | FR2HK12 | TGGATCCGTCAGCCCCCAGGGAAGGCCCTGG |
| 800 | FR2HK13 | TGGATCCGCCAGGCTCCAGGGAAGGGGCTGG |
| 801 | FR2HK14 | TGGGTCCGCCAAGCTACAGGAAAAGGTCTGG |
| 802 | FR2HK15 | TGGGTCCGCCAGGCTCCAGGGAAGGGGCTGG |
| 803 | FR2HK16 | TGGGCCCGCAAGGCTCCAGGAAAGGGGCTGG |
| 804 | FR2HK17 | TGGGTCCGCCAAGCTCCAGGGAAGGGGCTGG |
| 805 | FR2HK18 | TGGGTCCGCCAGGCTCCAGGGAAGGGGCTGG |
| 806 | FR2HK19 | TGGGTCCGCCAGGCTCCAGGGAAGGGGCTGG |
| 807 | FR2HK20 | TGGGTCCGCCAGGCTCCAGGCAAGGGGCTGG |
| 808 | FR2HK21 | TGGGTCCGCCAGGCTCCAGGCAAGGGGCTGG |
| 809 | FR2HK22 | TGGGTCCATCAGGCTCCAGGAAAGGGGCTGG |
| 810 | FR2HK23 | TGGATCCGCCAGGCTCCAGGGAAGGGGCTGG |
| 811 | FR2HK24 | TGGGTCCGTCAAGCTCCGGGGAAGGGTCTGG |
| 812 | FR2HK25 | TGGGTCCGCCAGGCTCCAGGGAAGGGGCTGG |
| 813 | FR2HK26 | TGGTTCCGCCAGGCTCCAGGGAAGGGGCTGG |
| 814 | FR2HK27 | TGGGTCCGCCAGGCTCCAGGGAAGGGGCTGG |
| 815 | FR2HK28 | TGGGTCCGCCAGGCTCCAGGGAAGGGACTGG |
| 816 | FR2HK29 | TGGGTCCGCCAGGCTCCAGGGAAGGGGCTGG |
| 817 | FR2HK30 | TGGGTCCGCCAGGCTCCAGGGAAGGGGCTGG |
| 818 | FR2HK31 | TGGGTCCGCCAGGCTCCAGGGAAGGGGCTGG |
| 819 | FR2HK32 | TGGGTCCGCCAGGCTTCCGGGAAAGGGCTGG |
| 820 | FR2HK33 | TGGGTCCGCCAAGCTCCAGGGAAGGGGCTGG |
| 821 | FR2HK34 | TGGGTCCGGCAAGCTCCAGGGAAGGGCCTGG |
| 522 | FR2HK35 | TGGATCCGGCAGCCCCCAGGGAAGGGACTGG |
| 823 | FR2HK36 | TGGATCCGCCAGCACCCAGGGAAGGGCCTGG |
| 824 | FR2HK37 | TGGATCCGCCAGCCCCCAGGGAAGGGGCTGG |
| 825 | FR2HK38 | TGGATCCGCCAGCCCCCAGGGAAGGGGCTGG |
| 826 | FR2HK39 | TGGATCCGGCAGCCCGCCGGGAAGGGACTGG |
| 827 | FR2HK40 | TGGATCCGGCAGCCCCCAGGGAAGGGACTGG |
| 828 | FR2HK41 | TGGATCCGGCAGCCCCCAGGGAAGGGACTGG |
| 829 | FR2HK42 | TGGGTGCGCCAGATGCCCGGGAAAGGCCTGG |
| 830 | FR2HK43 | TGGATCAGGCAGTCCCCATCGAGAGGCCTTG |
| 831 | FR2HK44 | TGGGTGCCACAGGCCCCTGGACAAGGGCTTG |

**Table 23. Heavy Chain FR2 (Kabat Definition) Reverse Primers (for Sub-Bank 6):**

| | | |
|---|---|---|
| 832 | FR2HK1' | TCCCATCCACTCAAGCCCTTGTCCAGGGGCCT |
| 833 | FR2HK2' | TCCCATCCACTCAAGCCCTTGTCCAGGGGCCT |
| 834 | FR2HK3' | TCCCATCCACTCAAGCCCTTTTCCAGGAGCCT |
| 835 | FR2HK4' | TCCCATCCACTCAAGCCTTTGTCCGGGGGCCT |
| 836 | FR2HK5' | TCCCATCCACTCAAGCGCTTGTCCGGGGGCCT |
| 837 | FR2HK6' | TCCCATCCACTCAAGCCCTTGTCCAGGGGCCT |
| 838 | FR2HK7' | TCCTATCCACTCAAGGCGTTGTCCACGAGCCT |
| 839 | FR2HK8' | TCCCATCCACTCAAGCCCTTGTCCAGGGGCCT |
| 840 | FR2HK9' | TCCCATCCACTCAAGCCCTTGTCCAGTGGCCT |
| 841 | FR2HK10' | TGCAAGCCACTCCAGGGCCTTCCCTGGGGGCT |
| 842 | FR2HK11' | TGCAAGCCACTCCAGGGCCTTTCCTGGGGGCT |
| 843 | FR2HK12' | TGCAAGCCACTCCAGGGCCTTCCCTGGGGGCT |
| 844 | FR2HK13' | TGAAACCCACTCCAGCCCCTTCCCTGGAGCCT |
| 845 | FR2HK14' | TGAGACCCACTCCAGACCTTTTCCTGTAGCTT |
| 846 | FR2HK15' | GCCAACCCACTCCAGCCCCTTCCCTGGAGCCT |
| 847 | FR2HK16' | CGATACCCACTCCAGCCCCTTTCCTGGAGCCT |
| 848 | FR2HK17' | AGAGACCCACTCCAGCCCCTTCCCTGGAGCTT |
| 849 | FR2HK18' | TGAGACCCACTCCAGCCCCTTCCCTGGAGCCT |
| 850 | FR2HK19' | TGAGACCCACTCCAGCCCCTTCCCTGGAGCCT |
| 851 | FR2HK20' | TGCCACCCACTCCAGCCCCTTGCCTGGAGCCT |
| 852 | FR2HK21' | TGCCACCCACTCCAGCCCCTTGCCTGGAGCCT |
| 853 | FR2HK22' | CGATACCCACTCCAGCCCCTTTCCTGGAGCCT |
| 854 | FR2HK23' | TGAGACCCACTCCAGCCCCTTCCCTGGAGCCT |
| 855 | FR2HK24' | AGAGACCCACTCCAGACCCTTCCCCGGAGCTT |
| 856 | FR2HK25' | TGAAACCCACTCCAGCCCCTTCCCTGGAGCCT |
| 857 | FR2HK26' | ACCTACCCACTCCAGCCCCTTCCCTGGAGCCT |
| 858 | FR2HK27' | TGAGACCCACTCCAGCCCCTTCCCTGGAGCCT |
| 859 | FR2HK28' | TGAAACATATTCCAGTCCCTTCCCTGGAGCCT |
| 860 | FR2HK29' | TGAGACCCACTCCAGCCCCTTCCCTGGAGCCT |
| 861 | FR2HK30 | GGCCACCCACTCCAGCCCCTTCCCTGGAGCCT |
| 862 | FR2HK31' | GCCAACCCACTCCAGCCCCTTCCCTGGAGCCT |
| 863 | FR2HK32' | GCCAACCCACTCCAGCCCTTTCCCGGAAGCCT |
| 864 | FR2HK33' | TGAGACCCACACCAGCCCCTTCCCTGGAGCTT |
| 865 | FR2HK34' | TGAGACCCACTCCAGGCCCTTCCCTGGAGCTT |
| 866 | FR2HK35' | CCCAATCCACTCCAGTCCCTTCCCTGGGGGCT |
| 867 | FR2HK36' | CCCAATCCACTCCAGGCCCTTCCCTGGGTGCT |
| 868 | FR2HK37' | CCCAATCCACTCCAGCCCCTTCCCTGGGGGCT |
| 869 | FR2HK38' | CCCAATCCACTCCAGCCCCTTCCCTGGGGGCT |
| 870 | FR2HK39' | CCCAATCCACTCCAGTCCCTTCCCGGCGGGCT |
| 871 | FR2HK40' | CCCAATCCACTCCAGTCCCTTCCCTGGGGGCT |
| 872 | FR2HK41' | CCCAATCCACTCCAGTCCCTTCCCTGGGGGCT |
| 873 | FR2HK42' | CCCCATCCACTCCAGGCCTTTCCCGGGCATCT |
| 874 | FR2HK43' | TCCCAGCCACTCAAGGCCTCTCGATGGGGACT |
| 875 | FR2HK44' | TCCCATCCACTCAAGCCCTTGTCCAGGGGCCT |

PCR is carried out using the following oligonucleotide combinations (44 in total): FR2HK1/FR2HK1', FR2HK2/FR2HK2', FR2HK3/FR2HK3', FR2HK4/FR2HK4', FR2HK5/FR2HK5', FR2HK6/FR2HK6', FR2HK7/FR2HK7', FR2HK8/FR2HK8', FR2HK9/FR2HK9', FR2HK10/FR2HK10', FR2HK11/FR2HK11', FR2HK12/FR2HK12', FR2HK13/FR2HK13', FR2HK14/FR2HK14', FR2HK15/FR2HK15', FR2HK16/FR2HK16', FR2HK17/FR2HK17', FR2HK18/FR2HK18', FR2HK19/FR2HK19', FR2HK20/FR2HK20', FR2HK21/FR2HK21', FR2HK22/FR2HK22', FR2HK23/FR2HK23', FR2HK24/FR2HK24', FR2HK25/FR2HK25', FR2HK26/FR2HK26', FR2HK27/FR2HK27', FR2HK28/FR2HK28', FR2HK29/FR2HK29', FR2HK30/FR2HK31', FR2HK32/FR2HK32', FR2HK33/FR2HK33', FR2HK34/FR2HK34', FR2HK35/FR2HK35', FR2HK36/FR2HK36', FR2HK37/FR2HK37', FR2HK38/FR2HK38', FR2HK39/FR2HK39', FR2HK40/FR2HK40', FR2HK41/FR2HK41', FR2HK42/FR2HK42', FR2HK43/FR2HK43', or FR2HK44/FR2HK44'. The pooling of the PCR products generates sub-bank 6.

By way of example but not limitation, the construction of heavy chain FR3 sub-bank (according to Kabat definition) is carried out using the Polymerase Chain Reaction by overlap extension using the oligonucleotides listed in Table 24 and Table 25 (all shown in the 5' to 3' orientation, name followed by sequence):

**Table 24. Heavy Chain FR3 (Kabat Definition) Forward Primers (for Sub-Bank 7):**

| | | |
|---|---|---|
| 876 | FR3HK1 | AGAGTCACCATGACCACAGACACATCCACGAGCACAGCCTACATGGAGCTGAGGAGCCTGAGATCTG |
| 877 | FR3HK2 | AGGGTCACCATGACCAGGGACACGTCCATCAGCACAGCCTACATGGAGCTGAGCAGGCTGAGATCTG |
| 878 | FR3HK3 | AGAGTCACCATGACCGAGGACACATCTACAGACACAGCCTACATGGAGCTGAGCAGCCTGAGATCTG |
| 879 | FR3HK4 | AGAGTCACCATTACCAGGGACACATCCGCGAGCACAGCCTACATGGAGCTGAGCAGCCTGAGATCTG |
| 880 | FR3HK5 | AGAGTCACCATTACCAGGGACAGGTCTATGAGCACAGCCTACATGGAGCTGAGCAGCCTGAGATCTG |
| 881 | FR3HK6 | AGAGTCACCATGACCAGGGACACGTCCACGAGCACAGTCTACATGGAGCTGAGCAGCCTGAGATCTG |
| 882 | FR3HK7 | AGAGTCACCATTACCAGGGACATGTCCACAAGCACAGCCTACATGGAGCTGAGCAGCCTGAGATCCG |
| 883 | FR3HK8 | AGAGTCACGATTACCGCGGACAAATCCACGAGCACAGCCTACATGGAGCTGAGCAGCCTGAGATCTG |
| 884 | FR3HK9 | AGAGTCACCATGACCAGGAACACCTCCATAAGCACAGCCTACATGGAGCTGAGCAGCCTGAGATCTG |
| 885 | FR3HK10 | AGGCTCACCATCTCCAAGGACACCTCCAAAAGCCAGGTGGTCCTTACCATGACCAACATGGACCCTG |
| 886 | FR3HK11 | AGGCTCACCATCACCAAGGACACCTCCAAAAACCAGGTGGTCCTACAATGACCAACATGGACCCTG |
| 887 | FR3HK12 | AGGCTCACCATCTCCAAGGACACCTCCAAAACCAGGTGGTCCTTACAATGACCAACATGGACCCTG |
| 888 | FR3HK13 | CGATTCACCATCTCCAGGGACAACGCCAAGAACTCACTGTATCTGCAAATGAACAGCCTGAGAGCCG |
| 889 | FR3HK14 | CGATTCACCATCTCCAGAGAAAATGCCAAGAACTCCTTGTATCTTCAAATGAACAGCCTGAGAGCCG |
| 890 | FR3HK15 | AGATTCACCATCTCAAGAGATGATTCAAAAAACACGCTGTATCTGCAAATGAACAGCCTGAAAACCG |
| 891 | FR3HK16 | CGATTCATCATCTCCAGAGACAATTCCAGGAACTCCCTGTATCTGCAAAAGAACAGACGGAGAGCCG |
| 892 | FR3HK17 | CGATTCACCATCTCCAGAGACAACGCCAAGAACTCCCTGTATCTGCAAATGAACAGTCTGAGAGCCG |
| 893 | FR3HK18 | CGATTCACCATCTCCAGAGACAACGCCAAGAACTCACTGTATCTGCAAATGAACAGCCTGAGAGCCG |
| 894 | FR3HK19 | CGGTTCACCATCTCCAGAGACAATTCCAAGAACACGCTGTATCTGCAAATGAACAGCCTGAGAGCCG |
| 895 | FR3HK20 | CGATTCACCATCTCCAGAGACAATTCCAAGAACACGCTGTATCTGCAAATGAACAGCCTGAGAGCTG |
| 396 | FR3HK21 | CGATTCACCATCTCCAGAGACAATTCCAAGAACACGCTGTATCTGCAAATGAACAGCCTGAGAGCCG |
| 897 | FR3HK22 | CGATTCATCATCTCCAGAGACAATTCCAGGAACACCCTGTATCTGCAAACGAATAGCCTGAGGGCCG |
| 898 | FR3HK23 | AGATTCACCATCTCCAGAGACAATTCCAAGAACACGCTGTATCTTCAAATGAACAACCTGAGAGCTG |
| 899 | FR3HK24 | CGATTCACCATCTCCAGAGACAACAGCAAAAACTCCCTGTATCTGCAAATGAACAGTCTGAGAACTG |
| 900 | FR3HK25 | CGATTCACCATCTCCAGAGACAATGCCAAGAACTCACTGTATCTCCAAATGAACAGCCTGAGAGACG |
| 901 | FR3HK26 | AGATTCACCATCTCAAGAGATGATTCCAAAAGCATCGCCTATCTGCAAATGAACAGCCTGAAAACCG |
| 902 | FR3HK27 | CGATTCACCATCTCCAGAGACAATTCCAAGAACACGCTGTATCTTCAAATGAACAGCCTGAGAGCCG |
| 903 | FR3HK28 | AGATTCACCATCTCCAGAGACAATTCCAAGAACACGCTGTATCTTCAAATGGGCAGCCTGAGAGCTG |
| 904 | FR3HK29 | CGATTCACCATCTCCAGAGACAATTCCAAGAACACGCTGTATCTTCAAATGAACAGCCTGAGAGCTG |
| 905 | FR3HK30 | CGATTCACCATCTCCAGAGACAACGCCAAGAACTCACTGTATCTGCAAATGAACAGCCTGAGAGCCG |
| 906 | FR3HK31 | AGATTCACCATCTCAAGAGATGATTCAAAGAACTCACTGTATCTGCAAATGAACAGCCTGAAAACCG |
| 907 | FR3HK32 | AGGTTCACCATCTCCAGAGATGATTCAAAGAACACGGCGTATCTGCAAATGAACAGCCTGAAAACCG |
| 908 | FR3HK33 | CGATTCACCATCTCCAGAGACAACGCCAAGAACACGCTGTATCTGCAAATGAACAGTCTGAGAGCCG |
| 909 | FR3HK34 | CGATTCACCATCTCCAGAGACAACGCCAAGAACTCCCTGTATCTGCAAATGAACAGTCTGAGAGCTG |
| 910 | FR3HK35 | CGAGTCACCATGTCAGTAGACACGTCCAAGAACCAGTTCTCCCTGAAGCTGAGCTCTGTGACCGCCG |
| 911 | FR3HK36 | CGAGTTACCATATCAGTAGACACGTCTAAGAACCAGTTCTCCCTGAAGCTGAGCTCTGTGACTGCCG |
| 912 | FR3HK37 | CGAGTCACCATATCAGTAGACACGTCCAAGAACCAGTTCTCCCTGAAGCTGAGCTCTGTGACCGCCG |
| 913 | FR3HK38 | CGAGTCACCATATCCGTAGACACGTCCAAGAACCAGTTCTCCCTGAAGCTGAGCTCTGTGACCGCCG |
| 914 | FR3HK39 | CGAGTCACCATGTCAGTAGACACGTCCAAGAACCAGTTCTCCCTGAAGCTGAGCTCTGTGACCGCCG |
| 915 | FR3HK40 | CGAGTCACCATATCAGTAGACACGTCCAAGAACCAGTTCTCCCTGAAGCTGAGCTCTGTGACCGCTG |
| 916 | FR3HK41 | CGAGTCACCATATCAGTAGACACGTCCAAGAACCAGTTCTCCCTGAAGCTGAGCTCTGTGACCGCTG |
| 917 | FR3HK42 | CAGGTCACCATCTCAGCCGACAAGTCCATCAGCACCGCCTACCTGCAGTGGAGCAGCCTGAAGGCCT |
| 918 | FR3HK43 | CGAATAACCATCAACCCAGACACATCCAAGAACCAGTTCTCCCTGCAGCTGAACTCTGTGACTCCCG |
| 919 | FR3HK44 | CGGTTTGTCTTCTCCATGGACACCTCTGCCAGCACAGCATACCTGCAGATCAGCAGCCTAAAGGCTG |

**Table 25. Heavy Chain FR3 (Kabat Definition) Reverse Primers (for Sub-Bank 7):**

| | | |
|---|---|---|
| 920 | FR3HK1' | TCTCGCACAGTAATACACGGCCGTGTCGTCAGATCTCAGGCTCCTCAGCT |
| 921 | FR3HK2' | TCTCGCACAGTAATACACGGCCGTGTCGTCAGATCTCAGCCTGCTCAGCT |
| 922 | FR3HK3' | TGTTGCACAGTAATACACGGCCGTGTCCTCAGATCTCAGGCTGCTCAGCT |
| 923 | FR3HK4' | TCTCGCACAGTAATACACAGCCATGTCCTCAGATCTCAGGCTGCTCAGCT |
| 924 | FR3HK5' | TCTTGCACAGTAATACATGGCTGTGTCCTCAGATCTCAGGCTGCTCAGCT |
| 925 | FR3HK6' | TCTCGCACAGTAATACACGGCCGTGTCCTCAGATCTCAGGCTGCTCAGCT |
| 926 | FR3HK7' | TGCCGCACAGTAATACACGGCCGTGTCCTCGGATCTCAGGCTGCTCAGCT |
| 927 | FR3HK8' | TCTCGCACAGTAATACACGGCCGTGTCCTCAGATCTCAGGCTGCTCAGCT |
| 928 | FR3HK9' | TCTCGCACAGTAATACACGGCCGTGTCCTCAGATCTCAGGCTGCTCAGCT |
| 929 | FR3HK10' | CCGTGCACAGTAATATGTGGCTGTGTCCACAGGGTCCATGTTGGTCATGG |
| 930 | FR3HK11' | GTGTGCACAGTAATATGTGGCTGTGTCCACAGGGTCCATGTTGGTCATTG |
| 931 | FR3HK12' | CCGTGCACAATAATACGTGGCTGTGTCCACAGGGTCCATGTTGGTCATTG |
| 932 | FR3HK13' | TCTCGCACAGTAATACACGGCCGTGTCCTCGGCTCTCAGGCTGTTCATTT |
| 933 | FR3HK14' | TCTTGCACAGTAATACACAGCCGTGTCCCCGGCTCTCAGGCTGTTCATTT |
| 934 | FR3HK15' | TGTGGTACAGTAATACACGGCTGTGTCCTCGGTTTTCAGGCTGTTCATTT |
| 935 | FR3HK16' | TCTCACACAGTAATACACAGCCATGTCCTCGGCTCTCCGTCTGTTCTTTT |
| 936 | FR3HK17' | TCTCGCACAGTGATACAAGGCCGTGTCCTCGGCTCTCAGACTGTTCATTT |
| 937 | FR3HK18' | TCTCGCACAGTAATACACAGCCGTGTCCTCGGCTCTCAGGCTGTTCATTT |
| 938 | FR3HK19' | TTTCGCACAGTAATATACGGCCGTGTCCTCGGCTCTCAGGCTGTTCATTT |
| 939 | FR3HK20' | TCTCGCACAGTAATACACAGCCGTGTCCTCAGCTCTCAGGCTGTTCATTT |
| 940 | FR3HK21' | CTCGCACAGTAATACACAGCCGTGTCCTCGGCTCTCAGGCTGTTCATTT |
| 941 | FR3HK22' | TCTCACACAGTAATACACAGCCGTGTCCTCGGCCCTCAGGCTATTCGTTT |
| 942 | FR3HK23' | TCTGGCACAGTAATACACGGCCGTGCCCTCAGCTCTCAGGTTGTTCATTT |
| 943 | FR3HK24' | TTTTGCACAGTAATACAAGGCGGTGTCCTCAGTTCTCAGACTGTTCATTT |
| 944 | FR3HK25' | TCTCGCACAGTAATACACAGCCGTGTCCTCGTCTCTCAGGCTGTTCATTT |
| 945 | FR3HK26' | TCTAGTACAGTAATACACGGCTGTGTCCTCGGTTTTCAGGCTGTTCATTT |
| 946 | FR3HK27' | TCTCGCACAGTAATACACGGCCGTGTCCTCGGCTGTCAGGCTGTTCATTT |
| 947 | FR3HK28' | TCTCGCACAGTAATACACAGCCATGTCCTCAGCTCTCAGGCTGCCCATTT |
| 948 | FR3HK29' | TCTCGCACAGTAATACACAGCCGTGTCCTCAGCTCTCAGGCTGTTCATTT |
| 949 | FR3HK30' | TCTCGCACAGTAATACACAGCCGTGTCGTCGGCTCTCAGGCTGTTCATTT |
| 950 | FR3HK31' | TCTAGCACAGTAATACACGGCCGTGTCCTCGGTTTTCAGGCTGTTCATTT |
| 951 | FR3HK32' | TCTAGTACAGTAATACACGGCCGTGTCCTCGGTTTTCAGGCTGTTCATTT |
| 952 | FR3HK33' | TCTTGCACAGTAATACACAGCCGTGTCCTCGGCTCTCAGACTGTTCATTT |
| 953 | FR3HK34' | TTTTGCACAGTAATACAAGGCCGTGTCCTCAGCTCTCAGACTGTTCATTT |
| 954 | FR3HK35' | TCTCGCACAGTAATACACGGCCGTGTCCACGGCGGTCACAGAGCTCAGCT |
| 955 | FR3HK36' | TCTCGCACAGTAATACACGGCCGTGTCCGCGGCAGTCACAGAGCTCAGCT |
| 956 | FR3HK37' | TCTCGCACAGTAATACACAGCCGTGTCCGCGGCGGTCACAGAGCTCAGGT |
| 957 | FR3HK38' | TCTCGCACAGTAATACACAGCCGTGTCTGCGGCGGTCACAGAGCTCAGCT |
| 958 | FR3HK39' | TCTCGCACAGTAATACACGGCCGTGTCCGCGGCGGTCACAGAGCTCAGCT |
| 959 | FR3HK40' | TCTCGCACAGTAATACACGGCCGTGTCCGCAGCGGTCACAGAGCTCAGCT |
| 960 | FR3HK41' | TCTCGCACAGTAATACACGGCCGTGTCCGCAGCGGTCACAGAGCTCAGCT |
| 961 | FR3HK42' | TCTCGCACAGTAATACATGGCGGTGTCCGAGGCCTTCAGGCTGCTCCACT |
| 962 | FR3HK43' | TCTTGCACAGTAATACACAGCCGTGTCCTCGGGAGTCACAGAGTTCAGCT |
| 963 | FR3HK44' | TCTCGCACAGTAATACATGGCCATGTCCTCAGCCTTTAGGCTGCTGATCT |

PCR is carried out using the following oligonucleotide combinations (44 in total): FR3HK1/FR3HK1', FR3HK2/FR3HK2', FR3HK3/FR3HK3', FR3HK4/FR3HK4', FR3HK5/FR3HK5', FR3HK6/FR3HK6', FR3HK7/FR3HK7', FR3HK8/FR3HK8', FR3HK9/FR3HK9', FR3HK10/FR3HK10', FR3HK11/FR3HK11', FR3HK12/FR3HK12', FR3HK13/FR3HK13', FR3HK14/FR3HK14', FR3HK15/FR3HK15', FR3HK16/FR3HK16', FR3HK17/FR3HK17', FR3HK18/FR3HK18', FR3HK19/FR3HK19', FR3HK20/FR3HK20', FR3HK21/FR3HK21', FR3HK22/FR3HK22', FR3HK23/FR3HK23', FR3HK24/FR3HK24', FR3HK25/FR3HK25', FR3HK26/FR3HK26', FR3HK27/FR3HK27', FR3HK28/FR3HK28', FR3HK29/FR3HK29', FR3HK30/FR3HK31', FR3HK32/FR3HK32', FR3HK33/FR3HK33', FR3HK34/FR3HK34', FR3HK35/FR3HK35', FR3HK36/FR3HK36', FR3HK37/FR3HK37', FR3HK38/FR3HK38', FR3HK39/FR3HK39', FR3HK40/FR3HK40', FR3HK41/FR3HK41', FR3HK42/FR3HK42', FR3HK43/FR3HK43', or FR3HK44/FR3HK44'. The pooling of the PCR products generates sub-bank 7.

By way of example but not limitation, the construction of heavy chain FR4 sub-bank is carried out using the Polymerase Chain Reaction by overlap extension using the oligonucleotides listed in Table 26 and Table 27 (all shown in the 5' to 3' orientation, name followed by sequence):

**Table 26. Heavy Chain FR4 Forward Primers (for Sub-Bank 11):**

| | | |
|---|---|---|
| 964 | FR4H1 | TGGGGCCAGGGCACCCTGGTCACCGTCTCCTCA |
| 965 | FR4H2 | TGGGGCCGTGGCACCCTGGTCACTGTCTCCTCA |
| 966 | FR4H3 | TGGGGCCAAGGGACAATGGTCACCGTCTCTTCA |
| 967 | FR4H4 | TGGGGCCAAGGAACCCTGGTCACCGTCTCCTCA |
| 968 | FR4H5 | TGGGGCCAAGGAACCCTGGTCACCGTCTCCTCA |
| 969 | FR4H6 | TGGGGGCAAGGGACCACGGTCACCGTCTCCTCA |

**Table 27. Heavy Chain FR4 Reverse Primers (for Sub-Bank 11):**

| | | |
|---|---|---|
| 970 | FR4H1' | TGAGGAGACGGTGACCAGGGTGCCCTGGCCCCA |
| 971 | FR4H2' | TGAGGAGACAGTGACCAGGGTGCCACGGCCCCA |
| 972 | FR4H3' | TGAAGAGACGGTGACCATTGTCCCTTGGCCCCA |
| 973 | FR4H4' | TGAGGAGACGGTGACCAGGGTTCCTTGGCCCCA |
| 974 | FR4H5' | TGAGGAGACGGTGACCAGGGTTCCTTGGCCCCA |
| 975 | FR4H6' | TGAGGAGACGGTGACCGTGGTCCCTTGCCCCCA |

PCR is carried out using the following oligonucleotide combinations (6 in total): FR4H1/FR4H1', FR4H2/FR4H2', FR4H3/FR4H3', FR4H4/FR4H4', FR4H5/FR4H5', or FR4H6/FR4H6'. The pooling of the PCR products generates sub-bank 11.

In some embodiments, heavy chain FR sub-banks 8, 9, 10 and 11 are constructed wherein sub-bank 8 comprises nucleic acids, each of which encodes a heavy chain FR1; sub-bank 9 comprises nucleic acids, each of which encodes a heavy chain FR2; sub-bank 10 comprises nucleic acids, each of which encodes a heavy chain FR3; and sub-bank 11 comprises nucleic acids, each of which encodes a heavy chain FR4, respectively, and wherein the heavy chain FR1, FR2, and FR3 are defined according to Chothia definition for CDR H1 and H2. In some embodiments, the FR sequences are derived from functional human antibody sequences. In other embodiments, the FR sequences are derived from human germline heavy chain sequences.

By way of example but not limitation, the following describes a method of generating 4 heavy chain FR sub-banks using Polymerase Chain Reaction (PCR), wherein human germline heavy chain sequences are used as templates. Heavy chain FR sub-banks 7, 8 and 9 (encoding FR1, 2, 3 respectively) encompass 44 human germline heavy chain sequences (VH1-18, VH1-2, VH1-24, VH1-3, VH1-45, VH1-46, VH1-58, VH1-69, VH1-8, VH2-26, VH2-5, VH2-70, VH3-11, VH3-13, VH3-15, VH3-16, VH3-20, VH3-21, VH3-23, VH3-30, VH3-33, VH3-35, VH3-38, VH3-43, VH3-48, VH3-49, VH3-53, VH3-64, VH3-66, VH3-7, VH3-72, VH3-73, VH3-74, VH3-9, VH4-28, VH4-31, VH4-34, VH4-39, VH4-4, VH4-59, VH4-61, VH5-51, VH6-1 and VH7-81). See Matsuda et al., 1998, J. Exp. Med., 188:1973-1975. The sequences are summarized at the NCBI website: www.ncbi.nlm.nih.gov/igblast/showGermline.cgi?organism=human&chainType=VH&seqT ype=nucleotide. Sub-bank 11 (encodes FR4) is the same sub-bank 11 as described above.

By way of example but not limitation, the construction of heavy chain FR1 sub-bank (according to Chothia definition) is carried out using the Polymerase Chain Reaction by overlap extension using the oligonucleotides listed in Table 28 and Table 29 (all shown in the 5' to 3' orientation, name followed by sequence):

**Table 28. Heavy Chain FR1 (Chothia Definition) Forward Primers (for Sub-Bank 8):**

| | | |
|---|---|---|
| 976 | FR1HC1 | CAGGTTCAGCTGGTGCAGTCTGGAGCTGAGGTGAAGAAGCCTGGGGCCTCA |
| 977 | FR1HC2 | CAGGTGCAGCTGGTGCAGTCTGGGGCTGAGGTGAAGAAGCCTGGGGCCTCA |
| 978 | FR1HC3 | CAGGTCCAGCTGGTACAGTCTGGGGCTGAGGTGAAGAAGCCTGGGGCCTCA |
| 979 | FR1HC4 | CAGGTTCAGCTGGTGCAGTCTGGGGCTGAGGTGAAGAAGCCTGGGGCCTCA |
| 980 | FR1HC5 | CAGATGCAGCTGGTGCAGTCTGGGGCTGAGGTGAAGAAGACTGGGTCCTCA |
| 981 | FR1HC6 | CAGGTGCAGCTGGTGCAGTCTGGGGCTGAGGTGAAGAAGCCTGGGGCCTCA |
| 982 | FR1HC7 | CAAATGCAGCTGGTGCAGTCTGGGCCTGAGGTGAAGAAGCCTGGGACCTCA |
| 983 | FR1HC8 | CAGGTGCAGCTGGTGCAGTCTGGGGCTGAGGTGAAGAAGCCTGGGTCCTCG |
| 984 | FR1HC9 | CAGGTGCAGCTGGTGCAGTCTGGGGCTGAGGTGAAGAAGCCTGGGGCCTCA |
| 985 | FR1HC10 | CAGGTCACCTTGAAGGAGTCTGGTCCTGTGCTGGTGAAACCCACAGAGACC |
| 986 | FR1HC11 | CAGATCACCTTGAAGGAGTCTGGTCCTACGCTGGTGAAACCCACACAGACC |
| 987 | FR1HC12 | CAGGTCACCTTGAGGGAGTCTGGTCCTGCGCTGGTGAAACCCACACAGACC |
| 988 | FR1HC13 | CAGGTGCAGCTGGTGGAGTCTGGGGGAGGCTTGGTCAAGCCTGGAGGGTCC |
| 989 | FR1HC14 | GAGGTGCAGCTGGTGGAGTCTGGGGGAGGCTTGGTACAGCCTGGGGGGTCC |
| 990 | FRHC15 | GAGGTGCAGCTGGTGGAGTCTGGGGGAGGCTTGGTAAAGCCTGGGGGGTCC |
| 991 | FR1HC16 | GAGGTGCAGCTGGTGGAGTCTGGGGGAGGCTTGGTACAGCCTGGGGGCTCC |
| 992 | FR1HC17 | GAGGTGCAGCTGGTGGAGTCTGGGGGAGGTGTGGTACGGCCTGGGGGGTCC |
| 993 | FR1HC18 | GAGGTGCAGCTGGTGGAGTCTGGGGGAGGCCTGGTCAAGCCTGGGGGGTCC |
| 994 | FR1HC19 | GAGGTGCAGCTGTTGGAGTCTGGGGGAGGCTTGGTACAGCCTGiGGGGGTCC |
| 995 | FR1HC20 | CAGGTGCAGCTGGTGGAGTCTGGGGGAGGCGTGGTCCAGCCTGGGAGGTCC |
| 996 | FR1HC21 | CAGGTGCAGCTGGTGGAGTCTGGGGGAGGCGTGGTCCAGCCTGGGAGGTCC |
| 997 | FR1HC22 | GAGGTGCAGCTGGTGGAGTCTGGGGGAGGCTTGGTACAGCCTGGGGGATCC |
| 998 | FR1HC23 | GAGGTGCAGCTGGTGGAGTCTGGGGGAGGCTTGGTACAGCCTAGGGGGTCC |
| 999 | FR1HC24 | GAAGTGCAGCTGGTGGAGTCTGGGGGAGTCGTGGTACAGCCTGGGGGGTCC |
| 1000 | FR1HC25 | GAGGTGCAGCTGGTGGAGTCTGGGGGAGGCTTGGTACAGCCTGGGGGGTCC |
| 1001 | FR1HC26 | GAGGTGCAGCTGGTGGAGTCTGGGGGAGGCTTGGTACAGCCAGGGCGGTCC |
| 1002 | FR1HC27 | GAGGTGCAGCTGGTGGAGTCTGGAGGAGGCTTGATCCAGCCTGGGGGGTCC |
| 1003 | FR1HC28 | GAGGTGCAGCTGGTGGAGTCTGGGGAAGGCTTGGTCCAGCCTGGGGGGTCC |
| 1004 | FR1HC29 | GAGGTGCAGCTGGTGGAGTCTGGAGGAGGCTTGATCCAGCCTGGGGGGTCC |
| 1005 | FR1HC30 | GAGGTGCAGCTGGTGGAGTCTGGGGGAGGCTTGGTCCAGCCTGGGGGGTCC |
| 1006 | FR1HC31 | GAGGTGCAGCTGGTGGAGTCTGGGGGAGGCTTGGTCCAGCCTGGAGGGTCC |
| 1007 | FR1HC32 | GAGGTGCAGCTGGTGGAGTCCGGGGGAGGCTTGGTCCAGCCTGGGGGGTCC |
| 1008 | FR1HC33 | GAGGTGCAGCTGGTGGAGTCCGGGGGAGGCTTAGTTCAGCCTGGGGGGTCC |
| 1009 | FR1HC34 | GAAGTGCAGCTGGTGGAGTCTGGGGGAGGCTTGGTACAGCCTGGCAGGTCC |
| 1010 | FR1HC35 | CAGGTGCAGCTGCAGGAGTCGGGCCCAGGACTGGTGAAGCCTTCGGACACC |
| 1011 | FR1HC36 | CAGGTGCAGCTGCAGGAGTCGGGCCCAGGACTGGTGAAGCCTTCACAGACC |
| 1012 | FR1HC37 | CAGGTGCAGCTACAGCAGTGGGGCGCAGGACTGTTGAAGCCTTCGGAGACC |
| 1013 | FR1HC38 | CAGCTGCAGCTGCAGGAGTCGGGCCCAGGACTGGTGAAGCCTTCGGAGACC |
| 1014 | FR1HC39 | CAGGTGCAGCTGCAGGAGTCGGGCCCAGGACTGGTGAAGCCTTCGGAGACC |
| 1015 | FR1HC40 | CAGGTGCAGCTGCAGGAGTCGGGCCCAGGACTGGTGAAGCCTTCGGAGACC |
| 1016 | FR1HC41 | CAGGTGCAGCTGCAGGAGTCGGGCCCAGGACTGGTGAAGCCTTCGGAGACC |
| 1017 | FR1HC42 | GAGGTGCAGCTGGTGCAGTCTGGAGCAGAGGTGAAAAAGCCCGGGGAGTCT |
| 1018 | FR1HC43 | CAGGTACAGCTGCAGCAGTCAGGTCCAGGACTGGTGAAGCCCTCGCAGACC |
| 1019 | FR1HC44 | CAGGTGCAGCTGGTGCAGTCTGGCCATGAGGTGAAGCAGCCTGGGGCCTCA |

**Table 29. Heavy Chain FR1 (Chothia Definition) Reverse Primers (for Sub-Bank 8):**

| | | |
|---|---|---|
| 1020 | FR1HC1' | AGAAGCCTTGCAGGAGACCTTCACTGAGGCCCCAGGCTTCTTCAC |
| 1021 | FR1HC2' | AGAAGCCTTGCAGGAGACCTTCACTGAGGCCCCAGGCTTCTTCAC |
| 1022 | FR1HC3' | GGAAACCTTGCAGGAGACCTTCACTGAGGCCCCAGGCTTCTTCAC |
| 1023 | FR1HC4' | AGAAGCCTTGCAGGAAACCTTCACTGAGGCCCCAGGCTTCTTCAC |
| 1024 | FR1HC5' | GGAAGCCTTGCAGGAAACCTTCACTGAGGACCCAGTCTTCTTCAC |
| 1025 | FR1HC6' | AGATGCCTTGCAGGAAACCTTCACTGAGGCCCCAGGCTTCTTCAC |
| 1026 | FR1HC7' | AGAAGCCTTGCAGGAGACCTTCACTGAGGTCCCAGGCTTCTTCAC |
| 1027 | FR1HC8' | AGAAGCCTTGCAGGAGACCTTCACCGAGGACCCAGGCTTCTTCAC |
| 1028 | FR1HC9' | AGAAGCCTTGCAGGAGACCTTCACTGAGGCCCCAGGCTTCTTCAC |
| 1039 | FR1HC10' | AGAGACGGTGCAGGTCAGCGTGAGGGTCTCTGTGGGTTTCACCAG |
| 1030 | FR1HC11' | AGAGAAGGTGCAGGTCAGCGTGAGGGTCTGTGTGGGTTTCACCAG |
| 1031 | FR1HC12' | AGAGAAGGTGCAGGTCAGTGTGAGGGTCTGTGTGGGTTTCACCAG |
| 1032 | FR1HC13' | AGAGGCTGCACAGGAGAGTCTCAGGGACCCTCCAGGCTTGACCAA |
| 1033 | FR1HC14' | AGAGGCTGCACAGGAGAGTCTCAGGGACCCCCCAGGCTGTACCAA |
| 1034 | FR1HC15' | AGAGGCTGCACAGGAGAGTCTAAGGGACCCCCCAGGCTTTACCAA |
| 1035 | FR1HC16' | AGAGGCTGCACAGGAGAGTCTCAGGGACCCCCCAGGCTGTACCAA |
| 1036 | FR1HC17' | AGAGGCTGCACAGGAGAGTCTCAGGGACCCCCCAGGCCGTACCAC |
| 1037 | FR1HC18' | AGAGGCTGCACAGGAGAGTCTCAGGGACCCCCCAGGCTTGACCAG |
| 1038 | FR1HC19' | AGAGGCTGCACAGGAGAGTCTCAGGGACCCCCCAGGCTGTACCAA |
| 1039 | FR1HC20' | AGAGGCTGCACAGGAGAGTCTCAGGGACCTCCCAGGCTGGACCAC |
| 1040 | FR1HC21' | AGACGCTGCACAGGAGAGTCTCAGGGACCTCCCAGGCTGGACCAC |
| 1041 | FR1HC22' | AGAGGCTGCACAGGAGAGTCTCAGGGATCCCCCAGGCTGTACCAA |
| 1042 | FR1HC23' | AGAGGCTGCACAGGAGAGTCTCAGGGACCCCCTAGGCTGTACCAA |
| 1043 | FR1HC24' | AGAGGCTGCACAGGAGAGTCTCAGGGACCCCCCAGGCTGTACCAC |
| 1044 | FR1HC25' | AGAGGCTGCACAGGAGAGTCTCAGGGACCCCCCAGGCTGTACCAA |
| 1045 | FR1HC26' | AGAAGCTGTACAGGAGAGTCTCAGGGACCGCCCTGGCTGTACCAA |
| 1046 | FR1HC27' | AGAGGCTGCACAGGAGAGTCTCAGGGACCCCCCAGGCTGGATCAA |
| 1047 | FR1HC28' | AGAGGCTGCACAGGAGAGTCTCAGGGACCCCCCAGGCTGGACCAA |
| 1048 | FR1HC29' | AGAGGCTGCACAGGAGAGTCTCAGGGACCCCCCAGGCTGGATCAA |
| 1049 | FR1HC30' | AGAGGCTGCACAGGAGAGTCTCAGGGACCCCCCAGGCTGGACCAA |
| 1050 | FR1HC31' | AGAGGCTGCACAGGAGAGTCTCAGGGACCCTCCAGGCTGGACCAA |
| 1051 | FR1HC32' | AGAGGCTGCACAGGAGAGTTTCAGGGACCCCCCAGGCTGGACCAA |
| 1052 | FR1HC33' | AGAGGCTGCACAGGAGAGTCTCAGGGACCCCCCAGGCTGAACTAA |
| 1053 | FR1HC34' | AGAGGCTGCACAGGAGAGTCTCAGGGACCTGCCAGGCTGTACCAA |
| 1054 | FR1HC35' | AGAGACAGCGCAGGTGAGGGACAGGGTGTCCGAAGGCTTCACCAG |
| 1055 | FR1HC36' | AGAGACAGTACAGGTGAGGGACAGGGTCTGTGAAGGCTTCACCAG |
| 1056 | FR1HC37' | ATAGACAGCGCAGGTGAGGGACAGGGTCTCCGAAGGCTTCAACAG |
| 1057 | FR1HC38' | AGAGACAGTGCAGGTGAGGGACAGGGTCTCCGAAGGCTTCACCAG |
| 1058 | FR1HC39' | AGAGACAGTGCAGGTGAGGGACAGGGTCTCCGAAGGCTTCACCAG |
| 1059 | FR1HC40' | AGAGACAGTGCAGGTGAGGGACAGGGTCTCCGAAGGCTTCACCAG |
| 1060 | FR21C41' | AGAGACAGTGCAGGTGAGGGACAGGGTCTCCGAAGGCTTCACCAG |
| 1061 | FR1HC42' | AGAACCCTTACAGGAGATCTTCAGAGACTCCCCGGGCTTTTTCAC |
| 1062 | FR1HC43' | GGAGATGGCACAGGTGAGTGAGAGGGTCTGCGAGGGCTTCACCAG |
| 1063 | FR1HC44' | AGAAGCCTTGCAGGAGACCTTCACTGAGGCCCCAGGCTGCTTCAC |

PCR is carried out using the following oligonucleotide combinations (44 in total): FR1HC1/FR1HC1', FR1HC2/FR1HC2', FR1HC3/FR1HC3', FR1HC4/FR1HC4', FR1HC5/FR1HC5', FR1HC6/FR1HC6', FR1HC7/FR1HC7', FR1HC8/FR1HC8', FR1HC9/FR1HC9', FR1HC10/FR1HC10', FR1HC11/FR1HC11', FR1HC12/FR1HC12', FR1HC13/FR1HC13', FR1HC14/FR1HC14', FR1HC15/FR1HC15', FR1HC16/FR1HC16', FR1HC17/FR1HC17', FR1HC18/FR1HC18', FR1HC19/FR1HC19', FR1HC20/FR1HC20', FR1HC21/FR1HC21', FR1HC22/FRI HC22', FR1HC23/FR1HC23', FR I HC24/FR I HC24', FR1HC25/FR1HC25', FR1HC26/FR1HC26', FR1HC27/FR1HC27', FR1HC28/FR1HC28', FR1HC29/FR1HC29', FR1HC30/FR1HC30', FR1HC31/FR1HC31', FR1HC32/FR1HC32', FR1HC33/FR1HC33', FR1HC34/FR1HC34', FR1HC35/FR1HC35', FR1HC36/FR1HC36', FR1HC37/FR1HC37', FR1HC38/FR1HC38', FR1HC39/FR1HC39', FR1HC40/FR1HC40', FR1HC41/FR1HC41', FR1HC42/FR1HC42', FR1HC43/FR1HC43', or FR1HC44/FR1HC44'. The pooling of the PCR products generates sub-bank 8.

By way of example but not limitation, the construction of heavy chain FR2 sub-bank (according to Chothia definition) is carried out using the Polymerase Chain Reaction by overlap extension using the oligonucleotides listed in Table 30 and Table 31 (all shown in the 5' to 3' orientation, name followed by sequence):

**Table 30. Heavy Chain FR2 (Chothia Definition) Forward Primers (for Sub-Bank 9):**

| | | |
|---|---|---|
| 1064 | FR2HC1 | TATGGTATCAGCTGGGTGCGACAGGCCCCTGGACAAGGGCTT |
| 1065 | FR2HC2 | TACTATATGCACTGGGTGCGACAGGCCCCTGGACAAGGGCTT |
| 1066 | FR3HC3 | TTATCCATGCACTGGGTGCGACAGGCTCCTGGAAAAGGGCTT |
| 1067 | FR2HC4 | TATGCTATGCATTGGGTGCGCCAGGCCCCCGGACAAAGGCTT |
| 1068 | FR2HC5 | CGCTACCTGCACTGGGTGCGACAGGCCCCCGGACAAGCGCTT |
| 1069 | FR2HC6 | TACTATATGCACTGGGTGCGACAGGCCCCTGGACAAGGGCTT |
| 1070 | FR2HC7 | TCTGCTATGCAGTGGGTGCGACAGGCTCGTGGACAACGCCTT |
| 1071 | FR2HC8 | TATGCTATCAGCTGGGTGCGACAGGCCCCTGGACAAGGGCTT |
| 1072 | FR2HC9 | TATGATATCAACTGGGTGCGACAGGCCACTGGACAAGGGCTT |
| 1073 | FR2HC10 | ATGGGTGTGAGCTGGATCCGTCAGCCCCCAGGGAAGGCCCTG |
| 1074 | FR2HC11 | GTGGGTGTGGGCTGGATCCGTCAGCCCCCAGGAAAGGCCCTG |
| 1075 | FR2HC12 | ATGTGTGTGAGCTGGATCCGTCAGCCCCCAGGGAAGGCCCTG |
| 1076 | FR2HC13 | TACTACATGAGCTGGATCCGCCAGGCTCCAGGGAAGGGGCTG |
| 1077 | FR2HC14 | TACGACATGCACTGGGTCCGCCAAGCTACAGGAAAAGGTCTG |
| 1078 | FR2HC15 | GCCTGGATGAGCTGGGTCCGCCAGGCTCCAGGGAAGGGGGTG |
| 1079 | FR2HC16 | AGTGACATGAACTGGGCCCGCAAGGCTCCAGGAAAGGGGCTG |
| 1080 | FR2HC17 | TATGGCATGAGCTGGGTCCGCCAAGCTCCAGGGAAGGGGCTG |
| 1081 | FR2HC18 | TATAGCATGAACTGGGTCCGCCAGGCTCCAGGGAAGGGGCTG |
| 1082 | FR2HC19 | TATGCCATGAGCTGGGTCCGCCAGGCTCCAGGGAAGGGGCTG |
| 1083 | FR2HC20 | TATGGCATGCACTGGGTCCGCCAGGCTCCAGGCAAGGGGCTG |
| 1084 | FR2HC21 | TATGGCATGCACTGGGTCCGCCAGGCTCCAGGCAAGGGGCTG |
| 1085 | FR2HC22 | AGTGACATGAACTGGGTCCATCAGGCTCCAGGAAAGGGGCTG |
| 1086 | FR2HC23 | AATGAGATGAGCTGGATCCGCCAGGCTCCAGGGAAGGGGCTG |
| 1087 | FR2HC24 | TATACCATGCACTGGGTCCGTCAAGCTCCGGGGAAGGGTCTG |
| 1088 | FR2HC25 | TATAGCATGAACTGGGTCCGCCAGGCTCCAGGGAAGGGGCTG |
| 1089 | FR2HC26 | TATGCTATGAGCTGGTTCCGCCAGGCTCCAGGGAAGGGGCTG |
| 1090 | FR2HC27 | AACTACATGAGCTGGGTCCGCCAGGCTCCAGGGAAGGGGCTG |
| 1091 | FR2HC28 | TATGCTATGCACTGGGTCCGCCAGGCTCCAGGGAAGGGACTG |
| 1092 | FR2HC29 | AACTACATGAGCTGGGTCCGCCAGGCTCCAGGGAAGGGGCTG |
| 1093 | FR2HC30 | TATTGGATGAGCTGGGTCCGCCAGGCTCCAGGGAAGGGGCTG |
| 1094 | FR2HC31 | CACTACATGGACTGGGTCCGCCAGGCTCCAGGGAAGGGGCTG |
| 1095 | FR2HC32 | TCTGCTATGCACTGGGTCCGCCAGGCTTCCGGGAAAGGGCTG |
| 1096 | FR2HC33 | TACTGGATGCACTGGGTCCGCCAAGCTCCAGGGAAGGGGCTG |
| 1097 | FR2HC34 | TATGCCATGCACTGGGTCCGGCAAGCTCCAGGGAAGGGCCTG |
| 1098 | FR2HC35 | AACTGGTGGGGCTGGATCCGGCAGCCCCCAGGGAAGGGACTG |
| 1099 | FR2HC36 | TACTACTGGAGCTGGATCCGCCAGCACCCAGGGAAGGGCCTG |
| 1100 | FR2HC37 | TACTACTGGAGCTGGATCCGCCAGCCCCCAGGGAAGGGGCTG |
| 1101 | FR2HC38 | TACTACTGGGGCTGGATCCGCCAGCCCCCAGGGAAGGGGCTG |
| 1102 | FR2HC39 | TACTACTGGAGCTGGATCCGGCAGCCCGCCGGGAAGGGACTG |
| 1103 | FR2HC40 | TACTACTGGAGCTGGATCCGGCAGCCCCCAGGGAAGGGACTG |
| 1104 | FR2HC41 | TACTACTGGAGCTGGATCCGGCAGCCCCCAGGGAAGGGACTG |
| 1105 | FR2HC42 | TACTGGATCGGCTGGGTGCGCCAGATGCCCGGGAAAGGCCTG |
| 1106 | FR2HC43 | GCTGCTTGGAACTGGATCAGGCAGTCCCCATCGAGAGGCCTT |
| 1107 | FR2HC44 | TATGGTATGAATTGGGTGCCACAGGCCCCTGGACAAGGGCTT |

**Table 31. Heavy Chain FR2 (Chothia Definition) Reverse Primers (for Sub-Bank 9):**

| | | |
|---|---|---|
| 1108 | FR2HC1' | GATCCATCCCATCCACTCAAGCCCTTGTCCAGGGGCCTG |
| 1109 | FR2HC2' | GATCCATCCCATCCAGTCAAGCCCTTGTCCAGGGGCCTG |
| 1110 | FR2HC3' | AAAACCTCCCATCCACTCAAGCCCTTTTCCAGGAGCCTG |
| 1111 | FR2HC4' | GCTCCATCCCATCCACTCAAGCCTTTGTCCGGGGGCCTG |
| 1112 | FR2HC5' | GATCCATCCCATCCACTCAAGCGCTTGTCCGGGGGCCTG |
| 1113 | FR2HC6' | GATTATTCCCATCCACTCAAGCCCTTGTCCAGGGGCCTG |
| 1114 | FR2HC7' | GATCCATCCTATCCACTCAAGGCGTTGTCCACGAGCCTG |
| 1115 | FR2HC8' | GATCCCTCCCATCCACTCAAGCCCTTGTCCAGGGGCCTG |
| 1116 | FR2HC9' | CATCCATCCCATCCACTCAAGCCCTTGTCCAGTGGCCTG |
| 1117 | FR2HC10' | AATGTGTGCAAGCCACTCCAGGGCCTTCCCTGGGGGCTG |
| 1118 | FR3HC11' | AATGAGTGCAAGCCACTCCAGGGCCTTTCCTGGGGGCTG |
| 1119 | FR2HC12' | AATGAGTGCAAGCCACTCCAGGGCCTTCCCTGGGGGCTG |
| 1130 | FR2HC13' | AATGTATGAAACCCACTCCAGCCCCTTCCCTGGAGCCTG |
| 1121 | FR2HC14' | AATAGCTGAGACCCACTCCAGACCTTTTCCTGTAGCTTG |
| 1122 | FR2HC15' | AATACGGCCAACCCACTCCAGCCCCTTCCCTGGAGCCTG |
| 1123 | FR2HC16' | AACACCCGATACCCACTCCAGCCCCTTTCCTGGAGCCTT |
| 1134 | FR2HC17' | AATACCAGAGACCCACTCCAGCCCCTTCCCTGGAGCTTG |
| 1125 | FR2HC18' | AATGGATGAGACCCACTCCAGCCCCTTCCCTGGAGCCTG |
| 1126 | FR2HC19' | AATAGCTGAGACCCACTCCAGCCCCTTCCCTGGAGCCTG |
| 1127 | FR2HC20' | TATAACTGCCACCCACTCCAGCCCCTTGCCTGGAGCCTG |
| 1128 | FR2HC21' | TATAACTGCCACCCACTCCAGCCCCTTGCCTGGAGCCTG |
| 1129 | FR2HC22' | AACACCCGATACCCACTCCAGCCCCTTTCCTGGAGCCTG |
| 1130 | FR2HC23' | AATGGATGAGACCCACTCCAGCCCCTTCCCTGGAGCCTG |
| 1131 | FR2HC24' | ATAAGAGAGACCCACTCCAGACCCTTCCCCGGAGCTTG |
| 1132 | FR2HC25' | AATGTATGAAACCCACTCCAGCCCCTTCCCTGGAGCCTG |
| 1133 | FR2HC26' | AATGAAACCTACCCACTCCAGCCCCTTCCCTGGAGCCTG |
| 1134 | FR2HC27' | AATAACTGAGACCCACTCCAGCCCCTTCCCTGGAGCCTG |
| 1135 | FR2HC28' | AATAGCTGAAACATATTCCAGTCCCTTCCCTGGAGCCTG |
| 1136 | FR2HC29' | AATAACTGAGACCCACTCCAGCCCCTTCCCTGGAGCCTG |
| 1137 | FR2HC30' | TATGTTGGCCACCCACTCCAGCCCCTTCCCTGGAGCCTG |
| 1138 | FR2HC31' | AGTACGGCCAACCCACTCCAGCCCCTTCCCTGGAGCCTG |
| 1139 | FR2HC32' | AATACGGCCAACCCACTCCAGCCCTTTCCCGGAAGCCTG |
| 1140 | FR2HC33' | AATACGTGAGACCCACACCAGCCCCTTCCCTGGAGCTTG |
| 1141 | FR2HC34' | AATACCTGAGACCCACTCCAGGCCCTTCCCTGGAGCTTG |
| 1142 | FR2HC35' | GATGTACCCAATCCACTCCAGTCCCTTCCCTGGGGGCTG |
| 1143 | FR2HC36' | GATGTACCCAATCCACTCCAGGCCCTTCCCTGGGTGCTG |
| 1144 | FR2HC37' | GATTTCCCCAATCCACTCCAGCCCCTTCCCTGGGGGCTG |
| 1145 | FR2HC38' | GATACTCCCAATCCACTCCAGCCCCTTCCCTGGGGGCTG |
| 1146 | FR2HC39' | GATACGCCCAATCCACTCCAGTCCCTTCCCGGCGGGCTG |
| 1147 | FR2HC40' | GATATACCCAATCCACTCCAGTCCCTTCCCTGGGGGCTG |
| 1148 | FR2HC41' | GATATACCCAATCCACTCCAGTCCCTTCCCTGGGGGCTG |
| 1149 | FR2HC42' | GATGATCCCCATCCACTCCAGGCCTTTCCCGGGCATCTG |
| 1150 | FR2HC43' | TGTCCTTCCCAGCCACTCAAGGCCTCTCGATGGGGACTG |
| 1151 | FR2HC44' | GAACCATCCCATCCACTCAAGCCCTTGTCCAGGGGCCTG |

PCR is carried out using the following oligonucleotide combinations (44 in total): FR2HC1/FR2HC1', FR2HC2/FR2HC2', FR2HC3/FR2HC3', FR2HC4/FR2HC4', FR2HC5/FR2HC5', FR2HC6/FR2HC6', FR2HC7/FR2HC7', FR2HC8/FR2HC8', FR2HC9/FR2HC9', FR2HC10/FR2HC10', FR2HC11/FR2HC11', FR2HC12/FR2HC12', FR2HC13/FR2HC13', FR2HC14/FR2HC14', FR2HC15/FR2HC15', FR2HC16/FR2HC16', FR2HC17/FR2HC17', FR2HC18/FR2HC18', FR2HC19/FR2HC19', FR2HC20/FR2HC20', FR2HC21/FR2HC21', FR2HC22/FR2HC22', FR2HC23/FR2HC23', FR2HC24/FR2HC24', FR2HC25/FR2HC25', FR2HC26/FR2HC26', FR2HC27/FR2HC27', FR2HC28/FR2HC28', FR2HC29/FR2HC29', FR2HC30/FR2HC30', FR2HC31/FR2HC31', FR2HC32/FR2HC32', FR2HC33/FR2HC33', FR2HC34/FR2HC34', FR2HC35/FR2HC35', FR2HC36/FR2HC36', FR2HC37/FR2HC37', FR2HC38/FR2HC38', FR2HC39/FR2HC39', FR2HC40/FR2HC40', FR2HC41/FR2HC41', FR2HC42/FR2HC42', FR2HC43/FR2HC43', or FR2HC44/FR2HC44'. The pooling of the PCR products generates sub-bank 9.

By way of example but not limitation, the construction of heavy chain **FR3** sub-bank (according to Chothia definition) is carried out using the Polymerase Chain Reaction by overlap extension using the oligonucleotides listed in Table 32 and Table 33 (all shown in the 5' to 3' orientation, name followed by sequence):

**Table 32. Heavy Chain FR3 (Chothia Definition) Forward Primers (for Sub-Bank 10):**

| | |
|---|---|
| 1152 | FR3HC1 |
| | ACAAACTATGCACAGAAGCTCCAGGGCAGAGTCACCATGACCACAGACACATCCACGAGCACAGCCTACATGG |
| 1153 | FR3HC2 |
| | ACAAACTATGCACAGAAGTTTCAGGGCAGGGTCACCATGACCAGGGACACGTCCATCAGCACAGCCTACATGG |
| 1154 | FR3HC3 |
| | ACAATCTACGCACAGAAGTTCCAGGGCAGAGTCACCATGACCGAGGACACATCTACAGACACAGCCTACATGG |
| 1155 | FR3HC4 |
| | ACAAAATATTCACAGGAGTTCCAGGGCAGAGTCACCATTACCAGGGACACATCCGCGAGCACAGCCTACATGG |
| 1156 | FR3HC5 |
| | ACCAACTACGCACAGAAATTCCAGGACAGAGTCACCATTACCAGGGACAGGTCTATGAGCACAGCCTACATGG |
| 1157 | FR3HC6 |
| | ACAAGCTACGCACAGAAGTTCCAGGGCAGAGTCACCATGACCAGGGACACGTCCACGAGCACAGTCTACATGG |
| 1158 | FR3HC7 |
| | ACAAACTACGCACAGAAGTTCCAGGAAAGAGTCACCATTACCAGGGACATGTCCACAAGCACAGCCTACATGG |
| 1159 | FR3HC8 |
| | GCAAACTACGCACAGAAGTTCCAGGGCAGAGTCACGATTACCGCGGACAAATCCACGAGCACAGCCTACATGG |
| 1160 | FR3HC9 |
| | ACAGGCTATGCACAGAAGTTCCAGGGCAGAGTCACCATGACCAGGAACACCTCCATAAGCACAGCCTACATGG |
| 1161 | FR3HC10 |
| | AAATCCTACAGCACATCTCTGAAGAGCAGGCTCACCATCTCCAAGGACACCTCCAAAAGCCAGGTGGTCCTTA |
| 1162 | FR3HC11 |
| | AAGCGCTACAGCCCATCTCTGAAGAGCAGGCTCACCATCACCAAGGACACCTCCAAAAACCAGGTGGTCCTTA |
| 1163 | FR3HC12 |
| | AAATACTACAGCACATCTCTGAAGACCAGGCTCACCATCTCCAAGGACACCTCCAAAAACCAGGTGGTCCTTA |
| 1164 | FR3HC13 |
| | ATATACTACGCAGACTCTGTGAAGGGCCGATTCACCATCTCCAGGGACAACGCCAAGAACTCACTGTATCTGC |
| 1165 | FR3HC14 |
| | ACATACTATCCAGGCTCCGTGAAGGGCCGATTCACCATCTCCAGAGAAAATGCCAAGAACTCCTTGTATCTTC |
| 1166 | FR3HC15 |
| | ACAGACTACGCTGCACCCGTGAAAGGCAGATTCACCATCTCAAGAGATGATTCAAAAAACACGCTGTATCTGC |
| 1167 | FR3HC16 |
| | ACGCACTATGTGGACTCCGTGAAGCGCCGATTCATCATCTCCAGAGACAATTCCAGGAACTCCCTGTATCTGC |
| 1168 | FR3HC17 |
| | ACAGGTTATGCAGACTCTGTGAAGGGCCGATTCACCATCTCCAGAGACAACGCCAAGAACTCCCTGTATCTGC |
| 1169 | FR3HC18 |
| | ATATACTACGCAGACTCAGTGAAGGGCCGATTCACCATCACCAGAGACAACGCCAAGAACTCACTGTATCTGC |
| 1170 | FR3HC19 |
| | ACATACTACGCAGACTCCGTGAAGGGCCGGTTCACCATCTCCAGAGACAATTCCAAGAACACGCTGTATCTGC |
| 1171 | FR3HC20 |
| | AAATACTATGCAGACTCCGTGAAGGGCCGATTCACCATCTCCAGAGACAATTCCAAGAACACGCTGTATCTGC |
| 1172 | FR3HC21 |
| | AAATACTATGCAGACTCCGTGAAGGGCCGATTCACCATCTCCAGAGACAATTCCAAGAACACGCTGTATCTGC |
| 1173 | FR3HC22 |
| | ACGCACTATGCAGACTCTGTGAAGGGCCGATTCATCATCTCCAGAGACAATTCCAGGAACACCCTGTATCTGC |
| 1174 | FR3HC23 |
| | ACATACTACGCAGACTCCAGGAAGGGCAGATTCACCATCTCCAGAGACAATTCCAAGAACACGCTGTATCTTC |
| 1175 | FR3HC24 |
| | ACATACTATGCAGACTCTGTGAAGGGCCGATTCACCATCTCCAGAGACAACAGCAAAAACTCCCTGTATCTGC |
| 1176 | FR3HC25 |
| | ATATACTACGCAGACTCTGTGAAGGGCCGATTCACCATCTCCAGAGACAATGCCAAGAACTCACTGTATCTGC |
| 1177 | FR3HC26 |
| | ACAGAATACGCCGCGTCTGTGAAAGGCAGATTCACCATCTCAAGAGATGATTCCAAAAGCATCGCCTATCTGC |
| 1178 | FR3HC27 |
| | ACATACTACGCAGACTCCGTGAAGGGCCGATTCACCATCTCCAGAGACAATTCCAAGAACACGCTGTATCTTC |
| 1179 | FR3HC28 |
| | ACATATTATGCAGACTCTGTGAAGGGCAGATTCACCATCTCCAGAGACAATTCCAAGAACACGCTGTATCTTC |
| 1180 | FR3HC29 |
| | ACATACTACGCAGACTCCGTGAAGGGCCGATTCACCATCTCCAGAGACAATTCCAAGAACACGCTGTATCTTC |
| 1181 | FR3HC30 |
| | AAATACTATGTGGACTCTGTGAAGGGCCGATTCACCATCTCCAGAGACAACGCCAAGAACTCACTGTATCTGC |
| 1182 | FR3HC31 |
| | ACAGAATACGCCGCGTCTGTGAAAGGCAGATTCACCATCTCAAGAGATGATTCAAAGAACTCACTGTATCTGC |
| 1183 | FR3HC32 |
| | ACAGCATATGCTGCGTCGGTGAAAGGCAGGTTCACCATCTCCAGAGATGATTCAAAGAACACGGCGTATCTGC |
| 1184 | FR3HC33 |
| | ACAAGCTACGCGGACTCCGTGAAGGGCCGATTCACCATCTCCAGAGACAACGCCAAGAACACGCTGTATCTGC |
| 1185 | FR3HC34 |
| | ATAGGCTATGCGGACTCTGTGAAGGGCCGATTCACCATCTCCAGAGACAACGCCAAGAACTCCCTGTATCTGC |
| 1186 | FR3HC35 |
| | ACCTACTACAACCCGTCCCTCAAGAGTCGAGTCACCATGTCAGTAGACACGTCCAAGAACCAGTTCTCCCTGA |
| 1187 | FR3HC36 |
| | ACCTACTACAACCCGTCCCTCAAGAGTCGAGTTACCATATCAGTAGACACGTCTAAGAACCAGTTCTCCCTGA |
| 1188 | FR3HC37 |
| | ACCAACTACAACCCGTCCCTCAAGAGTCGAGTCACCATATCAGTAGACACGTCCAAGAACCAGTTCTCCCTGA |
| 1189 | FR3HC38 |
| | ACCTACTACAACCCGTCCCTCAAGAGTCGAGTCACCATATCCGTAGACACGTCCAAGAACCAGTTCTCCCTGA |
| 1190 | FR3HC39 |
| | ACCAACTACAACCCCTCCCTCAAGAGTCGAGTCACCATGTCAGTAGACACGTCCAAGAACCAGTTCTCCCTGA |
| 1191 | FR3HC40 |
| | ACCAACTACAACCCCTCCCTCAAGAGTCGAGTCACCATATCAGTAGACACGTCCAAGAACCAGTTCTCCCTGA |
| 1192 | FR3HC41 |
| | ACCAACTACAACCCCTCCCTCAAGAGTCGAGTCACCATATCAGTAGACACGTCCAAGAACCAGTTCTCCCTGA |
| 1193 | FR3HC42 |
| | ACCAGATACAGCCCGTCCTTCCAAGGCCAGGTCACCATCTCAGCCGACAAGTCCATCAGCACCGCCTACCTGC |
| 1194 | FR3HC43 |
| | AATGATTATGCAGTATCTGTGAAAAGTCGAATAACCATCAACCCAGACACATCCAAGAACCAGTTCTCCCTGC |
| 1195 | FR3HC44 |
| | CCAACATATGCCCAGGGCTTCACAGGACGGTTTGTCTTCTCCATGGACACCTCTGCCAGCACAGCATACCTGC |

**Table 33. Heavy Chain FR3 (Chothia Definition) Reverse Primers (for Sub-Bank 10):**

| | |
|---|---|
| 1196 | FR3HC1' |
| | TCTCGCACAGTAATACACGGCCGTGTCGTCAGATCTCAGGCTCCTCAGCTCCATGTAGGCTGTGCTCGTGG |
| 1197 | FR3HC2' |
| | TCTCGCACAGTAATACACGGCCGTGTCGTCAGATCTCAGCCTGCTCAGCTCCATGTAGGCTGTGCTGATGG |
| 1198 | FR3HC3' |
| | TGTTGCACAGTAATACACGGCCGTGTCCTCAGATCTCAGGCTGCTCAGCTCCATGTAGGCTGTGTCTGTAG |
| 1199 | FR3HC4' |
| | TCTCGCACAGTAATACACAGCCATGTCCTCAGATCTCAGGCTGCTCAGCTCCATGTAGGCTGTGCTCGCGG |
| 1200 | FR3HC5' |
| | TCTrGCACAGTAATACATGGCTGTGTCCTCAGATCTCAGGCTGCTCAGCTCCATGTAGGCTGTGCTCATAG |
| 1201 | FR3HC6' |
| | TCTCGCACAGTAATACACGGCCGTOTCCTCAGATC^{T}CAGGCTGCTCACCTCCATGTAGACfGTGCTCGTGG |
| 1202 | FR3HC7' |
| | TGCCGCACAGTAATACACGGCCGTGTCCTCGGATCTCAGGCTGCTCAGCTCCATGTAGGCTGTGCTTGTGG |
| 1203 | FR3HC8' |
| | TCTCGCACAGTAATACACGGCCGTGTCCTCAGATCTCAGGCTGCTCAGCTCCATGTAGGCTGTGCTCGTGG |
| 1204 | FR3HC9' |
| | TCTCGCACAGTAATACACGGCCGTGTCCTCAGATCTCAGGCTGCTCAGCTCCATGTAGGCTGTGCTTATGG |
| 1205 | FR3HC10' |
| | CCGTGCACAGTAATATGTGGCTGTGTCCACAGGGTCCATGTTGGTCATGGTAAGGACCACCTGGCTTTTGG |
| 1206 | FR3HC11' |
| | GTGTGCACAGTAATATGTGGCTGTGTCCACAGGGTCCATGTTGGTCATTGTAAGGACCACCTGGTTTTTGG |
| 1207 | FR3HC12' |
| | CCGTGCACAATAATACGTGGCTGTGTCCACAGGGTCCATGTTGGTCATTGTAAGGACCACCTGGTTTTTGG |
| 1208 | FR3HC13' |
| | TCTCGCACAGTAATACACGGCCGTGTCCTCGGCTCTCAGGCTGTTCATTTGCAGATACAGTGAGTTCTTGG |
| 1209 | FR3HC14' |
| | TCTTGCACAGTAATACACAGCCGTGTCCCCGGCTCTCAGGCTGTTCATTTGAAGATACAAGGAGTTCTTGG |
| 1210 | FR3HC15' |
| | TGTGGTACAGTAATACACGGCTGTGTCCTCGGTTTTCAGGCTGTTCATTTGCAGATACAGCGTGTTTTTTG |
| 1211 | FR3HC16' |
| | TCTCACACAGTAATACACAGCCATGTCCTCGGCTCTCCGTCTGTTCTTTTGCAGATACAGGGAGTTCCTGG |
| 1212 | FR3HC17' |
| | TCTCGCACAGTGATACAAGGCCGTGTCCTCGGCTCTCAGACTGTTCATTTGCAGATACAGGGAGTTCTTGG |
| 1213 | FR3HC18' |
| | TCTCGCACAGTAATACACAGCCGTGTCCTCGGCTCTCAGGCTGTTCATTTGCAGATACAGTGAGTTCTTGG |
| 1214 | FR3HC19' |
| | TTTCGCACAGTAATATACGGCCGTGTCCTCGGCTCTCAGGCTGTTCATTTGCAGATACAGCGTGTTCTTGG |
| 1215 | FR3HC20' |
| | TCTCGCACAGTAATACACAGCCGTGTCCTCAGCTCTCAGGCTGTTCATTTGCAGATACAGCGTGTTCTTGG |
| 1216 | FR3HC21' |
| | TCTCGCACAGTAATACACAGCCGTGTCCTCGGCTCTCAGGCTGTTCATTTGCAGATACAGCGTGTTCTTGG |
| 1217 | FR3HC22' |
| | TCTCACACAGTAATACACAGCCGTGTCCTCGGCCCTCAGGCTATTCGTTTGCAGATACAGGGTGTTCCTGG |
| 1218 | FR3HC23' |
| | TCTGGCACAGTAATACACCGCCGTGCCCTCAGCTCTCAGGTTGTTCATTTGAAGATACAGCCTGTTCTTGG |
| 1219 | FR3HC24' |
| | TTTTGCACAGTAATACAAGGCGGTGTCCTCAGTTCTCAGACTGTTCATTTGCAGATACAGGGAGTTTTTGC |
| 1220 | FR3HC25' |
| | TCTCGCACAGTAATACACAGCCGTGTCCTCGTCTCTCAGGCTGTTCATTTGCAGATACAGTGAGTTCTTGG |
| 1221 | FR3HC26' |
| | TCTAGTACAGTAATACACGGCTGTGTCCTCGGTTTTCAGGCTGTTCATTTGCAGATAGGCGATGCTTTTGG |
| 1222 | FR3HC27' |
| | TCTCGCACAGTAATACACGGCCGTGTCCTCGGCTCTCAGGCTGTTCATTTGAAGATACAGCGTGTTCTTGG |
| 1223 | FR3HC28' |
| | TCTCGCACAGTAATACACAGCCATGTCCTCAGCTCTCAGGCTGCCCATTTGAAGATACAGCGTGTTCTTGG |
| 1224 | FR3HC29' |
| | TCTCGCACAGTAATACACAGCCGTGTCCTCAGCTCTCAGGCTGTTCATTTGAAGATACAGCGTGTTCTTGG |
| 1225 | FR3HC30' |
| | TCTCGCACAGTAATACACAGCCGTGTCCTCGGCTCTCAGGCTGTTCATTTGCAGATACAGTGAGTTCTTGG |
| 1226 | FR3HC31' |
| | TCTAGCACAGTAATACACGGCCGTGTCCTCGGTTTTCAGGCTGTTCATTTGCAGATACAGTGAGTTCTTTG |
| 1227 | FR3HC32' |
| | TCTAGTACAGTAATACACGGCCGTGTCCTCGGTTTTCAGGCTGTTCATTTGCAGATACGCCGTGTTCTTTG |
| 1228 | FR3HC33' |
| | TCTTGCACAGTAATACACAGCCGTGTCCTCGGCTCTCAGACTGTTCATTTGCAGATACAGCGTGTTCTTGG |
| 1229 | FR3HC34' |
| | TTTTGCACAGTAATACAAGGCCGTGTCCTCAGCTCTCAGACTGTTCATTTGCAGATACAGGGAGTTCTTGG |
| 1230 | FR3HC35' |
| | TCTCGCACAGTAATACACGGCCGTGTCCACGGCGGTCACAGAGCTCAGCTTCAGGGAGAACTGGTTCTTGG |
| 1231 | FR3HC36' |
| | TCTCGCACAGTAATACACGGCCGTGTCCCCGGCAGTCACAGAGCTCAGCTTCAGGGAGAACTGGTTCTTAG |
| 1232 | FR3HC37' |
| | TCTCGCACAGTAATACACAGCCGTGTCCGCGGCGGTCACAGAGCTCAGCTTCAGGGAGAACTGGTTCTTGG |
| 1233 | FR3HC38' |
| | TCTCGCACAGTAATACACAGCCGTGTCTGCGGCGGTCACAGAGCTCAGCTTCAGGGAGAACTGGTTCTTGG |
| 1234 | FR3HC39' |
| | TCTCGCACAGTAATACACGGCCGTGTCCGCGGCGGTCACAGAGCTCAGCTTCAGGGAGAACTGGTTCTTGG |
| 1233 | FR3HC40' |
| | TCTCGCACAGTAATACACGGCCGTGTCCGCAGCGGTCACAGAGCTCAGCTTCAGGGAGAACTGGTTCTTGG |
| 1236 | FR3HC41' |
| | TCTCGCACAGTAATACACGGCCGTGTCCGCAGCGGTCACAGAGCTCAGCTTCAGGGAGAACTGGTTCTTGG |
| 1237 | FR3HC42' |
| | TCTCGCACAGTAATACATGGCGGTGTCCGAGGCCTTCAGGCTGCTCCACTGCAGGTAGGCGGTGCTGATGG |
| 1238 | FR3HC43' |
| | TCTTGCACAGTAATACACAGCCGTGTCCTCGGGAGTCACAGAGTTCAGCTGCAGGGAGAACTGGTTCTTGG |
| 1239 | FR3HC44' |
| | TCTCGCACAGTAATACATGGCCATGTCCTCAGCCTTTAGGCTGCTGATCTGCAGGTATGCTGTGCTGGCAG |

PCR is carried out using the following oligonucleotide combinations (44 in total): FR3HC1/FR3HC1', FR3HC2/FR3HC2', FR3HC3/FR3HC3', FR3HC4/FR3HC4', FR3HC5/FR3HC5', FR3HC6/FR3HC6', FR3HC7/FR3HC7', FR3HC8/FR3HC8', FR3HC9/FR3HC9', FR3HC10/FR3HC10', FR3HC11/FR3HC11', FR3HC12/FR3HC12', FR3HC13/FR3HC13', FR3HC14/FR3HC14', FR3HC15/FR3HC15', FR3HC16/FR3HC16', FR3HC17/FR3HC17', FR3HC18/FR3HC18', FR3HC19/FR3HC19', FR3HC20/FR3HC20', FR3HC21/FR3HC21', FR3HC22/FR3HC22', FR3HC23/FR3HC23', FR3HC24/FR3HC24', FR3HC25/FR3HC25', FR3HC26/FR3HC26', FR3HC27/FR3HC27', FR3HC28/FR3HC28', FR3HC29/FR3HC29', FR3HC30/FR3HC30', FR3HC31/FR3HC31', FR3HC32/FR3HC32', FR3HC33/FR3HC33', FR3HC34/FR3HC34', FR3HC35/FR3HC35', FR3HC36/FR3HC36', FR3HC37/FR3HC37', FR3HC38/FR3HC38', FR3HC39/FR3HC39', FR3HC40/FR3HC40', FR3HC41/FR3HC41', FR3HC42/FR3HC42', FR3HC43/FR3HC43', or FR3HC44/FR3HC44'. The pooling of the PCR products generates sub-bank 10.

### 2.3 Selection of CDRs

In addition to the synthesis of framework region sub-banks, sub-banks of CDRs can be generated and randomly fused in frame with framework regions from framework region sub-banks to produce combinatorial libraries of antibodies (with or without constant regions) that can be screened for their immunospecificity for an antigen of interest, as well as their immunogenicity in an organism of interest. The combinatorial library methodology of the invention is exemplified herein for the production of humanized antibodies for use in human beings. However, the combinatorial library methodology can readily be applied to the production of antibodies for use in any organism of interest.

The present invention provides for a CDR sub-bank for each CDR of the variable light chain and variable heavy chain. Accordingly, the invention provides a CDR region sub-bank for variable light chain CDR1, variable light chain CDR2, and variable light CDR3 for each species of interest and for each definition of a CDR (e.g., Kabat and Chothia). The invention also provides a CDR sub-bank for variable heavy chain CDR1, variable heavy CDR2, and variable heavy chain CDR3 for each species of interest and for each definition of a CDR (e.g., Kabat and Chothia). CDR sub-banks may comprise CDRs that have been identified as part of an antibody that binds immunospecifically to an antigen of interest. The CDR sub-banks can be readily used to synthesize a combinatorial library of antibodies which can be screened for their immunospecificity for an antigen of interest, as well as their immunogencity in an organism of interest.

For example, light chain CDR sub-banks 12, 13 and 14 can be constructed, wherein CDR sub-bank 12 comprises a plurality of nucleic acid sequences comprising nucleotide sequences, each nucleotide sequence encoding light chain CDR1 according to Kabat system; CDR sub-bank 13 comprises a plurality of nucleic acid sequences comprising nucleotide sequences, each nucleotide sequence encoding light chain CDR2 according to Kabat system; and CDR sub-bank 14 comprises a plurality of nucleic acid sequences comprising nucleotide sequences, each nucleotide sequence encoding light chain CDR3 according to Kabat system. Light chain CDR sub-banks 15, 16 and 17 can be constructed, wherein CDR sub-bank 15 comprises a plurality of nucleic acid sequences comprising nucleotide sequences, each nucleotide sequence encoding light chain CDR1 according to Chothia system; CDR sub-bank 16 comprises a plurality of nucleic acid sequences comprising nucleotide sequences, each nucleotide sequence encoding light chain CDR2 according to Chothia system; and CDR sub-bank 17 comprises a plurality of nucleic acid sequences comprising nucleotide sequences, each nucleotide sequence ericoding light chain CDR3 according to Chothia system

Heavy chain CDR sub-banks 18, 19 and 20 can be constructed, wherein CDR sub-bank 18 comprises a plurality of nucleic acid sequences comprising nucleotide sequences, each nucleotide sequence encoding heavy chain CDR1 according to Kabat system; CDR sub-bank 19 comprises a plurality of nucleic acid sequences comprising nucleotide sequences, each nucleotide sequence encoding heavy chain CDR2 according to Kabat system; and CDR sub-bank 20 comprises a plurality of nucleic acid sequences comprising nucleotide sequences, each nucleotide sequence encoding heavy chain CDR3 according to Kabat system. Heavy chain CDR sub-banks 21, 22 and 23 can be constructed, wherein CDR sub-bank 21 comprises a plurality of nucleic acid sequences comprising nucleotide sequences, each nucleotide sequence encoding heavy chain CDR1 according to Chothia system; CDR sub-bank 22 comprises a plurality of nucleic acid sequences comprising nucleotide sequences, each nucleotide sequence encoding heavy chain CDR2 according to Chothia system; and CDR sub-bank 23 comprises a plurality of nucleic acid sequences comprising nucleotide sequences, each nucleotide sequence encoding heavy chain CDR3 according to Chothia system.

In some embodiments, the CDR sequences are derived from functional antibody sequences. In some embodiments, the CDR sequences are random sequences, which comprises at least 5, at least 6, at least 7, at least 8, at least 9, or at least 10 contiguous nucleotide sequences, synthesized by any methods known in the art. The CDR sub-banks can be used for construction of combinatorial sub-libraries. Alternatively, a CDR of particular interest can be selected and then used for the construction of combinatorial sub-libraries (see Section 5.3).

### 2.4 Construction of Combinatorial Sub-libraries

Combinatorial sub-libraries are constructed by fusing in frame non-human CDRs with corresponding human framework regions of the FR sub-banks. For example, combinatorial sub-library 1 is constructed by fusing in frame non-human CDRs with corresponding kappa light chain human framework regions using sub-banks 1; combinatorial sub-library 2 is constructed by fusing in frame non-human CDRs with corresponding kappa light chain human framework regions using sub-banks 2; combinatorial sub-library 3 is constructed by fusing in frame non-human CDRs with corresponding kappa light chain human framework regions using sub-banks 3; combinatorial sub-library 4 is constructed by fusing in frame non-human CDRs with corresponding kappa light chain human framework regions using sub-banks 4; combinatorial sub-libraries 5, 6, and 7 are constructed by fusing in frame non-human CDRs (Kabat definition for CDR H1 and H2) with the corresponding heavy chain human framework regions using sub-banks 5, 6 and 7, respectively; combinatorial sub-libraries 8, 9 and 10 are constructed by fusing in frame non-human CDRs (Chothia definition for CDR H1 and H2) with the corresponding heavy chain human framework regions using sub-banks 8, 9 and 10, respectively; combinatorial sub-library 11 is constructed by fusing in frame non-human CDR H3 (Kabat and Chothia definition) with the corresponding human heavy chain framework regions using sub-bank 11. In some embodiments, the non-human CDRs may also be selected from a CDR library.

The construction of combinatorial sub-libraries can be carried out using any method known in the art. By way of example but not limitation, the combinatorial sub-library 1 is constructed using the Polymerase Chain Reaction (PCR) by overlap extension using the oligonucleotides in Table 34 and Table 35 (all shown in the 5' to 3' orientation, name followed by sequence) where K= G or T, M= A or C, R= A or G, S= C or G, W= A or T and Y= C or T.

**Table 34. Light Chain FR1 Antibody-Specitic Forward Primers (for Sub-Library 1)**

| | | |
|---|---|---|
| 1240 | AL1 | GATGTTGTGATGACWCAGTCT |
| 1241 | AL2 | GACATCCAGATGAYCCAGTCT |
| 1242 | AL3 | GCCATCCAGWTGACCCAGTCT |
| 1243 | AL4 | GAAATAGTGATGAYGCAGTCT |
| 1244 | AL5 | GAAATTGTGTTGACRCAGTCT |
| 1245 | AL6 | GAKATTGTGATGACCCAGACT |
| 1246 | AL7 | GAAATTGTRMTGACWCAGTCT |
| 1247 | AL8 | GAYATYGTGATGACYCAGTCT |
| 1248 | AL9 | GAAACGACACTCACGCAGTCT |
| 1249 | AL10 | GACATCCAGTTGACCCAGTCT |
| 1250 | AL11 | AACATCCAGATGACCCAGTCT |
| 1251 | AL12 | GCCATCCGGATGACCCAGTCT |
| 1252 | AL13 | GTCATCTGGATGACCCAGTCT |

**Table 35. Light Chain FR1 Antibody-Specitic Reverse Primers (for Sub-Library 1)**

| | | |
|---|---|---|
| 1253 | AL1' | [first 70% of CDR L1]-GCAGGAGATGGAGGCCGGCTS |
| 1254 | AL2' | [first 70% of CDR L1]-GCAGGAGAGGGTGRCTCTTTC |
| 1255 | AL3' | [first 70% of CDR L1]-ACAASTGATGGTGACTCTGTC |
| 1356 | AL4' | [first 70% of CDR L1]-GAAGGAGATGGAGGCCGGCTG |
| 1357 | AL5' | [first 70% of CDR L1]-GCAGGAGATGGAGGCCTGCTC |
| 1258 | AL6' | [first 70% of CDR L1]-GCAGGAGATGTTGACTTTGTC |
| 1259 | AL7' | [first 70% of CDR L1]-GCAGGTGATGGTGACTTTCTC |
| 1360 | AL8' | [first 70% of CDR L1]-GCAGTTGATGGTGGCCCTCTC |
| 1261 | AL9' | [first 70% of CDR L1]-GCAAGTGATGGTGACTCTGTC |
| 1262 | AL10' | [first 70% of CDR L1]-GCAAATGATACTGACTCTGTC |

PCR is carried out with AL1 to AL13 in combination with AL1' to AL10' using sub-bank 1 as a template. This generates Combinatorial sub-library 1 or a pool of oligonucleotides corresponding to sequences described in Table 1.

By way of example but not limitation, the combinatorial sub-library 2 is constructed using the Polymerase Chain Reaction (PCR) by overlap extension using the oligonucleotides in Table 36 and Table 37 (all shown in the 5' to 3' orientation, name followed by sequence) where K= G or T, M= A or C, R= A or G, S= C or G, W= A or T and Y= C or T.

**Table 36. Light Chain FR2 Antibody-Specitic Forward Primers (for Sub-Library 2):**

| | | |
|---|---|---|
| 1263 | BL1 | [last 70% of CDR L1]-TGGYTTCAGCAGAGGCCAGGC |
| 1264 | BL2 | [last 70% of CDR L1]-TGGTACCTGCAGAAGCCAGGS |
| 1265 | BL3 | [last 70% of CDR L1]-TGGTATCRGCAGAAACCAGGG |
| 1266 | BL4 | [last 70% of CDR L1]-TGGTACCARCAGAAACCAGGA |
| 1267 | BL5 | [last 70% of CDR L1]-TGGTACCARCAGAAACCTGGC |
| 1268 | BL6 | [last 70% of CDR L1]-TGGTAYCWGCAGAAACCWGGG |
| 1269 | BL7 | [last 70% of CDR L1]-TGGTATCAGCARAAACCWGGS |
| 1270 | BL8 | [last 70% of CDR L1]-TGGTAYCAGCARAAACCAG |
| 1271 | BL9 | [last 70% of CDR L 1]-TGGTTTCTGCAGAAAGCCAGG |
| 1272 | BL10 | [last 70% of CDR L1]-TGGTTTCAGCAGAAACCAGGG |

**Table 37. Light Chain FR2 Antibody-Specific Reverse Primers (for Sub-Library 2)**

| | | |
|---|---|---|
| 1273 | BL1' | [first 70% of CDR L2]-ATAGATCAGGAGCTGTGGAGR |
| 1274 | BL2' | [first 70% of CDR L2]-ATAGATCAGGAGCTTAGGRGC |
| 1275 | BL3' | [first 70% of CDR L2]-ATAGATGAGGAGCCTGGGMGC |
| 1276 | BL4' | [first 70% of CDR L2]-RTAGATCAGGMGCTTAGGGGC |
| 1277 | BL5' | [first 70% of CDR L2]-ATAGATCAGGWGCTTAGGRAC |
| 1278 | BL6' | [first 70% of CDR L2]-ATAGATGAAGAGCTTAGGGGC |
| 1279 | BL7' | [first 70% of CDR L2]-ATAAATTAGGAGTCTTGGAGG |
| 1280 | BL8' | [first 70% of CDR L2]-GTAAATGAGCAGCTTAGGAGG |
| 1281 | BL9' | [first 70% of CDR L2]-ATAGATCAGGAGTGTGGAGAC |
| 1281 | BL10' | [first 70% of CDR L2]-ATAGATCAGGAGCTCAGGGGC |
| 1283 | BL11' | [first 70% of CDR L2]-ATAGATCAGGGACTTAGGGGC |
| 1284 | BL12' | [first 70% of CDR L2]-ATAGAGGAAGAGCTTAGGGGA |
| 1285 | BL13' | [first 70% of CDR L2]-CTTGATGAGGAGCTTTGGAGA |
| 1286 | BL14' | [first 70% of CDR L2]-ATAAATTAGGCGCCTTGGAGA |
| 1287 | BL15' | [first 70% of CDR L2]-CTTGATGAGGAGCTTTGGGGC |
| 1288 | BL16' | [first 70% of CDR L2]-TTGAATAATGAAAATAGCAGC |

PCR is carried out with BL1 to BL10 in combination with BL1' to BL16' using sub-bank 2 as a template. This generates combinatorial sub-library 2 or a pool of oligonucleotides corresponding to sequences described in Table 2.

By way of example but not limitation, the combinatorial sub-library 3 is constructed using the Polymerase Chain Reaction (PCR) by overlap extension using the oligonucleotides in Table 38 and Table 39 (all shown in the 5' to 3' orientation, name followed by sequence) where K= G or T, M= A or C, R= A or G, S= C or G, W= A or T and Y= C or T.

**Table 38. Light Chain FR3 Antibody-Specific Forward Primers (for Sub-Library 3):**

| | | |
|---|---|---|
| 1289 | CL1 | [Last 70% of CDR L2]-GGGGTCCCAGACAGATTCAGY |
| 1290 | CL2 | [Last 70% of CDR L2]-GGGGTCCCATCAAGGTTCAGY |
| 1291 | CL3 | [Last 70% of CDR L2]-GGYATCCCAGCCAGGTTCAGT |
| 1292 | CL4 | [Last 70% of CDR L2]-GGRGTCCCWGACAGGTTCAGT |
| 1293 | CL5 | [Last 70% of CDR L2]-AGCATCCCAGCCAGGTTCAGT |
| 1294 | CL6 | [Last 70% of CDR L2]-GGGGTCCCCTCGAGGTTCAGT |
| 1295 | CL7 | [Last 70% of CDR L2]-GGAATCCCACCTCGATTCAGT |
| 1296 | CL8 | [Last 70% of CDR L2]-GGGGTCCCTGACCGATTCAGT |
| 1297 | CL9 | [Last 70% of CDR L2]-GGCATCCCAGACAGGTTCAGT |
| 1298 | CL10 | [Last 70% of CDR L2]-GGGGTCTCATCGAGGTTCAGT |
| 1299 | CL11 | [Last 70% of CDR L2]-GGAGTCCCAGATAGGTTCAGT |

**Table 39. Light Chain FR3 Antibody-Specific Reverse Primers (for Sub-Library 3)**

| | | |
|---|---|---|
| 1300 | CL1' | [First 70% of CDR L3]-KCAGTAATAAACCCCAACATC |
| 1301 | CL2' | [First 70% of CDR L3]-ACAGTAATAYGTTGCAGCATC |
| 1302 | CL3' | [First 70% of CDR L3]-ACMGTAATAAGTTGCAACATC |
| 1303 | CL4' | [First 70% of CDR L3]-RCAGTAATAAGTTGCAAAATC |
| 1304 | CL5' | [First 70% of CDR L3]-ACAGTAATAARCTGCAAAATC |
| 1305 | CL6' | [First 70% of CDR L3]-ACARTAGTAAGTTGCAAAATC |
| 1306 | CL7' | [First 70% of CDR L3]-GCAGTAATAAACTCCAAMATC |
| 1307 | CL8' | [First 70% of CDR L3]-GCAGTAATAAACCCCGACATC |
| 1308 | CL9' | [First 70% of CDR L3]-ACAGAAGTAATATGCAGCATC |
| 1309 | CL10' | [First 70% of CDR L3]-ACAGTAATATGTTGCAATATC |
| 1310 | CL11' | [First 70% of CDR L3]-ACAGTAATACACTGCAAAATC |
| 1311 | CL12' | [First 70% of CDR L3]-ACAGTAATAAACTGCCACATC |

PCR is carried out with CL1 to CL11 in combination with CL1' to CL12' using-sub-bank 3 as a template. This generates combinatorial sub-library 3 or a pool of oligonucleotides corresponding to sequences described in Table 3.

By way of example but not limitation, the combinatorial sub-library 4 is constructed using the Polymerase Chain Reaction (PCR) by overlap extension using the oligonucleotides in Table 40 and Table 41 (all shown in the 5' to 3' orientation, name followed by sequence) where K= G or T, M= A or C, R= A or G, S= C or G, W= A or T and Y= C or T.

**Table 40. Light Chain FR4 Antibody-Specific Forward Primers (for Sub-Library 4):**

| | | |
|---|---|---|
| 1312 | DL1 | [Last 70% of CDR L3]-TTYGGCCARGGGACCAAGSTG |
| 1313 | DL2 | [Last 70% of CDR L3]-TTCGGCCAAGGGACACGACTG |
| 1314 | DL3 | [Last 70% of CDR L3]-TTCGGCCCTGGGACCAAAGTG |
| 1315 | DL4 | [Last 70% of CDR L3]-TTCGGCGGAGGGACCAAGGTG |

**Table 41. Light Chain FR4 Antibody-Specific Reverse Primers (for Sub-Library 4)**

| | | |
|---|---|---|
| 1316 | DL1' | TTCGATYTCCACCTTGGTCCC |
| 1317 | DL2' | TTTGATCTCCAGCTTGGTCCC |
| 1318 | DL3' | TTTGATATCCACTTTGGTCCC |
| 1319 | DL4' | TTTAATCTCCAGTCGTGTCCC |

PCR is carried out with DL1 to DL4 in combination with DL1' to DL14' using sub-bank 4 as a template. This generates combinatorial sub-library 4 or a pool of oligonucleotides corresponding to sequences described in Table 4.

By way of example but not limitation, the combinatorial sub-library 5 is constructed using the Polymerase Chain Reaction (PCR) by overlap extension using the oligonucleotides in Table 42 and Table 43 (all shown in the 5' to 3' orientation, name followed by sequence) where K= G or T, M= A or C, R= A or G, S= C or G, W= A or T and Y= C or T.

**Table 42. Heavy Chain FR1 (Kabat Definition) Antibody-Specific Forward Primers (for Sub-Library 5):**

| | | |
|---|---|---|
| 1320 | AH1 | CAGGTKCAGCTGGTGCAGTCT |
| 1321 | AH2 | GAGGTGCAGCTGKTGGAGTCT |
| 1322 | AH3 | CAGSTGCAGCTGCAGGAGTCG |
| 1323 | AH4 | CAGGTCACCTTGARGGAGTCT |
| 1324 | AH5 | CARATGCAGCTGGTGCAGTCT |
| 1325 | AH6 | GARGTGCAGCTGGTGSAGTC |
| 1326 | AH7 | CAGATCACCTTGAAGGAGTCT |
| 1327 | AH8 | CAGGTSCAGCTGGTRSAGTCT |
| 1328 | AH9 | CAGGTACAGCTGCAGCAGTCA |
| 1329 | AH10 | CAGGTGCAGCTACAGCAGTGG |

**Table 43. Heavy Chain FR1 (Kabat Definition) Antibody-Specific Reverse Primers (for Sub-Library 5):**

| | | |
|---|---|---|
| 1330 | AHK1' | [First 70% of CDR H1]-RGTGAAGGTGTATCCAGAAGC |
| 1331 | AHK2' | [First 70% of CDR H1]-GCTGAGTGAGAACCCAGAGAM |
| 1332 | AHK3' | [First 70% of CDR H1]-ACTGAARGTGAATCCAGAGGC |
| 1333 | AHK4' | [First 70% of CDR H1]-ACTGACGGTGAAYCCAGAGGC |
| 1334 | AHK5' | [First 70% of CDR H1]-GGTGAYGGAGCCACCAGAGAC |
| 1335 | AHK6' | [First 70% of CDR H1]-RGTAAAGGTGWAWCCAGAAGC |
| 1336 | AHK7' | [First 70% of CDR H1]-ACTRAAGGTGAAYCCAGAGGC |
| 1337 | AHK8' | [First 70% of CDR H1]-GGTRAARCTGTAWCCAGAASC |
| 1338 | AHK9' | [First 70% of CDR H1]-AYCAAAGGTGAATCCAGARGC |
| 1339 | AHK10' | [First 70% of CDR H1]-RCTRAAGGTGAATCCAGASGC |
| 1340 | AHK12 | [First 70% of CDR H1]-GGTGAAGGTGTATCCRGAWGC |
| 1341 | AHK13' | [First 70% of CDR H1]-ACTGAAGGACCCACCATAGAC |
| 1342 | AHK14' | [First 70% of CDR H1]-ACTGATGGAGCCACCAGAGAC |
| 1343 | AHK15' | [First 70% of CDR H1]-GCTGATGGAGTAACCAGAGAC |
| 1344 | AHK16 | [First 70% of CDR H1]-AGTGAGGGTGTATCCGGAAAC |
| 1345 | AHK17' | [First 70% of CDR H1]-GCTGAAGGTGCCTCCAGAAGC |
| 1346 | AHK18' | [First 70% of CDR H1]-AGAGACACTGTCCCCGGAGAT |

PCR is carried out with AH 1 to AH10 in combination with AHK1' to AHK18' using sub-bank 5 as a template. This generates combinatorial sub-library 5 or a pool of oligonucleotides corresponding to sequences described in Table 5.

By way of example but not limitation, the combinatorial sub-library 6 is constructed using the Polymerase Chain Reaction (PCR) by overlap extension using the oligonucleotides in Table 44 and Table 45 (all shown in the 5' to 3' orientation, name followed by sequence) where K= G or T, M= A or C, R= A or G, S= C or G, W= A or T and Y= C or T.

**Table 44. Heavy Chain FR2 (Kabat Definition) Antibody-Specific Forward Primers (for Sub-Library 6):**

| | | |
|---|---|---|
| 1347 | BHK1 | [Last 70% of CDR H1]-TGGGTGCGACAGGCYCCTGGA |
| 1348 | BHK2 | [Last 70% of CDR H1]-TGGGTGCGMCAGGCCCCCGGA |
| 1349 | BHK3 | [Last 70% of CDR H1]-TGGATCCGTCAGCCCCCAGGR |
| 1350 | BHK4 | [Last 70% of CDR H1]-TGGRTCCGCCAGGCTCCAGGG |
| 1351 | BHK5 | [Last 70% of CDR H1]-TGGATCCGSCAGCCCCCAGGG |
| 1352 | BHK6 | [Last 70% of CDR H1]-TGGGTCCGSCAAGCTCCAGGG |
| 1353 | BHK7 | [Last 70% of CDR H1]-TGGGTCCRTCARGCTCCRGGR |
| 1354 | BHK8 | [Last 70% of CDR H1]-TGGGTSCGMCARGCYACWGGA |
| 1355 | BHK9 | [Last 70% of CDR H1]-TGGKTCCGCCAGGCTCCAGGS |
| 1356 | BHK10 | [Last 70% of CDR H1]-TGGATCAGGCAGTCCCCATCG |
| 1357 | BHK11 | [Last 70% of CDR H1]-TGGGCCCGCAAGGCTCCAGGA |
| 1358 | BHK12 | [Last 70% of CDR H1]-TGGATCCGCCAGCACCCAGGG |
| 1359 | BHK13 | [Last 70% of CDR H1]-TGGGTCCGCCAGGCTTCCGGG |
| 1360 | BHK14 | [Last 70% of CDR H1]-TGGGTGCGCCAGATGCCCGGG |
| 1361 | BHK15 | [Last 70% of CDR H1]-TGGGTGCGACAGGCTCGTGGA |
| 1362 | BHK16 | [Last 70% of CDR H1]-TGGATCCGGCAGCCCGCCGGG |
| 1363 | BHK17 | [Last 70% of CDR H1]-TGGGTGCCACAGGCCCCTGGA |

**Table 45. Heavy Chain FR2 (Kabat Definition) Antibody-Specific Reverse Primers (for Sub-Library 6):**

| | | |
|---|---|---|
| 1364 | BHK1' | [First 70% of CDR H2]-TCCCATCCACTCAAGCCYTTG |
| 1365 | BHK2' | [First 70% of CDR H2]-TCCCATCCACTCAAGCSCTT |
| 1366 | BHK3' | [First 70% of CDR H2]-WGAGACCCACTCCAGCCCCTT |
| 1367 | BHK4' | [First 70% of CDR H2]-CCAATCCACTCCAGKCCCTT |
| 1368 | BHK5' | [First 70% of CDR H2]-TGAGACCCACTCCAGRCCCTT |
| 1369 | BHK6' | [First 70% of CDR H2]-GCCAACCCACTCCAGCCCYTT |
| 1370 | BHK7' | [First 70% of CDR H2]-KGCCACCCACTCCAGCCCCTT |
| 1371 | BHK8' | [First 70% of CDR H2]-TCCCAGCCACTCAAGGCCTC |
| 1372 | BHK9' | [First 70% of CDR H2]-CCCATCCACTCCAGGCCTT |
| 1373 | BHK10' | [First 70% of CDR H2]-TGARACCCACWCCAGCCCCTT |
| 1374 | BHK12' | [First 70% of CDR H2]-MGAKACCCACTCCAGMCCCTT |
| 1375 | BRK13' | [First 70% of CDR H2]-YCCMATCCACTCMAGCCCYTT |
| 1376 | BHK14' | [First 70% of CDR H2]-TCCTATCCACTCAAGGCGTTG |
| 1377 | BHK15' | [First 70% of CDR H2]-TGCAAGCCACTCCAGGGCCTT |
| 1378 | BHK16' | [First 70% of CDR H2]-TGAAACATATTCCAGTCCCTT |
| 1379 | BHK17' | [First 70% of CDR H2]-CGATACCCACTCCAGCCCCTT |

PCR is carried out with BHK1 to BHK17 in combination with BHK1' to BHK17' using sub-bank 6 as a template. This generates combinatorial sub-library 6 or a pool of oligonucleotides corresponding to sequences described in Table 6.

By way of example but not limitation, the combinatorial sub-library 7 is constructed using the Polymerase Chain Reaction (PCR) by overlap extension using the oligonucleotides in Table 46 and Table 47 (all shown in the 5' to 3' orientation, name followed by sequence) where K= G or T, M= A or C, R= A or G, S= C or G, W= A or T and Y= C or T.

**Table 46. Heavy Chain FR3 (Kabat Definition) Antibody-Specific Forward Primers (for Sub-Library 7):**

| | | |
|---|---|---|
| 1380 | CHK1 | [Last 70% of CDR H2]-AGAGTCACCATGACCAGGRAC |
| 1381 | CHK2 | [Last 70% of CDR H2]-AGGCTCACCATCWCCAAGGAC |
| 1382 | CHK3 | [Last 70% of CDR H2]-CGAGTYACCATATCAGTAGAC |
| 1383 | CHK4 | [Last 70% of CDR H2]-CGATTCACCATCTCCAGRGAC |
| 1384 | CHK5 | [Last 70% of CDR H2]-AGATTCACCATCTCMAGAGA |
| 1385 | CHK6 | [Last 70% of CDR H2]-MGGTTCACCATCTCCAGAGA |
| 1386 | CHK7 | [Last 70% of CDR H2]-CGATTCAYCATCTCCAGAGA |
| 1387 | CHK8 | [Last 70% of CDR H2]-CGAGTCACCATRTCMGTAGAC |
| 1388 | CHK9 | [Last 70% of CDR H2]-AGRGTCACCATKACCAGGGAC |
| 1389 | CHK10 | [Last 70% of CDR H2]-CAGGTCACCATCTCAGCCGAC |
| 1390 | CHK11 | [Last 70% of CDR H2]-CGAATAACCATCAACCCAGAC |
| 1391 | CHK12 | [Last 70% of CDR H2]-CGGTTTGTCTTCTCCATGGAC |
| 1392 | CHK13 | [Last 70% of CDR H2]-AGAGTCACCATGACCGAGGAC |
| 1393 | CHK14 | [Last 70% of CDR H2]-AGAGTCACGATTACCGCGGAC |
| 1394 | CHK15 | [Last 70% of CDR H2]-AGAGTCACCATGACCACAGAC |

**Table 47. Heavy Chain FR3 (Kabat Definition) Antibody (for Sub-Library 7)**

| | | |
|---|---|---|
| 1395 | CHK1' | [First 70% of CDR H3]-TCTAGYACAGTAATACACGGC |
| 1396 | CHK2' | [First 70% of CDR H3]-TCTCGCACAGTAATACAYGGC |
| 1397 | CHK3' | [First 70% of CDR H3]-TCTYGCACAGTAATACACAGC |
| 1398 | CHK4' | [First 70% of CDR H3]-TGYYGCACAGTAATACACGGC |
| 1399 | CHK5' | [First 70% of CDR H3]-CCGTGCACARTAATAYGTGGC |
| 1400 | CHK6' | [First 70% of CDR H3]-TCTGGCACAGTAATACACGGC |
| 1401 | CHK7' | [First 70% of CDR H3]-TGTGGTACAGTAATACACGGC |
| 1402 | CHK8' | [First 70% ofCDR H3]-TCTCGCACAGTGATACAAGGC |
| 1403 | CHK9' | (First 70% of CDR H3]-TTTTGCACAGTAATACAAGGC |
| 1404 | CHK10' | [First 70% of CDR H3]-TCTTGCACAGTAATACATGGC |
| 1405 | CHK11' | [First 70% of CDR H3]-GTGTGCACAGTAATATGTGGC |
| 1406 | CHK12' | [First 70% of CDR H3]-TTTCGCACAGTAATATACGGC |
| 1407 | CHK13' | [First 70% of CDR H3]-TCTCACACAGTAATACACAGC |

PCR is carried out with CHK1 to CHK15 in combination with CHK1' to CHK13' using sub-bank 7 as a template. This generates combinatorial sub-library 7 or a pool of oligonucleotides corresponding to sequences described in Table 7.

By way of example but not limitation, the combinatorial sub-library 8 is constructed using the Polymerase Chain Reaction (PCR) by overlap extension using the oligonucleotides in Table 48 and Table 49 (all shown in the 5' to 3' orientation, name followed by sequence) where K= G or T, M= A or C, R= A or G, S= C or G, W= A or T and Y= C or T.

**Table 48. Heavy Chain FR1 (Chothia Definition) Antibody-Specific Forward Primers (for Sub-Library 8):**

| | | |
|---|---|---|
| 1408 | AH1 | CAGGTKCAGCTGGTGCAGTCT |
| 1409 | AH2 | GAGGTGCAGCTGKTGGAGTCT |
| 1410 | AH3 | CAGSTGCAGCTGCAGGAGTCG |
| 1411 | AH4 | CAGGTCACCTTGARGGAGTCT |
| 1412 | AH5 | CARATGCAGCTGGTGCAGTCT |
| 1413 | AH6 | GARGTGCAGCTGGTGSAGTC |
| 1414 | AH7 | CAGATCACCTTGAAGGAGTCT |
| 1415 | AH8 | CAGGTSCAGCTGGTRSAGTCT |
| 1416 | AH9 | CAGGTACAGCTGCAGCAGTCA |
| 1417 | AH10 | CAGGTGCAGCTACAGCAGTGG |

**Table 49. Heavy Chain FR1 (Chothia Definition) Antibody-Specific Reverse Primers (for Sub-Library 8)**

| | | |
|---|---|---|
| 1418 | AHC1' | [First 70% of CDR H1]- RGAARCCTTGCAGGAGACCTT |
| 1419 | AHC2' | [First 70% of CDR H1]- RGAAGCCTTGCAGGAAACCTT |
| 1420 | AHC3' | [First 70% of CDR H1]- AGATGCCTRGCAGGAAACCTT |
| 1421 | AHC4' | [First 70% of CDR H1]- AGAGAMGGTGCAGGTCAGCGT |
| 1422 | AHC5' | [First 70% of CDR H1]- AGASGCTGCACAGGAGAGTCT |
| 1423 | AHC6' | [First 70% of CDR H1]- AGAGACAGTRCAGGTGAGGGA |
| 1424 | AHC7' | [First 70% of CDR H1]- AKAGACAGCGCAGGTGAGGGA |
| 1425 | AHC8' | [First 70% of CDR H1]- AGAGAAGGTGCAGGTCAGTGT |
| 1426 | AHC9' | [First 70% of CDR H1]- AGAAGCTGTACAGGAGAGTCT |
| 1427 | AHC10' | [First 70% of CDR H1]- AGAGGCTGCACAGGAGAGTTT |
| 1428 | AHC12' | [First 70% of CDR H1]- AGAACCCTTACAGGAGATCTT |
| 1429 | AHC13' | [First 70% of CDR H1]- GGAGATGGCACAGGTGAGTGA |

PCR is carried out with AH1 to AH10 in combination with AHC1' to AHC13' using sub-bank 8 as a template. This generates combinatorial sub-library 8 or a pool of oligonucleotides corresponding to sequences described in Table 8.

By way of example but not limitation, the combinatorial sub-library 9 is constructed using the Polymerase Chain Reaction (PCR) by overlap extension using the oligonucleotides in Table 50 and Table 51 (all shown in the 5' to 3' orientation, name followed by sequence) where K= G or T, M= A or C, R= A or G, S= C or G, W= A or T and Y= C or T.

**Table 50. Heavy Chain FR2 (Chothia Definition) Antibody-Specific Forward Primers (for Sub-Library 9):**

| | | |
|---|---|---|
| 1430 | BHC1 | [Last 70% of CDR H1]-TATGGYATSAGCTGGGTGCGM |
| 1431 | BHC2 | [Last 70% of CDR H 1]-ATGKGTGTGAGCTGGATCCGT |
| 1432 | BHC3 | [Last 70% of CDR H1]-TACTACTGGRGCTGGATCCGS |
| 1433 | BHC4 | [Last 70% of CDR H1]-TATGCYATSAGCTGGGTSCGM |
| 1434 | BHC5 | [Last 70% of CDR H1]-TCTGCTATGCASTGGGTSCGM |
| 1435 | BHC6 | [Last 70% of CDR H1]-TATGCYATGCAYTGGGTSCGS |
| 1436 | BHC7 | [Last 70% of CDR H1]-CGCTACCTGCACTGGGTGCGA |
| 1437 | BHC8 | [Last 70% of CDR H1]-TTATCCATGCACTGGGTGCGA |
| 1438 | BHC9 | [Last 70% of CDR H1]-GCCTGGATGAGCTGGGTCCGC |
| 1439 | BHC10 | [Last 70% of CDR H1]-GCTGCTTGGAACTGGATCAGG |
| 1440 | BHC11 | [Last 70% of CDR H1]-AATGAGATGAGCTGGATCCGC |
| 1441 | BHC12 | [Last 70% of CDR H1]-AACTACATGAGCTGGGTCCGC |
| 1442 | BHC13 | [Last 70% of CDR H1]-AACTGGTGGGGCTGGATCCGG |
| 1443 | BHC14 | [Last 70% of CDR H1]-GTGGGTGTGGGCTGGATCCGT |
| 1444 | BHC15 | [Last 70% of CDR H1]-CACTACATGGACTGGGTCCGC |
| 1445 | BHC16 | [Last 70% of CDR H1]-AGTGACATGAACTGGGCCCGC |
| 1446 | BHC17 | [Last 70% of CDR H1]-AGTGACATGAACTGGGTCCAT |
| 1447 | BHC18 | [Last 70% of CDR H1]-TATACCATGCACTGGGTCCGT |
| 1448 | BHC19 | [Last 70% of CDR H 1]-TATGCTATGCACTGGGTCCGC |
| 1449 | BHC20 | [Last 70% of CDR H1]-TATGCTATGAGCTGGTTCCGC |
| 1450 | BHC21 | [Last 70% of CDR H 1]-TATAGCATGAACTGGGTCCGC |
| 1451 | BHC22 | [Last 70% of CDR H1]-TATGGCATGCACTGGGTCCGC |
| 1452 | BHC23 | [Last 70% of CDR H1]-TATTGGATGAGCTGGGTCCGC |
| 1453 | BHC24 | [Last 70% of CDR H 1]-TACGACATGCACTGGGTCCGC |
| 1454 | BHC25 | [Last 70% of CDR H1]-TACTACATGAGCTGGATCCGC |
| 1455 | BHC26 | [Last 70% of CDR H1]-TACTGGATGCACTGGGTCCGC |
| 1456 | BHC27 | [Last 70% of CDR H 1]-TACTGGATCGGCTGGGTGCGC |
| 1457 | BHC28 | [Last 70% of CDR H 1]-TACTATATGCACTGGGTGCGA |
| 1458 | BHC29 | [Last 70% of CDR H1]-TATGATATCAACTGGGTGCGA |
| 1459 | BHC30 | [Last 70% of CDR H1]-TATGGTATGAATTGGGTGCCA |

**Table 51. Heavy Chain FR2 (Chothia Definition) Antibody-Specific Reverse Primers (for Sub-Library 9)**

| | | |
|---|---|---|
| 1460 | BHC1' | [First 70% of CDR H2]-AATASCWGAGACCCACTCCAG |
| 1461 | BHC2' | [First 70% of CDR H2]-AATAASWGAGACCCACTCCAG |
| 1462 | BHC3' | [First 70% of CDR H2]-GMTCCATCCCATCCACTCAAG |
| 1463 | BHC4' | [First 70% of CDR H2]-GATACKCCCAATCCACTCCAG |
| 1464 | BHC5' | [First 70% of CDR H2]-GATRTACCCAATCCACTCCAG |
| 1465 | BHC6' | [First 70% of CDR H2]-AATGWGTGCAAGCCACTCCAG |
| 1466 | BHC7' | [First 70% of CDR H2]-AAYACCYGAKACCCACTCCAG |
| 1467 | BHC8' | [First 70% of CDR H2]-AATGKATGARACCCACTCCAG |
| 1468 | BHC9' | [First 70% of CDR H2]-ARTACGGCCAACCCACTCCAG |
| 1469 | BHC10' | [First 70% of CDR H2]-AAAACCTCCCATCCACTCAAG |
| 1470 | BHC12' | [First 70% of CDR H2]-GATTATTCCCATCCACTCAAG |
| 1471 | BHC13' | [First 70% of CDR H2]-GATCCATCCTATCCACTCAAG |
| 1472 | BHC14' | [First 70% ofCDR H2]-GAACCATCCCATCCACTCAAG |
| 1473 | BHC15' | [First 70% of CDR H2]-GATCCCTCCCATCCACTCAAG |
| 1474 | BHC16' | [First 70% of CDR H2]-CATCCATCCCATCCACTCAAG |
| 1475 | BHC17' | [First 70% of CDR H2]-TGTCCTTCCCAGCCACTCAAG |
| 1476 | BHC18' | [First 70% of CDR H2]-AATACGTGAGACCCACACCAG |
| 1477 | BHC19' | [First 70% of CDR H2]-AATAGCTGAAACATATTCCAG |
| 1478 | BHC20' | [First 70% of CDR H2]-GATTTCCCCAATCCACTCCAG |
| 1479 | BHC21' | [First 70% of CDR H2]-GATGATCCCCATCCACTCCAG |
| 1480 | BHC22' | [First 70% of CDR H2]-TATAACTGCCACCCACTCCAG |
| 1481 | BHC23' | [First 70% of CDR H2]-AATGAAACCTACCCACTCCAG |
| 1482 | BHC24' | [First 70% of CDR H2]-TATGTTGGCCACCCACTCCAG |

PCR is carried out with BHC1 to BHC30 in combination with BHC1' to BHC24' using sub-bank 9 as a template. This generates combinatorial sub-library 9 or a pool of oligonucleotides corresponding to sequences described in Table 9.

By way of example but not limitation, the combinatorial sub-library 10 is constructed using the Polymerase Chain Reaction (PCR) by overlap extension using the oligonucleotides in Table 52 and Table 53 (all shown in the 5' to 3' orientation, name followed by sequence) where K= G or T, M= A or C, R= A or G, S= C or G, W= A or T and Y= C or T.

**Table 52. Heavy Chain FR3 (Chothia Definition) Antibody-Specific Forward Primers (for Sub-Library 10):**

| | | |
|---|---|---|
| 1483 | CHC1 | [Last 70% of CDR H2]-ACCAACTACAACCCSTCCCTC |
| 1484 | CHC2 | [Last 70% of CDR H2]-ATATACTACGCAGACTCWGTG |
| 1485 | CHC3 | [Last 70% of CDR H2]-ACATACTAYGCAGACTCYGTG |
| 1486 | CHC4 | [Last 70% of CDR H2]-ACMAACTACGCACAGAARTTC |
| 1487 | CHC5 | [Last 70% of CDR H2]-ACAAACTATGCACAGAAGYT |
| 1488 | CHC6 | [Last 70% of CDR H2]-ACARGCTAYGCACAGAAGTTC |
| 1489 | CHC7 | [Last 70% of CDR H2]-AYAGGYTATGCRGACTCTGTG |
| 1490 | CHC8 | [Last 70% of CDR H2]-AAATMCTACAGCACATCTCTG |
| 1491 | CHC9 | [Last 70% of CDR H2]-AAATACTATGTGGACTCTGTG |
| 1492 | CHC10 | [Last 70% of CDR H2]-CCAACATATGCCCAGGGCTTC |
| 1493 | CHC11 | [Last 70% of CDR H2]-GCAAACTACGCACAGAAGTTC |
| 1494 | CHC12 | [Last 70% of CDR H2]-AAATACTATGCAGACTCCGTG |
| 1495 | CHCl3 | [Last 70% of CDR H2]-AAGCGCTACAGCCCATCTCTG |
| 1496 | CHC14 | [Last 70% of CDR H2]-AATGATTATGCAGTATCTGTG |
| 1497 | CHC15 | [Last 70% of CDR H2]-ACCAGATACAGCCCGTCCTTC |
| 1498 | CHC16 | [Last 70% of CDR H2]-ACAGAATACGCCGCGTCTGTG |
| 1499 | CHC17 | [Last 70% of CDR H2]-ACGCACTATGCAGACTCTGTG |
| 1500 | CHC18 | [Last 70% of CDR H2]-ACGCACTATGTGGACTCCGTG |
| 1501 | CHC19 | [Last 70% of CDR H2]-ACAATCTACGCACAGAAGTTC |
| 1502 | CHC20 | [List 70% of CDR H2]-ACAAAATATTCACAGGAGTTC |
| 1503 | CHC21 | [Last 70% of CDR H2]-ACATACTACGCAGACTCCAGG |
| 1504 | CHC22 | [Last 70% of CDR H2]-ACAAGCTACGCGGACTCCGTG |
| 1505 | CHC23 | [Last 70% of CDR H2]-ACATATTATGCAGACTCTGTG |
| 1506 | CHC24 | [Last 70% of CDR H2]-ACAGACTACGCTGCACCCGTG |
| 1507 | CHC25 | [Last 70% of CDR H2]-ACAGCATATGCTGCGTCGGTG |
| 1508 | CHC26 | [Last 70% of CDR H2]-ACATACTATCCAGGCTCCGTG |
| 1509 | CHC27 | [Last 70% of CDR H2]-ACCTACTACAACCCGTCCCTC |

**Table 53. Heavy Chain FR3 (Chothia Definition) Antibody-Specific Reverse Primers (for Sub-Library 10):**

| | | |
|---|---|---|
| 1510 | CHC1' | [First 70% of CDR H3]- TSTYGCACAGTAATACACGGC |
| 1511 | CHC2' | [First 70% of CDR H3]- TCTYGCACAGTAATACATGGC |
| 1512 | CHC3' | [First 70% of CDR H3]- TCTAGYACAGTAATACACGGC |
| 1513 | CHC4' | [First 70% of CDR H3]- CCGTGCACARTAATAYGTGGC |
| 1514 | CHC5' | [First 70% of CDR H3]- TCTYGCACAGTAATACACAGC |
| 1515 | CHC6' | [First 70% of CDR H3]- GTGTGCACAGTAATATGTGGC |
| 1516 | CHC7' | [First 70% of CDR H3]- TGCCGCACAGTAATACACGGC |
| 1517 | CHC8' | [First 70% of CDR H3]- TGTGGTACAGTAATACACGGC |
| 1518 | CHC9' | [First 70% of CDR H3]- TCTCACACAGTAATACACAGC |
| 1519 | CHC10' | [First 70% of CDR H3]- TCTCGCACAGTGATACAAGGC |
| 1520 | CHC11' | [First 70% of CDR H3]-TTTCGCACAGTAATATACGGC |
| 1521 | CHC12' | [First 70% of CDR H3]- TCTGGCACAGTAATACACGGC |
| 1522 | CHC13' | [First 70% of CDR H3]- TTTTGCACAGTAATACAAGGC |

PCR is carried out with CHC1 to CHC27 in combination with CHC1' to CHC13' using sub-bank 10 as a template. This generates combinatorial sub-library 10 or a pool of oligonucleotides corresponding to sequences described in Table 10.

By way of example but not limitation, the combinatorial sub-library 11 is constructed using the Polymerase Chain Reaction (PCR) by overlap extension using the oligonucleotides in Table 54 and Table 55 (all shown in the 5' to 3' orientation, name followed by sequence) where K= G or T, M= A or C, R= A or G, S= C or G, W= A or T and Y= C or T.

**Table 54. Heavy Chain FR4 (Kabat and Chothia Definition) Antibody-Specific Forward Primers (for Sub-Library 11):**

| | | |
|---|---|---|
| 1523 | DH1 | [Last 70% of CDR H3]-TGGGGCCARGGMACCCTGGTC |
| 1524 | DH2 | [Last 70% of CDR H3]-TGGGGSCAAGGGACMAYGGTC |
| 1525 | DH3 | [Last 70% of CDR H3]-TGGGGCCGTGGCACCCTGGTC |

**Table 55. Heavy Chain FR4 (Kabat and Chothia Definition) Antibody-Specific Reverse Primers (for Sub-Library 11)**

| | | |
|---|---|---|
| 1526 | DH1' | TGAGGAGACRGTGACCAGGGT |
| 1527 | DH2' | TGARGAGACGGTGACCRTKGT |
| 1528 | DH3' | TGAGGAGACGGTGACCAGGGT |

PCR is carried out with DH1 to DHC3 in combination with DH1' to DH3' using sub-bank 11 as a template. This generates combinatorial sub-library 11 or a pool of oligonucleotides corresponding to sequences described in Table 11.

In some embodiments, nine combinatorial sub-libraries can be constructed using direct ligation of non-human CDRs and the human frameworks of the sub-banks. For example, combinatorial sub-libraries 1', 2' and 3' are built separately by direct ligation of the non-human CDRs L1, L2 and L3 (in a single stranded or double stranded form) to sub-banks 1, 2 and 3, respectively. In one embodiment, the non-human CDRs (L1, L2 and L3) are single strand nucleic acids. In another embodiment, the non-human CDRs (L1, L2 and L3) are double strand nucleic acids. Alternatively, combinatorial sub-libraries 1', 2' and 3' can be obtained by direct ligation of the non-human CDRs (L1, L2 and L3) in a single stranded (+) form to the nucleic acid 1-46 listed in Table 1, nucleic acid 47-92 listed in Table 2, and nucleic acid 93-138 listed in Table 3, respectively.

In some embodiments, combinatorial sub-libraries 5' and 6' are built separately by direct ligation of the non-human CDRs H1 and H2 (in a single stranded or double stranded form and according to Kabat definition) to sub-banks 5 and 6, respectively. Alternatively, sub-libraries 5' and 6' can be obtained by direct ligation of the non-human CDRs H1 and H2 (according to Kabat definition and in a single stranded (+) form) to nucleic acid 144 to 187 listed in Table 5 and 188 to 231 listed in Table 6, respectively.

In some embodiments, combinatorial sub-libraries 8' and 9'are built separately by direct ligation of the non-human CDRs H1 and H2 (in a single stranded or double stranded form and according to Chothia definition) to sub-banks 8 and 9, respectively. Alternatively, sub-libraries 8' and 9' can be obtained by direct ligation of the non-human CDRs H1 and H2 (according to Chothia definition and in a single stranded (+) form) to nucleic acid 276 to 319 listed in Table 8 and 320 to 363 of Table 9, respectively.

Combinatorial sub-libraries 11' and 12' are built separately by direct ligation of the non-human CDR H3 (in a single stranded or double stranded form) to sub-bank 7 (Kabat definition) and 10 (Chothia definition), respectively. Alternatively, sub-libraries 11' and 12' can be obtained by direct ligation of non-human CDR H3 (in a single stranded (+) form) to nucleic acid 232 to 275 listed in Table 7 and 364 to 407 of Table 10, respectively.

Direct ligation of DNA fragments can be carried out according to standard protocols. It can be followed by purification/separation of the ligated products from the unligated ones.

### 2.5 Construction of Combinatorial Libraries

Combinatorial libraries are constructed by assembling together combinatorial sub-libraries of corresponding variable light chain region or variable heavy chain region. For example, combinatorial library of human kappa light chain germline frameworks (combination library 1) can be built by assembling together sub-libraries 1, 2, 3 and 4 through overlapping regions in the CDRs as described below; two combinatorial libraries of human heavy chain germline frameworks (one for Kabat definition of the CDRs, combination library 2, and one for Chothia definition of the CDRs,combination library 3) can be built by assembling together sub-libraries 5, 6, 7, 11 (Kabat definition) or sub-libraries 8, 9, 10, 11 (Chothia definition) through overlapping regions in the CDRs as described below.

In one embodiment, the construction of combinatorial library 1 is carried out using the Polymerase Chain Reaction (PCR) by overlap extension using the oligonucleotides listed in Table 56 and Table 57 (all shown in the 5' to 3' orientation, the name of the primer followed by the sequence):

**Table 56. Light Chain Forward Primers (for Combinatorial Library 1):**

| | | |
|---|---|---|
| 1529 | AL1 | GATGTTGTGATGACWCAGTCT |
| 1530 | AL2 | GACATCCAGATGAYCCAGTCT |
| 1531 | AL3 | GCCATCCAGWTGACCCAGTCT |
| 1532 | AL4 | GAAATAGTGATGAYGCAGTCT |
| 1533 | AL5 | GAAATTGTGTTGACRCAGTCT |
| 1534 | AL6 | GAKATTGTGATGACCCAGACT |
| 1535 | AL7 | GAAATTGTRMTGACWCAGTCT |
| 1536 | AL8 | GAYATYGTGATGACYCAGTCT |
| 1537 | AL9 | GAAACGACACTCACGCAGTCT |
| 1538 | AL10 | GACATCCAGTTGACCCAGTCT |
| 1539 | AL11 | AACATCCAGATGACCCAGTCT |
| 1540 | AL12 | GCCATCCGGATGACCCAGTCT |
| 1541 | AL13 | GTCATCTGGATGACCCAGTCT |

**Table 57. Light Chain Reverse Primers (for Combinatorial Library 1):**

| | | |
|---|---|---|
| 1542 | DL1' | TTTGATYTCCACCTTGGTCCC |
| 1543 | DL2' | TTTGATCTCCAGCTTGGTCCC |
| 1544 | DL3' | TTTGATATCCACTTTGGTCCC |
| 1545 | DL4' | TTTAATCTCCAGTCGTGTCCC |

PCR is carried out with AL1 to AL 13 in combination with DL1' to DL4' using sub-libraries 1, 2, 3 and 4 together or a pool of oligonucleotides corresponding to sequences described in Table 1, 2, 3 and 4 as a template. This generates combinatorial library 1.

In one embodiment, the construction of combinatorial library 2 and 3 is carried out using the Polymerase Chain Reaction (PCR) by overlap extension using the oligonucleotides listed in Table 58 and Table 59 (all shown in the 5' to 3' orientation, name followed by sequence):

**Table 58. Heavy Chain Forward Primers (for Combinatorial Library 2 and 3, Kabat and Chothia Definition):**

| | | |
|---|---|---|
| 1546 | AH1 | CAGGTKCAGCTGGTGCAGTCT |
| 1547 | AH2 | GAGGTGCAGCTGKTGGAGTCT |
| 1548 | AH3 | CAGSTGCAGCTGCAGGAGTCG |
| 1549 | AH4 | CAGGTCACCTTGARGGAGTCT |
| 1550 | AH5 | CARATGCAGCTGGTGCAGTCT |
| 1551 | AH6 | GARGTGCAGCTGGTGSAGTC |
| 1552 | AH7 | CAGATCACCTTGAAGGAGTCT |
| 1553 | AH8 | CAGGTSCAGCTGGTRSAGTCT |
| 1554 | AH9 | CAGGTACAGCTGCAGCAGTCA |
| 1555 | AH10 | CAGGTGCAGCTACAGCAGTGG |

**Table 59. Heavy Chain Reverse Primers (for Combinatorial Library 2 and 3, Kabat and Chothia Definition):**

| | | |
|---|---|---|
| 1556 | DH1' | TGAGGAGACRGTGACCAGGGT |
| 1557 | DH2' | TGARGAGACGGTGACCRTKGT |
| 1558 | DH3' | TGAGGAGACGGTGACCAGGGT |

PCR is carried out with AH1 to AH10 in combination with DH1' to DH3' using sub-libraries 5, 6, 7, 11 together, or a pool of oligonucleotides corresponding to sequences described in Table 5, 6, 7 and 11, or sub-libraries 8, 9, 10, 11, or a pool of oligonucleotides corresponding to sequences described in Table 8, 9, 10 and 11, together as a template. This generates Combinatorial library 2 or 3, respectively.

In another embodiment, combinatorial libraries are constructed by direct ligation. For example, combinatorial library of human kappa light chain germline frameworks (combination library 1') is built by direct sequential ligation of sub-libraries 1', 2', 3' and sub-bank 4 (or nucleic acids 139 to 143, see Table 4) together. This is followed by a Polymerase Chain Reaction step using the oligonucleotides described in Table 60 and Table 61. Two combinatorial libraries of human heavy chain germline framework regions (one for Kabat definition of the CDRs, combination library 2'; and one for Chothia definition of the CDRs, combination library 3') are built by direct sequential ligation of sub-libraries 5', 6', 11' and sub-bank 11 (Kabat definition) or of sub-libraries 8', 9', 12' and sub-bank 11 (Chothia definition) together. Alternatively, sub-bank 11 can be substituted with nucleic acids 408 to 412 (see Table 11) in the ligation reactions. This is followed by a Polymerase Chain Reaction step using the oligonucleotides described in Table 62 and Table 63.

**Table 60. Light Chain Forward Primers (for Combinatorial Library 1'):**

| | | |
|---|---|---|
| 1559 | AL1 | GATGTTGTGATGACWCAGTCT |
| 1560 | AL2 | GACATCCAGATGAYCCAGTCT |
| 1561 | AL3 | GCCATCCAGWTGACCCAGTCT |
| 1562 | AL4 | GAAATAGTGATGAYGCAGTCT |
| 1563 | AL5 | GAAATTGTGTTGACRCAGTCT |
| 1564 | AL6 | GAKATTGTGATGACCCAGACT |
| 1565 | AL7 | GAAATTGTRMTGACWCAGTCT |
| 1566 | AL8 | GAYATYGTGATGACYCAGTCT |
| 1567 | AL9 | GAAACGACACTCACGCAGTCT |
| 1568 | AL10 | GACATCCAGTTGACCCAGTCT |
| 1569 | AL11 | AACATCCAGATGACCCAGTCT |
| 1570 | AL12 | GCCATCCGGATGACCCAGTCT |
| 1571 | AL13 | GTCATCTGGATGACCCAGTCT |

**Table 61. Light Chain Reverse Primers (for Combinatorial Library 1'):**

| | | |
|---|---|---|
| 1572 | DL1' | TTTGATYTCCACCTTGGTCCC |
| 1573 | DL2' | TTTGATCTCCAGCTTGGTCCC |
| 1574 | DL3' | TTTGATATCCACTTTGGTCCC |
| 1575 | DL4' | TTTAATCTCCAGTCGTGTCCC |

PCR is carried out with AL1 to AL 13 in combination with DL1' to DL4' using sub-libraries 1', 2', 3' and sub-bank 4 (or nucleic acids 139 to 143, see Table 4) previously ligated together as a template. This generates combinatorial library 1'.

**Table 62. Heavy Chain Forward Primers (for Combinatorial Library 2' and 3', Kabat and Chothia Definition):**

| | | |
|---|---|---|
| 1576 | AH1 | CAGGTKCAGCTGGTGCAGTCT |
| 1577 | AH2 | GAGGTGCAGCTGKTGGAGTCT |
| 1578 | AH3 | CAGSTGCAGCTGCAGGAGTCG |
| 1579 | AH4 | CAGGTCACCTTGARGGAGTCT |
| 1580 | AH5 | CARATGCAGCTGGTGCAGTCT |
| 1581 | AH6 | GARGTGCAGCTGGTGSAGTC |
| 1582 | AH7 | CAGATCACCTTGAAGGAGTCT |
| 1583 | AH8 | CAGGTSCAGCTGGTRSAGTCT |
| 1584 | AH9 | CAGGTACAGCTGCAGCAGTCA |
| 1585 | AH10 | CAGGTGCAGCTACAGCAGTGG |

**Table 63. Heavy Chain Reverse Primers (for Combinatorial Library 2' and 3', Kabat and Chothia Definition):**

| | | |
|---|---|---|
| 1586 | DH1' | TGAGGAGACRGTGACCAGGGT |
| 1587 | DH2' | TGARGAGACGGTGACCRTKGT |
| 1588 | DH3' | TGAGGAGACGGTGACCAGGGT |

PCR is carried out with AH1 to AH10 in combination with DH1' to DH3' using sub-libraries 5', 6', 11' and sub-bank 11 (or nucleic acids 408 to412, see Table 11) previously ligated together or sub-libraries 8', 9', 12' and sub-bank 11 (or nucleic acids 408 to413, see Table 11) previously ligated together as a template. This generates combinatorial library 2' or 3', respectively.

The sub-banks of framework regions, sub-banks of CDRs,combinatorial sub-libraries, and combinatorial libraries can be stored for a later use. The nucleic acids can be stored in a solution, as a dry sterilized lyophilized powder, or a water free concentrate in a hermetically sealed container. In cases where the nucleic acids are not stored in a solution, the nucleic acids can be reconstituted (e.g., with water or saline) to the appropriate concentration for a later use. The sub-banks, combinatorial sub-libraries and combinatorial libraries are preferably stored at between 2°C and 8°C in a container indicating the quantity and concentration of the nucleic acids.

### 2.6 Expression of the Combinatorial Libraries

The combinatorial libraries can be expressed using any methods known in the art, including but not limited to, bacterial expression system, mammalian expression system, and *in vitro* ribosomal display system.

In preferred embodiments, the present invention encompasses the use of phage vectors to express the combinatorial libraries. Phage vectors have particular advantages of providing a means for screening a very large population of expressed display proteins and thereby locate one or more specific clones that code for a desired binding activity.

The use of phage display vectors to express a large population of antibody molecules are well known in the art and will not be reviewed in detail herein. The method generally involves the use of a filamentous phage (phagemid) surface expression vector system for cloning and expressing antibody species of a library. See, *e.g.,* Kang et al., Proc. Natl. Acad. Sci., USA, 88:4363-4366 (1991); Barbas et al., Proc. Natl. Acad. Sci., USA, 88:7978-7982 (1991); Zebedee et al., Proc. Natl. Acad. Sci., USA, 89:3175-3179 (1992); Kang et al., Proc. Natl. Acad. Sci., USA, 88:11120-11123 (1991); Barbas et al., Proc. Natl. Acad. Sci., USA, 89:4457-4461 (1992); Gram et al., Proc. Natl. Acad. Sci., USA, 89:3576-3580 (1992); Brinkman et al., J. Immunol. Methods 182:41-50 (1995); Ames et al., J. Immunol. Methods 184:177-186 (1995); Kettleborough et al., Eur. J. Immunol. 24:952-958 (1994); Persic et al., Gene 187 9-18 (1997); Burton et al., Advances in Immunology 57:191-280 (1994); PCT application No. PCT/GB91/01134; PCT publication Nos. WO 90/02809; WO 91/10737; WO 92/01047; WO 92/18619; WO 93/11236; WO 95/15982; WO 95/20401; and U.S. Patent Nos. 5,698,426; 5,223,409; 5,403,484; 5,580,717; 5,427,908; 5,750,753; 5,821,047; 5,571,698; 5,427,908; 5,516,637; 5,780,225; 5,658,727; 5,733,743 and 5,969,108.

A preferred phagemid vector is a recombinant DNA molecule containing a nucleotide sequence that codes for and is capable of expressing a fusion polypeptide containing, in the direction of amino- to carboxy-terminus, (I) a prokaryotic secretion signal domain, (2) a heterologous polypeptide defining an immunoglobulin heavy or light chain variable region, and (3) a filamentous phage membrane anchor domain. The vector includes DNA expression control sequences for expressing the fusion polypeptide, preferably prokaryotic control sequences.

The filamentous phage membrane anchor is preferably a domain of the cpIII or cpVIII coat protein capable of associating with the matrix of a filamentous phage particle, thereby incorporating the fusion polypeptide onto the phage surface.

Preferred membrane anchors for the vector are obtainable from filamentous phage M13, fl, fd, and equivalent filamentous phage. Preferred membrane anchor domains are found in the coat proteins encoded by gene III and gene VIII. (See Ohkawa et al., J. Biol. Chem., 256:9951-9958, 1981). The membrane anchor domain of a filamentous phage coat protein is a portion of the carboxy terminal region of the coat protein and includes a region of hydrophobic amino acid residues for spanning a lipid bilayer membrane, and a region of charged amino acid residues normally found at the cytoplasmic face of the membrane and extending away from the membrane. For detailed descriptions of the structure of filamentous phage particles, their coat proteins and particle assembly, see the reviews by Rached et al., Microbiol. Rev., 50:401-427 (1986); and Model et al., in "The Bacteriophages: Vol. 2", R. Calendar, ed. Plenum Publishing Co., pp. 375-456 (1988).

The secretion signal is a leader peptide domain of a protein that targets the protein to the periplasmic membrane of gram negative bacteria. A preferred secretion signal is a pelB secretion signal. (Better et al., Science, 240:1041-1043 (1988); Sastry et al., Proc. Natl. Acad. Sci., USA, 86:5728-5732 (1989); and Mullinax et al., Proc. Natl. Acad. Sci., USA, 87:8095-8099 (1990)). The predicted amino acid residue sequences of the secretion signal domain from two pelB gene product variants from Erwinia carotova are described in Lei et al., Nature, 331:543-546 (1988). Amino acid residue sequences for other secretion signal polypeptide domains from *E. coli* useful in this invention as described in Oliver, Escherichia coli and Salmonella Typhimurium, Neidhard, F. C. (ed.), American Society for Microbiology, Washington, D.C., 1:56-69 (1987).

DNA expression control sequences comprise a set of DNA expression signals for expressing a structural gene product and include both 5' and 3' elements, as is well known, operatively linked to the gene. The 5' control sequences define a promoter for initiating transcription and a ribosome binding site operatively linked at the 5' terminus of the upstream translatable DNA sequence. The 3' control sequences define at least one termination (stop) codon in frame with and operatively linked to the heterologous fusion polypeptide.

Preferably, the vector used includes a prokaryotic origin of replication or replicon, *i.e.,* a DNA sequence having the ability to direct autonomous replication and maintenance of the recombinant DNA molecule extra-chromosomally in a prokaryotic host cell, such as a bacterial host cell, transformed therewith. Such origins of replication are well known in the art. Preferred origins of replication are those that are efficient in the host organism. A preferred host cell is *E*. *coli.* See Sambrook et al., in "Molecular Cloning: a Laboratory Manual", 2nd edition, Cold Spring Harbor Laboratory Press, New York (1989).

In addition, a prokaryotic replicon, where used, can also include a nucleic acid whose expression confers a selective advantage, such as drug resistance, to a bacterial host transformed therewith. Typical bacterial drug resistance genes are those that confer resistance to ampicillin, tetracycline, neomycin/kanamycin or chloramphenicol. Vectors typically also contain convenient restriction sites for insertion of translatable DNA sequences.

In someinstances, the vector is capable of co-expression of two cistrons contained therein, such as a nucleotide sequence encoding a variable heavy chain region and a nucleotide sequence encoding a variable light chain region. Co-expression has been accomplished in a variety of systems and therefore need not be limited to any particular design, so long as sufficient relative amounts of the two gene products are produced to allow assembly and expression of functional heterodimer.

A DNA expression vector can be designed for convenient manipulation in the form of a filamentous phage particle encapsulating a genome. In this instance, a DNA expression vector further contains a nucleotide sequence that defines a filamentous phage origin of replication such that the vector, upon presentation of the appropriate genetic complementation, can replicate as a filamentous phage in single stranded replicative form and be packaged into filamentous phage particles. This feature provides the ability of the DNA expression vector to be packaged into phage particles for subsequent segregation of the particle, and vector contained therein, away from other particles that comprise a population of phage particles.

A filamentous phage origin of replication is a region of the phage genome, as is well known, that defines sites for initiation of replication, termination of replication and packaging of the replicative form produced by replication (see for example, Rasched et al., Microbiol. Rev., 50:401-427, 1986; and Horiuchi, J. Mol. Biol., 188:215-223, 1986). A preferred filamentous phage origin of replication is an M13, fl or fd phage origin of replication (Short et al., Nucl. Acids Res., 16:7583-7600, 1988).

The method for producing a heterodimeric immunoglobulin molecule generally involves (1) introducing a large population of display vectors each capable of expressing different putative binding sites displayed on a phagemid surface display protein to a filamentous phage particle, (2) expressing the display protein and binding site on the surface of a filamentous phage particle, and (3) isolating (screening) the surface-expressed phage particle using affinity techniques such as panning of phage particles against a preselected antigen, thereby isolating one or more species of phagemid containing a display protein containing a binding site that binds a preselected antigen.

The isolation of a particular vector capable of expressing an antibody binding site of interest involves the introduction of the dicistronic expression vector able to express the phagemid display protein into a host cell permissive for expression of filamentous phage genes and the assembly of phage particles. Typically, the host is *E. coli.* Thereafter, a helper phage genome is introduced into the host cell containing the phagemid expression vector to provide the genetic complementation necessary to allow phage particles to be assembled.

The resulting host cell is cultured to allow the introduced phage genes and display protein genes to be expressed, and for phage particles to be assembled and shed from the host cell. The shed phage particles are then harvested (collected) from the host cell culture media and screened for desirable antibody binding properties. Typically, the harvested particles are "panned" for binding with a preselected antigen. The strongly binding particles are then collected, and individual species of particles are clonally isolated and further screened for binding to the antigen. Phages which produce a binding site of desired antigen binding specificity are selected.

After phage selection, the antibody coding regions from the phage can be isolated and used to generate whole antibodies or any other desired antigen binding fragment, and expressed in any desired host, including mammalian cells, insect cells, plant cells, yeast, and bacteria, e.g., as described in detail below. For example, techniques to recombinantly produce Fab, Fab' and F(ab')₂ fragments can also be employed using methods known in the art such as those disclosed in International Publication No. WO 92/22324; Mullinax et al., BioTechniques 12(6):864-869 (1992); and Sawai et al., AJRI 34:26-34 (1995); and Better et al., Science 240:1041-1043 (1988. Examples of techniques which can be used to produce single-chain Fvs and antibodies include those described in U.S. Patent Nos. 4,946,778 and 5,258,498; Huston et al., Methods in Enzymology 203:46-88 (1991); Shu et al., PNAS 90:7995-7999 (1993); and Skerra et al., Science 240:1038-1040 (1988).

A Host cell can contain a vector or nucleotide sequence of this invention, the host cell can be *E. coli.*

A combinatorial library can be cloned into a M 13-based phage vector. This vector allows the expression of Fab fragments that contain the first constant domain of the human γ1 heavy chain and the constant domain of the human kappa (κ) light chain under the control of the lacZ promoter. This can be carried out by hybridization mutagenesis as described in Wu & An, 2003, Methods Mol. Biol., 207, 213-233; Wu, 2003, Methods Mol. Biol., 207, 197-212; and Kunkel et al., 1987, Methods Enzymol. 154, 367-382. Briefly, purified minus strands corresponding to the heavy and light chains to be cloned are annealed to two regions containing each one palindromic loop. Those loops contain a unique XbaI site which allows for the selection of the vectors that contain both V_{L} and V_{H} chains fused in frame with the human kappa (κ) constant and first human γ1 constant regions, respectively (Wu & An, 2003, Methods Mol. Biol., 207, 213-233, Wu, 2003, Methods Mol. Biol., 207, 197-212). Synthesized DNA is then electroporated into XL1-blue for plaque formation on XL1-blue bacterial lawn or production of Fab fragments as described in Wu, 2003, Methods Mol. Biol., 207, 197-212.

In addition to bacterial/phage expression systems, other host-vector systems may be utilized to express the combinatorial libraries. These include, but are not limited to, mammalian cell systems transfected with a vector or infected with virus (e.g., vaccinia virus, adenovirus, etc.); insect cell systems transfected with a vector or infected with virus (e.g., baculovirus); microorganisms such as yeast containing yeast vectors; or bacteria transformed with DNA, plasmid DNA, or cosmid DNA. *See e.g.,* Verma et al., J Immunol Methods. 216(1-2):165-81 (1998).

The expression elements of vectors vary in their strengths and specificities. Depending on the host-vector system utilized, any one of a number of suitable transcription and translation elements may be used. In a preferred aspect, each nucleic acid of a combinatorial library is part of an expression vector that expresses the humanized heavy and/or light chain or humanized heavy and/or light variable regions in a suitable host. In particular, such nucleic acids have promoters, preferably heterologous promoters, operably linked to the antibody coding region, said promoter being inducible or constitutive, and, optionally, tissue-specific. (See Section 5.7 for more detail.) In another particular embodiment, nucleic acid molecules are used in which the antibody coding sequences and any other desired sequences are flanked by regions that promote homologous recombination at a desired site in the genome, thus providing for intrachromosomal expression of the antibody encoding nucleic acids (Koller and Smithies, 1989, Proc. Natl. Acad. Sci. USA 86:8932-8935; Zijlstra et al., 1989, Nature 342:435-438).

The combinatorial libraries can also be expressed using *in vitro* systems, such as the ribosomal display systems (see Section 5.6 for detail).

### 2.7 Selection of Humanized Antibodies

The expressed combinatorial libraries can be screened for binding to the antigen recognized by the donor antibody using any methods known in the art. Preferably, a phage display library constructed and expressed as described in section 5.4. and 5.6, respectively, is screened for binding to the antigen recognized by the donor antibody, and the phage expressing V_{H} and/or V_{L} domain with significant binding to the antigen can be isolated from a library using the conventional screening techniques (e.g. as described in Harlow, E., and Lane, D., 1988, supra Gherardi, E et al. 1990. J. Immunol. meth. 126 p61-68). The phage particles shed from host cells are harvested (collected) from the host cell culture media and screened for desirable antibody binding properties. Typically, the harvested particles are "panned" for binding with a preselected antigen. The strongly binding particles are then collected, and individual species of particles are clonally isolated and further screened for binding to the antigen. Phage which produce a binding site of desired antigen binding specificity are selected. Preferably, a humanized antibody of the invention has affinity of at least 1x10⁶ M⁻¹, preferably at least 1x10⁷ M⁻¹, at least 1x10⁸ M⁻¹, or at least 1x10⁹ M⁻¹ for an antigen of interest.

Preferably, a phage library is first screened using a modified plaque lifting assay, termed capture lift. See Watkins et al., 1997, Anal. Biochem., 253:37-45. Briefly, phage infected bacteria are plated on solid agar lawns and subsequently, are overlaid with nitrocellulose filters that have been coated with a Fab-specific reagent (*e.g.*, an anti-Fab antibody). Following the capture of nearly uniform quantities of phage-expressed Fab, the filters are probed with desired antigen-Ig fusion protein at a concentration substantially below the Kd value of the Fab.

Alternatively, the combinatorial libraries are expressed and screened using in *vitro* systems, such as the ribosomal display systems *(see, e.g.,* Graddis et al., Curr Pharm Biotechnol. 3(4):285-97 (2002); Hanes and Plucthau PNAS USA 94:4937-4942 (1997); He, 1999, J. Immunol. Methods, 231:105; Jermutus et al. (1998) Current Opinion in Biotechnology, 9:534-548. The ribosomal display system works by translating a library of antibody or fragment thereof *in vitro* without allowing the release of either antibody (or fragment thereof) or the mRNA from the translating ribosome. This is made possible by deleting the stop codon and utilizing a ribosome stabilizing buffer system. The translated antibody (or fragment thereof) also contains a C-terminal tether polypeptide extension in order to facilitate the newly synthesized antibody or fragment thereof to emerge from the ribosomal tunnel and fold independently. The folded antibody or fragment thereof can be screened or captured with a cognate antigen. This allows the capture of the mRNA, which is subsequently enriched *in vitro.* The *E*. *coli* and rabbit reticulocute systems are commonly used for the ribosomal display.

Other methods know in the art, *e.g*., PROfusion™ (U.S. Patent No. 6,281,344, Phylos Inc., Lexington, MA), Covalent Display (International Publication No. WO 9837186, Actinova Ltd., Cambridge, U.K.), can also be used.

An antigen can be bound to a solid support(s), which can be provided by a petri dish, chromatography beads, or magnetic beads. As used herein, the term "solid support" is not limited to a specific type of solid support. Rather a large number of supports are available and are known to one skilled in the art. Solid supports include silica gels, resins, derivatized plastic films, glass beads, cotton, plastic beads, polystyrene beads, alumina gels, and polysaccharides. A suitable solid support may be selected on the basis of desired end use and suitability for various synthetic protocols. For example, for peptide synthesis, a solid support can be a resin such as p-methylbenzhydrylamine (pMBHA) resin (Peptides International, Louisville, KY), polystyrenes (*e.g*., PAM-resin obtained from Bachem Inc., Peninsula Laboratories, etc.), including chloromethylpolystyrene, hydroxymethylpolystyrene and aminomethylpolystyrene, poly (dimethylacrylamide)-grafted styrene co-divinyl-benzene (*e.g*., POLYHIPE resin, obtained from Aminotech, Canada), polyamide resin (obtained from Peninsula Laboratories), polystyrene resin grafted with polyethylene glycol (*e.g.,* TENTAGEL or ARGOGEL, Bayer, Tubingen, Germany) polydimethylacrylamide resin (obtained from Milligen/Biosearch, California), or Sepharose (Pharmacia, Sweden).

The combinatorial library is then passed over the antigen, and those individual antibodies that bind are retained after washing, and optionally detected with a detection system. If samples of bound population are removed under increasingly stringent conditions, the binding affinity represented in each sample will increase. Conditions of increased stringency can be obtained, for example, by increasing the time of soaking or changing the pH of the soak solution, etc.

Alternatively, enzyme linked immunosorbent assay (ELISA) is used to screen for an antibody with desired binding activity. ELISAs comprise preparing antigen, coating the wells of a microtiter plate with the antigen, washing away antigen that did not bind the wells, adding the antibody of interest conjugated to a detectable compound such as an enzymatic substrate (*e.g*., horseradish peroxidase or alkaline phosphatase) to the wells and incubating for a period of time, washing away unbound antibodies or non-specifically bound antibodies, and detecting the presence of the antibodies specifically bound to the antigen coating the well. In ELISAs, the antibody of interest does not have to be conjugated to a detectable compound; instead, a second antibody (which recognizes the antibody of interest) conjugated to a detectable compound may be added to the well. Further, instead of coating the well with the antigen, the antibody may be coated to the well. In this case, the detectable molecule could be the antigen conjugated to a detectable compound such as an enzymatic substrate (*e.g.*, horseradish peroxidase or alkaline phosphatase). One of skill in the art would be knowledgeable as to the parameters that can be modified to increase the signal detected as well as other variations of ELISAs known in the art. For further discussion regarding ELISAs see, *e.g.*, Ausubel et al., eds, 1994, Current Protocols in Molecular Biology, Vol. I, John Wiley & Sons, Inc., New York at 11.2.1.

BIAcore kinetic analysis can be used to determine the binding on and off rates (Kd) of antibodies of the invention to a specific antigen. BIAcore kinetic analysis comprises analyzing the binding and dissociation of an antigen from chips with immobilized antibodies on their surface. *See* Wu et al., 1999, J. Mol. Biol., 294:151-162. Briefly, antigen-Ig fusion protein is immobilized to a (1-ethyl-3-[3-dimethylaminopropyl]-carbodiimide hydrochloride) and N-hydroxy-succinimide-activated sensor chip CM5 by injecting antigen-Ig in sodium acetate. Antigen-Ig is immobilized at a low density to prevent rebinding of Fabs during the dissociation phase. To obtain association rate constant (Kon), the binding rate at six different Fab concentrations is determined at certain flow rate. Dissociation rate constant (Koff) are the average of six measurements obtained by analyzing the dissociation phase. Sensorgrams are analyzed with the BIAevaluation 3.0 program. Kd is calculated from Kd = Koff/Kon. Residual Fab is removed after each measurement by prolonged dissociation. Preferably, positive plaques are picked, re-plated at a lower density, and screened again.

The binding affinity of an antibody (including a scFv or other molecule comprising, or alternatively consisting of, antibody fragments or variants thereof) to an antigen and the off-rate of an antibody-antigen interaction can be determined by competitive binding assays. One example of a competitive binding assay is a radioimmunoassay comprising the incubation of labeled antigen (*e.g*., ³H or ¹²¹I) with the antibody of interest in the presence of increasing amounts of unlabeled antigen, and the detection of the antibody bound to the labeled antigen. The affinity of the antibody and the binding off-rates can be determined from the data by Scatchard plot analysis. Competition with a second antibody can also be determined using radioimmunoassays. In this case, an antigen is incubated with an antibody conjugated to a labeled compound (*e.g*., ³H or ¹²¹I) in the presence of increasing amounts of an unlabeled second antibody.

Other assays, such as immunoassays, including but not limited to, competitive and non-competitive assay systems using techniques such as western blots, radioimmunoassays, ELISA (enzyme linked immunosorbent assay), sandwich immunoassays, immunoprecipitation assays, precipitin reactions, gel diffusion precipitin reactions, immunodiffusion assays, agglutination assays, complement-fixation assays, fluorescent immunoassays, and protein A immunoassays, can also be used to screen or further characterize the binding specificity of a humanized antibody. Such assays are routine and well known in the art (see, *e.g*., Ausubel et al., eds, 1994, Current Protocols in Molecular Biology, Vol. 1, John Wiley & Sons, Inc., New York. Exemplary immunoassays are described briefly below (which are not intended by way of limitation).

Preferably, ELISA is used as a secondary screening on supernatant prepared from bacterial culture expressing Fab fragments in order to confirm the clones identified by the capture lift assay. Two ELISAs can be carried out: (1) Quantification ELISA: this can be carried out essentially as described in Wu, 2003, Methods Mol. Biol., 207, 197-212. Briefly, concentrations can be determined by an anti-human Fab ELISA: individual wells of a 96-well Maxisorp Immunoplate are coated with 50 ng of a goat anti-human Fab antibody and then incubated with samples (supernatant-expressed Fabs) or standard (human IgG Fab). Incubation with a goat anti-human kappa horseradish peroxydase (HRP) conjugate then followed. HRP activity can be detected with TMB substrate and the reaction quenched with 0.2 M H₂SO₄. Plates are read at 450 nm. Clones that express detactable amount of Fab are then selected for the next part of the secondary screening. (2) Functional ELISA: briefly, a particular antigen binding activity is determined by the antigen-based ELISA: individual wells of a 96-well Maxisorp Immunoplate are coated with 50 ng of the antigen of interest, blocked with 1%BSA/0.1%Tween 20 and then incubated with samples (supernatant-expressed Fabs). Incubation with a goat anti-human kappa horseradish peroxydase (HRP) conjugate then followed. HRP activity is detected with TMB substrate and the reaction quenched with 0.2 M H₂SO₄. Plates are read at 450 nm.

Immunoprecipitation protocols generally comprise lysing a population of cells in a lysis buffer such as RIPA buffer (I % NP-40 or Triton X- 100, 1 % sodium deoxycholate, 0.1 % SDS, 0. 15 M NaCl, 0.0 1 M sodium phosphate at pH 7. 2, 1 % Trasylol) supplemented with protein phosphatase and/or protease inhibitors (*e.g*., EDTA, PMSF, 159 aprotinin, sodium vanadate), adding the antibody of interest to the cell lysate, incubating for a period of time (*e.g*., to 4 hours) at 40 degrees C, adding protein A and/or protein G sepharose beads to the cell lysate, incubating for about an hour or more at 40 degrees C, washing the beads in lysis buffer and re-suspending the beads in SDS/sample buffer. The ability of the antibody of interest to immunoprecipitate a particular antigen can be assessed by, *e.g.*, western blot analysis. One of skill in the art would be knowledgeable as to the parameters that can be modified to increase the binding of the antibody to an antigen and decrease the background (*e.g*., pre-clearing the cell lysate with sepharose beads). For further discussion regarding immunoprecipitation protocols see, *e.g.*, Ausubel et al., eds, 1994, Current Protocols in Molecular Biology, Vol. 1, John Wiley & Sons, Inc., New York, at 10. 16. 1.

Western blot analysis generally comprises preparing protein samples, electrophoresis of the protein samples in a polyacrylamide get (*e.g*., 8%- 20% SDS-PAGE depending on the molecular weight of the antigen), transferring the protein sample from the polyacrylamide get to a membrane such as nitrocellulose, PVDF or nylon, blocking the membrane in blocking solution (*e.g*., PBS with 3% BSA or non-fat milk), washing the membrane in washing buffer (*e.g.*, PBSTween 20), blocking the membrane with primary antibody (the antibody of interest) diluted in blocking buffer, washing the membrane in washing buffer, blocking the membrane with a secondary antibody (which recognizes the primary antibody, *e.g*., an anti-human antibody) conjugated to an enzymatic substrate (e.g., horseradish peroxidase or alkaline phosphatase) or radioactive molecule (*e.g*., 12P or 121I) diluted in blocking buffer, washing the membrane in wash buffer, and detecting the presence of the antigen. One of skill in the art would be knowledgeable as to the parameters that can be modified to increase the signal detected and to reduce the background noise. For further discussion regarding western blot protocols see, *e.g*., Ausubel et al., eds, 1994, GinTent Protocols in Molecular Biology, Vol. 1, John Wiley & Sons, Inc., New York at 10.8.1.

A nucleic acid encoding a modified (*e.g*., humanized) antibody or fragment thereof with desired antigen binding activity can be characterized by sequencing, such as dideoxynucleotide sequencing using a ABI300 genomic analyzer. Other immunoassays, such as the two-part secondary ELISA screen described above, can be used to compare the modified (*e.g*., humanized) antibodies to each other and to the donor antibody in terms of binding to a particular antigen of interest.

### 2.8 Production and Characterization of Humanized Antibodies

Once one or more nucleic acids encoding a humanized antibody or fragment thereof with desired binding activity are selected, the nucleic acid can be recovered by standard techniques known in the art. Preferably, selected phage particles are recovered and used to infect fresh bacteria before recovering the desired nucleic acids.

A phage displaying a protein comprising a humanized variable region with a desired specificity or affinity can be elution from an affinity matrix by any method known in the art. A ligand with better affinity to the matrix can be used. The corresponding non-humanized antibody can be used. An elution method which is not specific to the antigen-antibody complex can be used.

The method of mild elution uses binding of the phage antibody population to biotinylated antigen and binding to streptavidin magnetic beads. Following washing to remove non-binding phage, the phage antibody is eluted and used to infect cells to give a selected phage antibody population. A disulfide bond between the biotin and the antigen molecule allows mild elution with dithiothreitol. In one embodiment, biotinylated antigen can be used in excess, but at or below, a concentration equivalent to the desired dissociation constant for the antigen-antibody binding. This method is advantageous for the selection of high affinity antibodies (R. E. Hawkins, S. J. Russell and G. Winter J. Mol. Biol. 226 889-896, 1992). Antibodies may also be selected for slower off rates for antigen selection as described in Hawkins et al, 1992, *supra.* The concentration of biotinylated antigen may gradually be reduced to select higher affinity phage antibodies. As an alternative, the phage antibody may be in excess over biotinylated antigen in order that phage antibodies compete for binding, in an analogous way to the competition of peptide phage to biotinylated antibody described by J. K. Scott & G. P. Smith (Science 249 386-390, 1990).

In another embodiment, a nucleotide sequence encoding amino acids constituting a recognition site for cleavage by a highly specific protease can be introduced between the foreign nucleic acid inserted, *e.g.,* between a nucleic acid encoding an antibody fragment, and the sequence of the remainder of gene III. Non-limiting examples of such highly specific proteases are Factor X and thrombin. After binding of the phage to an affinity matrix and elution to remove non-specific binding phage and weak binding phage, the strongly bound phage would be removed by washing the column with protease under conditions suitable for digestion at the cleavage site. This would cleave the antibody fragment from the phage particle eluting the phage. These phage would be expected to be infective, since the only protease site should be the one specifically introduced. Strongly binding phage could then be recovered by infecting, *e.g., E. coli* TG I cells.

An alternative procedure to the above is to take the affinity matrix which has retained the strongly bound pAb and extract the DNA, for example by boiling in SDS solution. Extracted DNA can then be used to directly transform *E. coli* host cells or alternatively the antibody encoding sequences can be amplified, for example using PCR with suitable primers, and then inserted into a vector for expression as a soluble antibody for further study or a pAb for further rounds of selection.

Alternatively, a population of phage is bound to an affinity matrix which contains a low amount of antigen. There is competition between phage, displaying high affinity and low affinity proteins, for binding to the antigen on the matrix. Phage displaying high affinity protein is preferentially bound and low affinity protein is washed away. The high affinity protein is then recovered by elution with the ligand or by other procedures which elute the phage from the affinity matrix (International Publication No. WO92/01047 demonstrates this procedure).

The recovered nucleic acid encoding donor CDRs and humanized framework can be used by itself or can be used to construct nucleic acid for a complete antibody molecule by joining them to the constant region of the respective human template. When the nucleic acids encoding antibodies are introduced into a suitable host cell line, the transfected cells can secrete antibodies with all the desirable characteristics of monoclonal antibodies.

Once a nucleic acid encoding an antibody molecule or a heavy or light chain of an antibody, or fragment thereof (preferably, containing the heavy or light chain variable region) has been obtained, the vector for the production of the antibody molecule may be produced by recombinant DNA technology using techniques well known in the art. Thus, methods for preparing a protein by expressing a nucleic acid encoding an antibody are described herein. Methods which are well known to those skilled in the art can be used to construct expression vectors containing antibody coding sequences and appropriate transcriptional and translational control signals. These methods include, for example, in *vitro* recombinant DNA techniques, synthetic techniques, and *in vivo* genetic recombination. Replicable vectors may comprise a nucleotide sequence encoding an antibody molecule of the invention, a heavy or light chain of an antibody, a heavy or light chain variable domain of an antibody or a fragment thereof, or a heavy or light chain CDR, operably linked to a promoter The expression of an antibody molecule, a heavy or light chain of an antibody, a heavy or light chain variable domain of an antibody or a fragment thereof, or a heavy or light chain CDR can be regulated by a constitutive promoter Alternatively, the expression of an antibody molecule, a heavy or light chain of an antibody, a heavy or light chain variable domain of an antibody or a fragment thereof, or a heavy or light chain CDR is regulated by an inducible promoter. The expression of an antibody molecule, a heavy or light chain of an antibody, a heavy or light chain variable domain of an antibody or a fragment thereof, or a heavy or light chain CDR can be regulated by a tissue specific promoter. Such vectors may also include the nucleotide sequence encoding the constant region of the antibody molecule *(see, e.g.,* International Publication No. WO 86/05807; International Publication No. WO 89/01036; and U.S. Patent No. 5,122,464) and the variable domain of the antibody may be cloned into such a vector for expression of the entire heavy, the entire light chain, or both the entire heavy and light chains.

The expression vector is transferred to a host cell by conventional techniques and the transfected cells are then cultured by conventional techniques to produce an antibody. Thus, host cells may contain a polynucleotide encoding an antibody or fragments thereof, or a heavy or light chain thereof, or portion thereof, or a single chain antibody, operably linked to a heterologous promoter. Preferably, for the expression of double-chained antibodies, vectors encoding both the heavy and light chains may be co-expressed in the host cell for expression of the entire immunoglobulin molecule, as detailed below.

Preferably, the cell line which is transformed to produce the altered antibody is an immortalized mammalian cell line of lymphoid origin, including but not limited to, a myeloma, hybridoma, trioma or quadroma cell line. The cell line may also comprise a normal lymphoid cell, such as a B cell, which has been immortalized by transformation with a virus, such as the Epstein Barr virus. Most preferably, the immortalized cell line is a myeloma cell line or a derivative thereof.

It is known that some immortalized lymphoid cell lines, such as myeloma cell lines, in their normal state, secrete isolated immunoglobulin light or heavy chains. If such a cell line is transformed with the recovered nucleic acid from phage library, it will not be necessary to reconstruct the recovered fragment to a constant region, provided that the normally secreted chain is complementary to the variable domain of the immunoglobulin chain encoded by the recovered nucleic acid from the phage library.

Although the cell line used is preferably a mammalian cell line, any other suitable cell line may alternatively be used. These include, but are not limited to, microorganisms such as bacteria *(e.g., E. coli* and *B. subtilis*) transformed with recombinant bacteriophage DNA, plasmid DNA or cosmid DNA expression vectors containing antibody coding sequences; yeast (*e.g*., *Saccharomyces Pichia*) transformed with recombinant yeast expression vectors containing antibody coding sequences; insect cell systems infected with recombinant virus expression vectors *(e.g.,* baculovirus) containing antibody coding sequences; plant cell systems infected with recombinant virus expression vectors *(e.g.,* cauliflower mosaic virus, CaMV; tobacco mosaic virus, TMV) or transformed with recombinant plasmid expression vectors *(e.g.,* Ti plasmid) containing antibody coding sequences; or mammalian cell systems *(e.g.,* COS, CHO, BHK, 293, NS0, and 3T3 cells) harboring recombinant expression constructs containing promoters derived from the genome of mammalian cells *(e.g.,* metallothionein promoter) or from mammalian viruses *(e.g.,* the adenovirus late promoter; the vaccinia virus 7.5K promoter). Preferably, bacterial cells such as *Escherichia coli,* and more preferably, eukaryotic cells, especially for the expression of whole recombinant antibody molecule, are used for the expression of a recombinant antibody molecule. For example, mammalian cells such as Chinese hamster ovary cells (CHO), in conjunction with a vector such as the major intermediate early gene promoter element from human cytomegalovirus is an effective expression system for antibodies (Foecking et al., 1986, Gene 45:101; and Cockett et al., 1990, Bio/Technology 8:2).

In bacterial systems, a number of expression vectors may be advantageously selected depending upon the use intended for the antibody molecule being expressed. For example, when a large quantity of such a protein is to be produced, for the generation of pharmaceutical compositions of an antibody molecule, vectors which direct the expression of high levels of fusion protein products that are readily purified may be desirable. Such vectors include, but are not limited to, the *E*. *coli* expression vector pUR278 (Ruther et al., 1983, EMBO 12:1791), in which the antibody coding sequence may be ligated individually into the vector in frame with the lac Z coding region so that a fusion protein is produced; and pIN vectors (Inouye & Inouye, 1985, Nucleic Acids Res. 13:3101-3109; Van Heeke & Schuster, 1989, J. Biol. Chem. 24:5503-5509). pGEX vectors may also be used to express foreign polypeptides as fusion proteins with glutathione 5-transferase (GST). In general, such fusion proteins are soluble and can easily be purified from lysed cells by adsorption and binding to matrix glutathione agarose beads followed by elution in the presence of free glutathione. The pGEX vectors are designed to include thrombin or factor Xa protease cleavage sites so that the cloned target can be released from the GST moiety.

In an insect system, Autographa californica nuclear polyhedrosis virus (AcNPV) is used as a vector to express foreign genes. The virus grows in *Spodoptera frugiperda* cells. The antibody coding sequence may be cloned individually into non-essential regions (for example the polyhedrin gene) of the virus and placed under control of an AcNPV promoter (for example the polyhedrin promoter).

In mammalian host cells, a number of viral-based expression systems may be utilized. In cases where an adenovirus is used as an expression vector, the antibody coding sequence of interest may be ligated to an adenovirus transcription/translation control complex, e.g., the late promoter and tripartite leader sequence. This chimeric gene may then be inserted in the adenovirus genome by *in vitro* or *in vivo* recombination. Insertion in a non-essential region of the viral genome *(e.g.,* region El or E3) will result in a recombinant virus that is viable and capable of expressing the antibody molecule in infected hosts *(e.g.,* see Logan & Shenk, 1984, Proc. Natl. Acad. Sci. USA 8 1:355-359). Specific initiation signals may also be required for efficient translation of inserted antibody coding sequences. These signals include the ATG initiation codon and adjacent sequences. Furthermore, the initiation codon must be in phase with the reading frame of the desired coding sequence to ensure translation of the entire insert. These exogenous translational control signals and initiation codons can be of a variety of origins, both natural and synthetic. The efficiency of expression may be enhanced by the inclusion of appropriate transcription enhancer elements, transcription terminators, etc. *(see, e.g.,* Bittner et al., 1987, Methods in Enzymol. 153:516-544).

In addition, a host cell strain may be chosen which modulates the expression of the inserted sequences, or modifies and processes the nucleic acid in a specific fashion desired. Such modifications *(e.g.,* glycosylation) and processing *(e.g.,* cleavage) of protein products may be important for the function of the protein. Different host cells have characteristic and specific mechanisms for the post-translational processing and modification of proteins and gene products. Appropriate cell lines or host systems can be chosen to ensure the correct modification and processing of the foreign protein expressed. To this end, eukaryotic host cells which possess the cellular machinery for proper processing of the primary transcript, glycosylation, and phosphorylation of the gene product may be used. Such mammalian host cells include but are not limited to CHO, VERY, BHK, Hela, COS, MDCK, 293, 3T3, W 138, BT483, Hs578T, HTB2, BT2O and T47D, NS0 (a murine myeloma cell line that does not endogenously produce any immunoglobulin chains), CRL7O3O and HsS78Bst cells.

For long-term, high-yield production of recombinant proteins, stable expression is preferred. For example, cell lines which stably express the antibody molecule may be engineered. Rather than using expression vectors which contain viral origins of replication, host cells can be transformed with DNA controlled by appropriate expression control elements (*e.g*., promoter, enhancer, sequences, transcription terminators, polyadenylation sites, etc.), and a selectable marker. Following the introduction of the foreign DNA, engineered cells may be allowed to grow for 1-2 days in an enriched media, and then are switched to a selective media. The selectable marker in the recombinant plasmid confers resistance to the selection and allows cells to stably integrate the plasmid into their chromosomes and grow to form foci which in turn can be cloned and expanded into cell lines. This method may advantageously be used to engineer cell lines which express the antibody molecule. Such engineered cell lines may be particularly useful in screening and evaluation of compositions that interact directly or indirectly with the antibody molecule.

A number of selection systems may be used, including but not limited to, the herpes simplex virus thymidine kinase (Wigler et al., 1977, Cell 11:223), hypoxanthineguanine phosphoribosyltransferase (Szybalska & Szybalski, 1992, Proc. Natl. Acad. Sci. USA 48:202), and adenine phosphoribosyltransferase (Lowy et al., 1980, Cell 22:8-17) genes can be employed in tk-, hgprt- or aprt- cells, respectively. Also, antimetabolite resistance can be used as the basis of selection for the following genes: *dhfr*, which confers resistance to methotrexate (Wigler et al., 1980, Natl. Acad. Sci. USA 77:357; O'Hare et al., 1981, Proc. Natl. Acad. Sci. USA 78:1527); *gpt*, which confers resistance to mycophenolic acid (Mulligan & Berg, 1981, Proc. Natl. Acad. Sci. USA 78:2072); neo, which confers resistance to the aminoglycoside G-418 (Wu and Wu, 1991, Biotherapy 3:87-95; Tolstoshev, 1993, Ann. Rev. Pharmacol. Toxicol. 32:573-596; Mulligan, 1993, Science 260:926-932; and Morgan and Anderson, 1993, Ann. Rev. Biochem. 62: 191-217; May, 1993, TIB TECH 11(5):155-2 15); and *hygro,* which confers resistance to hygromycin (Santerre et al., 1984, Gene 30:147). Methods commonly known in the art of recombinant DNA technology may be routinely applied to select the desired recombinant clone, and such methods are described, for example, in Ausubel et al. (eds.), Current Protocols in Molecular Biology, John Wiley & Sons, NY (1993); Kriegler, Gene Transfer and Expression, A Laboratory Manual, Stockton Press, NY (1990); and in Chapters 12 and 13, Dracopoli et al. (eds), Current Protocols in Human Genetics, John Wiley & Sons, NY (1994); Colberre-Garapin et al., 1981, J. Mol. Biol. 150:1.

The expression levels of an antibody molecule can be increased by vector amplification (for a review, see Bebbington and Hentschel, The use of vectors based on gene amplification for the expression of cloned genes in mammalian cells in DNA cloning, Vol.3. (Academic Press, New York, 1987)). When a marker in the vector system expressing antibody is amplifiable, increase in the level of inhibitor present in culture of host cell will increase the number of copies of the marker gene. Since the amplified region is associated with the antibody gene, production of the antibody will also increase (Crouse et al., 1983, Mol. Cell. Biol. 3:257).

The host cell may be co-transfected with two expression vectors, the first vector encoding a heavy chain derived polypeptide and the second vector encoding a light chain derived polypeptide. The two vectors may contain identical selectable markers which enable equal expression of heavy and light chain polypeptides. Alternatively, a single vector may be used which encodes, and is capable of expressing, both heavy and light chain polypeptides. In such situations, the light chain should be placed before the heavy chain to avoid an excess of toxic free heavy chain (Proudfoot, 1986, Nature 322:52; and Kohler, 1980, Proc. Natl. Acad. Sci. USA 77:2 197). The coding sequences for the heavy and light chains may comprise cDNA or genomic DNA.

The antibodies can also be introduced into a transgenic animal *(e.g.,* transgenic mouse). *See, e.g.,* Bruggemann, Arch. Immunol. Ther. Exp. (Warsz). 49(3):203-8 (2001); Bruggemann and Neuberger, Immunol. Today 8:391-7 (1996), each of which is incorporated herein by reference. Transgene constructs or transloci can be obtained by, *e.g.,* plasmid assembly, cloning in yeast artificial chromosomes, and the use of chromosome fragments. Translocus integration and maintenance in transgenic animal strains can be achieved by pronuclear DNA injection into oocytes and various transfection methods using embryonic stem cells.

For example, nucleic acids encoding humanized heavy and/or light chain or humanized heavy and/or light variable regions may be introduced randomly or by homologous recombination into mouse embryonic stem cells. The mouse heavy and light chain immunoglobulin genes may be rendered non-functional separately or simultaneously with the introduction of nucleic acids encoding humanized antibodies by homologous recombination. In particular, homozygous deletion of the JH region prevents endogenous antibody production. The modified embryonic stem cells are expanded and microinjected into blastocysts to produce chimeric mice. The chimeric mice are then bred to produce homozygous offspring which express humanized antibodies.

Once an antibody molecule has been produced by recombinant expression, it may be purified by any method known in the art for purification of an immunoglobulin molecule, for example, by chromatography *(e.g.,* ion exchange, affinity, particularly by affinity for the specific antigen after Protein A, and sizing column chromatography), centrifugation, differential solubility, or by any other standard technique for the purification of proteins. Further, the antibodies or fragments thereof may be fused to heterologous polypeptide sequences described herein or otherwise known in the art to facilitate purification.

### 2.9 Antibody Conjugates

Antibodies or fragments thereof can be conjugated or fused to one or more moieties, including but not limited to, peptides, polypeptides, proteins, fusion proteins, nucleic acid molecules, small molecules, mimetic agents, synthetic drugs, inorganic molecules, and organic molecules.

Antibodies or fragments thereof can be recombinantly fused or chemically conjugated (including both covalent and non-covalent conjugations) to a heterologous protein or polypeptide (or fragment thereof, preferably to a polypepetide of at least 10, at least 20, at least 30, at least 40, at least 50, at least 60, at least 70, at least 80, at least 90 or at least 100 amino acids) to generate fusion proteins. The fusion does not necessarily need to be direct, but may occur through linker sequences. For example, antibodies may be used to target heterologous polypeptides to particular cell types, either *in vitro* or *in vivo,* by fusing or conjugating the antibodies to antibodies specific for particular cell surface receptors. Antibodies fused or conjugated to heterologous polypeptides may also be used in *in vitro* immunoassays and purification methods using methods known in the art. See *e.g.,* International publication No. WO 93/21232; European Patent No. EP 439,095; Naramura et al., 1994, Immunol. Lett. 39:91-99; U.S. Patent No. 5,474,981; Gillies et al., 1992, PNAS 89:1428-1432; and Fell et al.,1991, J. Immunol. 146:2446-2452.

Compositions may comprise heterologous proteins, peptides or polypeptides fused or conjugated to antibody fragments. For example, the heterologous polypeptides may be fused or conjugated to a Fab fragment, Fd fragment, Fv fragment, F(ab)₂ fragment, a VH domain, a VL domain, a VH CDR, a VL CDR, or fragment thereof. Methods for fusing or conjugating polypeptides to antibody portions are well-known in the art. See, *e.g.,* U.S. Patent Nos. 5,336,603, 5,622,929, 5,359,046, 5,349,053, 5,447,851, and 5,112,946; European Patent No.s EP 307,434 and EP 367,166; International publication Nos. WO 96/04388 and WO 91/06570; Ashkenazi et al., 1991, Proc. Natl. Acad. Sci. USA 88: 10535-10539; Zheng et al., 1995, J. Immunol. 154:5590-5600; and Vil et al., 1992, Proc. Natl. Acad. Sci. USA 89:11337-.

Additional fusion proteins may be generated through the techniques of gene-shuffling, motif-shuffling, exon-shuffling, and/or codon-shuffling (collectively referred to as "DNA shuffling"). DNA shuffling may be employed to alter the activities of antibodies of the invention or fragments thereof *(e.g.,* antibodies or fragments thereof with higher affinities and lower dissociation rates). *See,* generally, U.S. Patent Nos. 5,605,793; 5,811,238; 5,830,721; 5,834,252; and 5,837,458, and Patten et al., 1997, Curr. Opinion Biotechnol. 8:724-33 ; Harayama, 1998, Trends Biotechnol. 16(2):76-82; Hansson, et al., 1999, J. Mol. Biol. 287:265-76; and Lorenzo and Blasco, 1998, Biotechniques 24(2):308-313. Antibodies or fragments thereof, or the encoded antibodies or fragments thereof, may be altered by being subjected to random mutagenesis by error-prone PCR, random nucleotide insertion or other methods prior to recombination. One or more portions of a polynucleotide encoding an antibody or antibody fragment may be recombined with one or more components, motifs, sections, parts, domains, fragments, etc. of one or more heterologous molecules.

Moreover, the antibodies or fragments thereof can be fused to marker sequences, such as a peptide to facilitate purification. Preferably, the marker amino acid sequence is a hexa-histidine peptide, such as the tag provided in a pQE vector (QIAGEN, Inc., 9259 Eton Avenue, Chatsworth, CA, 91311), among others, many of which are commercially available. As described in Gentz et al., 1989, Proc. Natl. Acad. Sci. USA 86:821-824, for instance, hexa-histidine provides for convenient purification of the fusion protein. Other peptide tags useful for purification include, but are not limited to, the hemagglutinin "HA" tag, which corresponds to an epitope derived from the influenza hemagglutinin protein (Wilson et al., 1984, Cell 37:767) and the "flag" tag.

In other embodiments, antibodies or fragments, analogs or derivatives thereof can be conjugated to a diagnostic or detectable agent. Such antibodies can be useful for monitoring or prognosing the development or progression of a disorder as part of a clinical testing procedure, such as determining the efficacy of a particular therapy. Such diagnosis and detection can be accomplished by coupling the antibody to detectable substances including, but not limited to various enzymes, such as but not limited to horseradish peroxidase, alkaline phosphatase, beta-galactosidase, or acetylcholinesterase; prosthetic groups, such as but not limited to streptavidin/biotin and avidin/biotin; fluorescent materials, such as but not limited to, umbelliferone, fluorescein, fluorescein isothiocynate, rhodamine, dichlorotriazinylamine fluorescein, dansyl chloride or phycoerythrin; luminescent materials, such as but not limited to, luminol; bioluminescent materials, such as but not limited to, luciferase, luciferin, and aequorin; radioactive materials, such as but not limited to iodine (¹³¹I, ¹²⁵I, ¹²³I, ¹²¹I,), carbon (¹⁴C), sulfur (³⁵S), tritium (³H), indium (¹¹⁵In, ¹¹³In, ¹¹²In, ¹¹¹In,), and technetium (⁹⁹Tc), thallium (²⁰¹Ti), gallium (⁶⁸Ga, ⁶⁷Ga), palladium (¹⁰³Pd), molybdenum (⁹⁹Mo), xenon (¹³³Xe), fluorine (¹⁸F), ¹⁵³Sm, ¹⁷⁷Lu, ¹⁵⁹Gd, ¹⁴⁹Pm, ¹⁴⁰La, ¹⁷⁵Yb, ¹⁶⁶Ho, ⁹⁰Y, ⁴⁷Sc, ¹⁸⁶Re, ¹⁸⁸Re, ¹⁴²Pr, ¹⁰⁵Rh, ⁹⁷Ru, ⁶⁸Ge, ⁵⁷Co, ⁶⁵Zn, ⁸⁵Sr, ³²P, ¹⁵³Gd, ¹⁶⁹Yb, ⁵¹Cr, ⁵⁴Mn, ⁷¹Se, ¹¹³Sn, and ¹¹⁷Tin; positron emitting metals using various positron emission tomographies, noradioactive paramagnetic metal ions, and molecules that are radiolabelled or conjugated to specific radioisotopes.

Antibodies or fragments thereof can be conjugated to a therapeutic moiety. An antibody or fragment thereof may be conjugated to a therapeutic moiety such as a cytotoxin, *e.g.,* a cytostatic or cytocidal agent, a therapeutic agent or a radioactive metal ion, *e.g.,* alpha-emitters. A cytotoxin or cytotoxic agent includes any agent that is detrimental to cells. Therapeutic moieties include, but are not limited to, antimetabolites *(e.g.,* methotrexate, 6-mercaptopurine, 6-thioguanine, cytarabine, 5-fluorouracil decarbazine), alkylating agents *(e.g.,* mechlorethamine, thioepa chlorambucil, melphalan, carmustine (BCNU) and lomustine (CCNU), cyclothosphamide, busulfan, dibromomannitol, streptozotocin, mitomycin C, and cisdichlorodiamine platinum (II) (DDP) cisplatin), anthracyclines *(e.g.,* daunorubicin (formerly daunomycin) and doxorubicin), antibiotics *(e.g.,* dactinomycin (formerly actinomycin), bleomycin, mithramycin, and anthramycin (AMC)), Auristatin molecules (*e.g*., auristatin PHE, bryostatin 1, and solastatin 10; *see* Woyke et al., Antimicrob. Agents Chemother. 46:3802-8 (2002), Woyke et al., Antimicrob. Agents Chemother. 45:3580-4 (2001), Mohammad et al., Anticancer Drugs 12:735-40 (2001), Wall et al., Biochem. Biophys. Res. Commun. 266:76-80 (1999), Mohammad et al., Int. J. Oncol. 15:367-72 (1999), , hormones *(e.g.,* glucocorticoids, progestins, androgens, and estrogens), DNA-repair enzyme inhibitors *(e.g.,* etoposide or topotecan), kinase inhibitors (*e.g*., compound ST1571, imatinib mesylate (Kantarjian et al., Clin Cancer Res. 8(7):2167-76 (2002)), cytotoxic agents *(e.g.,* paclitaxel, cytochalasin B, gramicidin D, ethidium bromide, emetine, mitomycin, etoposide, tenoposide, vincristine, vinblastine, colchicin, doxorubicin, daunorubicin, dihydroxy anthracin dione, mitoxantrone, mithramycin, actinomycin D, 1-dehydrotestosterone, procaine, tetracaine, lidocaine, propranolol, and puromycin and analogs or homologs thereof) and those compounds disclosed in U.S. Pat. Nos. 6,245,759, 6,399,633, 6,383,790, 6,335,156, 6,271,242, 6,242,196, 6,218,410, 6,218,372, 6,057,300, 6,034,053, 5,985,877, 5,958,769, 5,925,376, 5,922,844, 5,911,995, 5,872,223, 5,863,904, 5,840,745, 5,728,868, 5,648,239, 5,587,459), farnesyl transferase inhibitors (*e.g*., R115777, BMS-214662, and those disclosed by, for example, U.S. Patent Nos: 6,458,935, 6,451,812, 6,440,974, 6,436,960, 6,432,959, 6,420,387, 6,414,145, 6,410,541, 6,410,539, 6,403,581, 6,399,615, 6,387,905, 6,372,747, 6,369,034, 6,362,188, 6,342,765, 6,342,487, 6,300,501, 6,268,363, 6,265,422, 6,248,756, 6,239,140, 6,232,338, 6,228,865, 6,228,856, 6,225,322, 6,218,406,6,211,193, 6,187,786, 6,169,096, 6,159,984, 6,143,766, 6,133,303, 6,127,366, 6,124,465, 6,124,295, 6,103,723, 6,093,737, 6,090,948, 6,080,870, 6,077,853, 6,071,935, 6,066,738, 6,063,930, 6,054,466, 6,051,582, 6,051,574, and 6,040,305), topoisomerase inhibitors *(e.g.,* camptothecin; irinotecan; SN-38; topotecan; 9-aminocamptothecin; GG-211 (GI 147211); DX-8951f; IST-622; rubitecan; pyrazoloacridine; XR-5000; saintopin; UCE6; UCE1022; TAN-1518A; TAN-1518B; KT6006; KT6528; ED-110; NB-506; ED-110; NB-506; and rebeccamycin); bulgarein; DNA minor groove binders such as Hoescht dye 33342 and Hoechst dye 33258; nitidine; fagaronine; epiberberine; coralyne; beta-lapachone; BC-4-1; bisphosphonates *(e.g.,* alendronate, cimadronte, clodronate, tiludronate, etidronate, ibandronate, neridronate, olpandronate, risedronate, piridronate, pamidronate, zolendronate) HMG-CoA reductase inhibitors, *(e.g.,* lovastatin, simvastatin, atorvastatin, pravastatin, fluvastatin, statin, cerivastatin, lescol, lupitor, rosuvastatin and atorvastatin) and pharmaceutically acceptable salts, solvates, clathrates, and prodrugs thereof. See, *e.g.,* Rothenberg, M.L., Annals of Oncology 8:837-855(1997); and Moreau, P., et al., J. Med. Chem. 41:1631-1640(1998)), antisense oligonucleotides *(e.g.,* those disclosed in the U.S. Pat. Nos. 6,277,832, 5,998,596, 5,885,834, 5,734,033, and 5,618,709), immunomodulators *(e.g.,* antibodies and cytokines), antibodies, and adenosine deaminase inhibitors *(e.g.,* Fludarabine phosphate and 2-Chlorodeoxyadenosine).

Further, an antibody or fragment thereof may be conjugated to a therapeutic moiety or drug moiety that modifies a given biological response. Therapeutic moieties or drug moieties are not to be construed as limited to classical chemical therapeutic agents. For example, the drug moiety may be a protein or polypeptide possessing a desired biological activity. Such proteins may include, for example, a toxin such as abrin, ricin A, pseudomonas exotoxin, cholera toxin, or diphtheria toxin; a protein such as tumor necrosis factor, α-interferon, β-interferon, nerve growth factor, platelet derived growth factor, tissue plasminogen activator, an apoptotic agent, *e.g*., TNF-α, TNF-β, AIM I (see, International publication No. WO 97/33899), AIM II (see, International Publication No. WO 97/34911), Fas Ligand (Takahashi et al., 1994, J. Immunol., 6:1567-1574), and VEGI (see, International publication No. WO 99/23105), a thrombotic agent or an anti-angiogenic agent, *e.g*., angiostatin, endostatin or a component of the coagulation pathway *(e.g.,* tissue factor); or, a biological response modifier such as, for example, a lymphokine *(e.g.,* interleukin-1 ("IL-1"), interleukin-2 ("IL-2"), interleukin-6 ("IL-6"), granulocyte macrophage colony stimulating factor ("GM-CSF"), and granulocyte colony stimulating factor ("G-CSF")), a growth factor *(e.g.,* growth hormone ("GH")), or a coagulation agent (e.g., calcium, vitamin K, tissue factors, such as but not limited to, Hageman factor (factor XII), high-molecular-weight kininogen (HMWK), prekallikrein (PK), coagulation proteins-factors II (prothrombin), factor V, XIIa, VIII, XIIIa, XI, XIa" IX, IXa, X, phospholipid. fibrinopeptides A and B from the α and β chains of fibrinogen, fibrin monomer).

Moreover, an antibody can be conjugated to therapeutic moieties such as a radioactive metal ion, such as alph-emiters such as ²¹³Bi or macrocyclic chelators useful for conjugating radiometal ions, including but not limited to, ¹³¹In, ¹³¹LU, ¹³¹Y, ¹³¹Ho, ¹³¹Sm, to polypeptides. In certain embodiments, the macrocyclic chelator is 1,4,7,10-tetraazacyclododecane-N,N',N",N"'-tetraacetic acid (DOTA) which can be attached to the antibody via a linker molecule. Such linker molecules are commonly known in the art and described in Denardo et al., 1998, Clin Cancer Res. 4(10):2483-90; Peterson et al., 1999, Bioconjug. Chem. 10(4):553-7; and Zimmerman et al., 1999, Nucl. Med. Biol. 26(8):943-50.

Techniques for conjugating therapeutic moieties to antibodies are well known, *see, e.g.,* Arnon et al., "Monoclonal Antibodies For Immunotargeting Of Drugs In Cancer Therapy", in Monoclonal Antibodies And Cancer Therapy, Reisfeld et al. (eds.), pp. 243-56 (Alan R. Liss, Inc. 1985); Hellstrom et al., "Antibodies For Drug Delivery", in Controlled Drug Delivery (2nd Ed.), Robinson et al. (eds.), pp. 623-53 (Marcel Dekker, Inc. 1987); Thorpe, "Antibody Carriers Of Cytotoxic Agents In Cancer Therapy: A Review", in Monoclonal Antibodies 84: Biological And Clinical Applications, Pinchera et al. (eds.), pp. 475-506 (1985); "Analysis, Results, And Future Prospective Of The Therapeutic Use Of Radiolabeled Antibody In Cancer Therapy", in Monoclonal Antibodies For Cancer Detection And Therapy, Baldwin et al. (eds.), pp. 303-16 (Academic Press 1985), and Thorpe et al., 1982, Immunol. Rev. 62:119-58.

Alternatively, an antibody can be conjugated to a second antibody to form an antibody heteroconjugate as described by Segal in U.S. Patent No. 4,676,980, which is incorporated herein by reference in its entirety.

The therapeutic moiety or drug conjugated to an antibody or fragment thereof should be chosen to achieve the desired prophylactic or therapeutic effect(s) for a particular disorder in a subject. A clinician or other medical personnel should consider the following when deciding on which therapeutic moiety or drug to conjugate to an antibody or fragment thereof: the nature of the disease, the severity of the disease, and the condition of the subject.

Antibodies may also be attached to solid supports, which are particularly useful for immunoassays or purification of the target antigen. Such solid supports include, but are not limited to, glass, cellulose, polyacrylamide, nylon, polystyrene, polyvinyl chloride or polypropylene.

### 2.10 Uses of the Antibodies of the Invention

The present invention provides methods of efficiently humanizing an antibody of interest. The humanized antibodies obtained by a method of the present invention can be used alone or in combination with other prophylactic or therapeutic agents for treating, managing, preventing or ameliorating a disorder or one or more symptoms thereof.

Methods for preventing, managing, treating, or ameliorating a disorder may comprise administering to a subject in need thereof one or more antibodies alone or in combination with one or more therapies *(e.g.,* one or more prophylactic or therapeutic agents) other than an antibody obtained by a method of the invention. Compositions may comprise one or more antibodies and one or more prophylactic or therapeutic agents other than antibodies obtained by a method of the invention and methods of preventing, managing, treating, or ameliorating a disorder or one or more symptoms thereof utilizing said compositions. Therapeutic or prophylactic agents include, but are not limited to, small molecules, synthetic drugs, peptides, polypeptides, proteins, nucleic acids *(e.g.,* DNA and RNA nucleotides including, but not limited to, antisense nucleotide sequences, triple helices, RNAi, and nucleotide sequences encoding biologically active proteins, polypeptides or peptides) antibodies, synthetic or natural inorganic molecules, mimetic agents, and synthetic or natural organic molecules.

Any therapy which is known to be useful, or which has been used or is currently being used for the prevention, management, treatment, or amelioration of a disorder or one or more symptoms thereof can be used in combination with an antibody obtained by a method of the invention as described herein. See, *e.g.,* Gilman et al., Goodman and Gilman's: The Pharmacological Basis of Therapeutics, 10th ed., McGraw-Hill, New York, 2001; The Merck Manual of Diagnosis and Therapy, Berkow, M.D. et al. (eds.), 17th Ed., Merck Sharp & Dohme Research Laboratories, Rahway, NJ, 1999; Cecil Textbook of Medicine, 20th Ed., Bennett and Plum (eds.), W.B. Saunders, Philadelphia, 1996 for information regarding therapies *(e.g.,* prophylactic or therapeutic agents) which have been or are currently being used for preventing, treating, managing, or ameliorating a disorder or one or more symptoms thereof. Examples of such agents include, but are not limited to, immunomodulatory agents, anti-inflammatory agents (*e.g*., adrenocorticoids, corticosteroids (*e.g*., beclomethasone, budesonide, flunisolide, fluticasone, triamcinolone, methlyprednisolone, prednisolone, prednisone, hydrocortisone), glucocorticoids, steroids, non-steriodal anti-inflammatory drugs *(e.g.,* aspirin, ibuprofen, diclofenac, and COX-2 inhibitors), pain relievers, leukotreine antagonists *(e.g.,* montelukast, methyl xanthines, zafirlukast, and zileuton), beta2-agonists *(e.g.,* albuterol, biterol, fenoterol, isoetharie, metaproterenol, pirbuterol, salbutamol, terbutalin formoterol, salmeterol, and salbutamol terbutaline), anticholinergic agents (*e.g*., ipratropium bromide and oxitropium bromide), sulphasalazine, penicillamine, dapsone, antihistamines, anti-malarial agents *(e.g.,* hydroxychloroquine), anti-viral agents, and antibiotics *(e.g.,* dactinomycin (formerly actinomycin), bleomycin, erythomycin, penicillin, mithramycin, and anthramycin (AMC)).

The humanized antibodies obtained by a method of the invention can be used directly against a particular antigen. Antibodies obtained by a method of the invention may belong to a subclass or isotype that is capable of mediating the lysis of cells to which the antibody binds. Antibodies obtained by a method of the invention may belong to a subclass or isotype that, upon complexing with cell surface proteins, activates serum complement and/or mediates antibody dependent cellular cytotoxicity (ADCC) by activating effector cells such as natural killer cells or macrophages.

The biological activities of antibodies are known to be determined, to a large extent, by the constant domains or Fc region of the antibody molecule (Uananue and Benacerraf, Textbook of Immunology, 2nd Edition, Williams & Wilkins, p. 218 (1984)). This includes their ability to activate complement and to mediate antibody-dependent cellular cytotoxicity (ADCC) as effected by leukocytes. Antibodies of different classes and subclasses differ in this respect, as do antibodies from the same subclass but different species; according to the present invention, antibodies of those classes having the desired biological activity are prepared. Preparation of these antibodies involves the selection of antibody constant domains and their incorporation in the humanized antibody by known technique. For example, mouse immunoglobulins of the IgG3 and IgG2a class are capable of activating serum complement upon binding to the target cells which express the cognate antigen, and therefore humanized antibodies which incorporate IgG3 and IgG2a effector functions are desirable for certain therapeutic applications.

In general, mouse antibodies of the IgG₂ₐ and IgG₃ subclass and occasionally IgG₁ can mediate ADCC, and antibodies of the IgG₃, IgG₂ₐ, and IgM subclasses bind and activate serum complement. Complement activation generally requires the binding of at least two IgG molecules in close proximity on the target cell. However, the binding of only one IgM molecule activates serum complement.

The ability of any particular antibody to mediate lysis of the target cell by complement activation and/or ADCC can be assayed. The cells of interest are grown and labeled in vitro; the antibody is added to the cell culture in combination with either serum complement or immune cells which may be activated by the antigen antibody complexes. Cytolysis of the target cells is detected by the release of label from the lysed cells. In fact, antibodies can be screened using the patient's own serum as a source of complement and/or immune cells. The antibody that is capable of activating complement or mediating ADCC in the *in vitro* test can then be used therapeutically in that particular patient.

Use of IgM antibodies may be preferred for certain applications, however IgG molecules by being smaller may be more able than IgM molecules to localize to certain types of infected cells.

In some embodiments, the antibodies obtained by a method of this invention are useful in passively immunizing patients.

The antibodies obtained by a method of the invention can also be used in diagnostic assays either *in vivo* or *in vitro* for detection/identification of the expression of an antigen in a subject or a biological sample *(e.g.,* cells or tissues). Non-limiting examples of using an antibody, a fragment thereof, or a composition comprising an antibody or a fragment thereof in a diagnostic assay are given in U.S. Patent Nos. 6,392,020; 6,156,498; 6,136,526; 6,048,528; 6,015,555; 5,833,988; 5,811,310; 8 5,652,114; 5,604,126; 5,484,704; 5,346,687; 5,318,892; 5,273,743; 5,182,107; 5,122,447; 5,080,883; 5,057,313; 4,910,133; 4,816,402; 4,742,000; 4,724,213; 4,724,212; 4,624,846; 4,623,627; 4,618,486; 4,176,174. Suitable diagnostic assays for the antigen and its antibodies depend on the particular antibody used. Non-limiting examples are an ELISA, sandwich assay, and steric inhibition assays. For *in vivo* diagnostic assays, the antibodies may be conjugated to a label that can be detected by imaging techniques, such as X-ray, computed tomography (CT), ultrasound, or magnetic resonance imaging (MRI). The antibodies can also be used for the affinity purification of the antigen from recombinant cell culture or natural sources.

### 2.11 Administration and Formulations

Compositions comprising antibodies obtained by a method of the invention are suitable for use in diagnosing, detecting, or monitoring a disorder, in preventing, treating, managing, or ameliorating of a disorder or one or more symptoms thereof, and/or in research. A composition may comprise one or more antibodies obtained by a method of the invention. A composition may comprise one or more antibodies obtained by a method of the invention and one or more prophylactic or therapeutic agents other than such antibodies. Preferably, the prophylactic or therapeutic agents are known to be useful for or having been or currently being used in the prevention, treatment, management, or amelioration of a disorder or one or more symptoms thereof. In accordance with these embodiments, the composition may further comprise of a carrier, diluent or excipient.

The compositions include, but are not limited to, bulk drug compositions useful in the manufacture of pharmaceutical compositions *(e.g.,* impure or non-sterile compositions) and pharmaceutical compositions (*i.e.,* compositions that are suitable for administration to a subject or patient) which can be used in the preparation of unit dosage forms. Such compositions comprise a prophylactically or therapeutically effective amount of a prophylactic and/or therapeutic agent disclosed herein or a combination of those agents and a pharmaceutically acceptable carrier. Preferably, compositions are pharmaceutical compositions and comprise an effective amount of one or more antibodies of the invention, a pharmaceutically acceptable carrier, and, optionally, an effective amount of another prophylactic or therapeutic agent.

The pharmaceutical composition can be formulated as an oral or non-oral dosage form, for immediate or extended release. The composition can comprise inactive ingredients ordinarily used in pharmaceutical preparation such as diluents, fillers, disintegrants, sweeteners, lubricants and flavors. The pharmaceutical composition is preferably formulated for intravenous administration, either by bolus injection or sustained drip, or for release from an implanted capsule. A typical formulation for intravenous administration utilizes physiological saline as a diluent.

Fab or Fab' portions of the antibodies produced by a method of the invention can also be utilized as the therapeutic active ingredient. Preparation of these antibody fragments is well-known in the art.

The composition can also include printed matter that describes clinical indications for which the antibodies can be administered as a therapeutic agent, dosage amounts and schedules, and/or contraindications for administration of the antibodies to a patient.

The compositions include, but are not limited to, bulk drug compositions useful in the manufacture of pharmaceutical compositions *(e.g.,* impure or non-sterile compositions) and pharmaceutical compositions (*i.e*., compositions that are suitable for administration to a subject or patient) which can be used in the preparation of unit dosage forms. Such compositions comprise a prophylactically or therapeutically effective amount of a prophylactic and/or therapeutic agent disclosed herein or a combination of those agents and a pharmaceutically acceptable carrier. Preferably, compositions are pharmaceutical compositions and comprise an effective amount of one or more antibodies, a pharmaceutically acceptable carrier, and, optionally, an effective amount of another prophylactic or therapeutic agent.

The term "pharmaceutically acceptable" means approved by a regulatory agency of the Federal or a state government or listed in the U.S. Pharmacopeia or other generally recognized pharmacopeia for use in animals, and more particularly in humans. The term "carrier" refers to a diluent, adjuvant (*e.g*., Freund's adjuvant (complete and incomplete)), excipient, or vehicle with which the therapeutic is contained in or administered. Such pharmaceutical carriers can be sterile liquids, such as water and oils, including those of petroleum, animal, vegetable or synthetic origin, such as peanut oil, soybean oil, mineral oil, or sesame oil. Water is a preferred carrier when the pharmaceutical composition is administered intravenously. Saline solutions and aqueous dextrose and glycerol solutions can also be employed as liquid carriers, particularly for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, or ethanol. The composition, if desired, can also contain minor amounts of wetting or emulsifying agents, or pH buffering agents. These compositions can take the form of solutions, suspensions, emulsion, tablets, pills, capsules, powders, or sustained-release formulations.

Generally, the ingredients of compositions of the invention are supplied either separately or mixed together in unit dosage form, for example, as a dry lyophilized powder or water free concentrate in a hermetically sealed container such as an ampoule or sachette indicating the quantity of active agent. Where the composition is to be administered by infusion, it can be dispensed with an infusion bottle containing sterile pharmaceutical grade water or saline. Where the composition is administered by injection, an ampoule of sterile water for injection or saline can be provided so that the ingredients may be mixed prior to administration.

The compositions can be formulated as neutral or salt forms. Pharmaceutically acceptable salts include those formed with anions such as those derived from hydrochloric, phosphoric, acetic, oxalic, tartaric acids, etc., and those formed with cations such as those derived from sodium, potassium, ammonium, calcium, ferric hydroxides, isopropylamine, triethylamine, 2-ethylamino ethanol, histidine, procaine, etc.

Various delivery systems are known and can be used to administer one or more antibodies or the combination of one or more antibodies and a prophylactic agent or therapeutic agent useful for preventing, managing, treating, or ameliorating a disorder or one or more symptoms thereof, *e.g.,* encapsulation in liposomes, microparticles, microcapsules, recombinant cells capable of expressing the antibody or antibody fragment, receptor-mediated endocytosis (see, *e.g.,* Wu and Wu, J. Biol. Chem. 262:4429-4432 (1987)), construction of a nucleic acid as part of a retroviral or other vector, etc. Methods of administering a prophylactic or therapeutic agent include, but are not limited to, parenteral administration *(e.g.,* intradermal, intramuscular, intraperitoneal, intravenous and subcutaneous), epidurala administration, intratumoral administration, and mucosal adminsitration (*e.g*., intranasal and oral routes). In addition, pulmonary administration can be employed, *e.g.,* by use of an inhaler or nebulizer, and formulation with an aerosolizing agent. See, *e.g.,* U.S. Patent Nos. 6,019,968, 5,985, 320, 5,985,309, 5,934,272, 5,874,064, 5,855,913, 5,290,540, and 4,880,078; and PCT Publication Nos. WO 92/19244, WO 97/32572, WO 97/44013, WO 98/31346, and WO 99/66903. An antibody of the invention, combination therapy, or a composition of the invention is administered using Alkermes AIR™ pulmonary drug delivery technology (Alkermes, Inc., Cambridge, MA). Prophylactic or therapeutic agents are administered intramuscularly, intravenously, intratumorally, orally, intranasally, pulmonary, or subcutaneously. The prophylactic or therapeutic agents may be administered by any convenient route, for example by infusion or bolus injection, by absorption through epithelial or mucocutaneous linings *(e.g.,* oral mucosa, rectal and intestinal mucosa, etc.) and may be administered together with other biologically active agents. Administration can be systemic or local.

It may be desirable to administer the prophylactic or therapeutic agents locally to the area in need of treatment; this may be achieved by, for example, and not by way of limitation, local infusion, by injection, or by means of an implant, said implant being of a porous or non-porous material, including membranes and matrices, such as sialastic membranes, polymers, fibrous matrices *(e.g.,* Tissuel^{®}), or collagen matrices. An effective amount of one or more antibodies is administered locally to the affected area to a subject to prevent, treat, manage, and/or ameliorate a disorder or a symptom thereof. In another embodiment, an effective amount of one or more antibodies is administered locally to the affected area in combination with an effective amount of one or more therapies *(e.g.,* one or more prophylactic or therapeutic agents) other than an antibody of a subject to prevent, treat, manage, and/or ameliorate a disorder or one or more symptoms thereof.

The prophylactic or therapeutic agent can be delivered in a controlled release or sustained release system. In one embodiment, a pump may be used to achieve controlled or sustained release (see Langer, *supra*; Sefton, 1987, CRC Crit. Ref. Biomed. Eng. 14:20; Buchwald et al., 1980, Surgery 88:507; Saudek et al., 1989, N. Engl. J. Med. 321:574). Polymeric materials can be used to achieve controlled or sustained release of the therapies (see *e.g*., Medical Applications of Controlled Release, Langer and Wise (eds.), CRC Pres., Boca Raton, Florida (1974); Controlled Drug Bioavailability, Drug Product Design and Performance, Smolen and Ball (eds.), Wiley, New York (1984); Ranger and Peppas, 1983, J., Macromol. Sci. Rev. Macromol. Chem. 23:61; see also Levy et al., 1985, Science 228:190; During et al., 1989, Ann. Neurol. 25:351; Howard et al., 1989, J. Neurosurg. 7 1:105); U.S. Patent No. 5,679,377; U.S. Patent No. 5,916,597; U.S. Patent No. 5,912,015; U.S. Patent No. 5,989,463; U.S. Patent No. 5,128,326; PCT Publication No. WO 99/15154; and PCT Publication No. WO 99/20253. Examples of polymers used in sustained release formulations include, but are not limited to, poly(2-hydroxy ethyl methacrylate), poly(methyl methacrylate), poly(acrylic acid), poly(ethylene-co-vinyl acetate), poly(methacrylic acid), polyglycolides (PLG), polyanhydrides, poly(N-vinyl pyrrolidone), poly(vinyl alcohol), polyacrylamide, poly(ethylene glycol), polylactides (PLA), poly(lactide-co-glycolides) (PLGA), and polyorthoesters. The polymer used in a sustained release formulation is inert, free of leachable impurities, stable on storage, sterile, and biodegradable. A controlled or sustained release system can be placed in proximity of the prophylactic or therapeutic target, thus requiring only a fraction of the systemic dose (see, *e.g.,* Goodson, in Medical Applications of Controlled Release, supra, vol. 2, pp. 115-138 (1984)).

Controlled release systems are discussed in the review by Langer (1990, Science 249:1527-1533). Any technique known to one of skill in the art can be used to produce sustained release formulations comprising one or more therapeutic agents. See, *e.g*., U.S. Patent No. 4,526,938, PCT publication WO 91/05548, PCT publication WO 96/20698,.Ning et al., 1996, "Intratumoral Radioimmunotheraphy of a Human Colon Cancer Xenograft Using a Sustained-Release Gel," Radiotherapy & Oncology 39:179-189, Song et al., 1995, "Antibody Mediated Lung Targeting of Long-Circulating Emulsions," PDA Journal of Pharmaceutical Science & Technology 50:372-397, Cleek et al., 1997, "Biodegradable Polymeric Carriers for a bFGF Antibody for Cardiovascular Application," Pro. Int'l. Symp. Control. Rel. Bioact. Mater. 24:853-854, and Lam et al., 1997, "Microencapsulation of Recombinant Humanized Monoclonal Antibody for Local Delivery," Proc. Int'l. Symp. Control Rel. Bioact. Mater. 24:759-760.

Where the composition is a nucleic acid encoding a prophylactic or therapeutic agent, the nucleic acid can be administered *in vivo* to promote expression of its encoded prophylactic or therapeutic agent, by constructing it as part of an appropriate nucleic acid expression vector and administering it so that it becomes intracellular, *e.g.,* by use of a retroviral vector (see U.S. Patent No. 4,980,286), or by direct injection, or by use of microparticle bombardment (*e.g.,* a gene gun; Biolistic, Dupont), or coating with lipids or cell-surface receptors or transfecting agents, or by administering it in linkage to a homeobox-like peptide which is known to enter the nucleus (see, *e.g.,* Joliot et al., 1991, Proc. Natl. Acad. Sci. USA 88:1864-1868). Alternatively, a nucleic acid can be introduced intracellularly and incorporated within host cell DNA for expression by homologous recombination.

A pharmaceutical composition is formulated to be compatible with its intended route of administration. Examples of routes of administration include, but are not limited to, parenteral, *e.g.,* intravenous, intradermal, subcutaneous, oral, intranasal (*e.g.,* inhalation), transdermal (*e.g.,* topical), transmucosal, and rectal administration. he composition is formulated in accordance with routine procedures as a pharmaceutical composition adapted for intravenous, subcutaneous, intramuscular, oral, intranasal, or topical administration to human beings. Typically, compositions for intravenous administration are solutions in sterile isotonic aqueous buffer. Where necessary, the composition may also include a solubilizing agent and a local anesthetic such as lignocaine to ease pain at the site of the injection.

If the compositions are to be administered topically, the compositions can be formulated in the form of an ointment, cream, transdermal patch, lotion, gel, shampoo, spray, aerosol, solution, emulsion, or other form well-known to one of skill in the art. See, *e.g*., Remington's Pharmaceutical Sciences and Introduction to Pharmaceutical Dosage Forms, 19th ed., Mack Pub. Co., Easton, PA (1995). For non-sprayable topical dosage forms, viscous to semi-solid or solid forms comprising a carrier or one or more excipients compatible with topical application and having a dynamic viscosity preferably greater than water are typically employed. Suitable formulations include, without limitation, solutions, suspensions, emulsions, creams, ointments, powders, liniments, or salves, which are, if desired, sterilized or mixed with auxiliary agents (*e.g.,* preservatives, stabilizers, wetting agents, buffers, or salts) for influencing various properties, such as, for example, osmotic pressure. Other suitable topical dosage forms include sprayable aerosol preparations wherein the active ingredient, preferably in combination with a solid or liquid inert carrier, is packaged in a mixture with a pressurized volatile (*e.g*., a gaseous propellant, such as freon) or in a squeeze bottle. Moisturizers or humectants can also be added to pharmaceutical compositions and dosage forms if desired. Examples of such additional ingredients are well-known in the art.

If the method comprises intranasal administration of a composition, the composition can be formulated in an aerosol form, spray, mist or in the form of drops. In particular, prophylactic or therapeutic agents can be conveniently delivered in the form of an aerosol spray presentation from pressurized packs or a nebuliser, with the use of a suitable propellant (*e.g.,* dichlorodifluoromethane, trichlorofluoromethane, dichlorotetrafluoroethane, carbon dioxide or other suitable gas). In the case of a pressurized aerosol the dosage unit may be determined by providing a valve to deliver a metered amount. Capsules and cartridges (composed of, *e.g.,* gelatin) for use in an inhaler or insufflator may be formulated containing a powder mix of the compound and a suitable powder base such as lactose or starch.

If the method comprises oral administration, compositions can be formulated orally in the form of tablets, capsules, cachets, gelcaps, solutions, or suspensions. Tablets or capsules can be prepared by conventional means with pharmaceutically acceptable excipients such as binding agents (*e.g.,* pregelatinised maize starch, polyvinylpyrrolidone, or hydroxypropyl methylcellulose); fillers (*e.g.,* lactose, microcrystalline cellulose, or calcium hydrogen phosphate); lubricants (*e.g.,* magnesium stearate, talc, or silica); disintegrants (*e.g.,* potato starch or sodium starch glycolate); or wetting agents (*e.g.,* sodium lauryl sulphate). The tablets may be coated by methods well-known in the art. Liquid preparations for oral administration may take the form of, but not limited to, solutions, syrups or suspensions, or they may be presented as a dry product for constitution with water or other suitable vehicle before use. Such liquid preparations may be prepared by conventional means with pharmaceutically acceptable additives such as suspending agents (*e.g*., sorbitol syrup, cellulose derivatives, or hydrogenated edible fats); emulsifying agents (*e.g.,* lecithin or acacia); non-aqueous vehicles (*e.g.,* almond oil, oily esters, ethyl alcohol, or fractionated vegetable oils); and preservatives (e*.g.,* methyl or propyl-p-hydroxybenzoates or sorbic acid). The preparations may also contain buffer salts, flavoring, coloring, and sweetening agents as appropriate. Preparations for oral administration may be suitably formulated for slow release, controlled release, or sustained release of a prophylactic or therapeutic agent(s).

The method may comprise pulmonary administration, *e.g.,* by use of an inhaler or nebulizer, of a composition formulated with an aerosolizing agent. See, *e.g.,* U.S. Patent Nos. 6,019,968, 5,985, 320, 5,985,309, 5,934,272, 5,874,064, 5,855,913, 5,290,540, and 4,880,078; and PCT Publication Nos. WO 92/19244, WO 97/32572, WO 97/44013, WO 98/31346, and WO 99/66903, each of which is incorporated herein by reference their entiretiesAn antibody, combination therapy, and/or composition can be administered using Alkermes AIR™ pulmonary drug delivery technology (Alkermes, Inc., Cambridge, MA).

The method may comprise administration of a composition formulated for parenteral administration by injection (*e.g.,* by bolus injection or continuous infusion). Formulations for injection may be presented in unit dosage form (*e.g.,* in ampoules or in multi-dose containers) with an added preservative. The compositions may take such forms as suspensions, solutions or emulsions in oily or aqueous vehicles, and may contain formulatory agents such as suspending, stabilizing and/or dispersing agents. Alternatively, the active ingredient may be in powder form for constitution with a suitable vehicle (*e.g.,* sterile pyrogen-free water) before use.

The methods may additionally comprise of administration of compositions formulated as depot preparations. Such long acting formulations may be administered by implantation (*e.g.,* subcutaneously or intramuscularly) or by intramuscular injection. Thus, for example, the compositions may be formulated with suitable polymeric or hydrophobic materials (*e.g.,* as an emulsion in an acceptable oil) or ion exchange resins, or as sparingly soluble derivatives (*e.g*., as a sparingly soluble salt).

The methods encompass administration of compositions formulated as neutral or salt forms. Pharmaceutically acceptable salts include those formed with anions such as those derived from hydrochloric, phosphoric, acetic, oxalic, tartaric acids, etc., and those formed with cations such as those derived from sodium, potassium, ammonium, calcium, ferric hydroxides, isopropylamine, triethylamine, 2-ethylamino ethanol, histidine, procaine, etc.

Generally, the ingredients of compositions are supplied either separately or mixed together in unit dosage form, for example, as a dry lyophilized powder or water free concentrate in a hermetically sealed container such as an ampoule or sachette indicating the quantity of active agent. Where the mode of administration is infusion, composition can be dispensed with an infusion bottle containing sterile pharmaceutical grade water or saline. Where the mode of administration is by injection, an ampoule of sterile water for injection or saline can be provided so that the ingredients may be mixed prior to administration.

One or more of the prophylactic or therapeutic agents, or pharmaceutical compositions can be packaged in a hermetically-sealed container such as an ampoule or sachette indicating the quantity of the agent. One or more of the prophylactic or therapeutic agents, or pharmaceutical compositions can be supplied as a dry sterilized lyophilized powder or water free concentrate in a hermetically-sealed container and can be reconstituted (*e.g.,* with water or saline) to the appropriate concentration for administration to a subject. Preferably, one or more of the prophylactic or therapeutic agents or pharmaceutical compositions is supplied as a dry sterile lyophilized powder in a hermetically-sealed container at a unit dosage of at least 5 mg, more preferably at least 10 mg, at least 15 mg, at least 25 mg, at least 35 mg, at least 45 mg, at least 50 mg, at least 75 mg, or at least 100 mg. The lyophilized prophylactic or therapeutic agents or pharmaceutical compositions should be stored at between 2°C and 8°C in its original container and the prophylactic or therapeutic agents, or pharmaceutical compositions should be administered within 1 week, preferably within 5 days, within 72 hours, within 48 hours, within 24 hours, within 12 hours, within 6 hours, within 5 hours, within 3 hours, or within 1 hour after being reconstituted. Alternatively, one or more of the prophylactic or therapeutic agents or pharmaceutical compositions is supplied in liquid form in a hermetically-sealed container indicating the quantity and concentration of the agent. Preferably, the liquid form of the administered composition is supplied in a hermetically-sealed container at least 0.25 mg/ml, more preferably at least 0.5 mg/ml, at least 1 mg/ml, at least 2.5 mg/ml, at least 5 mg/ml, at least 8 mg/ml, at least 10 mg/ml, at least 15 mg/kg, at least 25 mg/ml, at least 50 mg/ml, at least 75 mg/ml or at least 100 mg/ml. The liquid form should be stored at between 2°C and 8°C in its original container.

Generally, the ingredients of the compositions are derived from a subject that is the same species origin or species reactivity as recipient of such compositions. Thus, human or humanized antibodies are administered to a human patient for therapy or prophylaxis.

### 2.11.1 Gene Therapy

Nucleic acid sequences comprising nucleotide sequences encoding an antibody or another prophylactic or therapeutic agent are administered to treat, prevent, manage, or ameliorate a disorder or one or more symptoms thereof by way of gene therapy. Gene therapy refers to therapy performed by the administration to a subject of an expressed or expressible nucleic acid. In this therapy, the nucleic acids produce their encoded antibody or prophylactic or therapeutic agent that mediates a prophylactic or therapeutic effect.

Any of the methods for gene therapy available in the art can be used. For general reviews of the methods of gene therapy, see Goldspiel et al., 1993, Clinical Pharmacy 12:488-505; Wu and Wu, 1991, Biotherapy 3:87-95; Tolstoshev, 1993, Ann. Rev. Pharmacol. Toxicol. 32:573-596; Mulligan, Science 260:926-932 (1993); and Morgan and Anderson, 1993, Ann. Rev. Biochem. 62:191-217; May, 1993, TIBTECH 11(5):155-215. Methods commonly known in the art of recombinant DNA technology which can be used are described in Ausubel et al. (eds.), Current Protocols in Molecular Biology, John Wiley & Sons, NY (1993); and Kriegler, Gene Transfer and Expression, A Laboratory Manual, Stockton Press, NY (1990).

The method comprises administration of a composition comprising nucleic acids encoding antibodies or another prophylactic or therapeutic agent, said nucleic acids being part of an expression vector that expresses the antibody, another prophylactic or therapeutic agent, or fragments or chimeric proteins or heavy or light chains thereof in a suitable host. In particular, such nucleic acids have promoters, preferably heterologous promoters, operably linked to the antibody coding region, said promoter being inducible or constitutive, and, optionally, tissue- specific. Nucleic acid molecules can be used in which the coding sequences of an antibody or another prophylactic or therapeutic agent and any other desired sequences are flanked by regions that promote homologous recombination at a desired site in the genome, thus providing for intrachromosomal expression of the antibody encoding nucleic acids (Koller and Smithies, 1989, Proc. Natl. Acad. Sci. USA 86:8932-8935; Zijlstra et al., 1989, Nature 342:435-438). The expressed antibody or other prophylactic or therapeutic agent can be a single chain antibody; alternatively, the nucleic acid sequences include sequences encoding both the heavy and light chains, or fragments thereof, of the antibody or another prophylactic or therapeutic agent.

Delivery of the nucleic acids into a subject may be either direct, in which case the subject is directly exposed to the nucleic acid or nucleic acid-carrying vectors, or indirect, in which case, cells are first transformed with the nucleic acids *in vitro,* then transplanted into the subject. These two approaches are known, respectively, *as in vivo* or *ex vivo* gene therapy.

The nucleic acid sequences can be directly administered *in vivo,* where it is expressed to produce the encoded product. This can be accomplished by any of numerous methods known in the art, e.g., by constructing them as part of an appropriate nucleic acid expression vector and administering it so that they become intracellular, e.g., by infection using defective or attenuated retrovirals or other viral vectors (see U.S. Patent No. 4,980,286), or by direct injection of naked DNA, or by use of microparticle bombardment *(e.g.,* a gene gun; Biolistic, Dupont), or coating with lipids or cell-surface receptors or transfecting agents, encapsulation in liposomes, microparticles, or microcapsules, or by administering them in linkage to a peptide which is known to enter the nucleus, by administering it in linkage to a ligand subject to receptor-mediated endocytosis (see, *e.g.,* Wu and Wu, 1987, J. Biol. Chem. 262:4429-4432) (which can be used to target cell types specifically expressing the receptors). Alternatively, nucleic acid-ligand complexes can be formed in which the ligand comprises a fusogenic viral peptide to disrupt endosomes, allowing the nucleic acid to avoid lysosomal degradation. Yet further, the nucleic acid can be targeted *in vivo* for cell specific uptake and expression, by targeting a specific receptor (see, *e.g.,* International Publication Nos. WO 92/06180; WO 92/22635; W092/20316; W093/14188; and WO 93/20221). Alternatively, the nucleic acid can be introduced intracellularly and incorporated within host cell DNA for expression, by homologous recombination (Koller and Smithies, 1989, Proc. Natl. Acad. Sci. USA 86:8932-8935; and Zijlstra et al., 1989, Nature 342:435-438).

Viral vectors that contain nucleic acid sequences encoding an antibody, another prophylactic or therapeutic agent, or fragments thereof can be used. For example, a retroviral vector can be used (see Miller et al., 1993, Meth. Enzymol. 217:581-599). These retroviral vectors contain the components necessary for the correct packaging of the viral genome and integration into the host cell DNA. The nucleic acid sequences encoding the antibody or another prophylactic or therapeutic agent to be used in gene therapy are cloned into one or more vectors, which facilitate delivery of the gene into a subject. More detail about retroviral vectors can be found in Boesen et al., 1994, Biotherapy 6:291-302, which describes the use of a retroviral vector to deliver the mdr 1 gene to hematopoietic stem cells in order to make the stem cells more resistant to chemotherapy. Other references illustrating the use of retroviral vectors in gene therapy are: Clowes et al., 1994, J. Clin. Invest. 93:644-651; Klein et al., 1994, Blood 83:1467-1473; Salmons and Gunzberg, 1993, Human Gene Therapy 4:129-141; and Grossman and Wilson, 1993, Curr. Opin. in Genetics and Devel. 3:110-114.

Adenoviruses are other viral vectors that can be used in gene therapy. Adenoviruses are especially attractive vehicles for delivering genes to respiratory epithelia. Adenoviruses naturally infect respiratory epithelia where they cause a mild disease. Other targets for adenovirus-based delivery systems are liver, the central nervous system, endothelial cells, and muscle. Adenoviruses have the advantage of being capable of infecting non-dividing cells. Kozarsky and Wilson, 1993, Current Opinion in Genetics and Development 3:499-503 present a review of adenovirus-based gene therapy. Bout et al., 1994, Human Gene Therapy 5:3-10 demonstrated the use of adenovirus vectors to transfer genes to the respiratory epithelia of rhesus monkeys. Other instances of the use of adenoviruses in gene therapy can be found in Rosenfeld et al., 1991, Science 252:431-434; Rosenfeld et al., 1992, Cell 68:143-155; Mastrangeli et al., 1993, J. Clin. Invest. 91:225-234; PCT Publication W094/12649; and Wang et al., 1995, Gene Therapy 2:775-783. Adeno-associated virus (AAV) has also been proposed for use in gene therapy (Walsh et al., 1993, Proc. Soc. Exp. Biol. Med. 204:289-300; and U.S. Patent No. 5,436,146).

Another approach to gene therapy involves transferring a gene to cells in tissue culture by such methods as electroporation, lipofection, calcium phosphate mediated transfection, or viral infection. Usually, the method of transfer includes the transfer of a selectable marker to the cells. The cells are then placed under selection to isolate those cells that have taken up and are expressing the transferred gene. Those cells are then delivered to a subject.

In thismethod, the nucleic acid is introduced into a cell prior to administration *in vivo* of the resulting recombinant cell. Such introduction can be carried out by any method known in the art, including but not limited to transfection, electroporation, microinjection, infection with a viral or bacteriophage vector containing the nucleic acid sequences, cell fusion, chromosome-mediated gene transfer, microcell-mediated gene transfer, spheroplast fusion, etc. Numerous techniques are known in the art for the introduction of foreign genes into cells (see, e.g., Loeffler and Behr, 1993, Meth. Enzymol. 217:599-618; Cohen et al., 1993, Meth. Enzymol. 217:618-644; Clin. Pharma. Ther. 29:69-92 (1985)) and may be used, provided that the necessary developmental and physiological functions of the recipient cells are not disrupted. The technique should provide for the stable transfer of the nucleic acid to the cell, so that the nucleic acid is expressible by the cell and preferably heritable and expressible by its cell progeny.

The resulting recombinant cells can be delivered to a subject by various methods known in the art. Recombinant blood cells (*e.g.,* hematopoietic stem or progenitor cells) are preferably administered intravenously. The amount of cells envisioned for use depends on the several factors including, but not limited to, the desired effects and the patient state, and can be determined by one skilled in the art.

Cells into which a nucleic acid can be introduced for purposes of gene therapy encompass any desired, available cell type, and include but are not limited to epithelial cells, endothelial cells, keratinocytes, fibroblasts, muscle cells, hepatocytes; blood cells such as T lymphocytes, B lymphocytes, monocytes, macrophages, neutrophils, eosinophils, mast cells, megakaryocytes, granulocytes; various stem or progenitor cells, in particular hematopoietic stem or progenitor cells (*e.g.,* as obtained from bone marrow, umbilical cord blood, peripheral blood, fetal liver, etc.). Preferably, the cell used for gene therapy is autologous to the subject.

When recombinant cells are used in gene therapy, nucleic acid sequences encoding an antibody or fragment thereof can be introduced into the cells such that they are expressible by the cells or their progeny, and the recombinant cells are then administered *in vivo* for therapeutic effect. Stem or progenitor cells could be used. Any stem and/or progenitor cells which can be isolated and maintained *in vitro* can potentially be used (see e.g., PCT Publication WO 94/08598; Stemple and Anderson, 1992, Cell 7 1:973-985; Rheinwald, 1980, Meth. Cell Bio. 21A:229; and Pittelkow and Scott, 1986, Mayo Clinic Proc. 61:771).

The nucleic acid to be introduced for purposes of gene therapy comprises an inducible promoter operably linked to the coding region, such that expression of the nucleic acid is controllable by controlling the presence or absence of the appropriate inducer of transcription.

### 2.12 Dosage and Frequency of Administration

The amount of a prophylactic or therapeutic agent or a composition which will be effective in the treatment, management, prevention, or amelioration of a disorder or one or more symptoms thereof can be determined by standard clinical methods. The frequency and dosage will vary according to factors specific for each patient depending on the specific therapy or therapies (*e.g*., the specific therapeutic or prophylactic agent or agents) administered, the severity of the disorder, disease, or condition, the route of administration, as well as age, body, weight, response, the patient's immune status, and the past medical history of the patient. For example, the dosage of a prophylactic or therapeutic agent or a composition which will be effective in the treatment, prevention, management, or amelioration of a disorder or one or more symptoms thereof can be determined by administering the composition to an animal model such as, *e.g*., the animal models disclosed herein or known to those skilled in the art. In addition, *in vitro* assays may optionally be employed to help identify optimal dosage ranges. Suitable regimens can be selected by one skilled in the art by considering such factors and by following, for example, dosages reported in the literature and recommended in the Physician's Desk Reference (57th ed., 2003).

The toxicity and/or efficacy of the prophylactic and/or therapeutic protocols can be determined by standard pharmaceutical procedures in cell cultures or experimental animals, *e.g.,* for determining the LD₅₀ (the dose lethal to 50% of the population) and the ED₅₀ (the dose therapeutically effective in 50% of the population). The dose ratio between toxic and therapeutic effects is the therapeutic index and it can be expressed as the ratio LD₅₀/ED₅₀. Therapies that exhibit large therapeutic indices are preferred. While therapies that exhibit toxic side effects may be used, care should be taken to design a delivery system that targets such agents to the site of affected tissue in order to minimize potential damage to uninfected cells and, thereby, reduce side effects.

The data obtained from the cell culture assays and animal studies can be used in formulating a range of dosage of the prophylactic and/or therapeutic agents for use in humans. The dosage of such agents lies preferably within a range of circulating concentrations that include the ED₅₀ with little or no toxicity. The dosage may vary within this range depending upon the dosage form employed and the route of administration utilized. For any therapy used, the therapeutically effective dose can be estimated initially from cell culture assays. A dose may be formulated in animal models to achieve a circulating plasma concentration range that includes the IC₅₀ (*i.e*., the concentration of the test compound that achieves a half-maximal inhibition of symptoms) as determined in cell culture. Such information can be used to more accurately determine useful doses in humans. Levels in plasma may be measured, for example, by high performance liquid chromatography.

For peptides, polypeptides, proteins, fusion proteins, and antibodies, the dosage administered to a patient is typically 0.01 mg/kg to 100 mg/kg of the patient's body weight. Preferably, the dosage administered to a patient is between 0.1 mg/kg and 20 mg/kg of the patient's body weight, more preferably 1 mg/kg to 10 mg/kg of the patient's body weight. Generally, human and humanized antibodies have a longer half-life within the human body than antibodies from other species due to the immune response to the foreign polypeptides. Thus, lower dosages of human antibodies and less frequent administration is often possible.

Exemplary doses of a small molecule include milligram or microgram amounts of the small molecule per kilogram of subject or sample weight (*e.g.,* about 1 microgram per kilogram to about 500 milligrams per kilogram, about 100 micrograms per kilogram to about 5 milligrams per kilogram, or about I microgram per kilogram to about 50 micrograms per kilogram).

The dosages of prophylactic or therapeutically agents are described in the Physicians' Desk Reference (56th ed., 2002).

### 2.13 Biological Assays

Antibodies or fragments thereof produced by methods of the present invention may be characterized in a variety of ways well-known to one of skill in the art. In particular, such antibodies or fragments thereof may be assayed for the ability to bind immunospecifically to an antigen. Such an assay may be performed in solution (e.g., Houghten, 1992, Bio/Techniques 13:412 421), on beads (Lam, 1991, Nature 354:82 84), on chips (Fodor, 1993, Nature 364:555 556), on bacteria (U.S. Patent No. 5,223,409), on spores (U.S. Patent Nos. 5,571,698; 5,403,484; and 5,223,409), on plasmids (Cull et al., 1992, Proc. Natl. Acad. Sci. USA 89:1865 1869) or on phage (Scott and Smith, 1990, Science 249:386 390; Cwirla et al., 1990, Proc. Natl. Acad. Sci. USA 87:6378 6382; and Felici, 1991, J. Mol. Biol. 222:301 310). Antibodies or fragments thereof that have been identified can then be assayed for specificity and affinity.

The antibodies or fragments thereof may be assayed for immunospecific binding to a specific antigen and cross-reactivity with other antigens by any method known in the art. Immunoassays which can be used to analyze immunospecific binding and cross-reactivity include, but are not limited to, competitive and non-competitive assay systems using techniques such as western blots, radioimmunoassays, ELISA (enzyme linked immunosorbent assay), "sandwich" immunoassays, immunoprecipitation assays, precipitin reactions, gel diffusion precipitin reactions, immunodiffusion assays, agglutination assays, complement-fixation assays, immunoradiometric assays, fluorescent immunoassays, or protein A immunoassays. Such assays are routine and well-known in the art (see, *e.g*., Ausubel et al., eds., 1994, Current Protocols in Molecular Biology, Vol. 1, John Wiley & Sons, Inc., New York. Exemplary immunoassays are described briefly in Section 5.6.

The antibodies or fragments thereof can also be assayed for their ability to inhibit the binding of an antigen to its host cell receptor using techniques known to those of skill in the art. For example, cells expressing a receptor can be contacted with a ligand for that receptor in the presence or absence of an antibody or fragment thereof that is an antagonist of the ligand and the ability of the antibody or fragment thereof to inhibit the ligand's binding can measured by, for example, flow cytometry or a scintillation assay. The ligand or the antibody or antibody fragment can be labeled with a detectable compound such as a radioactive label (e.g., ³²P, ³⁵S, and ¹²⁵I) or a fluorescent label (*e.g.,* fluorescein isothiocyanate, rhodamine, phycoerythrin, phycocyanin, allophycocyanin, o-phthaldehyde and fluorescamine) to enable detection of an interaction between the ligand and its receptor. Alternatively, the ability of antibodies or fragments thereof to inhibit a ligand from binding to its receptor can be determined in cell-free assays. For example, a ligand can be contacted with an antibody or fragment thereof that is an antagonist of the ligand and the ability of the antibody or antibody fragment to inhibit the ligand from binding to its receptor can be determined. Preferably, the antibody or the antibody fragment that is an antagonist of the ligand is immobilized on a solid support and the ligand is labeled with a detectable compound. Alternatively, the ligand is immobilized on a solid support and the antibody or fragment thereof is labeled with a detectable compound. A ligand may be partially or completely purified (*e.g.,* partially or completely free of other polypeptides) or part of a cell lysate. Alternatively, a ligand can be biotinylated using techniques well known to those of skill in the art (*e.g.,* biotinylation kit, Pierce Chemicals; Rockford, IL).

An antibody or a fragment thereof constructed and/or identified in accordance with the present invention can be tested *in vitro* and/or *in vivo* for its ability to modulate the biological activity of cells. Such ability can be assessed by, *e.g.,* detecting the expression of antigens and genes; detecting the proliferation of cells; detecting the activation of signaling molecules (*e.g.,* signal transduction factors and kinases); detecting the effector function of cells; or detecting the differentiation of cells. Techniques known to those of skill in the art can be used for measuring these activities. For example, cellular proliferation can be assayed by ³H-thymidine incorporation assays and trypan blue cell counts. Antigen expression can be assayed, for example, by immunoassays including, but are not limited to, competitive and non-competitive assay systems using techniques such as western blots, immunohistochemistry radioimmunoassays, ELISA (enzyme linked immunosorbent assay), "sandwich" immunoassays, immunoprecipitation assays, precipitin reactions, gel diffusion precipitin reactions, immunodiffusion assays, agglutination assays, complement-fixation assays, immunoradiometric assays, fluorescent immunoassays, protein A immunoassays, and FACS analysis. The activation of signaling molecules can be assayed, for example, by kinase assays and electrophoretic shift assays (EMSAs).

The antibodies, fragments thereof, or compositions are preferably tested *in vitro* and then *in vivo* for the desired therapeutic or prophylactic activity prior to use in humans. For example, assays which can be used to determine whether administration of a specific pharmaceutical composition is indicated include cell culture assays in which a patient tissue sample is grown in culture and exposed to, or otherwise contacted with, a pharmaceutical composition, and the effect of such composition upon the tissue sample is observed. The tissue sample can be obtained by biopsy from the patient. This test allows the identification of the therapeutically most effective therapy (*e.g.,* prophylactic or therapeutic agent) for each individual patient. In various specific embodiments, *in vitro* assays can be carried out with representative cells of cell types involved a particular disorder to determine if a pharmaceutical composition has a desired effect upon such cell types. For example, *in vitro* asssay can be carried out with cell lines.

The effect of an antibody, a fragment thereof, or a composition on peripheral blood lymphocyte counts can be monitored/assessed using standard techniques known to one of skill in the art. Peripheral blood lymphocytes counts in a subject can be determined by, *e.g*., obtaining a sample of peripheral blood from said subject, separating the lymphocytes from other components of peripheral blood such as plasma using, *e.g.,* Ficoll-Hypaque (Pharmacia) gradient centrifugation, and counting the lymphocytes using trypan blue. Peripheral blood T-cell counts in subject can be determined by, *e.g*., separating the lymphocytes from other components of peripheral blood such as plasma using, *e.g.,* a use of Ficoll-Hypaque (Pharmacia) gradient centrifugation, labeling the T-cells with an antibody directed to a T-cell antigen which is conjugated to FITC or phycoerythrin, and measuring the number of T-cells by FACS.

The antibodies, fragments, or compositions used to treat, manage, prevent, or ameliorate a viral infection or one or more symptoms thereof can be tested for their ability to inhibit viral replication or reduce viral load in *in vitro* assays. For example, viral replication can be assayed by a plaque assay such as described, *e.g.,* by Johnson et al., 1997, Journal of Infectious Diseases 176:1215-1224 176:1215-1224. The antibodies or fragments thereof administered as described herein can also be assayed for their ability to inhibit or downregulate the expression of viral polypeptides. Techniques known to those of skill in the art, including, but not limited to, western blot analysis, northern blot analysis, and RT-PCR can be used to measure the expression of viral polypeptides.

The antibodies, fragments, or compositions used to treat, manage, prevent, or ameliorate a bacterial infection or one or more symptoms thereof can be tested in *in vitro* assays that are well-known in the art. *In vitro* assays known in the art can also be used to test the existence or development of resistance of bacteria to a therapy. Such *in vitro* assays are described in Gales et al., 2002, Diag. Nicrobiol. Infect. Dis. 44(3):301-311; Hicks et al., 2002, Clin. Microbiol. Infect. 8(11): 753-757; and Nicholson et al., 2002, Diagn. Microbiol. Infect. Dis. 44(1): 101-107.

The antibodies, fragments, or compositions used to treat, manage, prevent, or ameliorate a fungal infection or one or more symptoms thereof can be tested for anti-fungal activity against different species of fungus. Any of the standard anti-fungal assays well-known in the art can be used to assess the anti-fungal activity of a therapy. The anti-fungal effect on different species of fungus can be tested. The tests recommended by the National Committee for Clinical Laboratories (NCCLS) (See National Committee for Clinical Laboratories Standards. 1995, Proposed Standard M27T. Villanova, Pa. and other methods known to those skilled in the art (Pfaller et al., 1993, Infectious Dis. Clin. N. Am. 7: 435-444) can be used to assess the anti-fungal effect of a therapy. The antifungal properties of a therapy may also be determined from a fungal lysis assay, as well as by other methods, including, inter alia, growth inhibition assays, fluorescence-based fungal viability assays, flow cytometry analyses, and other standard assays known to those skilled in the art.

For example, the anti-fungal activity of a therapy can be tested using macrodilution methods and/or microdilution methods using protocols well-known to those skilled in the art (see, e.g., Clancy et al., 1997 Journal of Clinical Microbiology, 35(11): 2878-82; Ryder et al., 1998, Antimicrobial Agents and Chemotherapy, 42(5): 1057-61; U.S. 5,521,153; U.S. 5,883,120, U.S. 5,521,169. Briefly, a fungal strain is cultured in an appropriate liquid media, and grown at an appropriate temperature, depending on the particular fungal strain used for a determined amount of time, which is also depends on the particular fungal strain used. An innoculum is then prepared photometrically and the turbidity of the suspension is matched to that of a standard, e.g., a McFarland standard. The effect of a therapy on the turbidity of the inoculum is determined visually or spectrophotometrically. The minimal inhibitory concentration ("MIC") of the therapy is determined, which is defined as the lowest concentration of the lead compound which prevents visible growth of an inoculum as measured by determining the culture turbidity.

The anti-fungal activity of a therapy can also be determined utilizing colorimetric based assays well known to one of skill in the art. One exemplary colorimetric assay that can be used to assess the anti-fungal activity of a therapy is described by Pfaller et al. (1994, Journal of Clinical Microbiology, 32(8): 1993-6; also see Tiballi et al., 1995, Journal of Clinical Microbiology, 33(4): 915-7). This assay employs a colorimetric endpoint using an oxidation-reduction indicator (Alamar Biosciences, Inc., Sacramento CA).

The anti-fungal activity of a therapy can also be determined utilizing photometric assays well known to one of skill in the art (see, *e.g.,* Clancy et al., 1997 Journal of Clinical Microbiology, 35(11): 2878-82; Jahn et al., 1995, Journal of Clinical Microbiology, 33(3): 661-667. This photometric assay is based on quantifying mitochondrial respiration by viable fungi through the reduction of 3-(4,5-dimethyl-2thiazolyl)-2,5,-diphenyl-2H-tetrazolium bromide (MTT) to formazan. MIC's determined by this assay are defined as the highest concentration of the test therapy associated with the first precipitous drop in optical density. The therapy can be assayed for anti-fungal activity using macrodilution, microdilution and MTT assays in parallel.

Further, any *in vitro* assays known to those skilled in the art can be used to evaluate the prophylactic and/or therapeutic utility of an antibody therapy disclosed herein for a particular disorder or one or more symptoms thereof.

The antibodies, compositions, or combination therapies can be tested in suitable animal model systems prior to use in humans. Such animal model systems include, but are not limited to, rats, mice, chicken, cows, monkeys, pigs, dogs, rabbits, etc. Any animal system well-known in the art may be used. Several aspects of the procedure may vary; said aspects include, but are not limited to, the temporal regime of administering the therapies (*e.g.,* prophylactic and/or therapeutic agents) whether such therapies are administered separately or as an admixture, and the frequency of administration of the therapies.

Animal models can be used to assess the efficacy of the antibodies, fragments thereof, or compositions for treating, managing, preventing, or ameliorating a particular disorder or one or more symptom thereof.

The administration of antibodies, generated according to the methods of the invention or compositions, or combination therapies comprising such antibodies can be tested for their ability to decrease the time course of a particular disorder by at least 25%, preferably at least 50%, at least 60%, at least 75%, at least 85%, at least 95%, or at least 99%. The antibodies, compositions, or combination therapies can also be tested for their ability to increase the survival period of humans suffering from a particular disorder by at least 25%, preferably at least 50%, at least 60%, at least 75%, at least 85%, at least 95%, or at least 99%. Further, antibodies, compositions, or combination therapies can be tested for their ability reduce the hospitalization period of humans suffering from viral respiratory infection by at least 60%, preferably at least 75%, at least 85%, at least 95%, or at least 99%. Techniques known to those of skill in the art can be used to analyze the function of the antibodies, compositions, or combination therapies *in vivo.*

Further, any *in vivo* assays known to those skilled in the art can be used to evaluate the prophylactic and/or therapeutic utility of an antibody, a fragment thereof, a composition, a combination therapy disclosed herein for a particular disorder or one or more symptoms thereof.

The toxicity and/or efficacy of the prophylactic and/or therapeutic protocols can be determined by standard pharmaceutical procedures in cell cultures or experimental animals, e.g., for determining the LD50 (the dose lethal to 50% of the population) and the ED50 (the dose therapeutically effective in 50% of the population). The dose ratio between toxic and therapeutic effects is the therapeutic index and it can be expressed as the ratio LD50/ED50. Therapies that exhibit large therapeutic indices are preferred. While therapies that exhibit toxic side effects may be used, care should be taken to design a delivery system that targets such agents to the site of affected tissue in order to minimize potential damage to uninfected cells and, thereby, reduce side effects.

The data obtained from the cell culture assays and animal studies can be used in formulating a range of dosage of the prophylactic and/or therapeutic agents for use in humans. The dosage of such agents lies preferably within a range of circulating concentrations that include the ED50 with little or no toxicity. The dosage may vary within this range depending upon the dosage form employed and the route of administration utilized. For any therapy used in a methoddescribed herein, the therapeutically effective dose can be estimated initially from cell culture assays. A dose may be formulated in animal models to achieve a circulating plasma concentration range that includes the IC50 (i.e, the concentration of the test compound that achieves a half-maximal inhibition of symptoms) as determined in cell culture. Such information can be used to more accurately determine useful doses in humans. Levels in plasma may be measured, for example, by high performance liquid chromatography.

### 2.14 Kits

Kits may comprise sub-banks of antibody framework regions of a species of interest, or may comprise sub-banks of CDRs of a species of interest. Kits may comprise combinatorial sub-libraries that comprise a plurality of nucleic acid sequences comprising nucleotide sequences, each nucleotide sequence encoding one framework region (e.g., FR1) in frame fused to one corresponding CDR (e.g., CDR1). Kits may comprise combinatorial libraries that comprises plurality of nucleic acid sequences comprising nucleotide sequences, each nucleotide sequence encoding the framework regions and CDRs fused in-frame (e.g., FR1+CDR1+FR2+CDR2+FR3+CDR3+FR4).

Kits may comprise sub-banks of human immunoglobulin framework regions, sub-banks of CDRs, combinatorial sub-libraries, and/or combinatorial libraries. A kit may comprise a framework region sub-bank for variable light chain framework region 1, 2, 3, and/or 4, wherein the framework region is defined according to the Kabat system. Alternatively, a kit may comprise a framework region sub-bank for variable light chain framework region 1, 2, 3, and/or 4, wherein the framework region is defined according to the Chothia system. Yet further, a kit may comprise a framework region sub-bank for variable heavy chain framework region 1, 2, 3, and/or 4, wherein the framework region is defined according to the Kabat system. Alternatively a kit may comprise a framework region sub-bank for variable heavy chain framework region 1, 2, 3, and/or 4, wherein the framework region is defined according to the Chothia system. Yet another kit may comprise sub-banks of both the light chain and the heavy chain frameworks.

Apharmaceutical pack or kit may comprise one or more containers filled with a humanized antibody produced by a method of the invention. The pharmaceutical pack or kit may further comprise one or more other prophylactic or therapeutic agents useful for the treatment of a particular disease. A pharmaceutical pack or kit may comprising one or more containers filled with one or more of the ingredients of the pharmaceutical compositionsdescribed herein. Optionally associated with such container(s) can be a notice in the form prescribed by a governmental agency regulating the manufacture, use or sale of pharmaceuticals or biological products, which notice reflects approval by the agency of manufacture, use or sale for human administration.

### 2.15 Article of Manufacture

A finished packaged and labeled pharmaceutical product can be an article of manufacture that includes the appropriate unit dosage form in an appropriate vessel or container such as a glass vial or other container that is hermetically sealed. In the case of dosage forms suitable for parenteral administration the active ingredient is sterile and suitable for administration as a particulate free solution. We describe both parenteral solutions and lyophilized powders, each being sterile, and the latter being suitable for reconstitution prior to injection. Alternatively, the unit dosage form may be a solid suitable for oral, transdermal, topical or mucosal delivery.

The unit dosage form can be suitable for intravenous, intramuscular or subcutaneous delivery. Thus, we describe solutions, preferably sterile, suitable for each delivery route.

As with any pharmaceutical product, the packaging material and container are designed to protect the stability of the product during storage and shipment. Further, the products include instructions for use or other informational material that advise the physician, technician or patient on how to appropriately prevent or treat the disease or disorder in question. In other words, the article of manufacture includes instruction means indicating or suggesting a dosing regimen including, but not limited to, actual doses, monitoring procedures (such as methods for monitoring mean absolute lymphocyte counts, tumor cell counts, and tumor size) and other monitoring information.

More specifically, an article of manufacture ay comprise packaging material, such as a box, bottle, tube, vial, container, sprayer, insufflator, intravenous (i.v.) bag, or envelope; and at least one unit dosage form of a pharmaceutical agent contained within said packaging material. An article of manufacture may comprise packaging material, such as a box, bottle, tube, vial, container, sprayer, insufflator, intravenous (i.v.) bag, or envelope; and at least one unit dosage form of each pharmaceutical agent contained within said packaging material.

An article of manufacture may comprise packaging material and a pharmaceutical agent and instructions contained within said packaging material, wherein said pharmaceutical agent is a humanized antibody and a pharmaceutically acceptable carrier, and said instructions indicate a dosing regimen for preventing, treating or managing a subject with a particular disease. An article of manufacture may comprise packaging material and a pharmaceutical agent and instructions contained within said packaging material, wherein said pharmaceutical agent is a humanized antibody, a prophylactic or therapeutic agent other than the humanized antibody and a pharmaceutically acceptable carrier, and said instructions indicate a dosing regimen for preventing, treating or managing a subject with a particular disease. An article of manufacture may comprise packaging material and two pharmaceutical agents and instructions contained within said packaging material, wherein said first pharmaceutical agent is a humanized antibody and a pharmaceutically acceptable carrier and said second pharmaceutical agent is a prophylactic or therapeutic agent other than the humanized antibody, and said instructions indicate a dosing regimen for preventing, treating or managing a subject with a particular disease.

Adverse effects that may be reduced or avoided may be indicated in informational material enclosed in an article of manufacture for use in preventing, treating or ameliorating one or more symptoms associated with a disease. Adverse effects that may be reduced or avoided include but are not limited to vital sign abnormalities (e.g., fever, tachycardia, bardycardia, hypertension, hypotension), hematological events (e.g., anemia, lymphopenia, leukopenia, thrombocytopenia), headache, chills, dizziness, nausea, asthenia, back pain, chest pain (*e.g.,* chest pressure), diarrhea, myalgia, pain, pruritus, psoriasis, rhinitis, sweating, injection site reaction, and vasodilatation. Since some of the therapies may be immunosuppressive, prolonged immunosuppression may increase the risk of infection, including opportunistic infections. Prolonged and sustained immunosuppression may also result in an increased risk of developing certain types of cancer.

Further, the information material enclosed in an article of manufacture can indicate that foreign proteins may also result in allergic reactions, including anaphylaxis, or cytosine release syndrome. The information material should indicate that allergic reactions may exhibit only as mild pruritic rashes or they may be severe such as erythroderma, Stevens Johnson syndrome, vasculitis, or anaphylaxis. The information material should also indicate that anaphylactic reactions (anaphylaxis) are serious and occasionally fatal hypersensitivity reactions. Allergic reactions including anaphylaxis may occur when any foreign protein is injected into the body. They may range from mild manifestations such as urticaria or rash to lethal systemic reactions. Anaphylactic reactions occur soon after exposure, usually within 10 minutes. Patients may experience paresthesia, hypotension, laryngeal edema, mental status changes, facial or pharyngeal angioedema, airway obstruction, bronchospasm, urticaria and pruritus, serum sickness, arthritis, allergic nephritis, glomerulonephritis, temporal arthritis, or eosinophilia.

The information material can also indicate that cytokine release syndrome is an acute clinical syndrome, temporally associated with the administration of certain activating anti T cell antibodies. Cytokine release syndrome has been attributed to the release of cytokines by activated lymphocytes or monocytes. The clinical manifestations for cytokine release syndrome have ranged from a more frequently reported mild, self limited, "flu like" illness to a less frequently reported severe, life threatening, shock like reaction, which may include serious cardiovascular, pulmonary and central nervous system manifestations. The syndrome typically begins approximately 30 to 60 minutes after administration (but may occur later) and may persist for several hours. The frequency and severity of this symptom complex is usually greatest with the first dose. With each successive dose, both the incidence and severity of the syndrome tend to diminish. Increasing the amount of a dose or resuming treatment after a hiatus may result in a reappearance of the syndrome. Methods of treatment and prevention described herein may avoid or reduce one or more of the adverse effects discussed herein.

### 5.0 EXAMPLES

### Reagents

All chemicals were of analytical grade. Restriction enzymes and DNA-modifying enzymes were purchased from New England Biolabs, Inc. (Beverly, MA). *pfu* DNA polymerase and oligonucleotides were purchased from Invitrogen (Carlsbad, CA). Human EphA2-Fc fusion protein (consisting of human EphA2 fused with the Fc portion of a human IgG1 (Carles-Kinch et al. Cancer Res. 62: 2840-2847 (2002)) was expressed in human embryonic kidney (HEK) 293 cells and purified by protein G affinity chromatography using standard protocols. Streptavidin magnetic beads were purchased from Dynal (Lake Success, NY). Human EphA2-Fc biotinylation was carried out using an EZ-Link Sulfo-NHS-LC-Biotinylation Kit according to the manufacturer's instructions (Pierce, Rockford, IL).

### 5.1- Cloning and sequencing of the parental monoclonal antibody

A murine hybridoma cell line (B233) secreting a monoclonal antibody (mAb) raised against the human receptor tyrosine kinase EphA2 (Kinch et al. Clin. Exp. Metastasis. 20:59-68 (2003)) was acquired by MedImmune, Inc. This mouse mAb is referred to as mAb B233 hereafter. Cloning and sequencing of the variable heavy (V_{H}) and light (V_{L}) genes of mAb B233 were carried out after isolation and purification of the messenger RNA from B233 using a Straight A's mRNA Purification kit (Novagen, Madison, WI) according to the manufacturer's instructions. cDNA was synthesized with a First Strand cDNA synthesis kit (Novagen, Madison, WI) as recommended by the manufacturer. Amplification of both V_{H} and V_{L} genes was carried out using the IgGV_{H} and IgκV_{L} oligonucleotides from the Mouse Ig-Primer Set (Novagen, Madison, WI) as suggested by the manufacturer. DNA fragments resulting from productive amplifications were cloned into pSTBlue-1 using the Perfectly Blunt Cloning Kit (Novagen, Madison, WI). Multiple V_{H} and V_{L} clones were then sequenced by the dideoxy chain termination method (Sanger et al., Proc. Natl. Acad. Sci. USA. 74: 5463-5467 (1977)) using an ABI3000 sequencer (Applied Biosystems, Foster City, CA). The consensus sequences of mAb B233 V_{L} (V_{L}-233) and V_{H} (V_{H}-233) genes are shown in Figure 1.

### 5.2- Selection of the human frameworks

Human framework genes were selected from the publicly available pool of antibody germline genes. More precisely, this included 46 human germline kappa chain genes (A1, A10, A11, A14, A17, A18, A19, A2, A20, A23, A26, A27, A3, A30, A5, A7, B2, B3, L1, L10, L11, L12, L14, L 15, L16, L18, L19, L2, L20, L22, L23, L24, L25, L4/18a, L5, L6, L8, L9, O1, O11, O12, 014, O18, 02, 04 and 08; K.F. Schable, et al., Biol. Chem. Hoppe Seyler 374:1001-1022, (1993); J. Brensing-Kuppers, et al., Gene 191:173-181(1997)) for the 1^{st}, 2^{nd} and 3^{rd} frameworks and 5 human germline J sequences for the 4^{th} framework (Jκ1, Jκ2, Jκ3, Jκ4 and Jκ5; P.A. Hieter, et al., J. Biol. Chem. 257:1516-1522 (1982)). The heavy chain portion of the library included 44 human germline heavy chain sequences (VH1-18, VH1-2, VH1-24, VH1-3, VH1-45, V1-46, VH1-58, VH1-69, VH1-8, VH2-26, VH2-5, VH2-70, VH3-11, VH3-13, VH3-15, VH3-16, VH3-20, VH3-21, VH3-23, VH3-30, VH3-33, VH3-35, VH3-38, VH3-43, VH3-48, VH3-49, VH3-53, VH3-64, VH3-66, VH3-7, VH3-72, VH3-73, VH3-74, VH3-9, VH4-28, VH4-31, VH4-34, VH4-39, VH4-4, VH4-59, VH4-61, VH5-51, VH6-1 and VH7-8; F. Matsuda, et al., J. Exp. Med. 188:1973-1975 (1998)) for the 1^{st}, 2^{nd} and 3^{rd} frameworks and 6 human germline J sequences for the 4^{th} framework (JH1, JH2, JH3, JH4, JH5 and JH6; J.V. Ravetch, et al., Cell 27(3 Pt 2): 583-591 (1981)).

### 5.3- Construction of the framework-shuffled libraries

### 5.3.1- Description of the libraries

Three main framework-shuffled libraries (library A, B and C) were constructed. Library A included a light chain framework shuffled sub-library (V_{L} sub1) paired with the heavy chain of mAb B233 (V_{H}-233). Library B included a heavy chain framework shuffled sub-library (V_{H} sub1) paired with the fixed framework shuffled light chains V_{L}-12C8 and V_{L}-8G7 (see §5.4.1.1, §5.4.1.2 and §5.4.1.3). Library C included a light chain framework shuffled sub-library (V_{L} sub2) paired with a heavy chain framework shuffled sub-library (V_{H} sub2).

The construction of the framework shuffled V_{H} and V_{L} sub-libraries was carried out using the oligonucleotides shown in Tables 1-7 and 11. More precisely, the oligonucleotides described in Tables 1-7 and 11 encode the complete sequences of all known human framework germline genes for the light (κ) and heavy chains, Kabat definition. The oligonucleotides described in Tables 64 and 65 encode part of the CDRs of mAb B233 and are overlapping with the corresponding human germline frameworks. With respect to Table 64, with the exception of AL1-13 and DL1Ü-4Ü, each oligonucleotide encodes portions of one CDR of mAb B233 (underlined) and of one human germline light chain framework (Kabat definition; Kabat et al., Sequences of Proteins of Immunological Interest, U.S. Public Health Service, National Institutes of Health, Washington, DC, 1991). CDRL1, L2 and L3 are encoded by AL1Ü-10ÜBL1-10, BL1Ü-16Ü/CL1-11 and CL1Ü-12Ü/DL1-4, respectively. Oligonucleotides AL1-13 contain a M13 gene 3 leader overlapping sequence (bold) and oligonucleotides DL1Ü-4Ü contain a Cκ overlapping sequence (bold). With respect to table 65, with the exception of AH1-10 and DH1Ü-3Ü, each oligonucleotide encodes portions of one CDR of mAb B233 (underlined) and of one human germline heavy chain framework (Kabat definition). CDRH1, H2 and H3 are encoded by AH1Ü-17Ü/BH1-17, BH1Ü-16Ü/CH1-15 and CH1Ü-13Ü/DH1-3, respectively. Oligonucleotides AH1-10 contain a M13 gene 3 leader overlapping sequence (bold) whereas oligonucleotides DH1Ü-3Ü contain a Cκ1 overlapping sequence (bold). (K=G or T, M=A or C, R=A or G, S=C or G, W=A or T and Y=C or T).

**Table 64. Oligonucleotides used for the fusion of mAb B233 light chain CDRs with human germline light chain frameworks.**

| | | |
|---|---|---|
| 1589 | AL1 | 5'-GGTCGTTCCATTTTACTCCCACTCCGATGTTGTGATGACWCAGTCT-3' |
| 1590 | AL2 | 5'-GGTCGTTCCATTTTACTCCCACTCCGACATCCAGATGAYCCAGTCT-3' |
| 1591 | AL3 | 5'-GGTCGTTCCATTTTACTCCCACTCCGCCATCCAGWTGACCCAGTCT-3' |
| 1592 | AL4 | 5'-GGTCGTTCCATTTTACTCCCACTCCGAAATAGTGATGAYGCAGTCT-3' |
| 1593 | AL5 | 5' -GGTCGTTCCATTTTACTCCCACTCCGAAATTGTGTTGACRCAGTCT-3' |
| 1594 | AL6 | 5'-GGTCGTTCCATTTTACTCCCACTCCGAKATTGTGATGACCCAGACT-3' |
| 1595 | AL7 | 5'-GGTCGTTCCATTTTACTCCCACTCCGAAATTGTRMTGACWCAGTCT-3' |
| 1596 | AL8 | 5'-GGTCGTTCCATTTTACTCCCACTCCGAYATYGTGATGACYCAGTCT-3' |
| 1597 | AL9 | 5'-GGTCGTTCCATTTTACTCCCACTCCGAAACGACACTCACGCAGTCT-3' |
| 1598 | AL10 | 5'-GGTCGTTCCATTTTACTCCCACTCCGACATCCAGTTGACCCAGTCT-3' |
| 1599 | AL11 | 5'-GGTCGTTCCATTTTACTCCCACTCCAACATCCAGATGACCCAGTCT-3' |
| 1600 | AL12 | 5'-GGTCGTTCCATTTTACTCCCACTCCGCCATCCGGATGACCCAGTCT-3' |
| 1601 | AL13 | 5'-GGTCGTTCCATTTTACTCCCACTCCGTCATCTGGATGACCCAGTCT-3' |
| 1602 | AL1Ü | 5'-TAATACTTTGGCTGGCCCTGCAGGAGATGGAGGCCGGC-3' |
| 1603 | AL2Ü | 5'-TAATACTTTGGCTGGCCCTGCAGGAGAGGGTGRCTCTTTC-3' |
| 1604 | AL3Ü | 5'-TAATACTTTGGCTGGCCCTACAASTGATGGTGACTCTGTC-3' |
| 1605 | AL4Ü | 5'-TAATACTTTGGCTGGCCCTGAAGGAGATGGAGGCCGGCTG-3' |
| 1606 | AL5Ü | 5'-TAATACTTTGGCTGGCCCTGCAGGAGATGGAGGCCTGCTC-3' |
| 1607 | AL6Ü | 5'-TAATACTTTGGCTGGCCCTGCAGGAGATGTTGACTTTGTC-3' |
| 1608 | AL7Ü | 5'-TAATACTTTGGCTGGCCCTGCAGGTGATGGTGACTTTCTC-3' |
| 1609 | AL8Ü | 5'-TAATACTTTGGCTGGCCCTGCAGTTGATGGTGGCCCTCTC-3' |
| 1610 | AL9Ü | 5'-TAATACTTTGGCTGGCCCTGCAAGTGATGGTGACTCTGTC-3' |
| 1611 | AL10Ü | 5'-TAATACTTTGGCTGGCCCTGCAAATGATACTGACTCTGTC-3' |
| 1612 | BL1 | 5'-CCAGCCAAAGTATTAGCAACAACCTACACTGGYTTCAGCAGAGGCCAGGC-3' |
| 1613 | BL2 | 5'-CCAGCCAAAGTATTAGCAACAACCTACACTGGTACCTGCAGAAGCCAGGS-3' |
| 1614 | BL3 | 5'-CCAGCCAAAGTATTAGCAACAACCTACACTGGTATCRGCAGAAACCAGGG-3' |
| 1615 | BL4 | 5'-CCAGCCAAAGTATTAGCAACAACCTACACTGGTACCARCAGAAACCAGGA-3' |
| 1616 | BL5 | 5'-CCAGCCAAAGTATTAGCAACAACCTACACTGGTACCARCAGAAACCTGGC-3' |
| 1617 | BL6 | 5'-CCAGCCAAAGTATTAGCAACAACCTACACTGGTACCARCAGAAACCTGGC-3' |
| 1618 | BL7 | 5'-CCAGCCAAAGTATTAGCAACAACCTACACTGGTATCAGCARAAACCWGGS-3' |
| 1619 | BL8 | 5'-CCAGCCAAAGTATTAGCAACAACCTACACTGGTAYCAGCARAAACCAG-3' |
| 1620 | BL9 | 5'-CCAGCCAAAGTATTAGCAACAACCTACACTGGTTTCTGCAGAAAGCCAGG-3' |
| 1621 | BL10 | 5'-CCAGCCAAAGTATTAGCAACAACCTACACTGGTTTCAGCAGAAACCAGGG-3' |
| 1622 | BL1Ü | 5'-GATGGACTGGAAAACATAATAGATCAGGAGCTGTGGAG-3' |
| 1623 | BL20 | 5'-GATGGACTGGAAAACATAATAGATCAGGAGCTTAGGRGC-3' |
| 1624 | BL3Ü | 5'-GATGGACTGGAAAACATAATAGATGAGGAGCCTGGGMGC-3' |
| 1625 | BL4Ü | 5'-GATGGACTGGAAAACATARTAGATCAGGMGCTTAGGGGC-3' |
| 1626 | BL5Ü | 5'-GATGGACTGGAAAACATAATAGATCAGGWGCTTAGGRAC-3' |
| 1627 | BL6Ü | 5'-GATGGACTGGAAAACATAATAGATGAAGAGCTTAGGGGC-3' |
| 1628 | BL7Ü | 5'-GATGGACTGGAAAACATAATAAATTAGGAGTCTTGGAGG-3' |
| 1629 | BL8Ü | 5'-GATGGACTGGAAAACATAGTAAATGAGCAGCTTAGGAGG-3' |
| 1630 | BL9Ü | 5'-GATGGACTGGAAAACATAATAGATCAGGAGTGTGGAGAC-3' |
| 1631 | BL10Ü | 5'-GATGGACTGGAAAACATAATAGATCAGGAGCTCAGGGGC-3' |
| 1632 | BL11Ü | 5'-GATGGACTGGAAAACATAATAGATCAGGGACTTAGGGGC-3' |
| 1633 | BL12Ü | 5'-GATGGACTGGAAAACATAATAGAGGAAGAGCTTAGGGGA-3' |
| 1634 | BL13Ü | 5'-GATGGACTGGAAAACATACTTGATGAGGAGCTTTGGAGA-3' |
| 1635 | BL14Ü | 5'-GATGGACTGGAAAACATAATAAATTAGGCGCCTTGGAGA-3' |
| 1636 | BL15Ü | 5'-GATGGACTGGAAAACATACTTGATGAGGAGCTTTGGGGC-3' |
| 1637 | BL16Ü | 5'-GATGGACTGGAAAACATATTGAATAATGAAAATAGCAGC-3' |
| 1638 | CL1 | 5'-GTTTTCCAGTCCATCTCTGGGGTCCCAGACAGATTCAGY-3' |
| 1639 | CL2 | 5'-GTTTTCCAGTCCATCTCTGGGGTCCCATCAAGGTTCAGY-3' |
| 1744 | CL3 | 5'-GTTTTCCAGTCCATCTCTGGYATCCCAGCCAGGTTCAGT-3' |
| 1745 | CL4 | 5'-GTTTTCCAGTCCATCTCTGGRGTCCCWGACAGGTTCAGT-3' |
| 1746 | CL5 | 5'-GTTTTCCAGTCCATCTCTAGCATCCCAGCCAGGTTCAGT-3' |
| 1747 | CL6 | 5'-GTTTTCCAGTCCATCTCTGGGGTCCCCTCGAGGTTCAGT-3' |
| 1748 | CL7 | 5'-GTTTTCCAGTCCATCTCTGGAATCCCACCTCGATTCAGT-3' |
| 1749 | CL8 | 5'-GTTTTCCAGTCCATCTCTGGGGTCCCTGACCGATTCAGT-3' |
| 1750 | CL9 | 5'-GTTTTCCAGTCCATCTCTGGCATCCCAGACAGGTTCAGT-3' |
| 1751 | CL10 | 5'-GTTTTCCAGTCCATCTCTGGGGTCTCATCGAGGTTCAGT-3' |
| 1752 | CL11 | 5'-GTTTTCCAGTCCATCTCTGGAGTGCCAGATAGGTTCAGT-3' |
| 1753 | CL1Ü | 5'-CCAGCTGTTACTCTGTTGKCAGTAATAAACCCCAACATC-3' |
| 1754 | CL2Ü | 5'-CCAGCTGTTACTCTGTTGACAGTAATAYGTTGCAGCATC-3' |
| 1755 | CL3Ü | 5'-CCAGCTGTTACTCTGTTGACMGTAATAAGTTGCAACATC-3' |
| 1756 | CL4Ü | 5'-CCAGCTGTTACTCTGTTGRCAGTAATAAGTTGCAAAATC-3' |
| 1757 | CL5Ü | 5'-CCAGCTGTTACTCTGTTGACAGTAATAARCTGCAAAATC-3' |
| 1758 | CL6Ü | 5'-CCAGCTGTTACTCTGTTGACARTAGTAAGTTGCAAAATC-3' |
| 1759 | CL7Ü | 5'-CCAGCTGTTACTCTGTTGGCAGTAATAAACTCCAAMATC-3' |
| 1760 | CL8Ü | 5'-CCAGCTGTTACTCTGTTGGCAGTAATAAACCCCGACATC-3' |
| 1761 | CL9Ü | 5'-CCAGCTGTTACTCTGTTGACAGAAGTAATATGCAGCATC-3' |
| 1762 | CL210Ü | 5'-CCAGCTGTTACTCTGTTGACAGTAATATGTTGCAATATC-3' |
| 1763 | CL11Ü | 5'-CCAGCTGTTACTCTGTTGACAGTAATACACTGCAAAATC-3' |
| 1764 | CL12Ü | 5'-CCAGCTGTTACTCTGTTGACAGTAATAAACTGCCACATC-3' |
| 1765 | DL1 | 5'-CAGAGTAACAGCTGGCCGCTCACGTTYGGCCARGGGACCAAGSTG-3' |
| 1766 | DL2 | 5'-CAGAGTAACAGCTGGCCGCTCACGTTCGGCCAAGGGACACGACTG-3' |
| 1767 | DL3 | 5'-CAGAGTAACAGCTGGCCGCTCACGTTCGGCCCTGGGACCAAAGTG-3' |
| 1768 | DL4 | 5'-CAGAGTAACAGCTGGCCGCTCACGTTCGGCGGAGGGACCAAGGTG-3' |
| 1769 | DL1Ü | 5'-**GATGAAGACAGATGGTGCAGCCACAGTACG**TTTGATYTCCACCTTGG-3' |
| 1770 | DL2Ü | 5'-**GATGAAGACAGATGGTGCAGCCACAGTACG**TTTGATCTCCAGCTTGG-3' |
| 1771 | DL3Ü | 5'-**GATGAAGACAGATGGTGCAGCCACAGTACG**TTTGATATCCACTTTGG-3' |
| 1772 | DL4Ü | 5'-**GATGAAGACAGATGGTGCAGCCACAGTACG**TTTAATCTCCAGTCGTG-3' |

**Table 65. Oligonucleotides used for the fusion of mAb B233 heavy chain CDRs with human germline heavy chain frameworks.**

| | | |
|---|---|---|
| 1640 | AH1 | 5'-GCTGGTGGTGCCGTTCTATAGCCATAGCCAGGTKCAGCTGGTGCAGTCT-3' |
| 1641 | AH2 | 5'-**GCTGGTGGTGCCGTTCTATAGCCATAGC**GAGGTGCAGCTGKTGGAGTCT-3' |
| 1642 | AH3 | 5'-**GTGGTGGTGCCGTTCTATAGCCATAGC**CAGSTGCAGCTGCAGGAGTCG-3' |
| 1643 | AH4 | 5'-**GCTGGTGGTGCCGTTCTATAGCCATAGC**CAGGTCACCTTGARGGAGTCT-3' |
| 1644 | AH5 | 5'-**GCTGGTGGTGCCGTTCTATAGCCATAGC**CARATGCAGCTGGTGCAGTCT-3' |
| 1645 | AH6 | 5'-**GCTGGTGGTGCCGTTCTATAGCCATAGC**GARGTGCAGCTGGTGSAGTC-3' |
| 1646 | AH7 | 5'-**GCPGGTGGTGCCGTTCTATAGCCATAGC**CAGATCACCTTGAAGGAGTCT-3' |
| 1647 | AH8 | 5'-**GCTGGTGGTGCCGTTCTATAGCCATAGC**CAGGTSCAGCTGGTRSAGTCT-3' |
| 1648 | AH9 | 5'-**GCTGGTGGTGCCGTTCTATAGCCATAGC**CAGGTACAGCTGCAGCAGTCT-3' |
| 1649 | AH10 | 5'-**GCTGGTGGTGCCGTTCTATAGCCATAGC**CAGGTGCAGCTACAGCAGTGG-3' |
| 1650 | AH1Ü | **5'-GTTCATGGAGTAATCRGTGAAGGTGTATCCAGAAGC-3'** |
| 1651 | AH2Ü | 5'-GTTCATGGAGTAATCGCTGAGTGAGAACCCAGAGAM-3' |
| 1652 | AH3Ü | 5'-GTTCATGGAGTAATCACTGAARGTGAATCCAGAGGC-3' |
| 1653 | AH4Ü | 5'-GTTCATGGAGTAATCACTGACGGTGAAYCCAGAGGC-3' |
| 1654 | AH50 | 5'-GTTCATGGAGTAATCGCTGAYGGAGCCACCAGAGAC-3' |
| 1655 | AH6Ü | 5'-GTTCATGGAGTAATCRGTAAAGGTGWAWCCAGAAGC-3' |
| 1656 | AH7Ü | 5'-GTTCATGGAGTAATCACTRAAGGTGAAYCCAGAGGC-3' |
| 1657 | AH8Ü | 5'-GTTCATGGAGTAATCGGTRAARCTGTAWCCAGAASC-3' |
| 1658 | AH90 | 5'-GTTCATGGAGTAATCAYCAAAGGTGAATCCAGARGC-3' |
| 1659 | AH10Ü | 5'-GTTCATGGAGTAATCRCTRAAGGTGAATCCAGASGC-3' |
| 1660 | AH11Ü | 5'-GTTCATGGAGTAATCGGTGAAGGTGTATCCRGAWGC-3' |
| 1661 | AH12Ü | 5'-GTTCATGGAGTAATCACTGAAGGACCCACCATAGAC-3' |
| 1662 | AH13Ü | 5'-GTTCATGGAGTAATCACTGATGGAGCCACCAGAGAC-3' |
| 1663 | AH14Ü | 5'-GTTCATGGAGTAATCGCTGATGGAGTAACCAGAGAC-3' |
| 1664 | AH15Ü | 5'-GTTCATGGAGTAATCAGTGAGGGTGTATCCGGAAAC-3' |
| 1665 | AH16Ü | 5'-GTTCATGGAGTAATCGCTGAAGGTGCCTCCAGAAGC-3' |
| 1666 | AH17Ü | 5'-GTTCATGGAGTAATCAGAGACACTGTCCCCGGAGAT-3' |
| 1667 | BH1 | 5'-GATTACTCCATGAACTGGGTGCGACAGGCYCCTGGA-3' |
| 1668 | BH2 | 5'-GATTACTCCATGAACTGGGTGCGMCAGGCCCCCGGA-3' |
| 1669 | BH3 | 5'-GATTACTCCATGAACTGGATCCGTCAGCCCCCAGGR-3' |
| 1670 | BH4 | 5'-GATTACTCCATGAACTGGRTCCGCCAGGCTCCAGGG-3' |
| 1671 | BH5 | 5'-GATTACTCCATGAACTGGATCCGSCAGCCCCCAGGG-3' |
| 1672 | BH6 | 5'-GATTACTCCATGAACTGGGTCCGSCAAGCTCCAGGG-3' |
| 1673 | BH7 | 5'-GATTACTCCATGAACTGGGTCCRTCARGCTCCRGGR-3' |
| 1674 | BH8 | 5'-GATTACTCCATGAACTGGGTSCGMCARGCYACWGGA-3' |
| 1675 | BH9 | 5'-GATTACTCCATGAACTGGKTCCGCCAGGCTCCAGGS-3' |
| 1676 | BH10 | 5'-GATTACTCCATGAACTGGATCAGGCAGTCCCCATCG-3' |
| 1677 | BH11 | 5'-GATTACTCCATGAACTGGGCCCGCAAGGCTCCAGGA-3' |
| 1678 | BH12 | 5'-GATTACTCCATGAACTGGATCCGCCAGCACCCAGGG-3' |
| 1679 | BH13 | 5'-GATTACTCCATGAACTGGGTCCGCCAGGCTTCCGGG-3' |
| 1680 | BH14 | 5'-GATTACTCCATGAACTGGGTGCGCCAGATGCCCGGG-3' |
| 1681 | BH15 | 5'-GATTACTCCATGAACTGGGTGCGACAGGCTCGTGGA-3' |
| 1682 | BH16 | 5'-GATTACTCCATGAACTGGATCCGGCAGCCCGCCGGG-3' |
| 1683 | BH17 | 5'-GATTACTCCATGAACTGGGTGCCACAGGCCCCTGGA-3' |
| 1684 | BH1Ü | 5'-TGTGTAATCATTAGCTTTGTTTCTAATAAATCCCATCCACTCAAGCCYTTG-3' |
| 1685 | BH2Ü | 5'-TGTTGTGTAATCATTAGCTTTGTTTCTAATAAATCCCATCCACTCAAGCSCTT-3' |
| 1686 | BH3Ü | 5'-TGTTGTGTAATCATTAGCTTTGTTTCTAATAAAWGAGACCCACTCCAGCCCCTT-3' |
| 1687 | BH4Ü | 5'-TGTTGTGTAATCATTAGCTTTGTTTCTAATAAACCCAATCCACTCCAGKCCCTT-3' |
| 1688 | BH5Ü | 5'-TGTTGTGTAATCATTAGCTTTGTTTCTAATAAATGAGACCCACTCCAGRCCCTT-3' |
| 1689 | BH6Ü | 5'-TGTTGTGTAATCATTAGCTTTGTTTCTAATAAAGCCAACCCACTCCAGCCCYTT-3' |
| 1690 | BH7Ü | 5'-TGTTGTGTAATCATTAGCTTTGTTTCTAATAAAKGCCACCCACTCCAGCCCCTT-3' |
| 1691 | BH8Ü | 5'-TGTTGTGTAATCATTAGCTTTGTTTCTAATAAATCCCAGCCACTCAAGGCCTC-3' |
| 1692 | BH9Ü | 5'-TGTTGTGTAATCATTAGCTTTGTTTCTAATAAACCCCATCCACTCCAGGCCTT-3' |
| 1693 | BH10Ü | 5'-TGTTGTGTAATCATTAGCTTTGTTTCTAATAAATGARACCCACWCCAGCCCCTT-3' |
| 1694 | BH11Ü | 5'-TGTTGTGTAATCATTAGCTTTGTTTCTAATAAAMGAKACCCACTCCAGMCCCTT-3' |
| 1695 | B12Ü | 5'-TGTTGTGTAATCATTAGCTTTGTTTCTAATAAAYCCMATCCACTCMAGCCCYTT-3' |
| 1696 | 1BH13Ü | 5'-TGTTGTGTAATCATTAGCTTTGTTTCTAATAAATCCTATCCACTCAAGGCGTTG-3' |
| 1697 | BH14Ü | 5'-TGTTGTGTAATCATTAGCTTTGTTTCTAATAAATGCAAGCCACTCCAGGGCCTT-3' |
| 1698 | BH15Ü | 5'-TGTTGTGTAATCATTAGCTTTGTTTCTAATAAATGAAACATATTCCAGTCCCTT-3' |
| 1699 | BH16Ü | 5'-TGTTGTGTAATCATTAGCTTTGTTTCTAATAAACGATACCCACTCCAGCCCCTT-3' |
| 1700 | CH1 | 5'-GCTAATGATTACACAACAGAGTACAGTGCATCTGTGAAGGGTAGAGTCACCATGACCAGGRAC-3' |
| 1701 | CH2 | 5'-GCTAATGATTACACAACAGAGTACAGTGCATCTGTGAAGGGTAGGCTCACCATCWCCAAGGAC-3' |
| 1702 | CH3 | 5'-GCTAATGATTACACAACAGAGTACAGTGCATCTGTGAAGGGTCGAGTYACCATATCAGTAGAC-3' |
| 1703 | CH4 | 5'-GCTAATGATTACACAACAGAGTACAGTGCATCTGTGAAGGGTCGATTCACCATCTCCAGRGAC-3' |
| 1704 | CH5 | 5'-GCTAATGATTACACAACAGAGTACAGTGCATCTGTGAAGGGTAGATTCACCATCTCMAGAGA-3' |
| 1705 | CH6 | 5'-GCTAATGATTACACAACAGAGTACAGTGCATCTGTGAAGGGTMGGTTCACCATCTCCAGAGA-3' |
| 1706 | CH7 | 5'-GCTAATGATTACACAACAGAGTACAGTGCATCTGTGAAGGGTCGATTCAYCATCTCCAGAGA-3' |
| 1707 | CH8 | 5'-GCTAATGATTACACAACAGAGTACAGTGCATCTGTGAAGGGTCGAGTCACCATRTCMGTAGAC-3' |
| 1708 | CH9 | 5'-GCTAATGATTACACAACAGAGTACAGTGCATCTGTGAAGGGTAGRGTCACCATKACCAGGGAC-3' |
| 1709 | CH10 | 5'-GCTAATGATTACACAACAGAGTACAGTGCATCTGTGAAGGGTCAGGTCACCATCTCAGCCGAC-3' |
| 1710 | CH11 | 5-GCTAATGATTACACAACAGAGTACAGTGCATCTGTGAAGGGTCGAATAACCATCAACCCAGAC-3 |
| 1711 | CH12 | 5'-CTAATGATTACACAACAGAGTACAGTGCATCTGTGAAGGGTCGGTTTGTCTTCTCCATGGAC-3' |
| 1712 | CH13 | 5'-GCTAATGATTACACAACAGAGTACAGTGCATCTGTGAAGGGTAGAGTCACCATGACCGAGGAC-3' |
| 1713 | CH14 | 5'-GCTAATGATTACACAACAGAGTACAGTGCATCTGTGAAGGGTAGAGTCACGATTACCGCGGAC-3' |
| 1714 | CH15 | 5'-GCTAATGATTACACAACAGAGTACAGTGCATCTGTGAAGGGTAGAGTCACCATGACCACAGAC-3' |
| 1715 | CH1Ü | 5'-GTCCATAGCATGATACCTAGGGTATCTAGYACAGTAATACACGGC-3' |
| 1716 | CH2Ü | 5'-GTCCATAGCATGATACCTAGGGTATCTCGCACAGTAATACAYGGC-3' |
| 1717 | CH3Ü | 5'-GTCCATAGCATGATACCTAGGGTATCTYGCACAGTAATACACAGC-3' |
| 1718 | CH4Ü | 5'-GTCCATAGCATGATACCTAGGGTATGYYGCACAGTAATACACGGC-3' |
| 1719 | CH5Ü | 5'-GTCCATAGCATGATACCTAGGGTACCGTGCACARTAATAYGTGGC-3' |
| 1720 | CH6Ü | 5'-GTCCATAGCATGATACCTAGGGTATCTGGCACAGTAATACACGGC-3' |
| 1721 | CH7Ü | 5'-GTCCATAGCATGATACCTAGGGTATGTGGTACAGTAATACACGGC-3' |
| 1722 | CH8Ü | 5'-GTCCATAGCATGATACCTAGGGTATCTCGCACAGTGATACAAGGC-3' |
| 1723 | CH9Ü | 5'-GTCCATAGCATGATACCTAGGGTATTTTGCACAGTAATACAAGGC-3' |
| 1724 | CH10Ü | 5'-GTCCATAGCATGATACCTAGGGTATCTTGCACAGTAATACATGGC-3' |
| 1725 | CH11Ü | 5'-GTCCATAGCATGATACCTAGGGTAGTGTGCACAGTAATATGTGGC-3' |
| 1726 | CH12Ü | 5'-GTCCATAGCATGATACCTAGGGTATTTCGCACAGTAATATACGGC-3' |
| 1727 | CH13Ü | 5'-GTCCATAGCATGATACCTAGGGTATCTCACACAGTAATACACAGC-3' |
| 1728 | DH1 | 5'-CCTAGGTATCATGCTATGGACTCCTGGGGCCARGGMACCCTGGTC-3' |
| 1729 | DH2 | 5'-CCTAGGTATCATGCTATGGACTCCTGGGGSCAAGGGACMAYGGTC-3' |
| 1730 | DH3 | 5'-CCTAGGTATCATGCTATGGACTCCTGGGGCCGTGGCACCCTGGTC-3' |
| 1731 | DH1Ü | 5'-**GGAAGACCGATGGGCCCTTGGTGGAGGCT**GAGGAGACRGTGACCAGGGT-3' |
| 1732 | DH2Ü | 5'-**GGAAGACCGATGGGCCCTTGGTGGAGGC**TGAGGAGACRGTGACCRTKGT-3' |
| 1733 | H3Ü | 5'-**GGAAGACCGATGGGCCTTGGTGGAGGC**TGAGGAGACGGTGACCAGGGT-3' |

### 5.3.2- Construction of the V_{H} and V_{L} sub-libraries

V_{L} sub1 sub-library was assembled sequentially using the polymerase chain reaction (PCR) by overlap extension. Ho *et al*., Gene 77:51-59 (1989). More precisely, so-called "intermediate" PCRs were carried out to synthesize each individual human germline framework fused in frame with a portion of the corresponding donor CDRs using the following oligonucleotide combinations: AL1-13/AL1Ü-10Ü/1-46, BL1-10/BL1Ü-16Ü/47-92, CL1-11/CL1Ü-12Ü/93-138 and DL1-4/DL1Ü-4Ü/139-143 for the 1^{st}, 2^{nd}, 3^{rd} and 4^{th} frameworks, respectively. This was carried out using *pfu* DNA polymerase (PCR SuperMix, Invitrogen) in 100 µl volume and approximately 5 pmol of oligonucleotides AL1-13, AL1Ü-10Ü, BL1-10, BL1Ü-16Ü, CL1-11, CL1Ü-12Ü, DL1-4 and DL1Ü-4Ü and approximately 100 pmol of oligonucleotides 1-143. The PCR program consisted of 5 min at 95°C; 1 min at 94°C, 1 min at 45°C, 1 min at 72°C for 30 cycles then 8 min at 72°C. A second PCR ("assembly PCR") was then carried out using *pfu* DNA polymerase (PCR SuperMix, Invitrogen), 0.5-2 µl of each of the "intermediate" PCRs, 25 pmol of each of the oligonucleotides DL1Ü, DL2Ü, DL3Ü, DL4Ü (see Table 64) and 100 pmol of the biotinylated oligonucleotide 5'-GGTCGTTCCATTTTACTCCCAC-3' (SEQ ID NO. 1734) in a 100 µl reaction volume. The assembly PCR program consisted of 5 min at 95°C; 30 s at 94°C, 30 s at 50°C, 45 s at 72°C for 30 cycles then 8 min at 72°C.

V_{H} subl, V_{H} sub2 and V_{L} sub2 framework-shuffled sub-libraries were also synthesized using the PCR by overlap extension. Ho *et al*., Gene 77:51-59 (1989). This total *in vitro* synthesis of the framework shuffled V_{H} and V_{L} genes was done essentially as described H. Wu *et al*., Methods Mol. Biol. 207: 213-233 (2003). Briefly, a first so-called "fusion PCR" was carried out using *pfu* DNA polymerase (PCR SuperMix, Invitrogen). Construction of V₁₁ sub1 was carried out using approximately 3-10 pmol of each of the oligonucleotides described in Tables 5, 6, 7, 11 and 65 in a 100 µl reaction volume. Construction of V_{H} sub2 was carried out using approximately 0.5 pmol of each of the oligonucleotides described in Tables 5, 6, 7, 11 and 65 in a 100 µl reaction volume. Construction of V_{L} sub2 was carried out using approximately 0.5 pmol of each of the oligonucleotides described in Tables 1, 2, 3, 4, and 64 in a 100 µl reaction volume. For each V_{H} sub1, V_{H} sub2 and V_{L} sub2 sub-library, the fusion PCR program consisted of 1 min at 95°C; 20 s at 94°C, 30 s at 50°C, 30 s at 72°C for 5 cycles; 20 s at 94°C, 30 s at 55°C, 30 s at 72°C for 25 cycles then 7 min at 72°C. A second so-called "synthesis PCR" then followed. More precisely, V_{H} sub 1 and V_{H} sub2 sub-libraries were synthesized using pfu DNA polymerase (PCR SuperMix, Invitrogen), 2-3 µl of the corresponding "fusion PCR", 30 pmol of each of the oligonucleotides DH1Ü, DH2Ü, DH3Ü (see Table 65) and 100 pmol of the biotinylated oligonucleotide 5'-GCTGGTGGTGCCGTTCTATAGCC-3' (SEQ ID NO. 1735) in a 100 µl reaction volume. V_{L} sub2 sub-library was synthesized using *pfu* DNA polymerase (PCR SuperMix, Invitrogen), 3 µl of the corresponding "fusion PCR", 25 pmol of each of the oligonucleotides DL1Ü, DL2Ü, DL3Ü, DL4Ü (see Table 64) and 100 pmol of the biotinylated oligonucleotide 5'-GGTCGTTCCATTTTACTCCCAC-3' (SEQ ID NO. 1734) in a 100 µl reaction volume. For each V_{H} subl, V_{H} sub2 and V_{L} sub2 sub-library, the synthesis PCR program consisted of 5 min at 94°C; 1 min at 94°C, 1 min at 45°C, 1 min at 72°C for 30 cycles then 8 min at 72°C.

### 5.3.3- Synthesis of the V_{L}-12C8 and V_{L}-8G7 genes

V_{L}-12C8 and V_{L}-8G7 light chain genes, used in the context of library B (V_{L}-12C8+V_{L}-8G7+V_{H} sub 1), were synthesized by PCR from the corresponding V region-encoding M13 phage vector (see §§ 5.4.1.1, 5.4.1.2, 5.4.1.3) using the 12C8for/12C8back and 8G7for/8G7back oligonucleotide combinations, respectively (see below).
12C8for **5'-GGTCGTTCCATTTTACTCCCACTCCGCCATCCAGTTGACTCAGTCTCC-**3'(biotinylated) (SEQ ID NO. 1736)
12C8back 5'-GATGAAGACAGATGGTGCAGCCACAGTACGTTTGATCTCCAGCTTGGTCCCTCC-3' (SEQ ID NO. 1737)
**8G7for 5'-GGTCGTTCCATTTTACTCCCACTCCGAAA**TTGTGTTGACACAGTCTCCAG-3' (biotinylated) (SEQ ID NO. 1738)
8G7back 5'-GATGAAGACAGATGGTGCAGCCACAGTACGTTTGATATCCACTTTGGTCCCTC-3' (SEQ ID NO. 1739).
Oligonucleotides 12C8for and 8G7for contain a M13 gene 3 leader overlapping sequence (bold). Oligonucleotides 8G7back and 12C8back contain a Cκ overlapping sequence (underlined).

### 5.3.4- Synthesis of the V_{H}-233 and V_{L}-233 genes

V_{H}-233 and V_{L}-233 heavy and light chain genes, used in the context of a chimaeric Fab positive control (V_{H}-233+V_{L}-233) or of library A (V_{L} sub1+V_{H}-233), were synthesized by PCR from the corresponding pSTBlue-1 (see § 5.1) vector using the 233Hfor/233Hback and 233Lfor/233Lback oligonucleotide combinations, respectively (see below).
233Hfor 5'-**GCTGGTGGTGCCGTTCT**ATAGCCATAGCGAGGTGAAGCTGGTGGAGTCTGGAGGAG-3' (biotinylated) (SEQ ID NO. 1740)
233Hback 5'-GGAAGACCGATGGGCCCTTGGTGGAGGCTGAGGAGACGGTGACTGAGGTTCCTTG-3' (SEQ ID NO. 1741)
233Lfor 5'-**GGTCGTTCCATTTTACTCCCACTCCGAT**ATTGTGCTAACTCAGTCTCCAGCCACCCTG -3' (biotinylated) (SEQ ID NO. 1742)
233Lback 5'-GATGAAGACAGATGGTGCAGCCACAGTACGTTTCAGCTCCAGCTTGGTCCCAGCACCG AACG-3' (SEQ ID NO. 1743)
Oligonucleotides 233Hfor and 233Lfor contain a M13 gene 3 leader overlapping sequence (bold). Oligonucleotide 233Hback contains a C_{κ1} overlapping sequence (underlined). Oligonucleotide 233Lback contains a Cκ overlapping sequence (underlined).

### 5.3.5- Cloning of the various V regions into a phage expression vector

Libraries A, B and C as well as the chimaeric Fab version of mAb B233 were cloned into a M13-based phage expression vector. This vector allows the expression of Fab fragments that contain the first constant domain of a human γ1 heavy chain and the constant domain of a human kappa (κ) light chain under the control of the *lacZ* promoter (Figure 2). The cloning was carried out by hybridization mutagenesis, Kunkel et al., Methods Enzymol. 154:367-382 (1987), as described Wu et al., Methods Mol. Biol. 207: 213-233 (2003). Briefly, minus single-stranded DNA corresponding to the various V regions of interest (see §5.3.2, §5.3.3 and §5.3.4) was purified from the final PCR products by ethanol precipitation after dissociation of the double-stranded PCR product using sodium hydroxide and elimination of the biotinylated strand by streptavidin-coated magnetic beads as described (H. Wu, et al., Methods Mol. Biol. 207: 213-233(2003); H. Wu, Methods Mol. Biol. 207: 197-212 (2003)). Equimolar amounts of different minus strands were mixed as follows: V_{H}-233/V_{L}subl, V_{H} subl/V_{L}-8G7/V_{L}-12C8, V_{H} sub2/V_{L} sub2 and V_{H}-233 N_{L}-233 to construct library A, library B, library C and chimaeric Fab 233, respectively. These different mixes were then individually annealed to two regions of the vector containing each one palindromic loop. Those loops contained a unique XbaI site which allows for the selection of the vectors that contain both V_{L} and V_{H} chains fused in frame with the human kappa (κ) constant and first human γ constant regions, respectively. Synthesized DNA was then electroporated into XL1-Blue for plaque formation on XL1-Blue bacterial lawn or production of Fab fragments as described Wu et al., Methods Mol. Biol. 207: 213-233 (2003).

### 5.4- Screening of the libraries

### 5.4.1- Primary screen

### 5.4.1.1- Description

The primary screen consisted of a single point ELISA (SPE) which was carried out using periplasmic extracts prepared from 1 ml-bacterial culture grown in 96 deep-well plates and infected with individual recombinant M13 clones (see §5.3.5) essentially as described in Wu et al., Methods Mol. Biol. 207: 213-233 (2003). Briefly, individual wells of a 96-well Maxisorp Immunoplate were coated with 20-500 ng of a goat anti-human Fab antibody, blocked with 3% BSA/PBS for 2h at 37°C and incubated with samples (periplasm-expressed Fabs) for 1h at room temperature. 300 ng/well of biotinylated human EphA2-Fc was then added for 1h at room temperature. This was followed by incubation with neutravidin-horseradish peroxidase (HRP) conjugate for 40 min at room temperature. HRP activity was detected with tetra-methyl-benzidine (TMB) substrate and the reaction quenched with 0.2 M H₂SO₄. Plates were read at 450 nm.

### 5.4.1.2- Results of the primary screen

Out of ∼ 500 clones from library A that were screened using 100 ng of the goat anti-human Fab capture reagent, 14 exhibited a significant signal (OD₄₅₀ ranging from 0.2-0.6). This typically corresponded to a signal at least 1.3-fold above the corresponding background signal (OD₄₅₀ ranged from 0.1-0.4) of an irrelevant antibody (MEDI-493; S. Johnson et al., J. Infect. Dis. 176: 1215-1224 (1997)). Under these conditions, Fab 233 exhibited an OD₄₅₀ ranging from 0.4-0.6.

Out of ∼ 200 clones from library A that were screened using 20 ng of the goat anti-human Fab capture reagent, 4 exhibited a significant signal (OD₄₅₀ ranging from 0.2-0.4). This typically corresponded to a signal at least 2-fold above the corresponding background signal of an irrelevant antibody (OD₄₅₀ of 0.1). Under these conditions, Fab 233 exhibited an OD₄₅₀ ranging from 0.2-0.3.

Out of ∼ 750 clones from library A that were screened using 500 ng of the goat anti-human Fab capture reagent, 16 exhibited a significant signal (OD₄₅₀ ranging from 0.1-0.7). This typically corresponded to a signal at least 1.3-fold above the corresponding background signal of an irrelevant antibody (OD₄₅₀ ranged from 0.06-0.2). Under these conditions, Fab 233 exhibited an OD₄₅₀ ranging from 0.1-0.6. Clones V_{H}-233/V_{L}-12C8 and V_{H}-233N_{L}-8G7 were isolated from this round of screening and both exhibited an OD₄₅₀ of 0.4 (same plate background OD₄₅₀ values were 0.1 and 0.2, respectively; same plate Fab 233 OD₄₅₀ values were 0.2 and 0.5, respectively).

Out of ∼ 750 clones from library B that were screened using 500 ng of the goat anti-human Fab capture reagent, 27 exhibited a significant signal (OD₄₅₀ ranging from 0.3-2.8). This typically corresponded to a signal at least 1.3-fold above the corresponding background signal of an irrelevant antibody (OD₄₅₀ ranged from 0.2-0.3). Under these conditions, both V_{H}-233/V_{L}-12C8 and V_{H}-233/V_{L}-8G7 exhibited OD₄₅₀ values ranging from 0.2-0.4. Clones V_{H}-2G6/V_{L}-12C8, V_{H}-6H11/V_{L}-8G7 and V_{H}-7E8/V_{L}-8G7 were isolated from this round of screening and exhibited an OD₄₅₀ of 2.8, 2.5 and 1.6, respectively (same plate background OD₄₅₀ values were 0.3, 0.2 and 0.2, respectively, same plate V_{H}-233/V_{L}-12C8 OD₄₅₀ values were 0.4, 0.3 and 0.3, respectively; same plate V_{H}-233/V_{L}-8G7 OD₄₅₀ values were 0.4, 0.3 and 0.3, respectively).

Out of ∼ 1150 clones from library C that were screened using 500 ng of the goat anti-human Fab capture reagent, 36 exhibited a significant signal (OD₄₅₀ ranging from 0.1-0.3). This typically corresponded to a signal at least 1.3-fold above the corresponding background signal of an irrelevant antibody (OD₄₅₀ ranged from 0.07-0.1). Under these conditions, Fab 233 exhibited an OD₄₅₀ ranging from 0.1-0.2.

### 5.4.1.3- Validation of the positive clones

Altogether, 9 clones from library A, 7 clones from library B and 0 clone from library C were re-confirmed in a second, independent, single point ELISA using periplasmic extracts prepared from 15 ml-bacterial culture and 500 ng of the goat anti-human Fab capture reagent. Specifically, two clones from library A (V_{H}-233N_{L}-12C8 and V_{H}-233/V_{L}-8G7) that exhibited amongst the highest [specific OD₄₅₀/background OD₄₅₀] ratio (ranging from approximately 15-50) were further characterized by dideoxynucleotide sequencing using a ABI3000 genomic analyzer. DNA sequence analysis of clone V_{H}-233/V_{L}-12C8 revealed that its heavy chain contained a single base substitution at base 104 resulting in a substitution (N to S) at position H35 (Kabat numbering). This mutation was corrected using the QuickChange XL site-directed mutagenesis Kit (Stratagene, La Jolla, CA) according to the manufacturer's instructions. Corrected clone V_{H}-233/V_{L}-12C8 exhibited a [specific OD₄₅₀/background OD₄₅₀] ratio up to approximately 50 (similar to mutated V_{H}-233/V_{L}-12C8) which indicated retention of binding to EphA2-Fc. Partially humanized clones V_{H}-233/V_{L}-12C8 and V_{H}-233/V_{L}-8G7 were then selected for further characterization by a secondary screen (see §5.4.2). The sequences of V_{L}-12C8 and V_{L}-8G7 are indicated in Figure 3. As mentioned above, these two humanized light chains were then included in the design of Library B. Three clones from this library that exhibited amongst the highest [specific OD₄₅₀/background OD₄₅₀] ratio (approximately 40) were further characterized by dideoxynucleotide sequencing. This led to the identification of three different humanized heavy chains (V_{H}-2G6, V_{H}-6H11 and V_{H}-7E8; see Figure 3). V_{H}-2G6, V_{H}-6H11 and V_{H}-7E8 were found to be paired with V_{L}-12C8, V_{L}-8G7 and V_{L}-8G7, respectively. These three fully humanized clones were then selected for further characterization by a secondary screen (see §5.4.2).

### 5.4.2- Secondary screen

### 5.4.2.1- Description

In order to further characterize the previously identified humanized clones (see §5.4.1.3), a secondary screen using Fab fragments expressed in periplasmic extracts prepared from 15 ml-bacterial culture was carried out. More precisely, two ELISAs were used: (i) a functional ELISA in which individual wells of a 96-well Maxisorp Immunoplate were coated with 500 ng of human EphA2-Fc and blocked with 3%BSA/PBS for 2h at 37°C. 2-fold serially diluted samples were then added and incubated for 1h at room temperature. Incubation with a goat anti-human kappa horseradish peroxidase (HRP) conjugate then followed. HRP activity was detected with TMB substrate and the reaction quenched with 0.2 M H₂SO₄. Plates were read at 450 nm; (ii) an anti-human Fab quantification ELISA which was carried out essentially as described. Wu *et al.,* Methods Mol. Biol. 207: 213-233 (2003). Briefly, individual wells of a 96-well Immulon Immunoplate were coated with 100 ng of a goat anti-human Fab antibody and then incubated with 2-fold serially diluted samples (starting at a 1/25 dilution) or standard (human IgG Fab, 500-3.91 ng/ml). Incubation with a goat anti-human kappa horseradish peroxidase (HRP) conjugate then followed. HRP activity was detected with TMB substrate and the reaction quenched with 0.2 M H₂SO₄. Plates were read at 450 nm.

### 5.4.2.2- Results of the secondary screen

The two-part secondary ELISA screen allowed us to compare Fab clones V_{H}-233/V_{L}-12C8, V_{H}-233N_{L}-8G7, V_{H}-2G6/V_{L}-12C8, V_{H}-6H11/V_{L}-8G7 and V_{H}-7E8/V_{L}-8G7 to each other and to the chimaeric Fab of mAb B233 (V_{H}-233/V_{L}-233) in terms of binding to human EphA2. As shown in Figure 4, all framework shuffled Fabs retain binding to human EphA2 as compared with the chimaeric Fab of mAb B233. Interestingly, some clones whose heavy and light chains are both humanized (V_{H}-2G6/V_{L}-12C8 and V_{H}-7E8/V_{L}-8G7) exhibit better apparent binding to human EphA2-Fc than clones in which only the same light chains are humanized (V_{H}-233/V_{L}-12C8 and V_{H}-233/V_{L}-8G7). This indicates the existence of a process whereby humanized heavy chains are specifically selected for optimal binding to the antigen in the context of a given humanized light chain. In order to further characterize the different fully humanized molecules, clones V_{H}-2G6/V_{L}-12C8, V_{H}-6H11/V_{L}-8G7 and V_{H}-7E8/V_{L}-8G7 as well as the chimaeric form of mAb B233 (V_{H}-233/V_{L}-233) were then cloned and expressed as a full length human IgG1 (see § 5.5).

### 5.5 Cloning, expression and purification of the various humanized versions of mAb B233 in a human IgG1 format

The variable regions of framework shuffled clones V_{H}-2G6, V_{H}-6H11, V_{H}-7E8, V_{L}-12C8 and V_{L}-8G7 and of V_{H}-233 and V_{L}-233 were PCR-amplified from the corresponding V region-encoding M 13 phage vectors *using pfu* DNA polymerase. They were then individually cloned into mammalian expression vectors encoding a human cytomegalovirus major immediate early (hCMVie) enhancer, promoter and 5'-untranslated region. M. Boshart, *et al.,* Cell 41:521-530 (1985). In this system, a human γ chain is secreted along with a human κ chain. S. Johnson, *et al.,* Infect. Dis. 176:1215-1224 (1997). The different constructs were expressed transiently in human embryonic kidney (HEK) 293 cells and harvested 72 hours post-transfection. The secreted, soluble human IgG1s were purified from the conditioned media directly on 1 ml HiTrap protein A or protein G columns according to the manufacturer's instructions (APBiotech, Inc., Piscataway, NJ). Purified human IgG1s (typically >95%homogeneity, as judged by SDS-PAGE) were recovered in yields varying from 2-13 µg/ml conditioned media, dialyzed against phosphate buffered saline (PBS), flash frozen and stored at -70°C.

### 5.6 BIAcore analysis of the binding of framework-shuffled, chimaeric and mAb B233 IgGs to EphA2-Fc

The interaction of soluble V_{H}-2G6/V_{L}-12C8, V_{H}-6H11/V_{L}-8G7, V_{H}-7E8/V_{L}-8G7 and V_{H}-233/V_{L}-233 IgGs as well as of mAb B233 with immobilized EphA2-Fc was monitored by surface plasmon resonance detection using a BIAcore 3000 instrument (Pharmacia Biosensor, Uppsala, Sweden). EphA2-Fc was coupled to the dextran matrix of a CM5 sensor chip (Pharmacia Biosensor) using an Amine Coupling Kit as described (B. Johnsson et al., Anal. Biochem. 198: 268-277 (1991)) at a surface density of between 105 and 160 RU. IgGs were diluted in 0.01 M HEPES pH 7.4 containing 0.15 M NaCl, 3 mM EDTA and 0.005% P20. All subsequent dilutions were made in the same buffer. All binding experiments were performed at 25°C with IgG concentrations typically ranging from 100 nM to 0.2 nM at a flow rate of 75 µL/min; data were collected for approximately 25 min and one 1-min pulse of 1M NaCl, 50 mM NaOH was used to regenerate the surfaces. IgGs were also flowed over an uncoated cell and the sensorgrams from these blank runs subtracted from those obtained with EphA2-Fc-coupled chips. Data were fitted to a 1:1 Langmuir binding model. This algorithm calculates both the *k*ₒₙ and the *k_{off}*, from which the apparent equilibrium dissociation constant, K_{D}, is deduced as the ratio of the two rate constants (*k_{off}*/*kₒₙ*). The values obtained are indicated in Table 66.

**Table 66. Affinity measurements for the binding of different IgGs to human EphA2-Fc^{a}**

| **Antibody** Constant (*K_{D}*)^{c} | Association rate (*kₒₙ*)^{b} (M⁻¹.s⁻¹) | Dissociation rate (*k_{off}*)^{b} (s⁻¹) | Dissociation (nM) |
|---|---|---|---|
| B233 (murine) | 2.8 x 10⁵ | 1.1 x 10⁻⁴ | 0.4V_{H}- |
| B233N_{L}-B233 (chimaeric) | 2.4 x 10⁵ | 8.0 x 10⁻⁵ | 0.3 |
| V_{H}-2G6/V_{L}-12C8 (humanized) | 6.4 x 10⁴ | 1.9 x 10⁻⁴ | 3.0 |
| V_{H}-6H11/V_{L}-8G7 (humanized) | 9.6 x 10⁴ | 1.8 x 10⁴ | 1.9 |
| V_{H}-7E8/V_{L}-8G7 (humanized) | 9.3 x 10³ | 4.5 x 10⁻⁴ | 48 |
| ³ Affinity measurements were carried out by BIAcore as reported in Description of Method. ^{b}Kinetic parameters represent the average of 5-18 individual measurements. *^{c}K_{D}* was calculated as a ration of the rate constants (*k_{off}*/*kₒₙ*). | | | |

### 5.7- Analysis of the framework-shuffled variants

### 5.7.1- Sequence analysis

Overall, two unique humanized light chains (V_{L}-12C8 and V_{L}-8G7) and three unique humanized heavy chains (V_{H}-2G6, V_{H}-6H11 and V_{H}-7E8) were found that supported efficient binding to human EphA2-Fc. The promiscuous nature of humanized light chain V_{L}-8G7 is highlighted by its ability to mediate productive binding in the context of two different heavy chains (V_{H}-7E8 and V_{H}-6H11). All of these humanized variants exhibited a high level of global amino acid identity to mAb B233 in the corresponding framework regions, ranging from 76-83% for the heavy chains and from 64-69% for the light chains (Figure 5). This can be explained by the fact that high-homology human frameworks are more likely to retain parental key residues. Analysis of individual frameworks revealed a wider range of differences, ranging from 48% for the first framework of V_{L}-12C8 to 91% for the fourth framework of V_{H}-2G6, V_{H}-6H11 and V_{H}-7E8.

Interestingly, humanized heavy chain V_{H}-7E8 consisted exclusively of human frameworks that were a perfect match with human framework germline sequences (Figure 5). Humanized heavy chains V_{H}-6H11 and V_{H}-2G6 contained one and two human frameworks, respectively, that exhibited a near-perfect match with the most related human framework germline sequences (Figure 5). The differences amounted to a maximum of three residues per chain (V_{H}-2G6) and two residues per framework (first framework of V_{H}-2G6). In no cases did these differences encode amino acids not found in other most distant human framework germline sequences. Thus, arguably, these clones may also be referred to as "fully humanized". Humanized light chains V_{L}-12C8 and V_{L}-8G7 contained one and three human frameworks, respectively, that exhibited a near-perfect match with the most related human framework germline sequences (Figure 5). The number of differences amounted to a maximum of three residues per chain (V_{L}-8G7) and one residue per framework (first, second and fourth framework of V_{L}-8G7; fourth framework of V_{L}-12C8). However, here again, the residues at these positions were also found in other, less homologous human framework sequences; therefore these variants may also be referred to as fully humanized. Since these differences were not built-in within our libraries, we attribute their origin to a combination of factors such as PCR fidelity and/or oligonucleotides quality.

### 5.7.2- Binding analysis

It is worth nothing that only a two-step humanization process in which the light and heavy chains of mAb B233 were successively humanized (Library A and B) allowed us to isolate humanized clones retaining binding to human EphA2-Fc. Indeed, screening of a library in which both the light and heavy chains were simultaneously humanized (Library C) did not allow us to recover molecules exhibiting detectable binding to this antigen. This probably reflects factors such as sub-optimal library quality, incomplete library sampling and/or inefficient prokaryotic expression of a portion of the library. We anticipate that screening a larger number of clones would have resulted in the identification of humanized antibody fragments retaining binding to human EphA2.

As expected in light of their identical heavy and light chains variable regions, parental mAb B233 and its chimaeric IgG version exhibited virtually identical dissociation constant (K_{D} = 0.4 and 0.3 nM, respectively; Table 66). Humanized clones V_{H}-6H11/V_{L}-8G7 and V_{H}-2G6/V_{L}-12C8, when formatted as a human IgG1, exhibited avidities towards human EphA2 which were similar to the parental and chimaeric version of mAb B233 (K_{D} = 1.9 and 3.0 nM, respectively; Table 66). This corresponded to a small avidity decrease of 6 and 10-fold, respectively, when compared with parental mAb B233. Humanized clone V_{H}-7E8/V_{L}-8G7 exhibited the lowest avidity (K_{D} = 48 nM), which corresponded to a larger decrease of 160-fold when compared with parental mAb B233. It is worth noting that in terms of strength of binding to EphA2-Fc, the BIAcore-based ranking of humanized IgG clones V_{H}-6H11N_{L}-8G7, V_{H}-2G6/V_{L}-12C8 and V_{H}-7E8/V_{L}-8G7 (Table 66) was different from the ELISA-based ranking that utilized their Fab counterparts (Figure 4). This is particularly striking in the case of clone V_{H}-7E8/V_{L}-8G7 which showed the lowest avidity (Table 66), yet consistently exhibited the highest signal by ELISA titration (Figure 4). We do not know what accounts for this difference but think that it is likely attributable to the format of the assays and/or imprecision in the quantification ELISA. Alternatively, it is possible that this discrepancy reflects unique, clone-specific correlations between affinity (as measured in Figure 4) and avidity (as measured in Table 66). Indeed, individual bivalent binding measurements depend on various factors such as the particular spatial arrangements of the corresponding antigen binding sites or the local antigen surface distribution (D.M. Crothers, et al. Immunochemistry 9: 341-357(1972); K.M. Müller, et al., Anal. Biochem. 261: 49-158(1998)).

## Claims

1. A method of generating a combinatorial library comprising a plurality of nucleotide sequences encoding humanized heavy chain variable regions, said nucleotide sequences encoding the humanized heavy chain variable regions each produced by fusing together a nucleic acid sequence encoding a human heavy chain framework region 1, a nucleic acid sequence encoding a heavy chain CDR1, a nucleic acid sequence encoding a human heavy chain framework region 2, a nucleic acid sequence encoding a heavy chain CDR2, a nucleic acid sequence encoding a human heavy chain framework region 3, a nucleic acid sequence encoding a heavy chain CDR3, and a nucleic acid sequence encoding a human heavy chain framework region 4, wherein the CDRs are derived from a non-human donor antibody heavy chain variable region and each nucleic acid sequence encoding a heavy chain framework region is from a sub-bank comprising a plurality of nucleic acid sequences encoding said human heavy chain framework region from human germline framework sequences and/or framework regions from functional human antibody sequences.

2. A method of generating a combinatorial library comprising a plurality of nucleotide sequences encoding humanized light chain variable regions, said nucleotide sequences encoding the humanized light chain variable regions each produced by fusing together a nucleic acid sequence encoding a human light chain framework region 1, a nucleic acid sequence encoding a light chain CDR1, a nucleic acid sequence encoding a human light chain framework region 2, a nucleic acid sequence encoding a light chain CDR2, a nucleic acid sequence encoding a human light chain framework region 3, a nucleic acid sequence encoding a light chain CDR3, and a nucleic acid sequence encoding a human light chain framework region 4, wherein the CDRs are derived from a non-human donor antibody light chain variable region and each nucleic acid sequence encoding a light chain framework region is from a sub-bank comprising a plurality of nucleic acid sequences encoding said human light chain framework region from human germline framework sequences and/or framework regions from functional human antibody sequences.

3. A method of generating a combinatorial library comprising: (i) a first set of nucleotide sequences encoding humanized heavy chain variable regions, said first set of nucleotide sequences encoding humanized heavy chain variable regions each produced by fusing together a nucleic acid sequence encoding a human heavy chain framework region 1, a nucleic acid sequence encoding a heavy chain CDR1, a nucleic acid sequence encoding a human heavy chain framework region 2, a nucleic acid sequence encoding a heavy chain CDR2, a nucleic acid sequence encoding a human heavy chain framework region 3, a nucleic acid sequence encoding a heavy chain CDR3, and a nucleic acid sequence encoding a human heavy chain framework region 4, and (ii) a second set of nucleotide sequences encoding humanized light chain variable regions, said second set of nucleotide sequences encoding humanized light chain variable regions each produced by fusing together a nucleic acid sequence encoding a human light chain framework region 1, a nucleic acid sequence encoding a light chain CDR1, a nucleic acid sequence encoding a human light chain framework region 2, a nucleic acid sequence encoding a light chain CDR2, a nucleic acid sequence encoding a human light chain framework region 3, a nucleic acid sequence encoding a light chain CDR3, and a nucleic acid sequence encoding a human light chain framework region 4, wherein the heavy chain variable region CDRs are derived from a non-human donor antibody heavy chain variable region, the light chain variable region CDRs are derived from a non-human donor antibody light chain variable region, each nucleic acid sequence encoding a human heavy chain framework region is from a sub-bank comprising a plurality of nucleic acid sequences encoding said human heavy chain framework region from human germline framework sequences and/or framework regions from functional human antibody sequences and each nucleic acid sequence encoding a human light chain framework region is from a sub-bank comprising a plurality of nucleic acid sequences encoding said human light chain framework region from human germline framework sequences and/or framework regions from functional human antibody sequences.

4. The method of claim 1, 2 or 3, further comprising introducing the combinatorial library of nucleotide sequences into a population of cells.

5. A method of producing a humanized antibody that immunospecifically binds to an antigen, said method comprising: (a) synthesizing a plurality of polynucleotide sequences each comprising a nucleotide sequence encoding a humanized heavy chain variable region, said nucleotide sequence produced by fusing together nucleic acid sequence encoding a human heavy chain framework region 1, a nucleic acid sequence encoding a heavy chain CDR1, a nucleic acid sequence encoding a human heavy chain framework region 2, a nucleic acid sequence encoding a heavy chain CDR2, a nucleic acid sequence encoding a human heavy chain framework region 3, a nucleic acid sequence encoding a heavy chain CDR3, and a nucleic acid sequence encoding a human heavy chain framework region 4, wherein the CDRs are derived from a nonhuman donor antibody heavy chain variable region that immunospecifically binds said antigen and each heavy chain framework region nucleic acid sequence is from a subbank comprising a plurality of nucleic acid sequences encoding said human heavy chain framework region from human germline framework sequences and/or framework regions from functional human antibody sequences; (b) introducing the polynucleotide sequences into a population of cells each containing a polynucleotide sequence encoding a light chain variable region; and (c) expressing the polynucleotide sequences encoding the heavy chain variable region and the light chain variable region.

6. A method of producing a humanized antibody that immunospecifically binds to an antigen, said method comprising: (a) synthesizing a plurality of polynucleotide sequences each comprising a nucleotide sequence encoding a humanized light chain variable region, said nucleotide sequence produced by fusing together a nucleic acid sequence encoding a human light chain framework region 1, a nucleic acid sequence encoding a light chain CDR1, a nucleic acid sequence encoding a human light chain framework region 2, a nucleic acid sequence encoding light chain CDR2, a nucleic acid sequence encoding a human light chain framework region 3, a nucleic acid sequence encoding a light chain CDR3, and a nucleic acid sequence encoding a human light chain framework region 4, wherein the CDRs are derived from a nonhuman donor antibody light chain variable region that immunospecifically binds said antigen and each light chain framework region nucleic acid sequence is from a subbank comprising a plurality of nucleic acid sequences encoding said human light chain framework region from human germline framework sequences and/or framework regions from functional human antibody sequences; (b) introducing the polynucleotide sequences into a population of cells each containing a polynucleotide sequence encoding a heavy chain variable region and (c) expressing the polynucleotide sequences encoding the heavy chain variable region and the light chain variable region.

7. A method of producing a humanized antibody that immunospecifically binds to an antigen, said method comprising: (a) synthesizing a plurality of first polynucleotide sequences each comprising a nucleotide sequence encoding a humanized heavy chain variable region, said nucleotide sequence produced by fusing together a nucleic acid sequence encoding a human heavy chain framework region 1, a nucleic acid sequence encoding a heavy chain CDR1, a nucleic acid sequence encoding a human heavy chain framework region 2, a nucleic acid sequence encoding a heavy chain CDR2, a nucleic acid sequence encoding a human heavy chain framework region 3, a nucleic acid sequence encoding a heavy chain CDR3, and a nucleic acid sequence encoding a human heavy chain framework region 4, wherein the CDRs are derived from a non-human donor antibody heavy chain variable region that immunospecifically binds said antigen and each heavy chain framework region nucleic acid sequence is from a sub-bank comprising a plurality of nucleic acid sequences encoding said heavy chain framework region from human germline framework sequences and/or framework regions from functional human antibody sequences; (b) synthesizing a plurality of second polynucleotide sequences each comprising a nucleotide sequence encoding a humanized light chain variable region, said nucleotide sequence produced by fusing together a nucleic acid sequence encoding a human light chain framework region 1, a nucleic acid sequence encoding a light chain CDR1, a nucleic acid sequence encoding a human light chain framework region 2, a nucleic acid sequence encoding light chain CDR2, a nucleic acid sequence encoding a human light chain framework region 3, a nucleic acid sequence encoding a light chain CDR3, and a nucleic acid sequence encoding a human light chain framework region 4, wherein the CDRs are derived from a non-human donor antibody light chain variable region that immunospecifically binds said antigen and each light chain framework region nucleic acid sequence is from a sub-bank comprising a plurality of nucleic acid sequences encoding said human light chain framework region from human germline framework sequences and/or framework regions from functional human antibody sequences; (c) introducing the nucleic acid sequences generated in steps (a) and (b) into a population of cells; and (d) expressing the nucleotide sequences encoding the heavy chain variable region and the light chain variable region.

8. The method of claim 5, 6 or 7, further comprising, after the step of expressing the nucleotide sequences, the step of screening for an antibody that immunospecifically binds to the antigen.

9. The method of claim 5, 6, 7 or 8, further comprising, prior to step (a), the step of generating the sub-banks of framework region sequences.

10. The method of any of the preceding claims, wherein the human framework region sub-bank(s) comprise framework regions from human germline framework sequences and/or framework regions from functional human antibody sequences into which one or mutations have been introduced.

## Patentansprüche

1. Verfahren zum Erzeugen einer kombinatorischen Bibliothek, umfassend eine Mehrzahl von Nukleotidsequenzen, die für humanisierte variable Regionen der schweren Kette kodieren, wobei die Nukleotidsequenzen, die für die humanisierte variablen Regionen der schweren Kette kodieren, jeweils durch Fusion einer Nukleinsäuresequenz, die für eine humane Framework-Region 1 der schweren Kette kodiert, eine Nukleinsäuresequenz, die für eine CDR1 der schweren Kette kodiert, eine Nukleinsäuresequenz, die für eine humane Framework-Region 2 der schweren Kette kodiert, eine Nukleinsäuresequenz, die für eine CDR2 der schweren Kette kodiert, eine Nukleinsäuresequenz, die für eine humane Framework-Region 3 der schweren Kette kodiert, eine Nukleinsäuresequenz, die für eine CDR3 der schweren Kette kodiert, und eine Nukleinsäuresequenz, die für eine humane Framework-Region 4 der schweren Kette kodiert, erzeugt werden, wobei die CDRs von einer nicht humanen variablen Region der schweren Kette eines Spenderantikörpers abgeleitet sind und jede Nukleinsäuresequenz, die für eine Framework-Region der schweren Kette kodiert, von einer Sub-Bank stammt, umfassend mehrere Nukleinsäuresequenzen, die für die humane FrameworkRegion der schweren Kette kodieren, von humanen Keimlinien-Framework-Sequenzen und/oder Framework-Regionen von funktionellen humanen Antikörpersequenzen.

2. Verfahren zum Erzeugen einer kombinatorischen Bibliothek, umfassend eine Mehrzahl von Nukleotidsequenzen, die für humanisierte variable Regionen der leichten Kette kodieren, wobei die Nukleotidsequenzen, die für die humanisierten variablen Regionen der leichten Kette kodieren, jeweils durch Fusion einer Nukleinsäuresequenz, die für eine humane Framework-Region 1 der leichten Kette kodiert, eine Nukleinsäuresequenz, die für eine CDR1 der leichten Kette kodiert, eine Nukleinsäuresequenz, die für eine humane Framework-Region 2 der leichten Kette kodiert, eine Nukleinsäuresequenz, die für eine CDR2 der leichten Kette kodiert, eine Nukleinsäuresequenz, die für eine humane Framework-Region 3 der leichten Kette kodiert, eine Nukleinsäuresequenz, die für eine CDR3 der leichten Kette kodiert, und eine Nukleinsäuresequenz, die für eine humane Framework-Region 4 der leichten Kette kodiert, erzeugt werden, wobei die CDRs von einer nicht humanen variablen Region der leichten Kette eines Spenderantikörpers abgeleitet sind und jede Nukleinsäuresequenz, die für eine Framework-Region der leichten Kette kodiert, von einer Sub-Bank stammt, umfassend mehrere Nukleinsäuresequenzen, die für die humane FrameworkRegion der leichten Kette kodieren, von humanen Keimlinien-Framework-Sequenzen und/oder Framework-Regionen von funktionellen humanen Antikörpersequenzen.

3. Verfahren zum Erzeugen einer kombinatorischen Bibliothek, umfassend: (i) einen ersten Satz von Nukleotidsequenzen, die für humanisierte variable Regionen der schweren Kette kodieren, wobei der erste Satz von Nukleotidsequenzen, die für humanisierte variable Regionen der schweren Kette kodieren, jeweils durch Fusion einer Nukleinsäuresequenz, die für eine humane Framework-Region 1 der schweren Kette kodiert, eine Nukleinsäuresequenz, die für eine CDR1 der schweren Kette kodiert, eine Nukleinsäuresequenz, die für eine humane Framework-Region 2 der schweren Kette kodiert, eine Nukleinsäuresequenz, die für eine CDR2 der schweren Kette kodiert, eine Nukleinsäuresequenz, die für eine humane Framework-Region 3 der schweren Kette kodiert, eine Nukleinsäuresequenz, die für eine CDR3 der schweren Kette kodiert, und eine Nukleinsäuresequenz, die für eine humane Framework-Region 4 der schweren Kette kodiert, erzeugt wird, und (ii) einen zweiten Satz von Nukleotidsequenzen, die für humanisierte variable Regionen der leichten Kette kodieren, wobei der zweite Satz von Nukleotidsequenzen, die für humanisierte variable Regionen der leichten Kette kodieren, jeweils durch Fusion einer Nukleinsäuresequenz, die für eine humane Framework-Region 1 der leichten Kette kodiert, eine Nukleinsäuresequenz, die für eine CDR1 der leichten Kette kodiert, eine Nukleinsäuresequenz, die für eine humane Framework-Region 2 der leichten Kette kodiert, eine Nukleinsäuresequenz, die für eine CDR2 der leichten Kette kodiert, eine Nukleinsäuresequenz, die für eine humane Framework-Region 3 der leichten Kette kodiert, eine Nukleinsäuresequenz, die für eine CDR3 der leichten Kette kodiert, und eine Nukleinsäuresequenz, die für eine humane Framework-Region 4 der leichten Kette kodiert, erzeugt wird, wobei die CDRs einer variablen Region der schweren Kette von einer nicht humanen variablen Region der schweren Kette eines Spenderantikörpers abgeleitet sind, die CDRs einer variablen Region der leichten Kette von einer nicht humanen variablen Region der leichten Kette eines Spenderantikörpers abgeleitet sind, jede Nukleinsäuresequenz, die für eine Framework-Region der schweren Kette kodiert, von einer Sub-Bank stammt, umfassend mehrere Nukleinsäuresequenzen, die für die humane Framework-Region der schweren Kette kodieren, von humanen Keimlinien-Framework-Sequenzen und/oder Framework-Regionen von funktionellen humanen Antikörpersequenzen, und jede Nukleinsäuresequenz, die für eine Framework-Region der leichten Kette kodiert, von einer Sub-Bank stammt, umfassend mehrere Nukleinsäuresequenzen, die für die humane Framework-Region der leichten Kette kodieren, von humanen Keimlinien-Framework-Sequenzen und/oder Framework-Regionen von funktionellen humanen Antikörpersequenzen.

4. Verfahren nach Anspruch 1, 2 oder 3, des Weiteren umfassend das Einführen der kombinatorischen Bibliothek von Nukleotidsequenzen in eine Population von Zellen.

5. Verfahren zum Erzeugen eines humanisierten Antikörpers, der immunspezifisch an ein Antigen bindet, wobei das Verfahren umfasst: (a) Synthetisieren mehrerer Polynukleotidsequenzen, jeweils umfassend eine Nukleotidsequenz, die für eine humanisierte variable Region der schweren Kette kodiert, wobei die Nukleotidsequenz durch Fusion einer Nukleinsäuresequenz, die für eine humane Framework-Region 1 der schweren Kette kodiert, eine Nukleinsäuresequenz, die für eine CDR1 der schweren Kette kodiert, eine Nukleinsäuresequenz, die für eine humane Framework-Region 2 der schweren Kette kodiert, eine Nukleinsäuresequenz, die für eine CDR2 der schweren Kette kodiert, eine Nukleinsäuresequenz, die für eine humane Framework-Region 3 der schweren Kette kodiert, eine Nukleinsäuresequenz, die für eine CDR3 der schweren Kette kodiert, und eine Nukleinsäuresequenz, die für eine humane Framework-Region 4 der schweren Kette kodiert, erzeugt wird, wobei die CDRs von einer nicht humanen variablen Region der schweren Kette eines Spenderantikörpers abgeleitet sind, die immunspezifisch das Antigen bindet, und jede Nukleinsäuresequenz einer Framework-Region der schweren Kette von einer Sub-Bank stammt, umfassend mehrere Nukleinsäuresequenzen, die für die humane Framework-Region der schweren Kette kodieren, von humanen KeimlinienFramework-Sequenzen und/oder Framework-Regionen von funktionellen humanen Antikörpersequenzen; (b) Einführen der Polynukleotidsequenzen in eine Population von Zellen, die jeweils eine Polynukleotidsequenz enthalten, die für eine variable Region der leichten Kette kodiert; und (c) Exprimieren der Polynukleotidsequenzen, die für die variable Region der schweren Kette und die variable Region der leichten Kette kodieren.

6. Verfahren zum Erzeugen eines humanisierten Antikörpers, der immunspezifisch an ein Antigen bindet, wobei das Verfahren umfasst: (a) Synthetisieren mehrerer Polynukleotidsequenzen, jeweils umfassend eine Nukleotidsequenz, die für eine humanisierte variable Region der leichten Kette kodiert, wobei die Nukleotidsequenz durch Fusion einer Nukleinsäuresequenz, die für eine humane Framework-Region 1 der leichten Kette kodiert, eine Nukleinsäuresequenz, die für eine CDR1 der leichten Kette kodiert, eine Nukleinsäuresequenz, die für eine humane Framework-Region 2 der leichten Kette kodiert, eine Nukleinsäuresequenz, die für eine CDR2 der leichten Kette kodiert, eine Nukleinsäuresequenz, die für eine humane Framework-Region 3 der leichten Kette kodiert, eine Nukleinsäuresequenz, die für eine CDR3 der leichten Kette kodiert, und eine Nukleinsäuresequenz, die für eine humane Framework-Region 4 der leichten Kette kodiert, erzeugt wird, wobei die CDRs von einer nicht humanen variablen Region der leichten Kette eines Spenderantikörpers abgeleitet sind, die immunspezifisch das Antigen bindet, und jede Nukleinsäuresequenz einer Framework-Region der leichten Kette von einer Sub-Bank stammt, umfassend mehrere Nukleinsäuresequenzen, die für die humane Framework-Region der leichten Kette kodieren, von humanen KeimlinienFramework-Sequenzen und/oder Framework-Regionen von funktionellen humanen Antikörpersequenzen; (b) Einführen der Polynukleotidsequenzen in eine Population von Zellen, die jeweils eine Polynukleotidsequenz enthalten, die für eine variable Region der schweren Kette kodiert; und (c) Exprimieren der Polynukleotidsequenzen, die für die variable Region der leichten Kette und die variable Region der schweren Kette kodieren.

7. Verfahren zum Erzeugen eines humanisierten Antikörpers, der immunspezifisch an ein Antigen bindet, wobei das Verfahren umfasst: (a) Synthetisieren mehrerer erster Polynukleotidsequenzen, jeweils umfassend eine Nukleotidsequenz, die für eine humanisierte variable Region der schweren Kette kodiert, wobei die Nukleotidsequenz durch Fusion einer Nukleinsäuresequenz, die für eine humane Framework-Region 1 der schweren Kette kodiert, eine Nukleinsäuresequenz, die für eine CDR1 der schweren Kette kodiert, eine Nukleinsäuresequenz, die für eine humane Framework-Region 2 der schweren Kette kodiert, eine Nukleinsäuresequenz, die für eine CDR2 der schweren Kette kodiert, eine Nukleinsäuresequenz, die für eine humane Framework-Region 3 der schweren Kette kodiert, eine Nukleinsäuresequenz, die für eine CDR3 der schweren Kette kodiert, und eine Nukleinsäuresequenz, die für eine humane Framework-Region 4 der schweren Kette kodiert, erzeugt wird, wobei die CDRs von einer nicht humanen variablen Region der schweren Kette eines Spenderantikörpers abgeleitet sind, die immunspezifisch das Antigen bindet, und jede Nukleinsäuresequenz einer Framework-Region der schweren Kette von einer Sub-Bank stammt, umfassend mehrere Nukleinsäuresequenzen, die für die humane FrameworkRegion der schweren Kette kodieren, von humanen Keimlinien-Framework-Sequenzen und/oder Framework-Regionen von funktionellen humanen Antikörpersequenzen; (b) Synthetisieren mehrerer zweiter Polynukleotidsequenzen, jeweils umfassend eine Nukleotidsequenz, die für eine humanisierte variable Region der leichten Kette kodiert, wobei die Nukleotidsequenz durch Fusion einer Nukleinsäuresequenz, die für eine humane Framework-Region 1 der leichten Kette kodiert, eine Nukleinsäuresequenz, die für eine CDR1 der leichten Kette kodiert, eine Nukleinsäuresequenz, die für eine humane Framework-Region 2 der leichten Kette kodiert, eine Nukleinsäuresequenz, die für eine CDR2 der leichten Kette kodiert, eine Nukleinsäuresequenz, die für eine humane Framework-Region 3 der leichten Kette kodiert, eine Nukleinsäuresequenz, die für eine CDR3 der leichten Kette kodiert, und eine Nukleinsäuresequenz, die für eine humane Framework-Region 4 der leichten Kette kodiert, erzeugt wird, wobei die CDRs von einer nicht humanen variablen Region der leichten Kette eines Spenderantikörpers abgeleitet sind, die immunspezifisch das Antigen bindet, und jede Nukleinsäuresequenz einer Framework-Region der leichten Kette von einer Sub-Bank stammt, umfassend mehrere Nukleinsäuresequenzen, die für die humane Framework-Region der leichten Kette kodieren, von humanen Keimlinien-Framework-Sequenzen und/oder Framework-Regionen von funktionellen humanen Antikörpersequenzen; (c) Einführen der Nukleinsäuresequenzen, die in Schritt (a) und (b) erzeugt wurden, in eine Population von Zellen; und (d) Exprimieren der Nukleotidsequenzen, die für die variable Region der schweren Kette und die variable Region der leichten Kette exprimieren.

8. Verfahren nach Anspruch 5, 6 oder 7, des Weiteren umfassend nach dem Schritt des Exprimierens der Nukleotidsequenzen den Schritt des Durchmusterns auf einen Antikörper, der immunspezifisch an das Antigen bindet.

9. Verfahren nach Anspruch 5, 6, 7 oder 8, des Weiteren umfassend vor Schritt (A) den Schritt des Erzeugens der Sub-Banken von Framework-Region-Sequenzen.

10. Verfahren nach einem der vorangehenden Ansprüche, wobei die humane(n) Framework-Region-Sub-Bank(en) Framework-Regionen von humanen Keimlinien-Framework-Sequenzen und/oder Framework-Regionen von funktionellen humanen Antikörper-Sequenzen umfasst bzw. umfassen, in die eine oder mehrere Mutationen eingeführt wurden.

## Revendications

1. Procédé de génération d'une bibliothèque combinatoire comprenant une pluralité de séquences nucléotidiques codant des régions variables à chaîne lourde humanisées, lesdites séquences nucléotidiques codant les régions variables à chaîne lourde humanisées, chacune étant produite par une fusion avec une séquence d'acides nucléiques codant une région charpente à chaîne lourde humaine 1, une séquence d'acides nucléiques codant une CDR à chaîne lourde 1, une séquence d'acides nucléiques codant une région charpente à chaîne lourde humaine 2, une séquence d'acides nucléiques codant une CDR à chaîne lourde 2, une séquence d'acides nucléiques codant une région charpente à chaîne lourde humaine 3, une séquence d'acides nucléiques codant une CDR à chaîne lourde 3, et une séquence d'acides nucléiques codant une région charpente à chaîne lourde humaine 4, dans lesquelles les CDR sont dérivées d'une région variable à chaîne lourde d'anticorps de donneur non humain et chaque séquence d'acides nucléiques codant une région charpente à chaîne lourde est d'une sous-banque comprenant une pluralité de séquences d'acides nucléiques codant ladite région charpente à chaîne lourde humaine de séquences charpentes et/ou de régions charpentes germinales humaines de séquences d'anticorps humains fonctionnels.

2. Procédé de génération d'une bibliothèque combinatoire comprenant une pluralité de séquences nucléotidiques codant des régions variables à chaîne légère humanisées, lesdites séquences nucléotidiques codant les régions variables à chaîne légère humanisées, chacune étant produite par une fusion avec une séquence d'acides nucléiques codant une région charpente à chaîne légère humaine 1, une séquence d'acides nucléiques codant une CDR à chaîne légère 1, une séquence d'acides nucléiques codant une région charpente à chaîne légère humaine 2, une séquence d'acides nucléiques codant une CDR à chaîne légère 2, une séquence d'acides nucléiques codant une région charpente à chaîne légère humaine 3, une séquence d'acides nucléiques codant une CDR à chaîne légère 3, et une séquence d'acides nucléiques codant une région charpente à chaîne légère humaine 4, dans lesquelles les CDR sont dérivées d'une région variable à chaîne légère d'anticorps de donneur non humain et chaque séquence d'acides nucléiques codant une région charpente à chaîne légère est d'une sous-banque comprenant une pluralité de séquences d'acides nucléiques codant ladite région charpente à chaîne légère humaine de séquences charpentes et/ou de régions charpentes germinales humaines de séquences d'anticorps humains fonctionnels.

3. Procédé de génération d'une bibliothèque combinatoire comprenant : (i) un premier ensemble de séquences nucléotidiques codant des régions variables à chaîne lourde humanisées, ledit premier ensemble de séquences nucléotidiques codant des régions variables à chaîne lourde humanisées, chacune étant produite par une fusion avec une séquence d'acides nucléiques codant une région charpente à chaîne lourde humaine 1, une séquence d'acides nucléiques codant une CDR à chaîne lourde 1, une séquence d'acides nucléiques codant une région charpente à chaîne lourde humaine 2, une séquence d'acides nucléiques codant une CDR à chaîne lourde 2, une séquence d'acides nucléiques codant une région charpente à chaîne lourde humaine 3, une séquence d'acides nucléiques codant une CDR à chaîne lourde 3, et une séquence d'acides nucléiques codant une région charpente à chaîne lourde humaine 4, et (ii) un deuxième ensemble de séquences nucléotidiques codant des régions variables à chaîne légère humanisées, ledit deuxième ensemble de séquences nucléotidiques codant des régions variables à chaîne légère humanisées, chacune étant produite par une fusion avec une séquence d'acides nucléiques codant une région charpente à chaîne légère humaine 1, une séquence d'acides nucléiques codant une CDR à chaîne légère 1, une séquence d'acides nucléiques codant une région charpente à chaîne légère humaine 2, une séquence d'acides nucléiques codant une CDR à chaîne légère 2, une séquence d'acides nucléiques codant une région charpente à chaîne légère humaine 3, une séquence d'acides nucléiques codant une CDR à chaîne légère 3, et une séquence d'acides nucléiques codant une région charpente à chaîne légère humaine 4, dans lesquelles les CDR à région variable à chaîne lourde sont dérivées d'une région variable à chaîne lourde d'anticorps de donneur non humain, les CDR à région variable à chaîne légère sont dérivées d'une région variable à chaîne légère d'anticorps de donneur non humain, chaque séquence d'acides nucléiques codant une région charpente à chaîne lourde humaine est d'une sous-banque comprenant une pluralité de séquences d'acides nucléiques codant ladite région charpente à chaîne lourde humaine de séquences charpentes et/ou de régions charpentes germinales humaines de séquences d'anticorps humains fonctionnels et chaque séquence d'acides nucléiques codant une région charpente à chaîne légère humaine est d'une sous-banque comprenant une pluralité de séquences d'acides nucléiques codant ladite région charpente à chaîne légère humaine de séquences charpentes et/ou de régions charpentes germinales humaines de séquences d'anticorps humains fonctionnels.

4. Procédé selon la revendication 1, la revendication 2 ou la revendication 3, comprenant en outre l'introduction de la bibliothèque combinatoire de séquences nucléotidiques dans une population de cellules.

5. Procédé de production d'un anticorps humanisé qui se lie de manière immunospécifique à un antigène, ledit procédé comprenant les étapes consistant à : (a) synthétiser une pluralité de séquences polynucléotidiques, chacune comprenant une séquence nucléotidique codant une région variable à chaîne lourde humanisée, ladite séquence nucléotidique étant produite par une fusion avec une séquence d'acides nucléiques codant une région charpente à chaîne lourde humaine 1, une séquence d'acides nucléiques codant une CDR à chaîne lourde 1, une séquence d'acides nucléiques codant une région charpente à chaîne lourde humaine 2, une séquence d'acides nucléiques codant une CDR à chaîne lourde 2, une séquence d'acides nucléiques codant une région charpente à chaîne lourde humaine 3, une séquence d'acides nucléiques codant une CDR à chaîne lourde 3, et une séquence d'acides nucléiques codant une région charpente à chaîne lourde humaine 4, dans lesquelles les CDR sont dérivées d'une région variable à chaîne lourde d'anticorps de donneur non humain qui se lie de manière immunospécifique au dit antigène et chaque séquence d'acides nucléiques de régions charpentes à chaîne lourde est d'une sous-banque comprenant une pluralité de séquences d'acides nucléiques codant ladite région charpente à chaîne lourde humaine de séquences charpentes et/ou de régions charpentes germinales humaines de séquences d'anticorps humains fonctionnels ; (b) introduire les séquences polynucléotidiques dans une population de cellules contenant chacune une séquence polynucléotidique codant une région variable à chaîne légère ; et (c) exprimer les séquences polynucléotidiques codant la région variable à chaîne lourde et la région variable à chaîne légère.

6. Procédé de production d'un anticorps humanisé qui se lie de manière immunospécifique à un antigène, ledit procédé comprenant les étapes consistant à : (a) synthétiser une pluralité de séquences polynucléotidiques, chacune comprenant une séquence nucléotidique codant une région variable à chaîne légère humanisée, ladite séquence nucléotidique étant produite par une fusion avec une séquence d'acides nucléiques codant une région charpente à chaîne légère humaine 1, une séquence d'acides nucléiques codant une CDR à chaîne légère 1, une séquence d'acides nucléiques codant une région charpente à chaîne légère humaine 2, une séquence d'acides nucléiques codant une CDR à chaîne légère 2, une séquence d'acides nucléiques codant une région charpente à chaîne légère humaine 3, une séquence d'acides nucléiques codant une CDR à chaîne légère 3, et une séquence d'acides nucléiques codant une région charpente à chaîne légère humaine 4, dans lesquelles les CDR sont dérivées d'une région variable à chaîne légère d'anticorps de donneur non humain qui se lie de manière immunospécifique au dit antigène et chaque séquence d'acides nucléiques de régions charpentes à chaîne légère est d'une sous-banque comprenant une pluralité de séquences d'acides nucléiques codant ladite région charpente à chaîne légère humaine de séquences charpentes et/ou de régions charpentes germinales humaines de séquences d'anticorps humains fonctionnels ; (b) introduire les séquences polynucléotidiques dans une population de cellules contenant chacune une séquence polynucléotidique codant une région variable à chaîne lourde ; et (c) exprimer les séquences polynucléotidiques codant la région variable à chaîne lourde et la région variable à chaîne légère.

7. Procédé de production d'un anticorps humanisé qui se lie de manière immunospécifique à un antigène, ledit procédé comprenant les étapes consistant à : (a) synthétiser une pluralité de premières séquences polynucléotidiques, chacune comprenant une séquence nucléotidique codant une région variable à chaîne lourde humanisée, ladite séquence nucléotidique étant produite par une fusion avec une séquence d'acides nucléiques codant une région charpente à chaîne lourde humaine 1, une séquence d'acides nucléiques codant une CDR à chaîne lourde 1, une séquence d'acides nucléiques codant une région charpente à chaîne lourde humaine 2, une séquence d'acides nucléiques codant une CDR à chaîne lourde 2, une séquence d'acides nucléiques codant une région charpente à chaîne lourde humaine 3, une séquence d'acides nucléiques codant une CDR à chaîne lourde 3, et une séquence d'acides nucléiques codant une région charpente à chaîne lourde humaine 4, dans lesquelles les CDR sont dérivées d'une région variable à chaîne lourde d'anticorps de donneur non humain qui se lie de manière immunospécifique au dit antigène et chaque séquence d'acides nucléiques de régions charpentes à chaîne lourde est d'une sous-banque comprenant une pluralité de séquences d'acides nucléiques codant ladite région charpente à chaîne lourde de séquences charpentes et/ou de régions charpentes germinales humaines de séquences d'anticorps humains fonctionnels ; (b) synthétiser une pluralité de deuxièmes séquences polynucléotidiques, chacune comprenant une séquence nucléotidique codant une région variable à chaîne légère humanisée, ladite séquence nucléotidique étant produite par une fusion avec une séquence d'acides nucléiques codant une région charpente à chaîne légère humaine 1, une séquence d'acides nucléiques codant une CDR à chaîne légère 1, une séquence d'acides nucléiques codant une région charpente à chaîne légère humaine 2, une séquence d'acides nucléiques codant une CDR à chaîne légère 2, une séquence d'acides nucléiques codant une région charpente à chaîne légère humaine 3, une séquence d'acides nucléiques codant une CDR à chaîne légère 3, et une séquence d'acides nucléiques codant une région charpente à chaîne légère humaine 4, dans lesquelles les CDR sont dérivées d'une région variable à chaîne légère d'anticorps de donneur non humain qui se lie de manière immunospécifique au dit antigène et chaque séquence d'acides nucléiques de régions charpentes à chaîne légère est d'une sous-banque comprenant une pluralité de séquences d'acides nucléiques codant ladite région charpente à chaîne légère humaine de séquences charpentes et/ou de régions charpentes germinales humaines de séquences d'anticorps humains fonctionnels ; (c) introduire les séquences d'acides nucléiques générées aux étapes (a) et (b) dans une population de cellules ; et (d) exprimer les séquences nucléotidiques codant la région variable à chaîne lourde et la région variable à chaîne légère.

8. Procédé selon la revendication 5, la revendication 6 ou la revendication 7, comprenant en outre, après l'étape consistant à exprimer les séquences nucléotidiques, l'étape consistant à dépister un anticorps qui se lie de manière immunospécifique à l'antigène.

9. Procédé selon la revendication 5, la revendication 6, la revendication 7 ou la revendication 8, comprenant en outre, avant l'étape (a), l'étape consistant à générer les sous-banques de séquences de régions charpentes.

10. Procédé selon l'une quelconque des revendications précédentes, dans lequel la/les sous-banque(s) de régions charpentes humaines comprennent des régions charpentes de séquences charpentes et/ou de régions charpentes germinales humaines de séquences d'anticorps humains fonctionnels dans lesquelles une ou plusieurs mutations ont été introduites.
